# EUROPEAN PATENT APPLICATION

(11) **EP 4 696 305 A1**
(43) Date of publication of application: **18.02.2026**
(21) Application number: 24788195.6
(22) Date of filing: 12.04.2024
(51) Int. Cl.: A61K 9/51, C07J 41/00, C07J 43/00, C07J 9/00, C07C 67/00, C07C 235/04, A61K 47/60, A61K 47/18, A61K 47/28, A61K 31/7088

(54) **LIPID NANOPARTICLES TARGETING SPLEEN**

(30) Priority: 14.04.2023 CN 202310400925; 29.12.2023 WO PCT/CN2023/143111
(71) Applicant: Beijing Jitai Pharmaceutical Technology Co., Ltd., Beijing 100094 (CN); Hangzhou Jitai Pharmaceutical Technology Co., Ltd., Hangzhou, Zhejiang 310051 (CN)
(72) Inventor: SUN, Zhuorui, Beijing 100094 (CN); LIU, Shaoli, Beijing 100094 (CN); LIU, Andong, Beijing 100094 (CN); ZHANG, Lin, Beijing 100094 (CN); SHI, Feng, Beijing 100094 (CN); SHAO, Shuaibo, Beijing 100094 (CN); WARRINGTON, Jeffrey Michael, Cambridge Massachusetts 02140 (US)
(74) Representative: Vossius & Partner Patentanwälte Rechtsanwälte mbB
(86) International application number: PCT/CN2024/087429
(87) International publication number: WO 2024/213098

(57) **Abstract**

The present application relates to lipid nanoparticles comprising steroid compound, as well as the preparation and uses of such lipid nanoparticles. Such lipid nanoparticles are useful in the delivery of payloads, such as nucleic acids, *in vivo* to non-hepatic organs (*e.g.,* spleen) for the treatment or prevention of certain diseases or disorders, particularly spleen diseases.

## Description

### 1. TECHNICAL FIELD

The present application relates to the field of pharmaceutical therapies, particularly lipid nanoparticles. This application also relates to the preparation of said lipid nanoparticles, and the use of said lipid nanoparticles for targeted delivery of biologically active molecules, such as nucleic acids (e.g. mRNA, miRNA, siRNA, saRNA, ASO, DNA) and polypeptides (e.g. antibodies) to certain organs, particularly the spleen.

### 2. BACKGROUND

Nucleic acid-based gene therapy has achieved rapid progress in various fields, such as the treatment of infectious diseases and cancers over the past few years. Lipid nanoparticles (LNPs) are one of the most advanced non-viral delivery platforms that can safely and efficiently deliver nucleic acid drugs (e.g. mRNA, or siRNA) to specific target organs of interest while also protecting them from degradation, such as by nuclease. LNPs possess several advantages, including high encapsulation efficiency, good cellular transfection efficiency, strong tissue penetration, and relatively low cytotoxicity and immunogenicity. Currently, LNPs have been successfully applied to several commercial products. For example, FDA-approved drugs such as the mRNA COVID-19 vaccines developed by Moderna and Pfizer-BioNTech, as well as the siRNA drug Onpattro developed by Alnylam, all utilize LNP drug delivery platform. It was found that after entering the circulation in vivo, pegylated lipids dissociate from LNP nanoparticles, allowing ApoE to adsorb onto LNP surface, which forms an efficient ligand for liver targeting. The LNPs subsequently bind to lipoprotein receptors on the surface of liver cells, triggering endocytosis. Therefore, the current LNP delivery systems predominantly exhibit passive liver targeting. In order to fully unleash the therapeutic potential of nucleic acid therapies, to expand their application to different types of diseases, and to minimize the toxic side effects on healthy organs, it is necessary to develop LNP delivery systems that target non-hepatic organs.

The present application provides certain steroid compounds that provide targeted delivery of payloads to non-hepatic organ, particularly the spleen.

### 3. SUMMARY

In one aspect, provided herein is a lipid nanoparticle, which has a higher delivery or expression level of the payload in the spleen of a subject than the delivery or expression level in other organs of the subject such as the liver or lung.

In one embodiment, the lipid nanoparticle comprising a steroid compound and an ionizable lipid.

In one embodiment, the amount of the steroid compound is from 3 mol % to 15 mol % of the total lipid present in the lipid nanoparticle, preferably 5-10 mol%, such as 3 mol%, 4 mol%, 5 mol%, 6 mol%, 7 mol%, 8 mol% , 9 mol%, 10 mol%, 11 mol%, 12 mol%, 13 mol%, 14 mol% or 15 mol% of the total lipid present in the lipid nanoparticle.

In one embodiment, the amount of the ionizable lipid is from 35 mol % to 70 mol % of the total lipid present in the lipid nanoparticle, more preferably from 40 mol % to 60 mol % of the total lipid present in the lipid nanoparticle, such as 35 mol %, 40 mol %, 45 mol %, 50 mol %, 55 mol % or 60 mol % of the total lipid present in the lipid nanoparticle_{∘}

In one embodiment, the lipid nanoparticle further comprising phospholipid.

In a preferred embodiment, the phospholipid is DSPC, DMPC, DOPC, DPPC, POPC, DOPE, DMPE, POPOE or DPPE.

In a preferred embodiment, the amount of the phospholipid is from 5 mol% to 30 mol% of the total lipid present in the lipid nanoparticle, preferably 5-20 mol%, more preferably 5-15 mol%, such as 5 mol%, 6 mol%, 7 mol%, 8 mol%, 9mol%, 10mol%, 11mol%, 12mol%, 13mol%, 14mol% or 15mol% of the total lipid present in the lipid nanoparticle.

In one embodiment, the the spleen-targeted lipid nanoparticle further comprising a structural lipid, such as cholesterol.

In a preferred embodiment, the structural lipid is selected from the group consisting of cholesterol, campesterol, stigmasterol, sitosterol, brassicasterol, ergosterol, solanine, ursolic acid, alpha-tocopherol, beta-sitosterol, avenasterol, and canola sterol.

In a preferred embodiment, the amount of the structural lipid is from 10 mol% to 80 mol% of the total lipid in the lipid nanoparticle, more preferably 15-70 mol%, more preferably 30-50 mol%, more preferably 30-40 mol%, such as 30 mol%, 35mol%, 40mol%, 45mol%, 50mol%, 55mol% or 60mol% of the total lipid in the lipid nanoparticle.

In one embodiment, the spleen-targeted lipid nanoparticle further comprising a polymer conjugated lipid, such as a pegylated lipid.

In a preferred embodiment, the pegylated lipid comprises a pegylated moiety having a molecule weight of from about 1000 Da to about 20, 000 Da, from about 1000 Da to about 5000 Da, or from about 1000 Da to about 2000 Da.

In a preferred embodiment, the polymer conjugated lipid is ALC-0159, DMG-PEG2000, DMPE-PEG1000, DPPE-PEG1000, DSPE-PEG1000, DOPE-PEG1000, Ceramide-PEG2000, DMPE-PEG2000, DPPE-PEG2000, DSPE-PEG2000, DSPE-PEG2000-Mannose, Ceramide-PEG5000, DSPE-PEG5000, or DSPE-PEG2000 amine.

In a preferred embodiment, the amount of the polymer conjugated lipid is from 0.25 mol% to 10 mol% of the total lipid in the lipid nanoparticle , more preferably 0.5-5 mol%, more preferably 0.5-3 mol%, such as 0.5 mol%, 0.6 mol%, 0.7mol%, 0.8mol%, 0.9mol%, 1.0mol%, 1.1mol%, 1.2mol%, 1.3mol%, 1.4mol%, 1.5mol%, 1.6mol%, 1.7mol%, 1.8mol%, 1.9mol %, 2.0mol%, 2.1mol%, 2.2mol%, 2.3mol%, 2.4mol%, 2.5mol%, 2.6mol%, 2.7mol%, 2.8mol%, 2.9mol%, 3.0mol%, 3.1mol%, 3.2 mol%, 3.3 mol%, 3.4 mol%, 3.5 mol%, 3.6 mol%, 3.7 mol%, 3.8 mol%, 3.9 mol% or 4.0 mol% of the total lipid in the lipid nanoparticle.

In one embodiment, the average particle size of the spleen-targeted lipid nanoparticle is from 50 nm to 800 nm, more preferably 50-400 nm, more preferably 80-200 nm, such as 80, 90, 100, 110, 120, 130, 140, 150, 160, 170, 180, 190 or 200nm.

In one embodiment, the lipid nanoparticle comprising (1) steroid compound; (2) ionizable lipid; (3) phospholipid; (4) structural lipid; and (5) polymer conjugated lipid.

In a preferred embodiment, the lipid nanoparticle further comprises a payload.

In one embodiment, the payload is a therapeutic agent, a prophylactic agent, or a diagnostic agent.

In one embodiment, the payload is nucleic acid.

In a preferred embodiment, the nucleic acid is antisense oligonucleotide (ASO), DNA, or RNA, optionally wherein the RNA is RNA interference (RNAi), small interfering RNA (siRNA), short hairpin RNA (shRNA), antisense RNA (aRNA), messenger RNA (mRNA), modified messenger RNA (mmRNA), long noncoding RNA (lncRNA), microRNA (miRNA), small activating RNA (saRNA), multicoding nucleic acid (MCNA), polymer-coded nucleic acid (PCNA), guide RNA (gRNA), CRISPR RNA (crRNA), or any other RNA in the ribozyme.

In a preferred embodiment, the spleen-targeted lipid nanoparticle comprises the following components in the molar percentages:
1) Steroid compound 3 mol%-15 mol%;
2) Ionizable lipid 35 mol%-70 mol%;
3) Phospholipid 5 mol%-30 mol%;
4) Structural lipid 10 mol%-80 mol%; and
5) Polymer conjugated lipid 0.25 mol%-10 mol%.

In a preferred embodiment, the spleen-targeting lipid nanoparticle comprises the following components in the molar percentages:
1) Steroid compound 5 mol%-10 mol%;
2) Ionizable lipid 40 mol%-60 mol%;
3) Phospholipid 5 mol%-20 mol%;
4) Structural lipid 15 mol%-70 mol%; and
5) Polymer conjugated lipid 0.5 mol%-5 mol%.

In a preferred embodiment, the spleen-targeting lipid nanoparticle comprises the following components in the molar percentages:
1) Steroid compound 5 mol%-10 mol%;
2) Ionizable lipid 40 mol%-60 mol%;
3) Phospholipid 5 mol%-15 mol%;
4) Structural lipid 30 mol%-50 mol%; and
5) Polymer conjugated lipid 0.5 mol%-3 mol%.

In a particularly preferred embodiment, the lipid nanoparticle comprises the following components in the molar percentages:
1) Steroid compound 5 mol%;
2) Ionizable lipid 50 mol%;
3) Phospholipid 10 mol%;
4) Structural lipid 33.5 mol%; and
5) Polymer conjugated lipid 1.5 mol%.

In one embodiment, the lipid nanoparticles have a particle size polydispersity index (PDI) of no greater than 0.2, preferably no greater than 0.15.

In one embodiment, the delivery or expression level of the payload in the spleen of the subject is more than 1 times, more preferably 2 times, more preferably 3 times, more preferably 4 times, more preferably 5 times, more preferably 10 times, more preferably 20 times, more preferably 30 times, more preferably 40 times, more preferably 50 times, or more preferably 100 times higher than the delivery or expression level of the payload in the liver of the subject, after administration of the lipid nanoparticle.

In one embodiment, the delivery or expression level of the payload in the spleen of the subject is more than 1 times, more preferably 2 times, more preferably 3 times, more preferably 4 times, more preferably 5 times, more preferably 10 times, more preferably 20 times, more preferably 30 times, more preferably 40 times, more preferably 50 times, or more preferably 100 times higher than the delivery or expression level of the payload in the lung of the subject, after administration of the lipid nanoparticle.

In one aspect, provided herein is a lipid nanoparticle comprising an ionizable lipid and a steroid compound, wherein the steroid compound is a compound of formula (I): 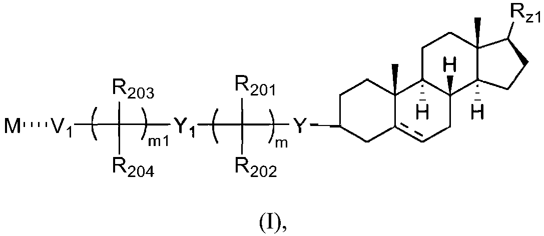 or a stereoisomer, a mixture of stereoisomers, or a pharmaceutically acceptable salt thereof, wherein:
- - - is a single bond or double bond;
Y is -C(O)O-, -O-, -NH-, -SC(O)O-, -OC(O)NR₂,-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C_{1 -8} alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl;
R_{z1} is C₁₋₁₄ alkyl or C₁₋₁₄ alkenyl, wherein the alkyl and alkenyl are optionally substituted; and
provided that when Y is -NHC(O)-, at least one of the following conditions are met:
   (i) m is an integer from 5 to 12; or
   (ii) V₁ is not N(CH₃)₂.

In one aspect, provided herein is a lipid nanoparticle comprising an ionizable lipid and a steroid compound, wherein the steroid compound is a compound of formula (I-P): 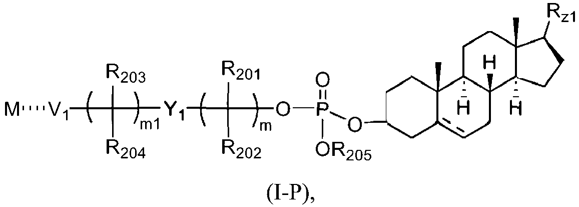 or a stereoisomer, a mixture of stereoisomers, or a pharmaceutically acceptable salt thereof, wherein:
- - - is a single bond or double bond;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂,-, -NR₂ₐC(S)O-, -S(O)₀₋₂-, or -OPO₃-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, -L_{2b}-N⁺R₂₁R₂₂R₂₃, L_{2b}-OC(O)R₂₁, or L_{2b}-C(O)OR₂₁;
R₂₀₅ is absent, a cation, H, C₁₋₆ alkyl, C_{I}-₆ haloalkyl, C₂₋₆ alkenyl, or C₂₋₆ alkynyl, and wherein alkyl, haloalkyl, alkenyl, and alkynyl, is optionally substituted;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

In one embodiment, Y is -C(O)O-, -OC(O)-, -O-, -NHC(O)O-, -NCH₃-C(O)O-, -OC(O)NH-, or - OC(O)O-, and wherein the attachment to the left is to the direction of V₁. In one embodiment, Y₁ is absent, - C(O)O-, -OC(O)-, -O-, -S-, SO, S(O)₂, -O(CO)O-, -NHC(O)-, or -C(O)NH-, and wherein the attachment to the left is to the direction of V₁.

In one embodiment, the steroid compound is a compound of Formula (I-A1), (I-A2), (I-A3), (I-A4), or (I-A5): 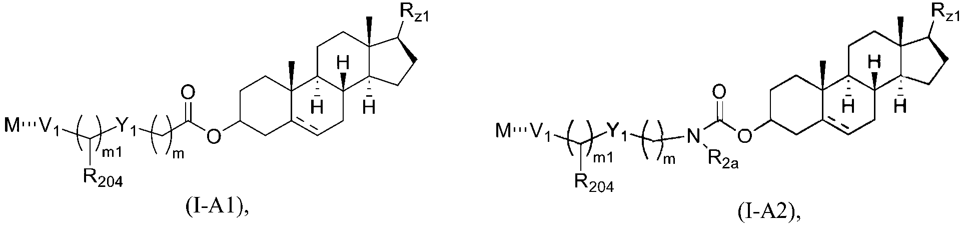 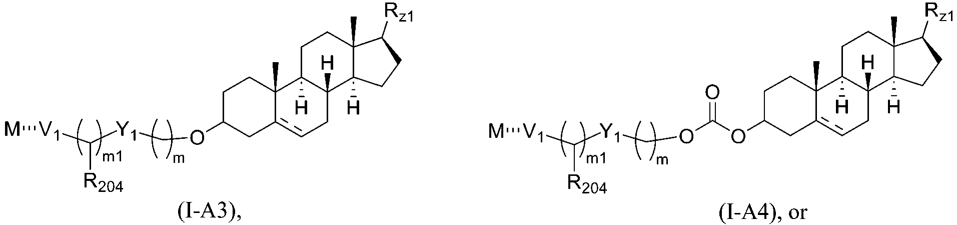 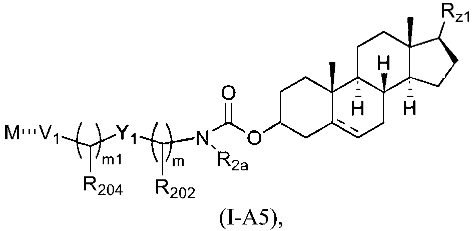 or a stereoisomer, a mixture of stereoisomers, or a pharmaceutically acceptable salt thereof,

In one embodiment, the steroid compound is a compound of Formula (I-B1), (I-B2), (I-B3), (I-B4), (I-B5), (I-B6), (I-B7), or (I-B8): 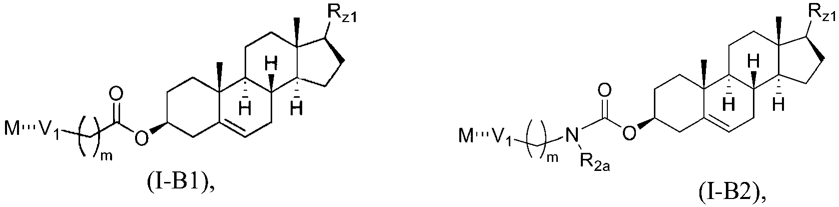 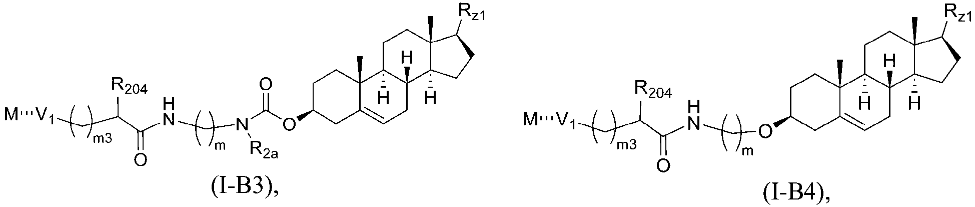 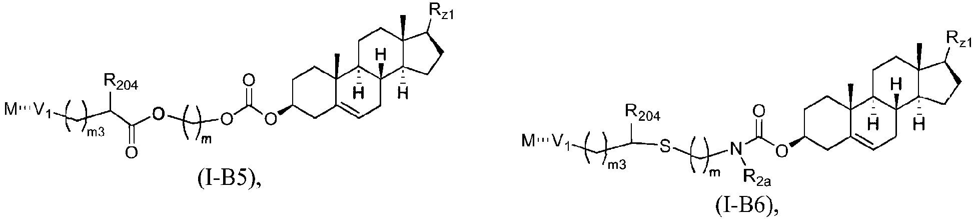 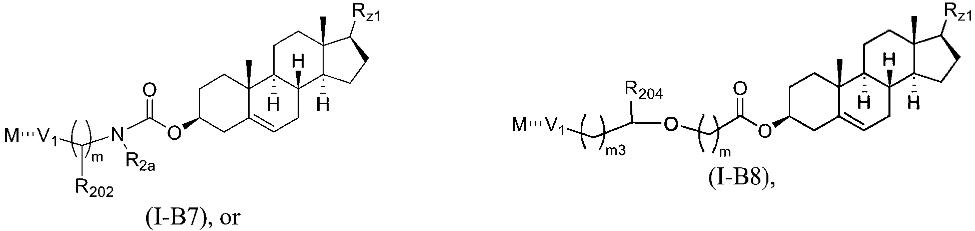 or a stereoisomer, a mixture of stereoisomers, or a pharmaceutically acceptable salt thereof, wherein m3 is an integer from 1 to 11.

In one embodiment, each instance of R₂₀₁, R₂₀₂, R₂₀₃, and R₂₀₄ are independently H, alkyl, -NR₂₁R₂₂, -N⁺R₂₁R₂₂R₂₃, -(CH₂)₀₋₆-OC(O)R₂₁, or -(CH₂)₀₋₆-C(O)OR₂₁.

In one embodiment, each instance of R₂₀₁, R₂₀₂, R₂₀₃, and R₂₀₄ are independently H, NH₂, NH₃⁺, - CH₂OC(O)CH₃, -CH₂C(O)OCH₃, -OC(O)CH₃, or -C(O)OCH₃.

In one embodiment, m is an integer from 5 to 12.

In one embodiment, the steroid compound is a compound of formula (I-P1): 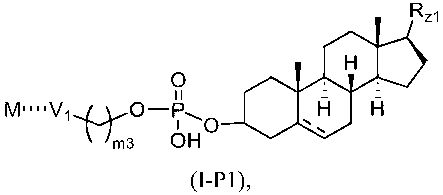 or a stereoisomer, a mixture of stereoisomers, or a pharmaceutically acceptable salt thereof, and wherein m3 is an integer from 1 to 11.

In one embodiment, V₁ is NR₂₁R₂₂, N⁺R₂₁R₂₂R₂₃, or an optionally substituted radical of amidine, guanidine, or urea. In one embodiment, R₂₁, R₂₂, R₂₃ are each independently H, hydroxyl, C₁₋₃ alkyl, or C₁₋₃ hydroxyalkyl. In one embodiment, V₁ is NH₂, NH₃⁺, N(CH₃)₂, N⁺(CH₃)₃, N⁺CH₃(CH₂CH₂OH)₂, N⁺(CH₃)₂(CH₂CH₂OH), NHC(=N⁺H₂)NH₂, NHC(O)NH₂, CH₂C(=NH)NH₂, N(CH₃)(CH₂CH₂CO₂CH₃), N⁺(CH₃)₂(CH₂CH₂CO₂CH₃), N(CH₃)(CH₂CO₂CH₃), or N⁺(CH₃)₂(CH₂CO₂CH₃). In one embodiment, V₁ is N⁺CH₃(CH₂CH₂OH)₂, N⁺(CH₃)₂(CH₂CH₂OH), or N⁺(CH₃)₃.

In one embodiment, M is absent, or one or more of bromide, chloride, iodide, or trifluoroacetate.

In one embodiment, R_{z1} is C₁₋₁₄ alkyl or C₁₋₁₄ alkenyl, wherein the alkyl and alkenyl are optionally substituted with one or more hydroxyl, oxo, nitro, cyano, halogen, C₁₋₆ alkoxy, C₂-C₆ alkenyl, C₂-C₆ alkynyl, C₃-C₈ cycloalkyl, C₆-C₁₀ aryl, 5 to 10-membered heteroaryl, or 3 to 8-membered heterocyclyl. In one embodiment, R_{z1} is C₆₋₁₂ alkyl or C₆₋₁₂ alkenyl, and wherein the alkyl and alkenyl are optionally substituted with one or more hydroxyl, oxo, or halogen. In one embodiment, R_{z1} is

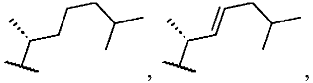

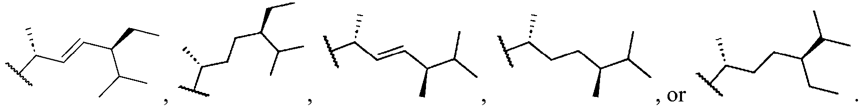

In another embodiment, provided herein is a steroid compound of formula (II-A): 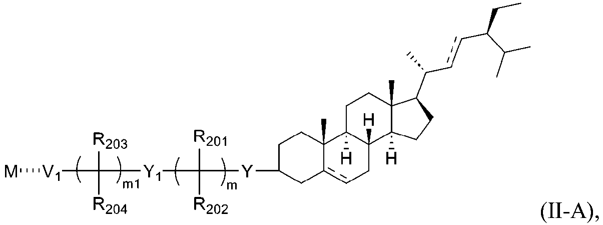 or a stereoisomer, a mixture of stereoisomers, or a pharmaceutically acceptable salt thereof, wherein:
each - - - is independently a single bond or double bond;
Y is -C(O)O-, -O-, -NH-, -SC(O)O-, -OC(O)NR₂,-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂,-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂,-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂,-, -NR₂ₐC(O)-, -NR₂ₐC(O)S-, -SC(O)NR₂,-, - C(O)-, -OC(S)-, -C(S)O-, -OC(S)NR₂,-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 3 to 12;
m1 is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted; and provided that the compound is not:

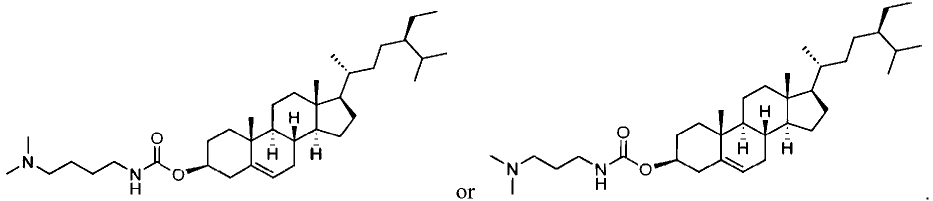

In one embodiment, provided herein is a steroid compound of formula (II-B): 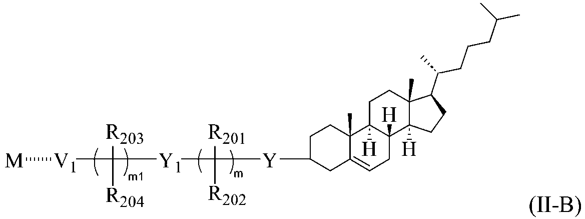 or a stereoisomer, a mixture of stereoisomers, or a pharmaceutically acceptable salt thereof, wherein:
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 5 to 12;
m1 is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently selected from H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene; and
each of R_{2b}, R'_{2b} and R"_{2b} are independently H or C₁₋₁₀ alkyl.

In one embodiment of formula (II-B), Y₁ is -C(O)O-, -OC(O)-, -O-, -S-, -NR₂ₐ-, -OC(O)NR₂ₐ-, or - NR₂ₐC(O)-.

In another embodiment, provided herein is a steroid compound of formula (II-C1): 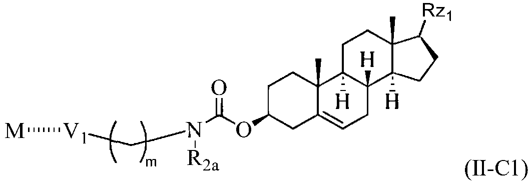 or a stereoisomer, a mixture of stereoisomers, or a pharmaceutically acceptable salt thereof, wherein:
R_{z1} is 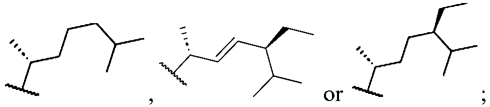
R₂ₐ is H, optionally substituted C₁₋₈ alkyl, -L_{2b}-OR_{2b}, L_{2b}-OC(O)R_{2b}, or L_{2b}-C(O)OR_{2b}, preferably H, - CH₃, -CH₂C(O)OCH₃, or -CH₂C(O)OH;V₁ is NR₂₁R₂₂ or N⁺R₂₁R₂₂R₂₃, wherein each instance of R₂₁, R₂₂, R₂₃ is independently selected from H, optionally substitutedC₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}, provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 10, such as 1, 2, 3, 4, 5, 6, 7, 8, 9, or 10.

In another aspect, provided herein is a steroid compound of formula (II-C2): 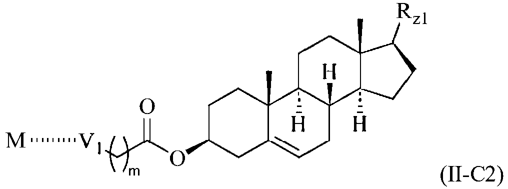 or a stereoisomer, a mixture of stereoisomers, or a pharmaceutically acceptable salt thereof, wherein:
R_{z1} is 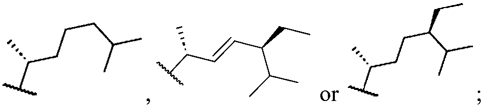 V₁ is NR₂₁R₂₂ or N⁺R₂₁R₂₂R₂₃, wherein each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}, provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl; and
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 10, such as 1, 2, 3, 4, 5, 6, 7, 8, 9, or 10.

In another aspect, provided herein is a lipid nanoparticle comprising an ionizable lipid and a steroid compound, wherein the steroid compound is: 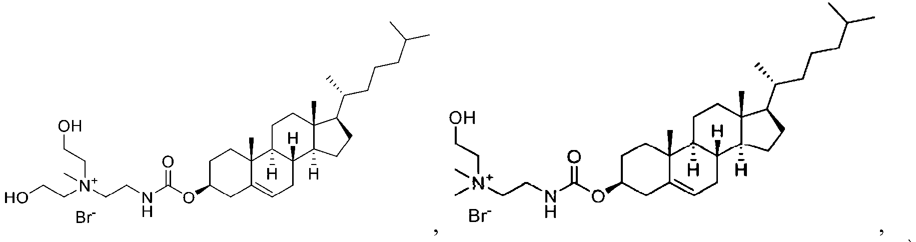 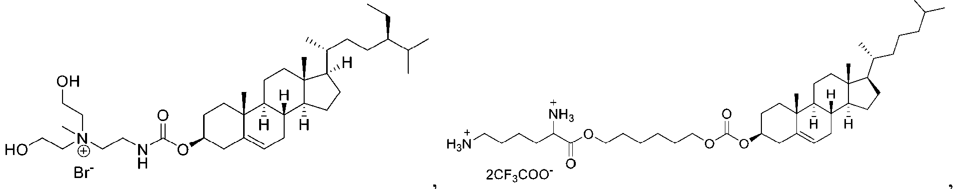 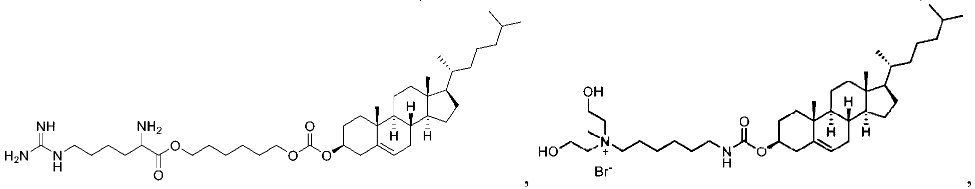 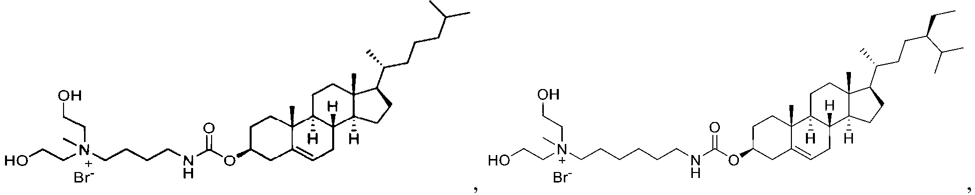 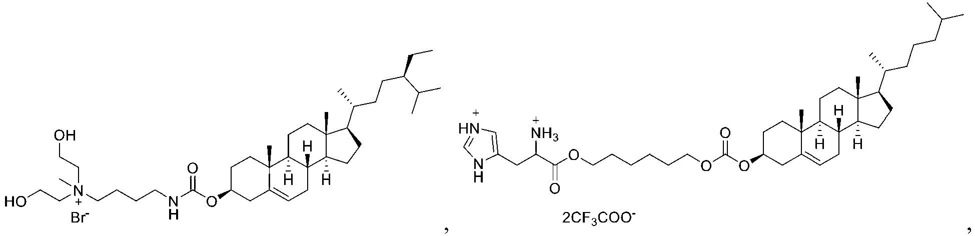 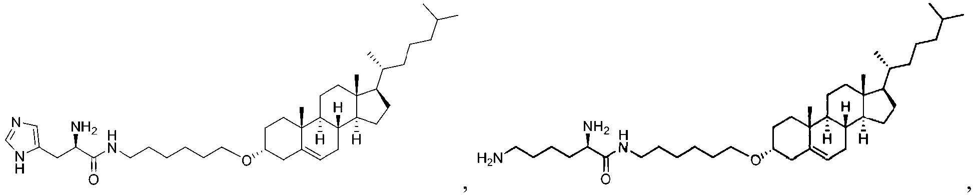 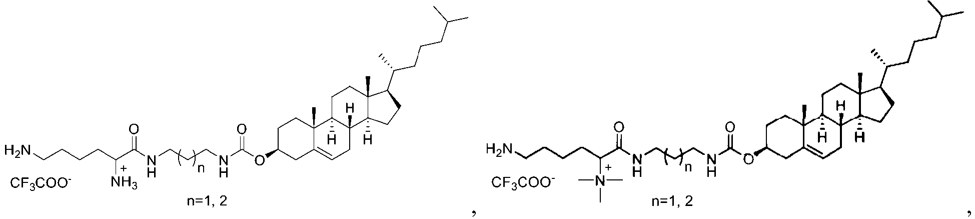 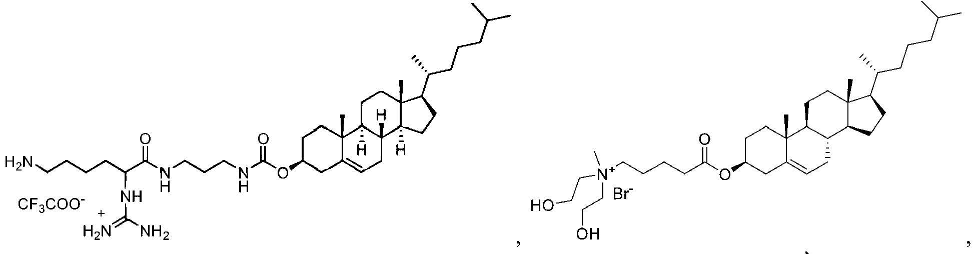 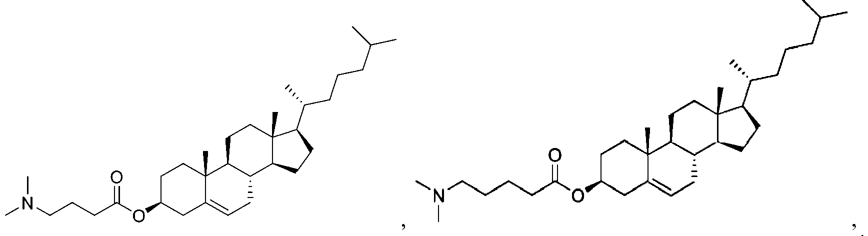 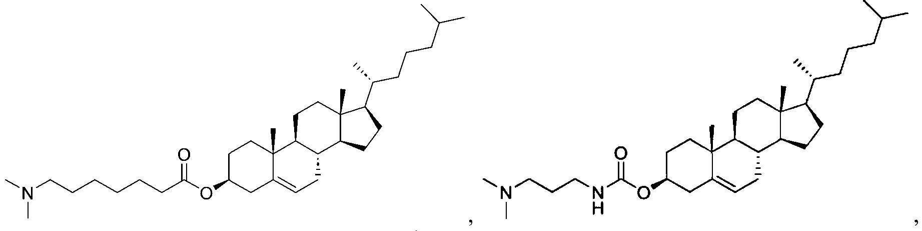 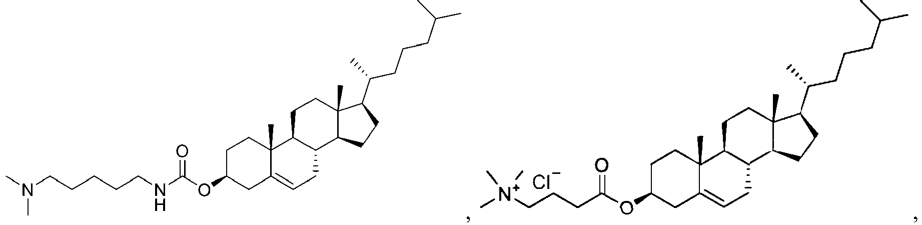 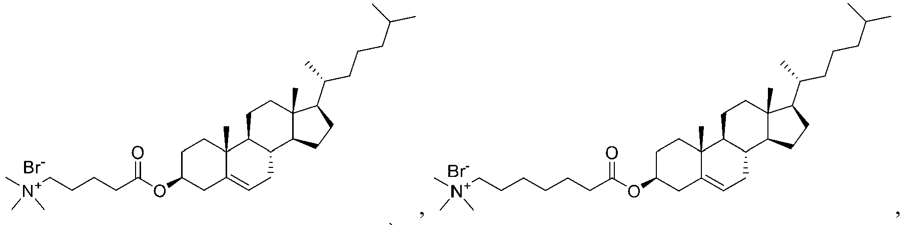 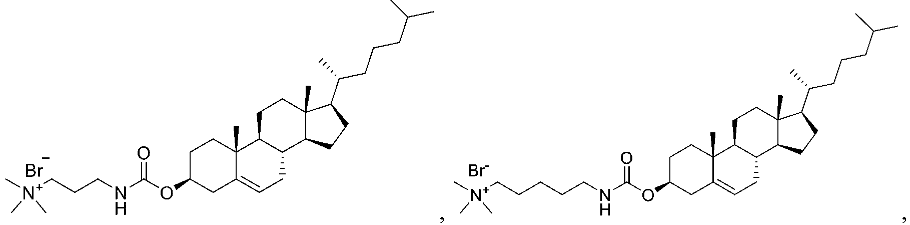 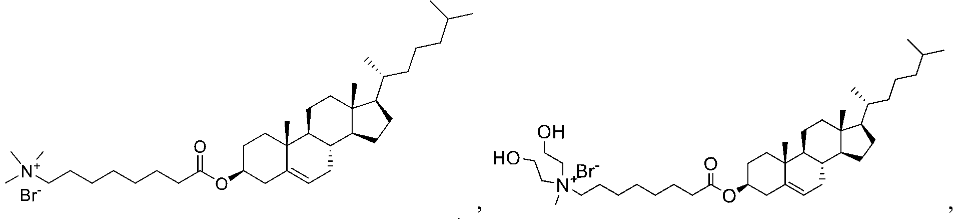 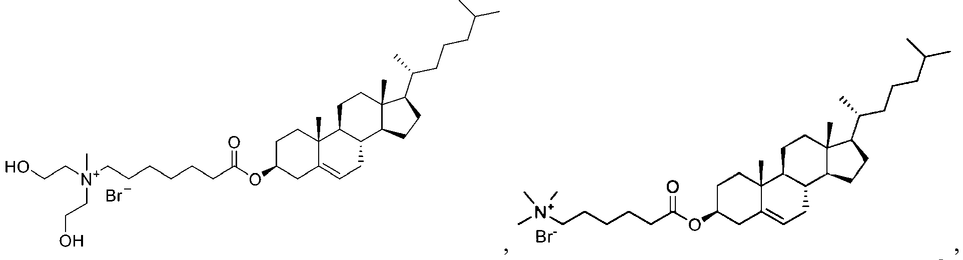 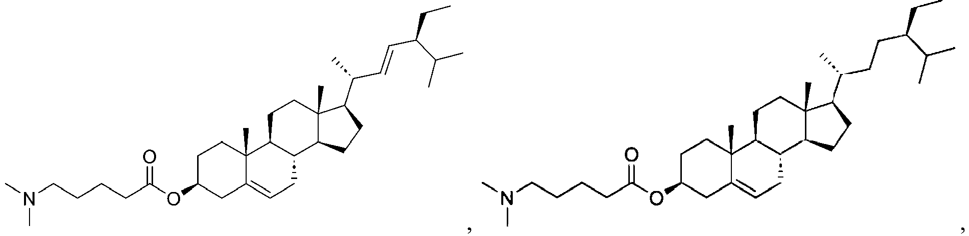 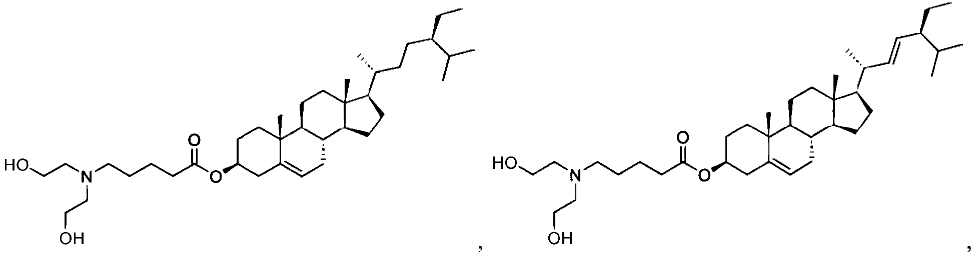 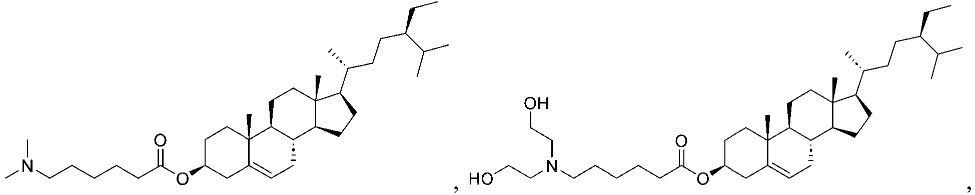 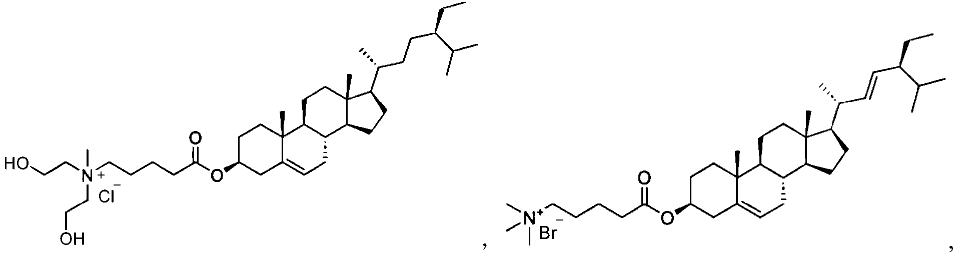 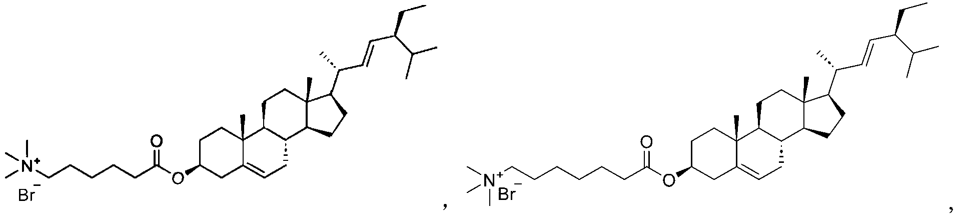 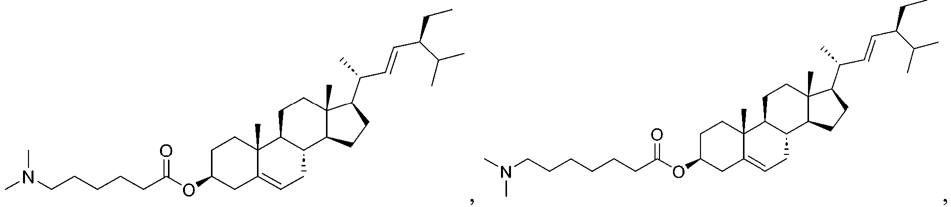 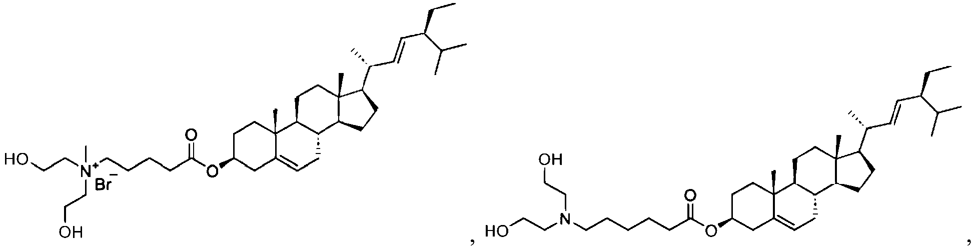 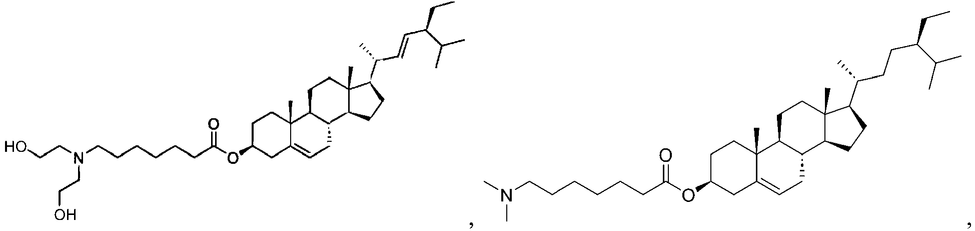 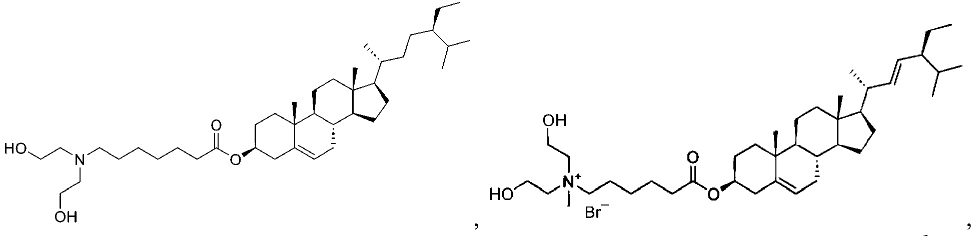 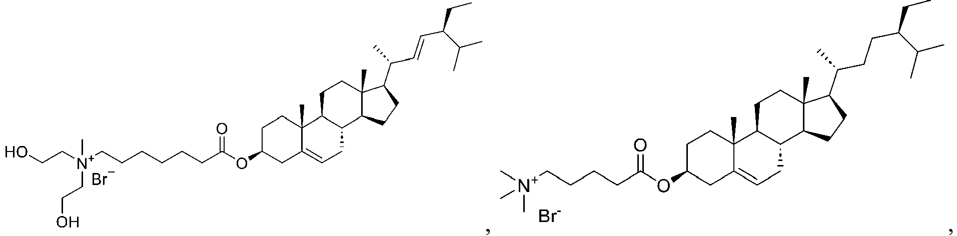 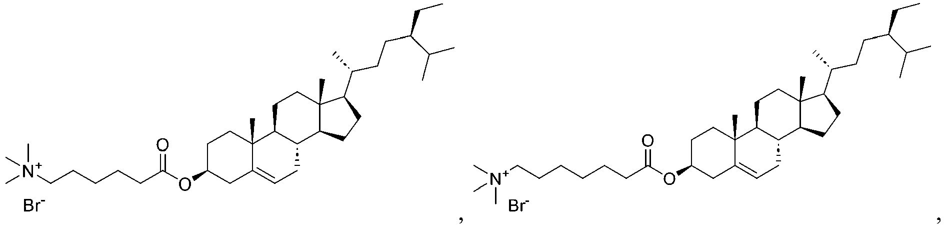 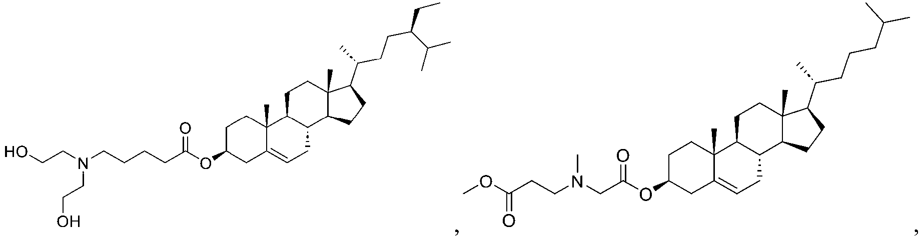 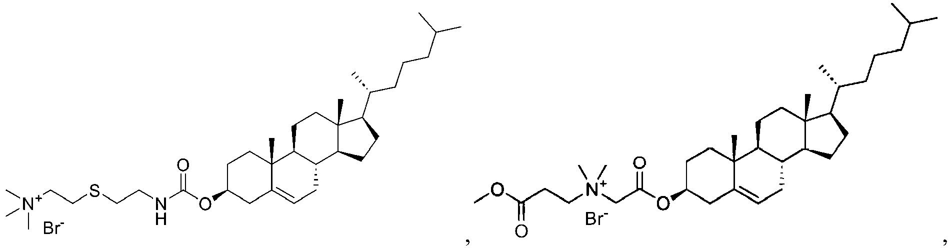 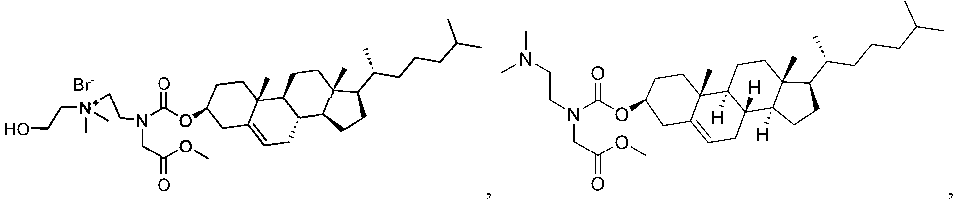 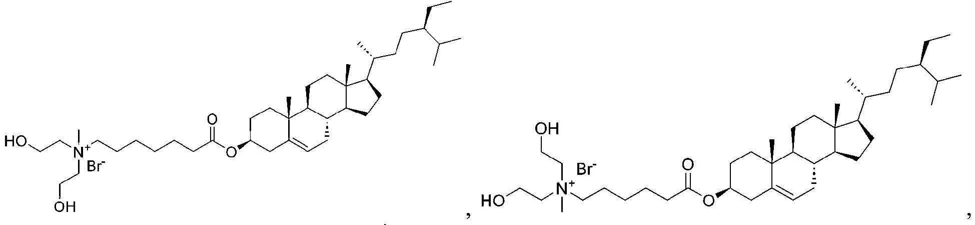 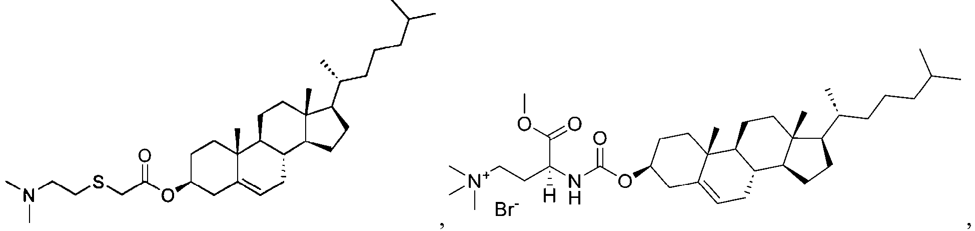 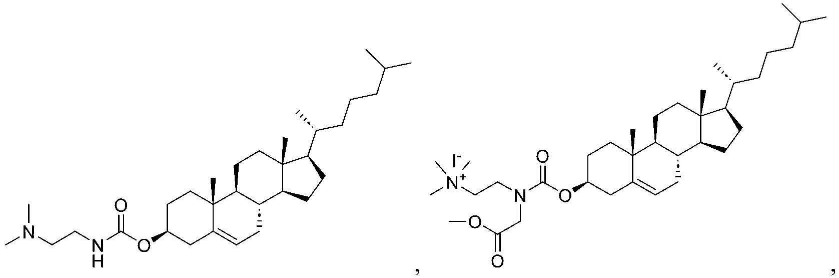 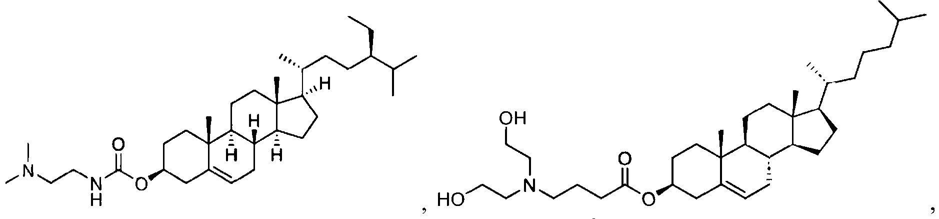 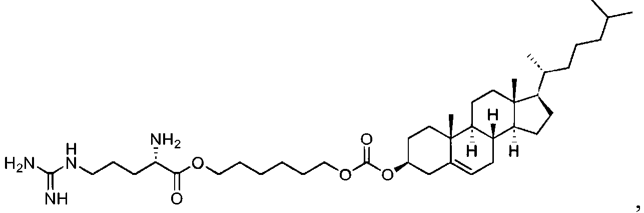 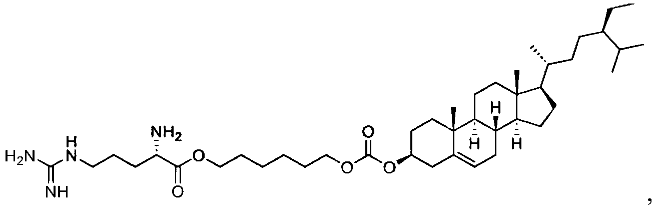 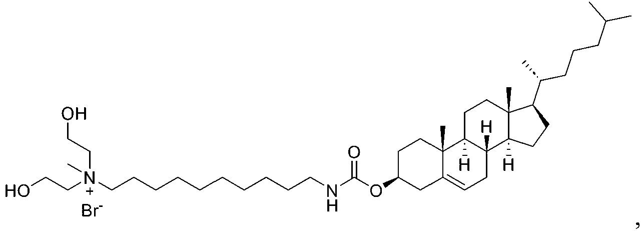 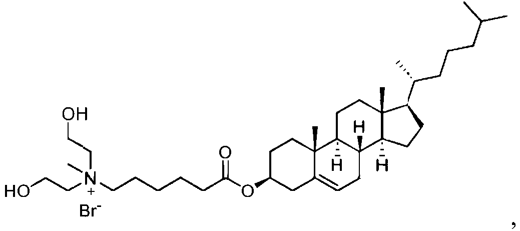 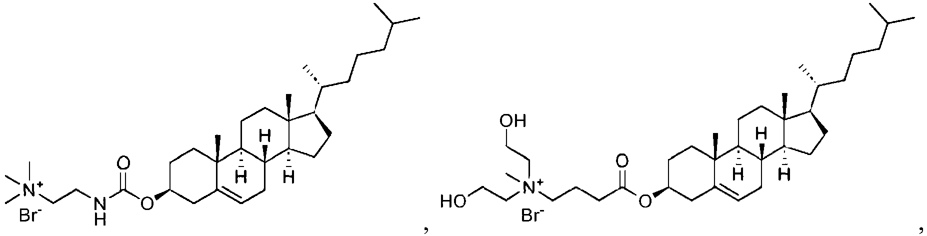 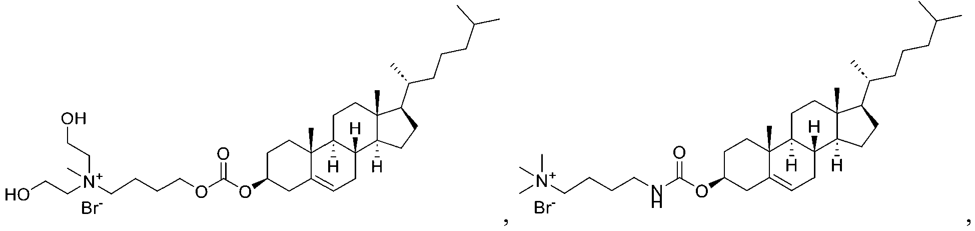 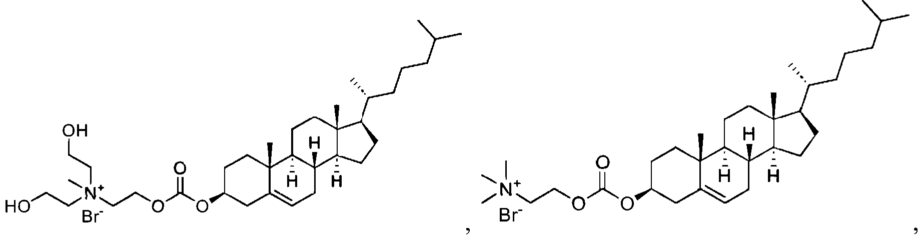 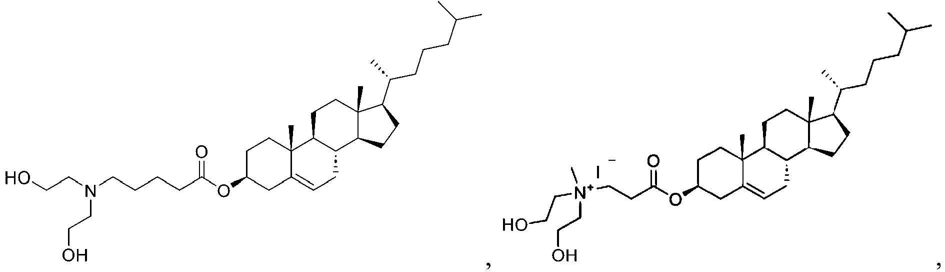 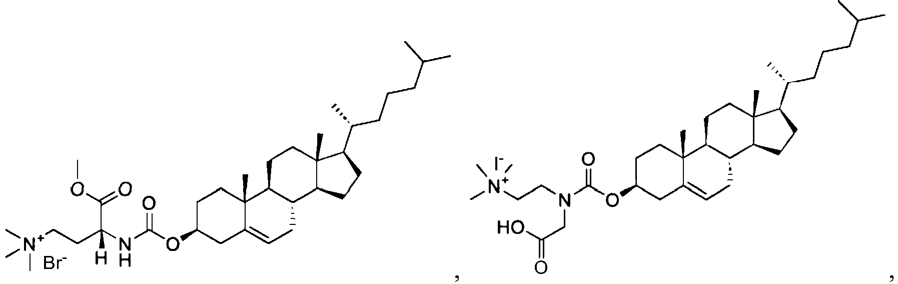 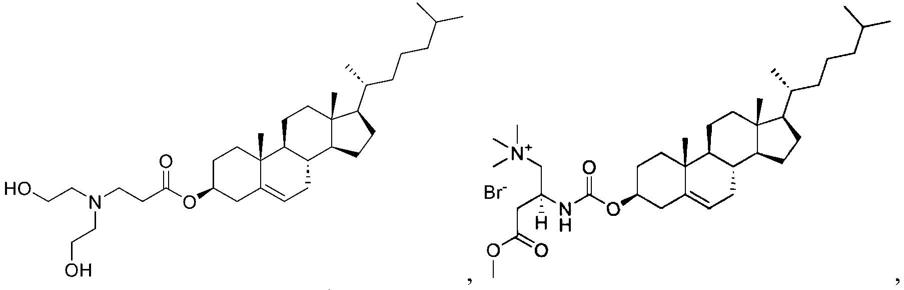 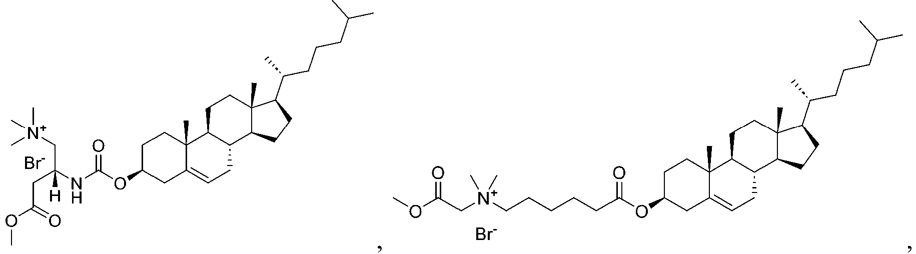 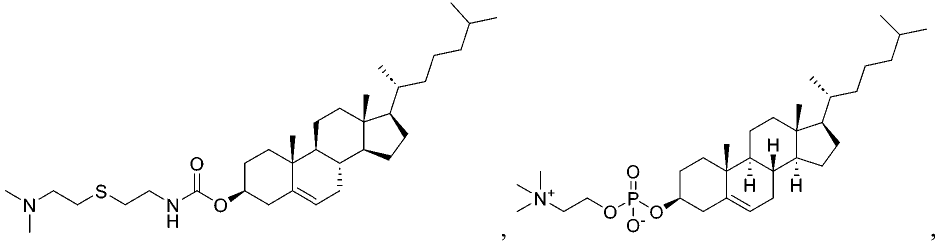 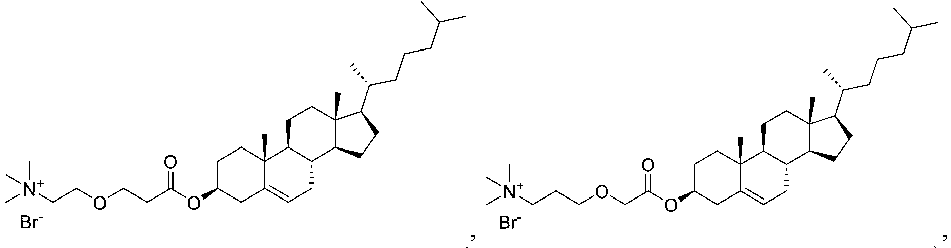 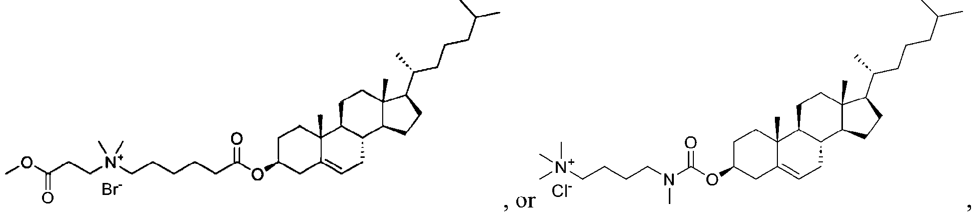 or a stereoisomer, a mixture of stereoisomers, or a pharmaceutically acceptable salt thereof.

In another aspect, provided herein is a lipid nanoparticle for use in delivering or expressing a payload in the spleen of a subject, wherein the lipid nanoparticle is administered to the subject via a systemic administration route, wherein the lipid nanoparticle comprises an ionizable lipid and a steroid compound, and wherein the steroid compound comprises one or more cationic groups.

In one embodiment, the systemic administration route is intravenous administration, intraarterial administration, or intraperitoneal administration.

In one embodiment, the cationic group is a tertiary amine, quaternary ammonium, primary amine, ammonium, imidazole, pyridine, pyrrole, pyrrolidine, pyrazole, isothiazole, isooxazole, oxadiazine, oxazoline, dithiourazole, isotriazole, tetrazole, pentazole, indole, dihydroindole, isooxazole, pyrimidine, pyrazine, quinazoline, piperazine, piperidine, morpholine, pyran, quinoxaline, quinoline, quinolinone, isoquinoline, amidine, guanidine, or urea.

In one embodiment, the steroid compound comprises a Z₁ moiety, and wherein the Z₁ is: 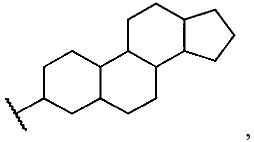 or a stereoisomer, or a mixture of stereoisomers thereof, wherein one or more ring C-C bond in Z₁ is independently optionally replaced by a C=C bond as valency permits, wherein Z₁ is optionally substituted with one or more groups selected from hydroxyl, halogen, and C₁₋₁₄ alkyl or C₁₋₁₄ alkenyl, wherein the alkyl is optionally substituted with one or more hydroxyl, nitro, cyano, halogen, C₁₋₆ alkoxy, C₂-C₆ alkenyl, C₂-C₆ alkynyl, C₃-C₈ cycloalkyl, C₆-C₁₀ aryl, 5 to 10-membered heteroaryl, or 3 to 8-membered heterocyclyl, and wherein the attachment in Z₁ is to the rest of the steroid compound. In one embodiment, the Z₁ moiety is a monovalent radical of cholesterol, cholestane, campesterol, sitosterol, sitostanol, beta-sitosterol, stigmasterol, stigmastanol, brassicasterol, avenasterol, ergosterol, ursolic acid, tocopherol, lumisterol, or a stereoisomer, a mixture of stereoisomers, or a pharmaceutically acceptable salt thereof.

In one embodiment, the steroid compound is a compound of formula (I): 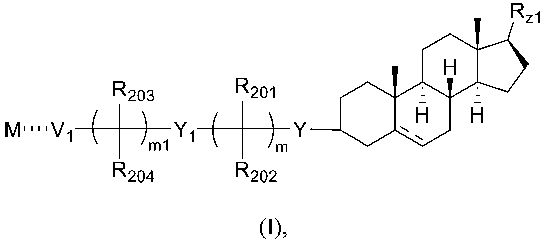 or a stereoisomer, a mixture of stereoisomers, or a pharmaceutically acceptable salt thereof, wherein:
- - - is a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, -NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, -C(O)-, -OC(S)-, - C(S)O-, -OC(S)NR₂,-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

In one embodiment, the amount of the steroid compound is from about 15 mol% to about 80 mol%, about 20 mol% to about 50 mol%, about 20 mol% to about 40 mol%, or about 15 mol% to about 30 mol% of the total lipid present in the lipid nanoparticle. In one embodiment, the amount of the steroid compound is from about 20 mole % to about 30 mole % of the total lipid present in the lipid nanoparticle.

In one embodiment, the ionizable lipid comprises at least one ionizable headgroup and at least one biodegradable hydrophobic chain, wherein the ionizable lipid has a logP of at least about 10.1 and a pKa from about 4 to about 11; and the biodegradable hydrophobic chain has a formula of -R₁₆-M₀-R₁₇; wherein R₁₆ is a C₄-₁₄ alkylene, C₄-₁₄ alkenylene, or C₄-₁₄ alkynylene; R₁₇ is a C₆-₂₀ alkyl; and M₀ is a biodegradable group, and wherein the alkylene, alkenylene, alkynylene and alkyl are optionally substituted.

In one embodiment, M₀ is an ester group, an amide group, a thioester group, a carbonate group, a carbamate group, a carbamothioester group, a urea group, or a disulfide group.

In one embodiment, the ionizable lipid is a compound of formula (III): 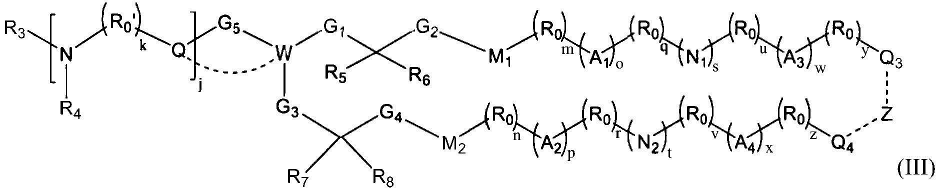 or a stereoisomer, a mixture of stereoisomers, tautomer, isotopologue, or a pharmaceutically acceptable salt thereof, wherein:
each instance of R₃ and R₄ are independently C₁₋₁₀ alkyl, C₂₋₁₀ alkenyl, C₂₋₁₀ alkynyl, C₃-C₁₄ cycloalkyl, 3 to 14-membered heterocyclyl, (C₁₋₁₀ alkylene)-(C₃-C₁₄ cycloalkyl), or (C₁₋₁₀ alkylene)-(3 to 14-membered heterocyclyl), or R₃ and R₄ together with the nitrogen atom they are attached to form a 3 to 14-membered heterocyclyl, or R₄ and one of the R₀' together with the atoms connecting them form a 4 to 10-membered heterocyclyl or 5 to 10-membered heteroaryl, and wherein the alkyl, alkenyl, alkynyl, cycloalkyl, heterocyclyl, heteroaryl, and alkylene are independently optionally substituted with one or more R*;
each instance of R₀' is an independently optionally substituted methylene, or wherein two R₀' together with the carbons they are attached to form a 3 to 8-membered cycloalkylene, and wherein the cycloalkylene is optionally substituted with one or more R**;
k is 0, 1, 2, 3, 4, 5, or 6;
j is 0 or 1;
the dashed line connecting Q and W is absent or a bond;
W is C, CH, or N, provided that when W is N, j is 0 and the dashed bond connecting Q and W is absent;
G₃ is absent, C₁₋₂₄ alkylene, C₂₋₂₄ alkenylene, C₃-C₈ cycloalkylene, C₃-C₈ cycloalkenylene, wherein the alkylene, alkenylene, cycloalkylene, cycloalkenylene are optionally substituted with one or more R**;
G₁, G₂, G₃ and G₄ are each independently absent, C₁₋₁₃ alkylene, C₂₋₁₃ alkenylene, or C₂₋₁₃ alkynylene, wherein the alkylene, alkenylene, and alkynylene are optionally substituted by one or more R⁵;
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R₅, R₆, R₇ and R₈ are each independently hydrogen or optionally substituted C₁₋₈ alkyl;
when the dashed line connecting Q and W is absent, Q is absent or is selected from -C(O)O-, -O-, -NH-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, - NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, - NR_{b}C(S)O-, -S-S-or -S(O)₀₋₂-, wherein the attachment to the right is to the direction of W;
when the dashed line connecting Q and W is a bond, G₅ is absent, and Q and W form a 5 to 10-membered single ring or fused ring, optionally wherein the ring is substituted with one or more R**;
M₁ and M₂ are each independently absent, or selected from -C(O)O-, -O-, -SC(O)O-, -OC(O)NRₐ-, - NRₐC(O)NRₐ-, -OC(O)S-, -OC(O)O-, -NRₐC(O)O-, -OC(O)-, -SC(O)-, -C(O)S-, -NRₐ-, -C(O)NRₐ-, - NRₐC(O)-, -NRₐC(O)S-, -SC(O)NRₐ-, -C(O)-, -OC(S)-, -C(S)O-, -OC(S)NRₐ-, -NRₐC(S)O-, -S-S-or -S(O)₀₋₂-;
each instance of R₀ is independently -(CRR')-;
each instance of R and R' are independently H, C₁₋₂₀ alkyl, Lₐ-ORₐ, -Lₐ-SRₐ, or -Lₐ-NRₐR'ₐ, or R and R' together with the carbon atom they are attached to form a C₃₋₈ cycloalkylene;
Q₃ and Q₄ are each independently H, -(CRR')-, C₆₋₁₀ aryl, or a steroid moiety;
each instance of A₁, A₂, A₃, A₄ are independently -(CR₁₈R₁₈-CR₁₈=CR₁₈)- or -(CR₁₈R₁₈-C≡C)-;
each instance of R₁₈ is independently H or C₁₋₂₀ alkyl;
each instance of N₁ and N₂ are independently a biodegradable group;
Z is absent, C₁₋₁₀ alkylene, or -O-P(O)(OH)-O-;
each of the dashed lines between Z-Q₃ or Z-Q₄ is independently absent or a bond, provided that when Z is absent, both dashed lines are absent;
m, n, q, r, u, v, y, and z are each independently 0, 1, 2, 3, 4, 5, 6, 7, 8, 9 or 10;
o, p, w, and x are each independently 0, 1, or 2;
s and t are each independently 0 or 1;
the number of carbon atoms between G₁ and Q₃ or between G₃ and Q₄ is from 8 to 30;
Lₐ and Lₑ are each independently absent or C₁₋₂₀ alkylene;
L_{b} and L_{f} are each independently absent or C₁₋₁₀ alkylene;
L_{c} is absent or C₁₋₈ alkylene;
L_{d} is absent or C₁₋₁₄ alkylene;
R* is hydroxyl, halogen, C₁₋₁₀ alkyl, C₁₋₁₀ haloalkyl, C₁₋₁₀ hydroxyalkyl, -L_{b}-OR_{b}, -L_{b}-SR_{b}, or -L_{b}-NR_{b}R'_{b};
R** is hydroxyl, halogen, C₁₋₈ alkyl, C₁₋₈ haloalkyl, C₁₋₈ hydroxyalkyl, -L_{b}-OR_{b}, -L_{b}-SR_{b}, or -L_{b}-NR₆R'_{b};
R^{s} is C₁₋₁₄ alkyl, -L_{b}-OR_{b}, -L_{b}-SR_{b}, or -L_{b}-NR_{b}R'_{b};
Rₐ and R'ₐ are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
R_{b} and R'_{b} are each independently H, C₁₋₁₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₁₀ alkyl, -L_{f}-OR_{f}, -L_{f}-SR_{f}, or -L_{f}-NR_{f}R'_{f},
R_{c} and R'_{c} are each independently hydrogen or C₁₋₈ alkyl;
R_{d} and R'_{d} are each independently hydrogen or C₁₋₁₄ alkyl;
Rₑ and R'ₑ are each independently hydrogen or C₁₋₂₀ alkyl; and
R_{f} and R'_{f} are each independently hydrogen or C₁₋₁₀ alkyl.

In one embodiment, the ionizable lipid is a compound of formula (IV'): 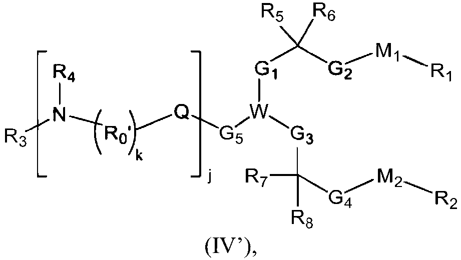 or a stereoisomer, a mixture of stereoisomers, or a pharmaceutically acceptable salt thereof, wherein
j is 0 or 1;
W is CH or N, provided that when W is N, j is 0;
k is 0, 1, 2, 3, 4, 5, 6, 7, or 8;
each instance of R₀' is an independently optionally substituted methylene, or wherein two R₀' together with the carbons they are attached to form a 3 to 8-membered cycloalkylene, and wherein the cycloalkylene is optionally substituted with one or more R**;
M₁ and M₂ are each independently -C(O)O-, -O-, -SC(O)O-, -OC(O)NRₐ-, -NRₐC(O)NRₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, or -S(O)₀₋₂-;
Q is absent, -C(O)O-, -O-, -NH-, -SC(O)O-, -OC(O)NR_{b}-, -NR_{b}C(O)NR_{b}-, -OC(O)S-, -OC(O)O-, - NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S-, or -S(O)₀₋₂-;
Rₐ and R_{b} are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
G₅ is absent or optionally substituted C₁₋₈ alkylene;
R₁ and R₂ are each independently C₄₋₂₀ alkyl, C₄₋₂₀ alkenyl or C₄₋₂₀ alkynyl, wherein one or more methylene units in R₁ and R₂ are independently optionally replaced by -NH- or -N(C₁₋₂₀ alkyl), wherein the alkyl, alkenyl and alkynyl are optionally substituted;
each instance of R₃ and R₄ are independently H, C₁₋₁₀ alkyl, C₂₋₁₀ alkenyl, C₂₋₁₀ alkynyl, C₃-C₁₄ cycloalkyl, or 3- to 14-membered heterocyclyl, or R₃ and R₄ together with the N atom to which they are attached form 3 to 14-membered heterocyclyl, or R₄ and one of the R₀' together with the atoms connecting them form a 4 to 10-membered heterocyclyl; and wherein the alkyl, alkenyl, alkynyl, cycloalkyl, heterocyclyl are optionally substituted with one or more R*; and
R₅, R₆, R₇ and R₈ are each independently hydrogen or optionally substituted C₁₋₈ alkyl.

In one embodiment, the ionizable lipid is a compound of formula (V'), (VI') or (VII'): 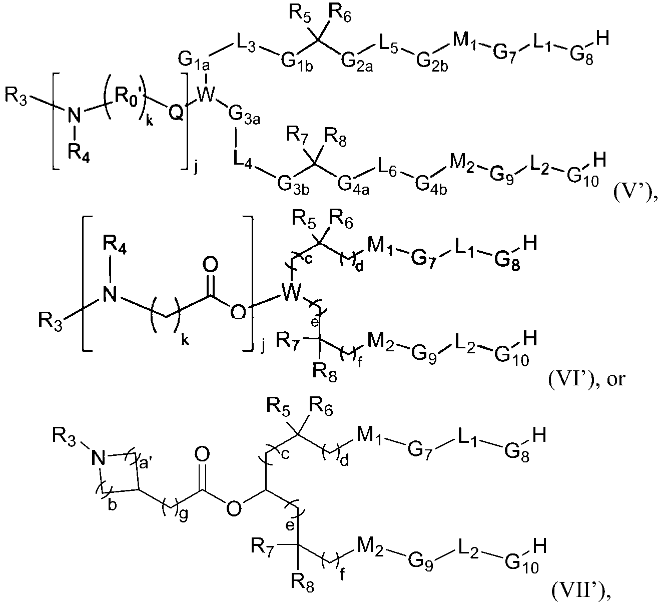 or a stereoisomer, a mixture of stereoisomers, or a pharmaceutically acceptable salt thereof, wherein
k is 0, 1, 2, 3, 4, or 5;
L₁ and L₂ are each independently -(CRR')₂-, -CH=CH-, -C≡C- or -NR"-;
one of L₃ and L₅ is -(CR^{s}R^{s}')₂-, -CH=CH-, or -C≡C-, and the other is absent;
one of L₄ and L₆ is -(CR^{s}R^{s}')₂-, -CH=CH-, or -C≡C-, and the other is absent;
each of G₁ₐ, G_{1b}, G₂ₐ, G_{2b}, G₃ₐ, G_{3b}, G₄ₐ and G_{4b} is independently absent or optionally substituted C₁₋₇ alkylene;
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms;
R₅, R₆, R₇ and R₈ are each independently hydrogen or optionally substituted C₁₋₆ alkyl;
G₇, G₈, G₉ andG₁₀ are each independently absent or optionally substituted C₁₋₁₂ alkylene, provided that G₇ and G₈ have a total length of 4, 5, 6, 7, 8, 9, 10, 11 or 12 carbon atoms, and G₉ and G₁₀ have a total length of 4, 5, 6, 7, 8, 9, 10, 11 or 12 carbon atoms;
R^{s} and R^{s}' are each independently H, C₁₋₁₀ alkyl, -L_{d}-OR_{d} or -L_{d}-NR_{d}R'_{d};
R' is H, C₁₋₁₄ alkyl, -Lₐ-ORₐ or -Lₐ-NRₐR'ₐ;
R" is H or C₁₋₁₄ alkyl;
L_{d} is absent or C₁₋₁₀ alkylene;
Lₐ is absent or C₁₋₁₄ alkylene;
Rₐ and R'ₐ are each independently H, C₁₋₁₄ alkyl, C₃₋₁₀ cycloalkyl or 3- to 10-membered heterocyclyl, and wherein the alkyl, cycloalkyl and heterocyclyl are optionally substituted;
R_{d} and R'_{d} are each independently H or C₁₋₁₀ alkyl;
a' and b are each independently 0, 1, 3, 4, 5, provided that at least one of a' and b is not 0;
g is 0, 1, 2, 3, 4, or 5;
a'+g is 0, 1, 2, 3, 4, or 5; and
c, d, e, and f are each independently 0, 1, 2, 3, 4, 5, 6, or 7, provided that c+d is an integer between 2 and 9, and e+f is an integer between 2 and 9.

In one embodiment, the ionizable lipid is a compound of formula (VIII): 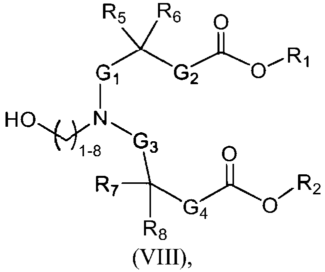 or a stereoisomer, a mixture of stereoisomers, or a pharmaceutically acceptable salt thereof.

In one embodiment, R₅, R₆, R₇ and R₈ are each independently hydrogen or methyl.

In one embodiment, G₁, G₂, G₃ and G₄ are each independently absent or C₁₋₈ alkylene.

In one embodiment, the ionizable lipid is a compound of formula (IX): 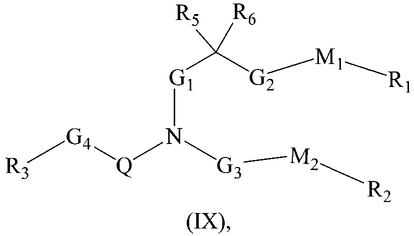 or a stereoisomer, a mixture of stereoisomers, or a pharmaceutically acceptable salt thereof, wherein:
G₁ and G₂ are each independently a bond, C₁₋₁₃ linear alkylene, C₂₋₁₃ linear alkenylene, or C₂₋₁₃ linear alkynylene, wherein the alkylene, alkenylene, and alkynylene are optionally substituted by one or more R_{G1};
G₁ and G₂ have a total length of 3, 4, 5, 6, 7, 8, 9, 10, 11, 12 or 13 carbon atoms;
each instance of R_{G1} is independently H, C₁₋₁₄ alkyl, -Lₐ-ORₐ, -Lₐ-SRₐ, or -Lₐ-NRₐR'ₐ;
G₃ is C₄-₁₄ linear alkylene, C₄₋₁₄ linear alkenylene, or C₄-₁₄ linear alkynylene, wherein the alkylene, alkenylene, and alkynylene are optionally substituted by one or more R_{G3};
each instance of R_{G3} is independently H, -Lₐ-ORₐ, -Lₐ-SRₐ or -Lₐ-NRₐR'ₐ;
Lₐ is independently a bond or C₁₋₁₄ alkylene;
Rₐ and R'ₐ are each independently H, C₁₋₁₄ alkyl, C₃₋₁₄ cycloalkyl, or 3- to 14-membered heterocyclyl;
G₄ is a bond, C₁₋₆ alkylene, C₂₋₆ alkenylene, or C₂₋₆ alkynylene, and wherein the alkylene, alkenylene, and alkynylene are optionally substituted by one or more R_{G4};
each of instance of R_{G4} is independently H, alkyl, -L_{b}-OR_{b}, -L_{b}-SR_{b} or -L_{b}-NR_{b}R'_{b};
L_{b} is independently a bond or C₁₋₆ alkylene;
R_{b} and R'_{b} are independently H, alkyl, C₃₋₁₀ cycloalkyl or 3-to 10-membered heterocyclyl;
or two R_{G4} together with the same carbon atom they are attached to form a C₃₋₁₄ cycloalkylene or 3- to 14-membered heterocyclylene, wherein the cycloalkylene and heterocyclylene are optionally substituted by one or more R_{4g};
each of instance of R_{4g} is independently H, halogen, cyano, C₁₋₈ alkyl, C₁₋₈ haloalkyl, -Lₑ-ORₑ, -Lₑ-SRₑ, or -Lₑ-NRₑR'ₑ;
Lₑ is independently a bond or C₁₋₈ alkylene;
Rₑ and R'ₑ are independently H, C₁₋₈ alkyl, C₃₋₁₄ cycloalkyl, or 3- to 14-membered heterocyclyl;
M₁ and M₂ are each independently -C(O)O-, -OC(O)-, -O-, -SC(O)O-, -OC(O)NR-, -NRC(O)NR-, - OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S- or -S(O)₀₋₂-;
Q is a bond, -C(O)O-, -O-, -SC(O)O-, -OC(O)NR_{f}-, -NR_{f}C(O)NR_{f}-, -OC(O)S-, -OC(O)O-, -NR_{f}C(O)O-, -OC(O)-, -SC(O)-, -C(O)S-, -NR_{f}-, -C(O)NR_{f}-, -NR_{f}C(O)-, -NR_{f}C(O)S-, -SC(O)NR_{f}-, -C(O)-, -OC(S)-, - C(S)O-, -OC(S)NR_{f}-, -NR_{f}C(S)O-, -S-S-, -S(O)₀₋₂-, phenylene, or pyridylidene, wherein the phenylene and pyridylidene are optionally substituted with one or more R*;
R* is independently H, halogen, cyano, C₁₋₁₀ alkyl, C₁₋₁₀ haloalkyl, -L_{f}-OR_{f}, -L_{f}-SR_{f}, or -L_{f}-NR_{f}R'_{f},
L_{f} is independently a bond or C₁₋₈ alkylene;
R_{f} and R'_{f} are independently H, C₁₋₁₀ alkyl, C₃₋₁₄ cycloalkyl, or 3- to 14-membered heterocyclyl;
R₁ and R₂ are independently C₄₋₂₀ alkyl, C₄₋₂₀ alkenyl, or C₄₋₂₀ alkynyl, which is optionally substituted with one or more R₁ₛ, and wherein one or more methylene units are optionally and independently replaced with -NR'-;
R₁ₛ is independently H, C₁₋₂₀ alkyl, -L_{c}-OR_{c}, -L_{c}-SR_{c}, or -L_{c}-NR_{c}R'_{c};
R are R' are each independently H or C₁₋₂₀ alkyl;
L_{c} is independently a bond or C₁₋₂₀ alkylene;
R_{c} and R'_{c} are each independently H, C₁₋₂₀ alkyl, C₃₋₁₄ cycloalkyl, or 3- to 14-membered heterocyclyl;
R₃ is -CN, -OR_{g}, -C(O)R_{g}, -OC(O)R_{g}, -NR"C(O)R_{g}, -NR_{g}R'_{g}, -NR"C(O)NR_{g}R'_{g}, -NR"C(O)R_{g}, - NR"S(O)₂R_{g}, -OC(O)NR_{g}R'_{g}, -NR"C(O)OR_{g}, -N(OR_{g})C(O)R_{g}, -N(OR_{g})S(O)₂R_{g}, -N(OR_{g})C(O)OR_{g}, - N(OR_{g})C(O)R_{g}R'_{g}, 3- to 14-membered heterocyclyl, or 5- to 14-membered heteroaryl;
R_{g} and R'_{g} are each independently H, C₁₋₁₀ alkyl, C₃₋₁₀ cycloalkyl, or 3- to 10-membered heterocyclyl;
R" is independently H or alkyl;
R₅ and R₆ are independently C₁₋₈ alkyl, wherein the alkyl is optionally substituted by one or more R₄ₛ;
or R₅ and R₆ together with the carbon atom to which they are attached form a C₃₋₁₄ cycloalkylene or 3-to 14-membered heterocyclylene, wherein the cycloalkylene and heterocyclylene are optionally substituted by one or more R₄ₛ;
each instance of R₄ₛ is independently H, halogen, cyano, C₁₋₈ alkyl, C₁₋₈ haloalkyl, -L_{d}-OR_{d}, -Lₐ-SRₐ, or - L_{d}-NR_{d}R'_{d};
L_{d} is independently a bond or C₁₋₈ alkylene; and
R_{d} and R'_{d} are independently H, C₁₋₈ alkyl, C₃₋₁₄ cycloalkyl or 3- to-14-membered heterocyclyl.

In one embodiment, the ionizable lipid is a compound of formula (X): 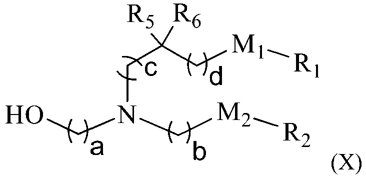 or a stereoisomer, a mixture of stereoisomers, or a pharmaceutically acceptable salt thereof, wherein:
a is 1, 2, 3, 4, 5 or 6;
b is 4, 5, 6, 7, 8, 9 or 10;
c is 1, 2, 3, 4, 5 or 6;
d is 0, 1, 2, 3 or 4; and
c+d is 3, 4, 5, 6, 7, 8 or 9.

In one embodiment, the ionizable lipid is: 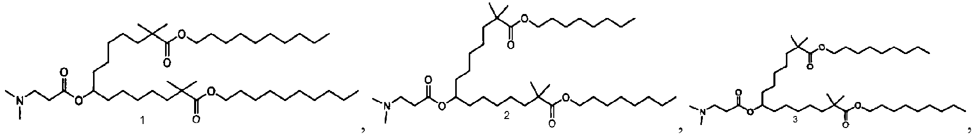 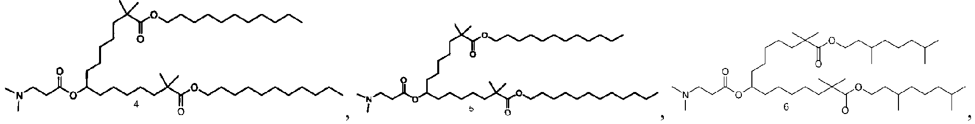 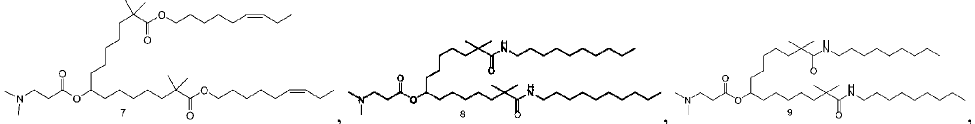 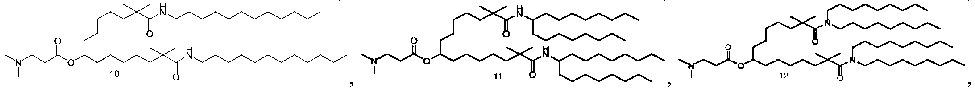 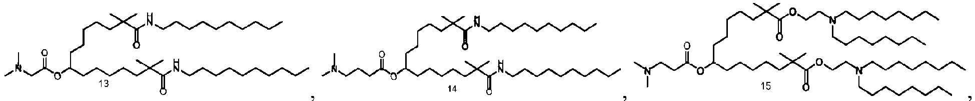 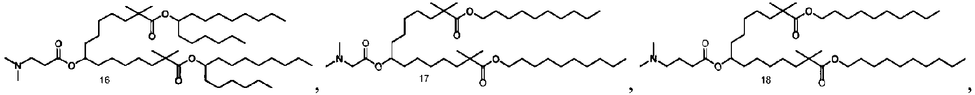 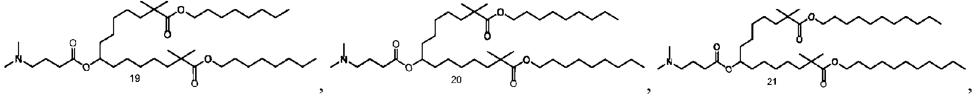 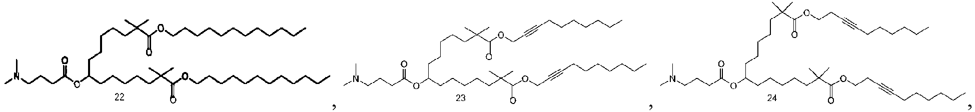 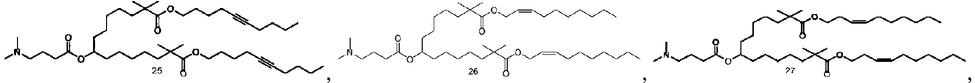 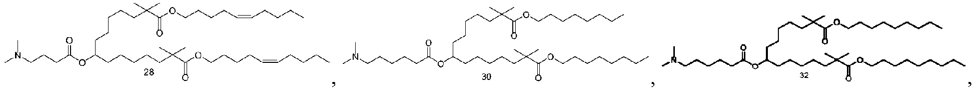 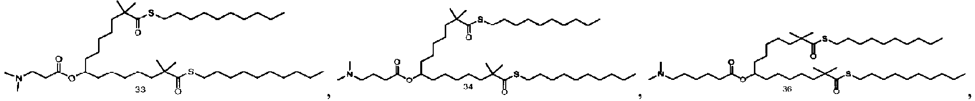 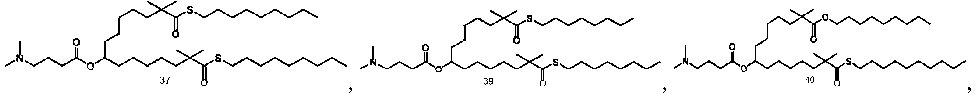 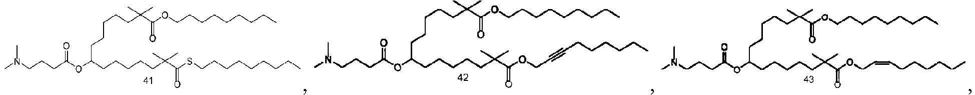 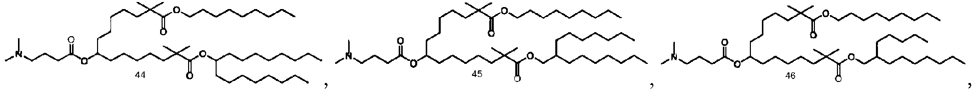 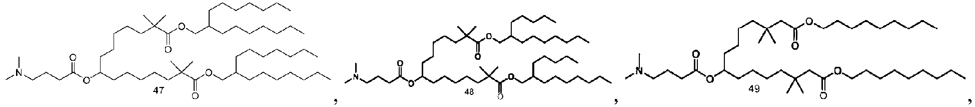 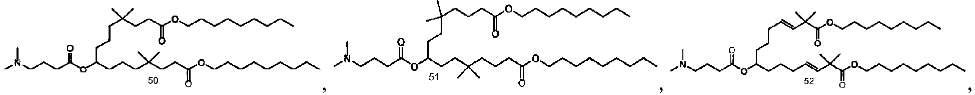 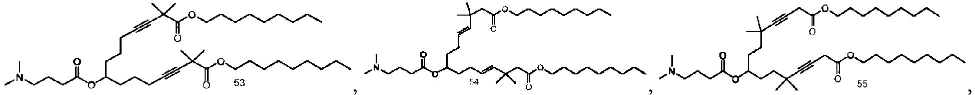 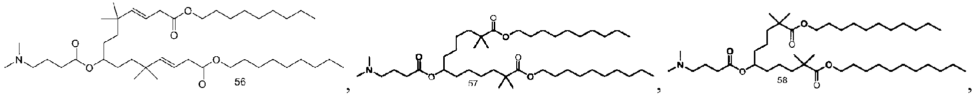 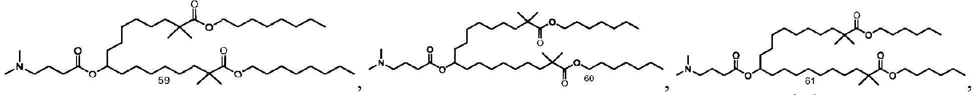 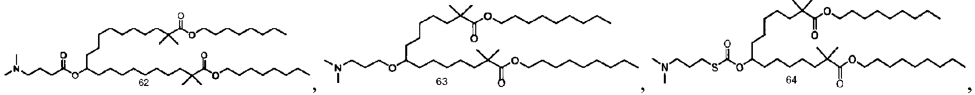 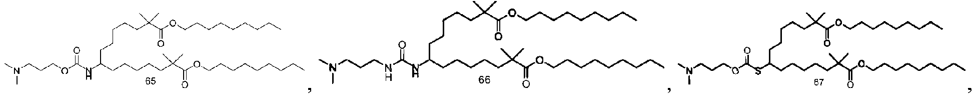 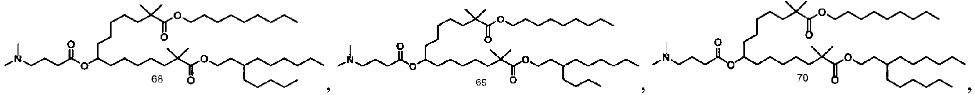 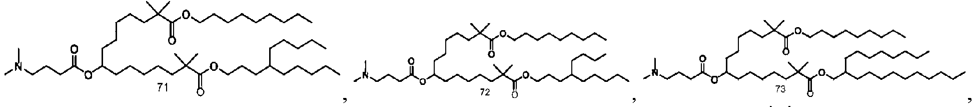 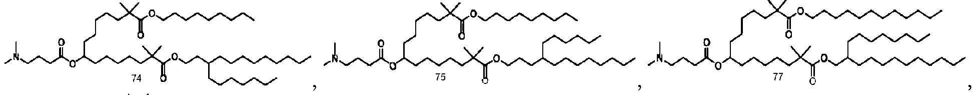 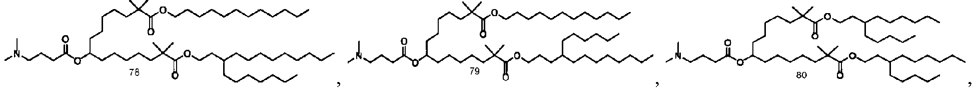 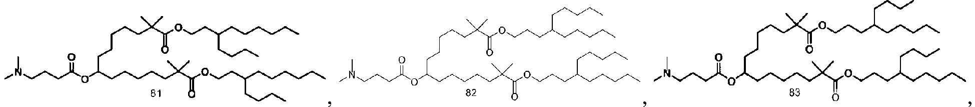 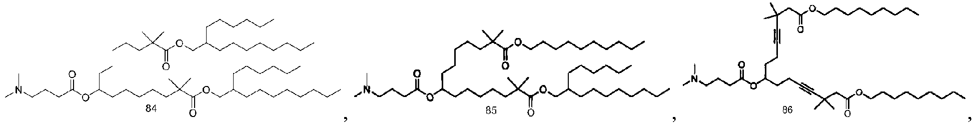 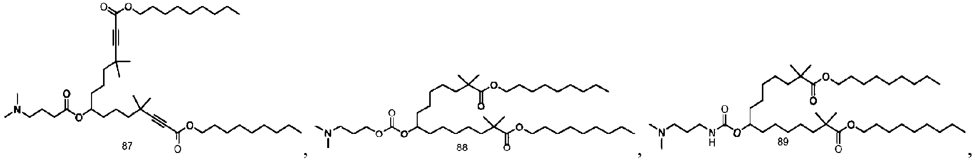 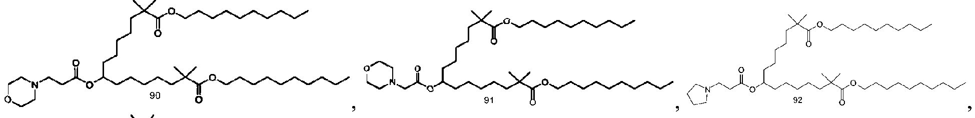 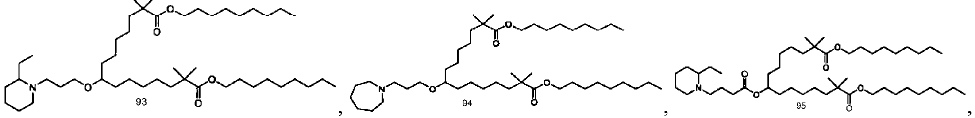 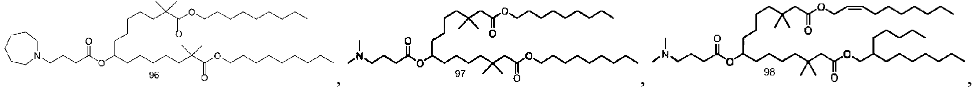 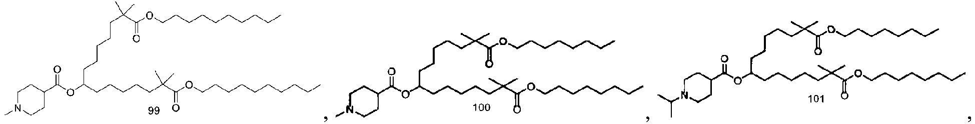 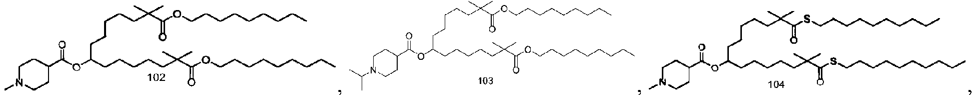 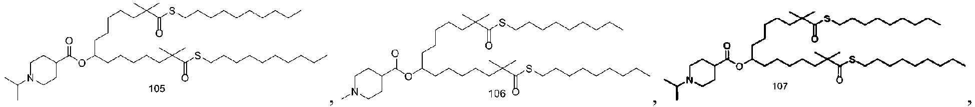 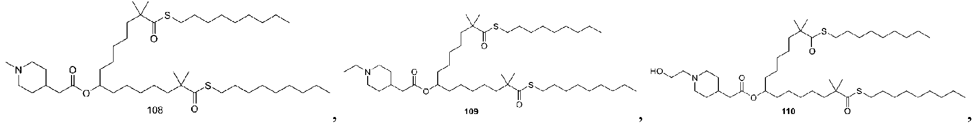 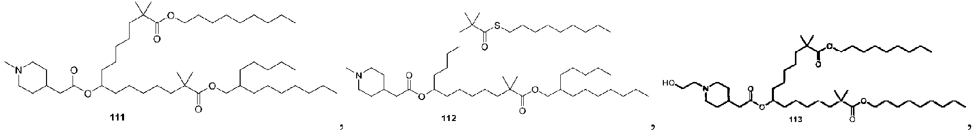 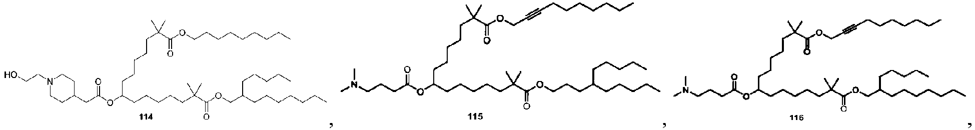 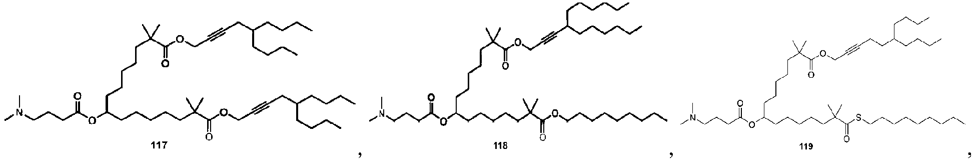 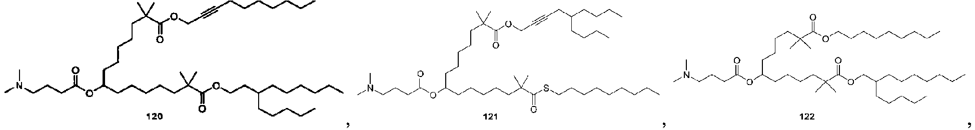 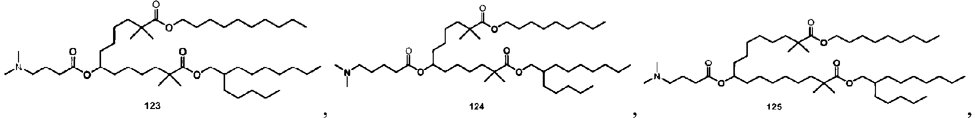 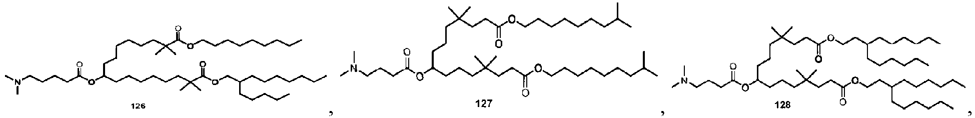 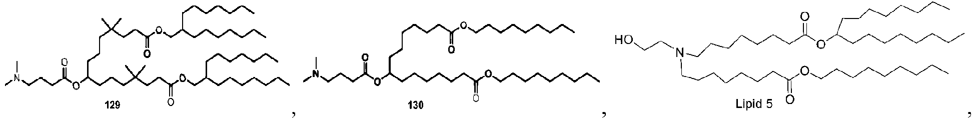 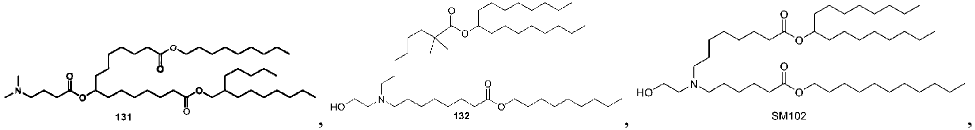 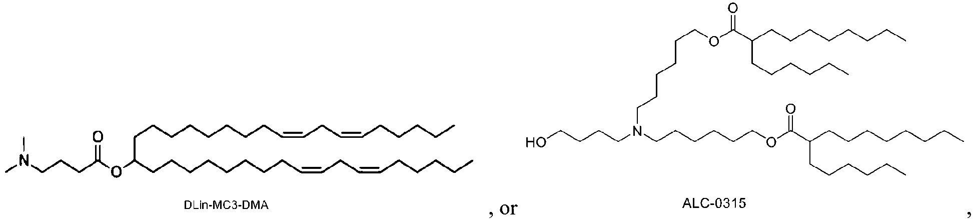 or a stereoisomer, a mixture of stereoisomers, or a pharmaceutically acceptable salt thereof.

In one embodiment, the ionizable lipid selected from the group consisting of: 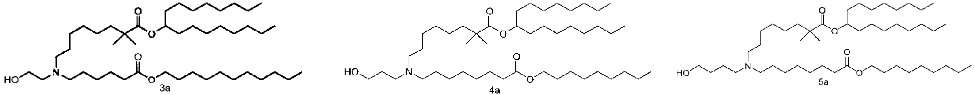 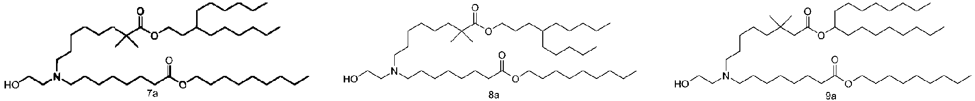 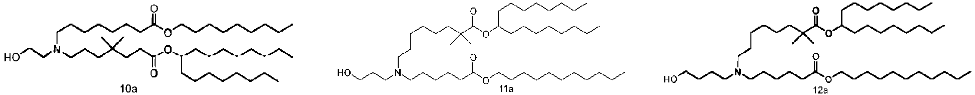 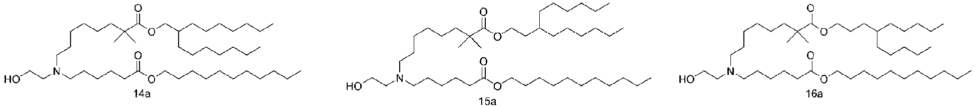 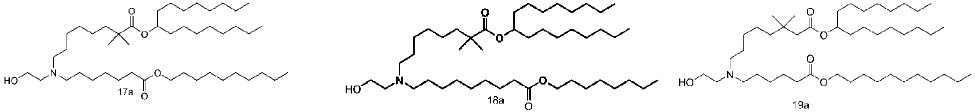 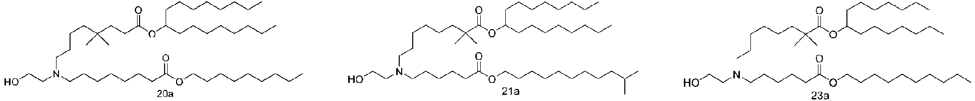 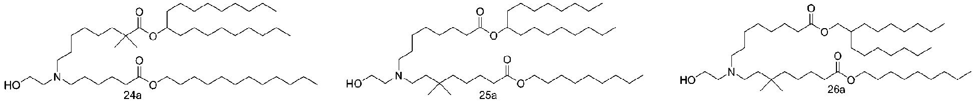 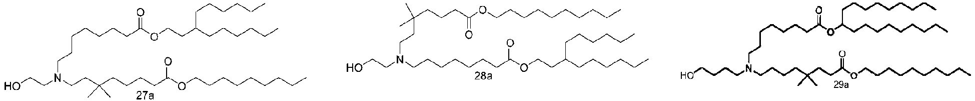 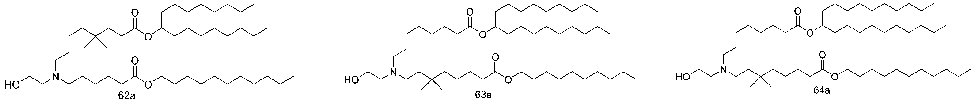 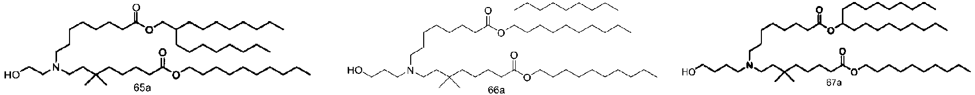 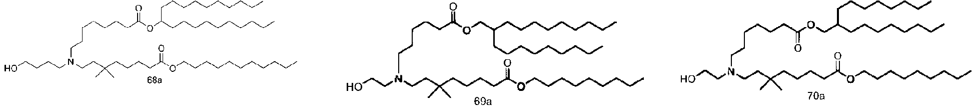 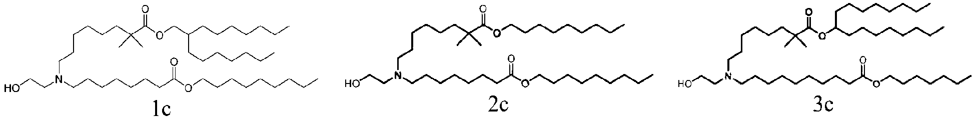 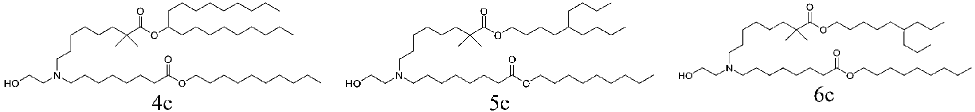 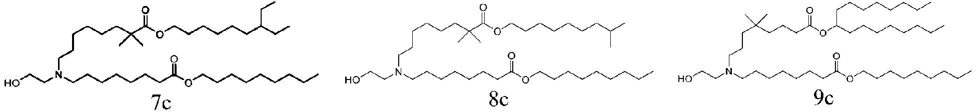 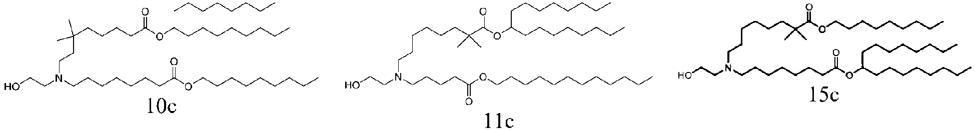 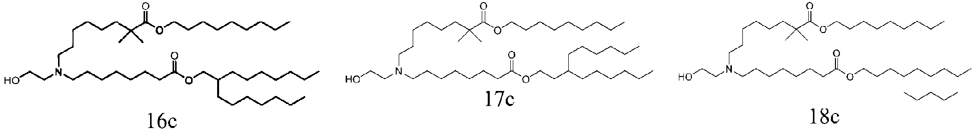 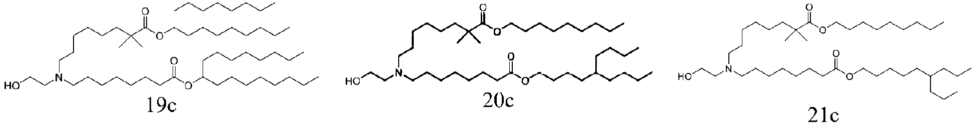 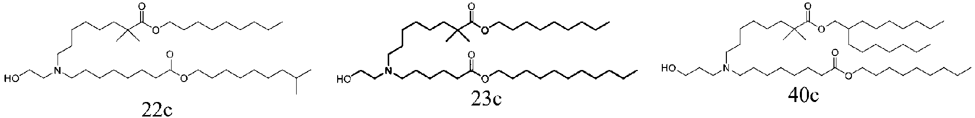 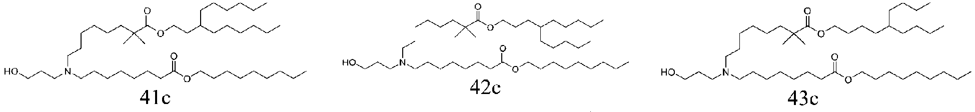 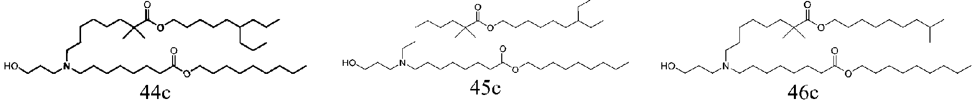 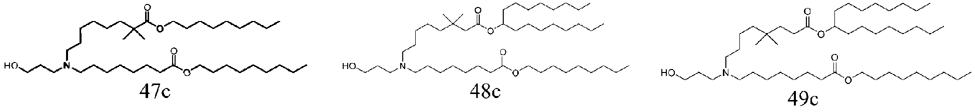 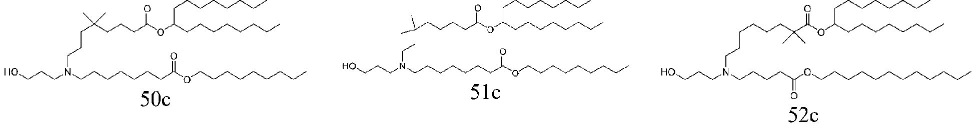 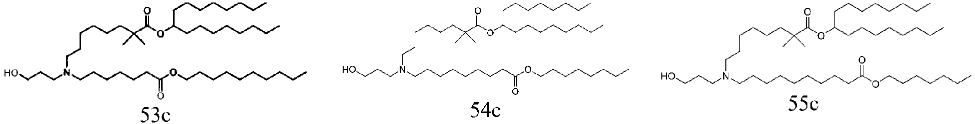 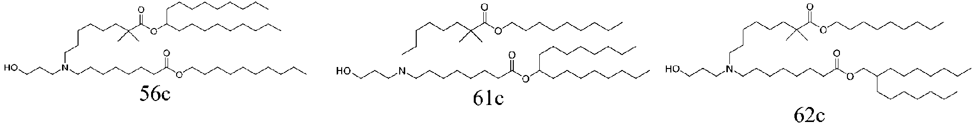 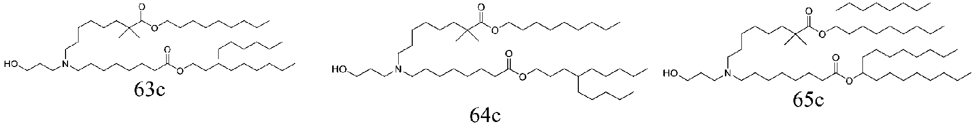 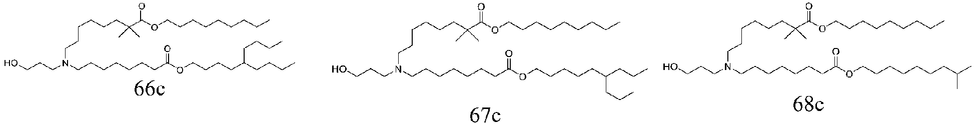 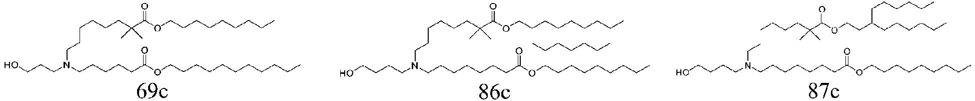 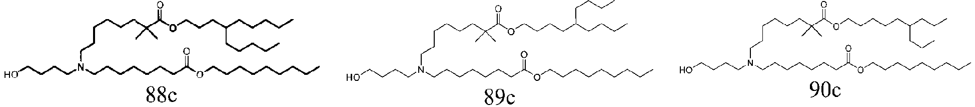 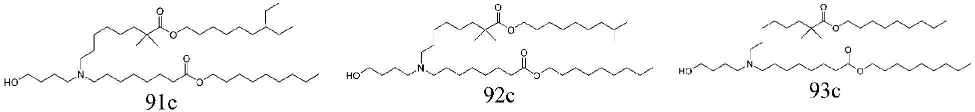 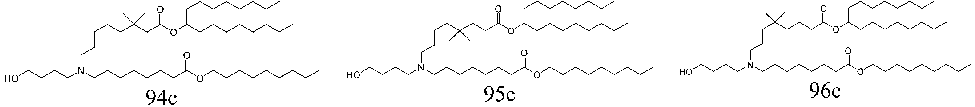 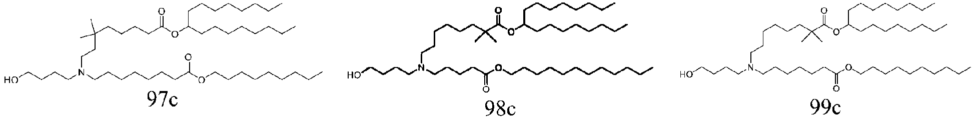 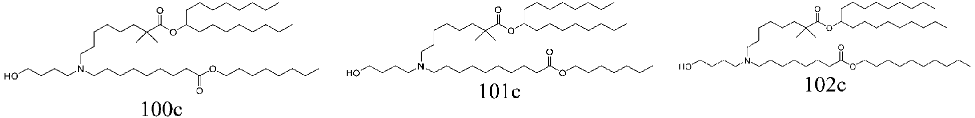 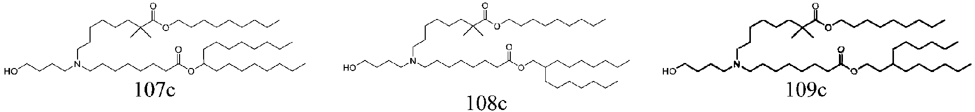 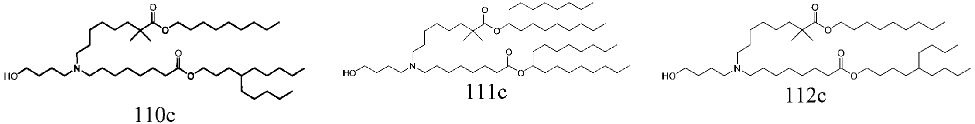 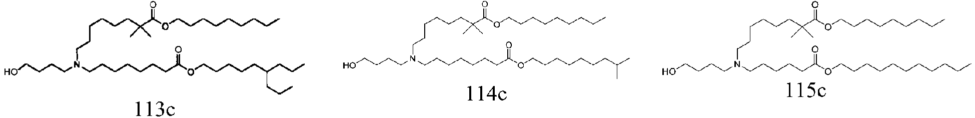 or a stereoisomer, a mixture of stereoisomers, or a pharmaceutically acceptable salt thereof.

In one embodiment, the ionizable lipid is DODAP, DODMA, DLinDMA, DLin-KC2-DMA, DLin-MC3-DMA, SM-102, or ALC-0315, or a combination thereof, or a stereoisomer, a mixture of stereoisomers, or a pharmaceutically acceptable salt thereof.

In one embodiment, the amount of the ionizable lipid is 35-70 mol % of the total lipid present in the lipid nanoparticle, more preferably 40-60 mol%, such as 35 mol%, 40 mol%, 45 mol%, 50 mol%, 55 mol% or 60 mol% of the total lipid present in the lipid nanoparticle.

In one embodiment, the lipid nanoparticle further comprises phospholipid. In one embodiment, the phospholipid is selected from the group consisting of DSPC, DMPC, DOPC, DPPC, POPC, DOPE, DMPE, POPOE, or DPPE, or a combination thereof.

In one embodiment, the lipid nanoparticle comprises a phospholipid in an amount of 5 mol%-30 mol%, preferably 5 mol%-20 mol%, more preferably 5 mol%-15 mol%, such as 5 mol%, 6 mol%, 7 mol%, 8 mol%, 9 mol%, 10 mol%, 11 mol%, 12 mol%, 13 mol%, 14 mol% or 15 mol% of the total lipid present in the lipid nanoparticle.

In one embodiment, the lipid nanoparticle further comprises a structural lipid. In one embodiment, the structural lipid is selected from the group consisting of cholesterol, campesterol, stigmasterol, sitosterol, brassicasterol, ergosterol, solanine, ursolic acid, alpha-tocopherol, beta-sitosterol, avenasterol, and canola sterol.

In one embodiment, the amount of the structural lipid is 10 mol%-80 mol%, more preferably 15 mol%-70 mol%, more preferably 30 mol%-50 mol%, more preferably 30 mol%-40 mol%, such as 30 mol%, 35 mol%, 40 mol%, 45 mol%, 50 mol%, 55 mol% or 60 mol% of the total lipid present in the lipid nanoparticle.

In one embodiment, the lipid nanoparticle further comprises a polymer conjugated lipid. In one embodiment, the polymer conjugated lipid is a pegylated lipid. In one embodiment, the pegylated lipid comprises a pegylated moiety having a molecule weight of from about 1000 Da to about 20, 000 Da, from about 1000 Da to about 5000 Da, or from about 1000 Da to about 2000 Da. In one embodiment, the polymer conjugated lipid is ALC-0159, DMG-PEG2000, DMPE-PEG1000, DPPE-PEG1000, DSPE-PEG1000, DOPE-PEG1000, Ceramide-PEG2000, DMPE-PEG2000, DPPE-PEG2000, DSPE-PEG2000, DSPE-PEG2000-Mannose, Ceramide-PEG5000, DSPE-PEG5000, or DSPE-PEG2000 amine.

In one embodiment, the amount of the polymer conjugated lipid is from 0.25 mol% to 10 mol%, more preferably from 0.5 mol% to 5 mol%, more preferably from 0.5 mol% to 3 mol%, such as 0.5 mol%, 0.6 mol%, 0.7 mol%, 0.8 mol%, 0.8 mol%, 0.9 mol%, 1.0 mol%, 1.1 mol%, 1.2 mol%, 1.3 mol%, 1.4 mol%, 1.5 mol%, 1.6 mol%, 1.7mol%, 1.8 mol%, 1.9 mol%, 2.0 mol%, 2.1 mol%, 2.2 mol%, 2.3 mol%, 2.4 mol%, 2.5 mol%, 2.6 mol%, 2.7 mol%, 2.8 mol%, 2.9 mol%, 3.0 mol%, 3.1 mol%, 3.2 mol%, 3.3 mol%, 3.4 mol%, 3.5 mol%, 3.6 mol%, 3.7 mol%, 3.8 mol%, 3.9 mol%, or 4.0 mol% of the total lipid present in the lipid nanoparticle.

In one embodiment, the lipid nanoparticle comprises a payload, and wherein the payload is a therapeutic agent, a prophylactic agent, or a diagnostic agent. In one embodiment, the payload is nucleic acid. In one embodiment, the nucleic acid is antisense oligonucleotide (ASO), DNA, or RNA, optionally wherein the RNA is RNA interference (RNAi), small interfering RNA (siRNA), short hairpin RNA (shRNA), antisense RNA (aRNA), messenger RNA (mRNA), modified messenger RNA (mmRNA), long noncoding RNA (lncRNA), microRNA (miRNA), small activating RNA (saRNA), multicoding nucleic acid (MCNA), polymer-coded nucleic acid (PCNA), guide RNA (gRNA), CRISPR RNA (crRNA), or any other RNA in the ribozyme.

In one embodiment, the ratio of total number of nitrogen atoms in the steroid compound and ionizable lipid and total number of phosphorus atoms in the nucleic acid is from about 1:1 to about 20:1, from about 1:1 to about 20:1, from about 2:1 to about 10:1, or from about 4:1 to about 8:1.

In one embodiment, the delivery or expression level of the payload in the spleen of a subject is higher than the delivery or expression level of the payload in the liver of the subject, when the lipid nanoparticle comprising the payload is systematically administered to the subject.

In one embodiment, the delivery or expression level of the payload in the spleen of the subject is at least 2 times, at least 5 times, at least 10 times, at least 20 times, at least 40 times, at least 60 times, or at least 100 times higher than the delivery or expression level of the payload in the liver of the subject.

In one embodiment, provided herein is a lipid nanoparticle composition comprising the lipid nanoparticle described herein.

In one embodiment, provided herein is a pharmaceutical composition comprising the lipid nanoparticle or the lipid nanoparticle composition described herein.

In one embodiment, provided herein is a method for the treatment or prevention of splenic disease or spleen-related disease in subjects with splenic disease or spleen-related disease, comprising administration of the effective amounts of lipid nanoparticles described herein, lipid nanoparticle compositions described herein, or pharmaceutical compositions described herein to said subjects.

In one embodiment, provided herein is use of lipid nanoparticles described herein, lipid nanoparticle compositions described herein, or pharmaceutical compositions described herein in manufacture of a medicament for the treatment or prevention of splenic disease or spleen-related disease.

In one embodiment, provided herein is lipid nanoparticles described herein, lipid nanoparticle compositions described herein, or pharmaceutical compositions described herein for use in the treatment or prevention of splenic disease or spleen-related disease.

In a preferred embodiment, the above-mentioned splenic disease or spleen-related disease includes hematologic disorders, infectious diseases, immune diseases, or cancer.

In a preferred embodiment, the above-mentioned splenic disease or spleen-related disease includes hemolytic anemia; thrombocytopenic purpura; chronic leukemia; lymphoma such as Hodgkin lymphoma or non-Hodgkin lymphoma; myelodysplastic syndrome; myelofibrosis; splenomegaly; lipid metabolism disorders such as Gaucher disease and sphingomyelinosis; splenomegaly and hypersplenism associated with sepsis, typhoid, infectious mononucleosis or subacute bacterial endocarditis; splenomegaly and hypersplenism associated with malaria, tuberculosis, Kala-azar or other related diseases; congestive splenomegaly and hypersplenism due to cirrhosis of the liver and portal hypertension; splenic cysts such as parasitic splenic cysts or pseudosplenic cysts; splenic abscesses; primary splenic tumors; splenic metastases; splenic artery aneurysms; splenic infarction; splenic purpura; splenic rupture; and the like.

In one embodiment, provided herein is process of preparing the lipid nanoparticle described herein or the lipid nanoparticle compositions described herein, comprising the steps of:
(i) dissolving in a first solution a mixture comprising the lipids, wherein the lipid solution is formed in an organic solvent;
(ii) dissolving in a second solution a payload to form a payload solution; and
(iii) mixing the lipid solution and the payload solution to form the lipid nanoparticles.

In one embodiment, the mixing is performed using a microfluidizer system.

In another aspect, provided herein is a steroid compound of formula (II-A): 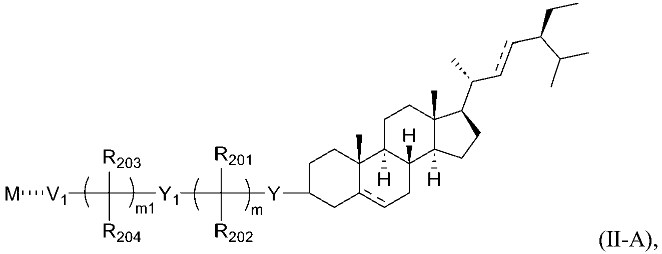 or a stereoisomer, a mixture of stereoisomers, or a pharmaceutically acceptable salt thereof, wherein:
each - - - is independently a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, or is selected from -C(O)O-, -O-, -NH-, -SC(O)O-, -OC(O)NR₂ₐ-, -NR₂ₐC(O)NR₂ₐ-, - OC(O)S-, -OC(O)O-, -NR₂ₐC(O)O-, -OC(O)-, -SC(O)-, -C(O)S-, -NR₂ₐ-, -C(O)NR₂ₐ-, -NR₂ₐC(O)-, - NR₂ₐC(O)S-, -SC(O)NR₂ₐ-, -C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 3 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent, or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted; and provided that the compound is not: 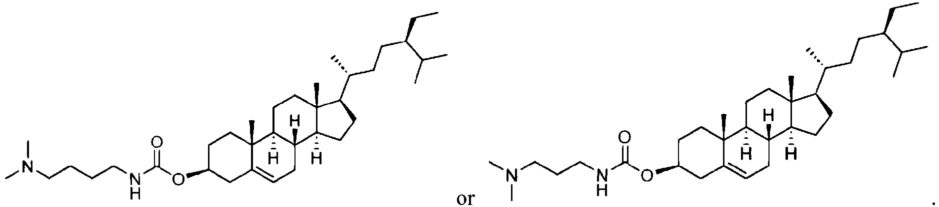
In one embodiment, provided herein is a steroid compound of formula (II-B): 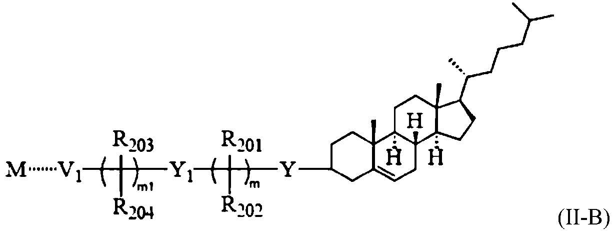 or a stereoisomer, a mixture of stereoisomers, or a pharmaceutically acceptable salt thereof, wherein:
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)- , -SC(O)- , -C(O)S-, -NR₂ₐ-, -C(O)NR₂ₐ-, -NR₂ₐC(O)- , -NR₂ₐC(O)S-, -SC(O)NR₂ₐ-, - C(O)- , -OC(S)- , -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)- , -SC(O)- , -C(O)S-, -NR₂ₐ-, -C(O)NR₂ₐ-, -NR₂ₐC(O)- , -NR₂ₐC(O)S-, -SC(O)NR₂ₐ-, - C(O)- , -OC(S)- , -C(S)O-, -OC(S)NR₂ₐ-, -NR₂ₐC(S)O-, or -S(O)₀₋₂-, and wherein the attachment to the left is to the direction of V₁;
each instance of R₂ₐ is H, C₁₋₁₀ alkyl, C₃₋₁₄ cycloalkyl, or 3 to 14-membered heterocyclyl, and wherein the alkyl, cycloalkyl, and heterocyclyl are independently optionally substituted by C₁₋₁₀ alkyl, L_{2b}-OR_{2b}, L_{2b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 5 to 12;
ml is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent, or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} are independently H or C₁₋₁₀ alkyl.

In one embodiment, provided herein is a steroid compound of formula (I-P): 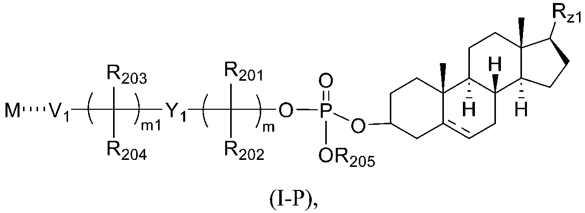 or a stereoisomer, a mixture of stereoisomers, or a pharmaceutically acceptable salt thereof, wherein:
- - - is a single bond or double bond;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, -S(O)₀₋₂-, or -OPO₃-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{2b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
R₂₀₅ is absent, a cationc, H, C₁₋₆ alkyl, C₁₋₆ haloalkyl, C₂₋₆ alkenyl, or C₂₋₆ alkynyl, and wherein alkyl, haloalkyl, alkenyl, and alkynyl, is optionally substituted;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

In one embodiment, provided herein is a steroid compound of formula (II-C1): 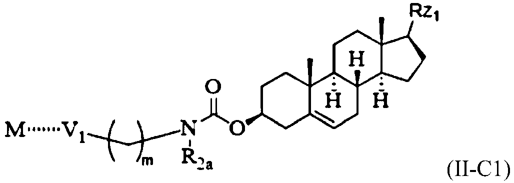 or a stereoisomer, a mixture of stereoisomers, or a pharmaceutically acceptable salt thereof, wherein:
R_{z1} is 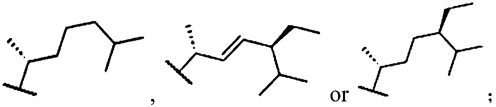
R₂ₐ is H, optionally substituted C₁₋₈ alkyl, -L_{2b}-OR_{2b}, L_{2b}-OC(O)R_{2b}, or L_{2b}-C(O)OR_{2b}, preferably H, - CH₃, -CH₂C(O)OCH₃, or -CH₂C(O)OH;
V₁ is NR₂₁R₂₂ or N⁺R₂₁R₂₂R₂₃, wherein each instance of R₂₁, R₂₂, R₂₃ is independently selected from H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}, provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent, or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
m is an integer from 1 to 10, such as 1, 2, 3, 4, 5, 6, 7, 8, 9, or 10.

In one embodiment, provided herein is a steroid compound of formula (II-C2): 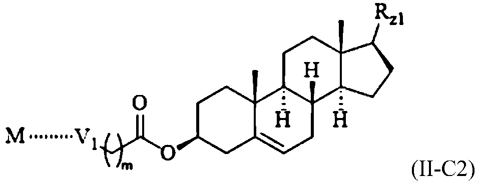 or a stereoisomer, a mixture of stereoisomers, or a pharmaceutically acceptable salt thereof, wherein:
R_{z1} is 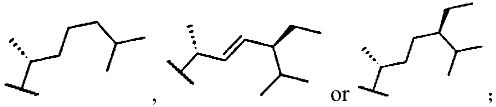
V₁ is NR₂₁R₂₂ or N⁺R₂₁R₂₂R₂₃, wherein each instance of R₂₁, R₂₂, R₂₃ is independently selected from H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}, provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 10, such as 1, 2, 3, 4, 5, 6, 7, 8, 9, or 10.

In one embodiment, the steroid compound is: 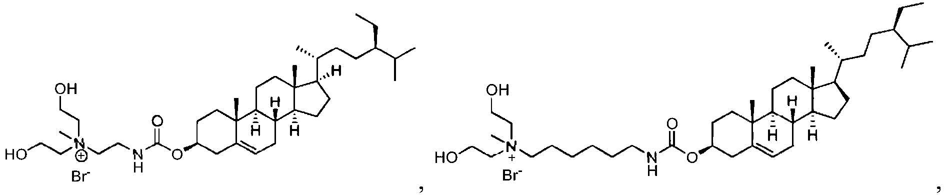 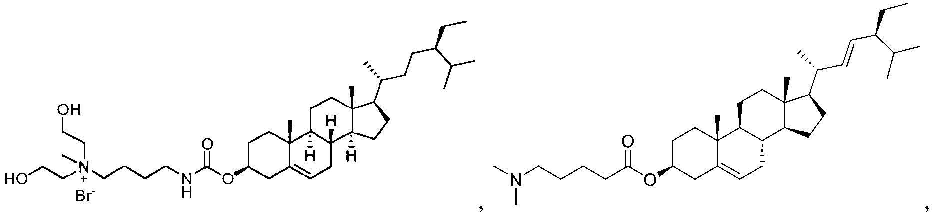 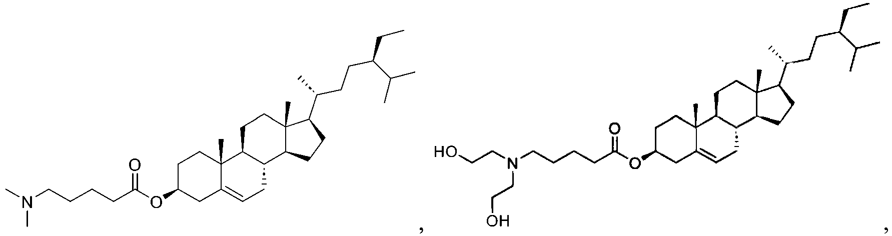 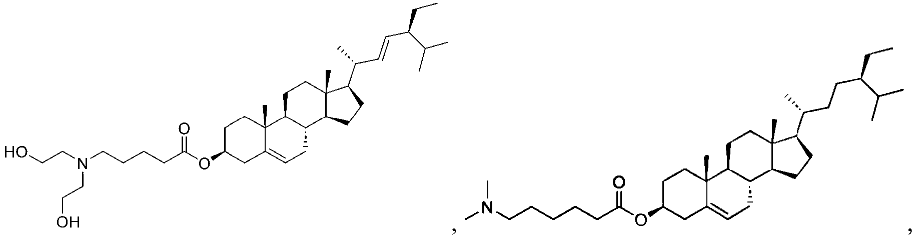 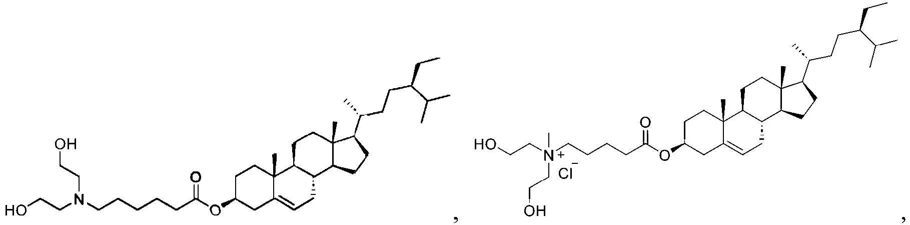 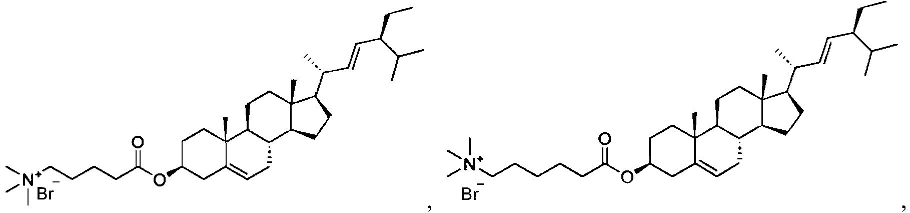 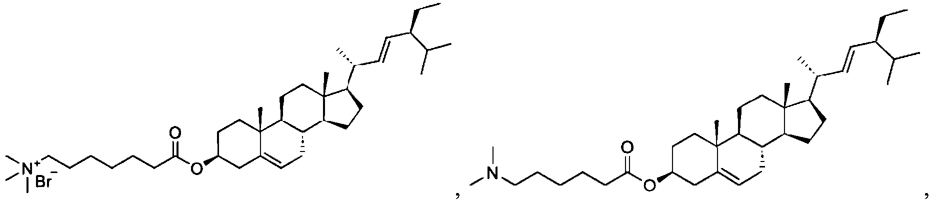 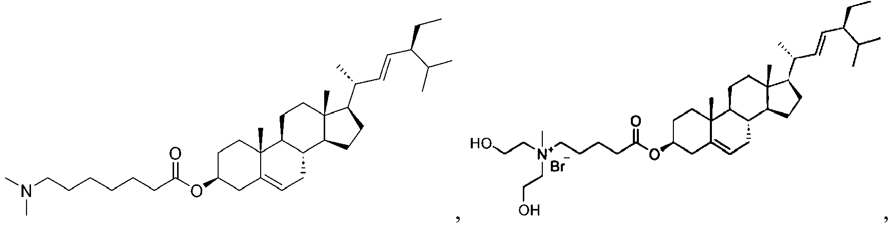 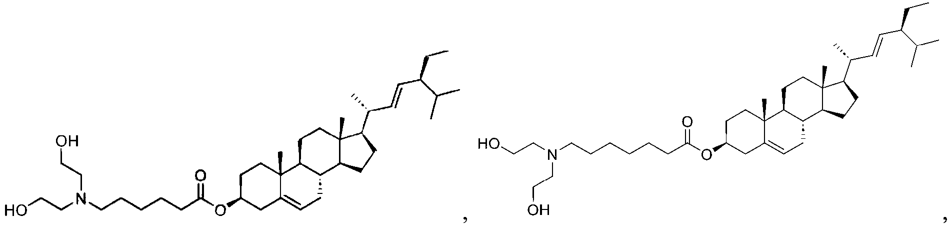 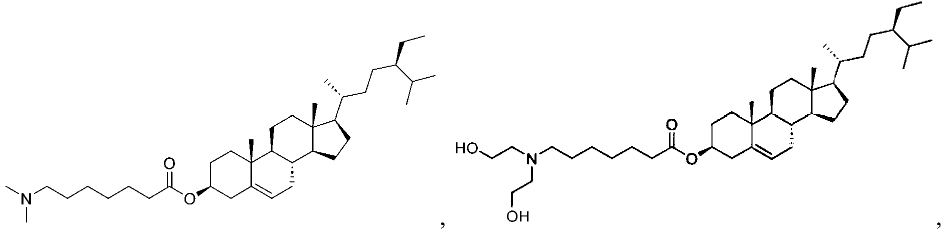 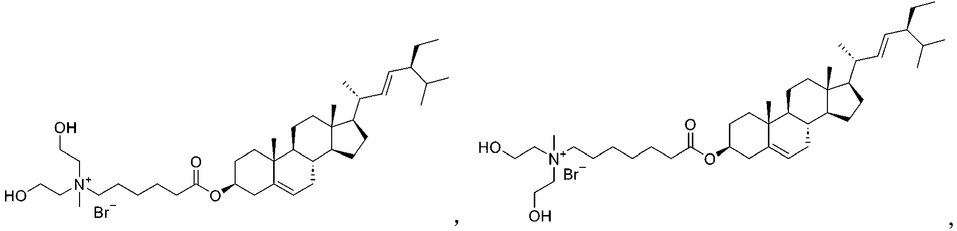 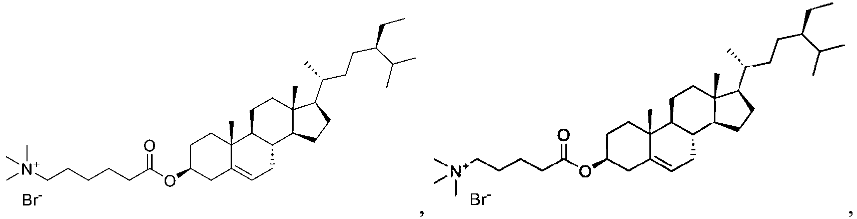 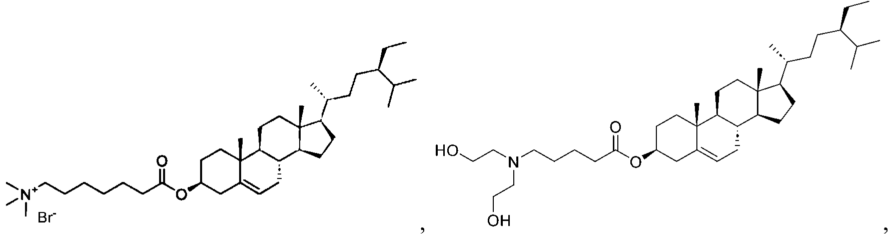 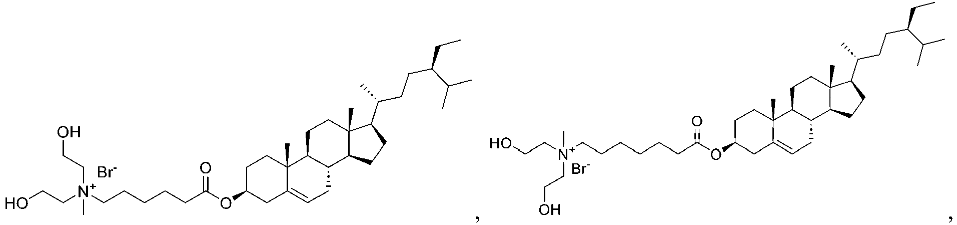 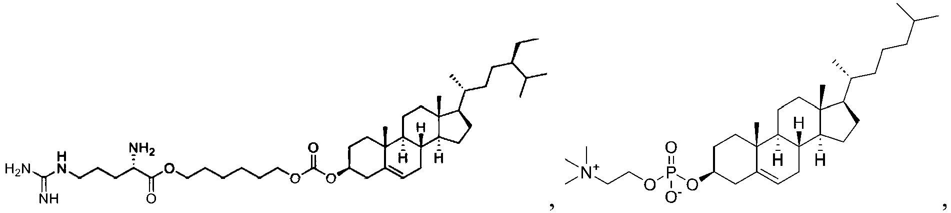 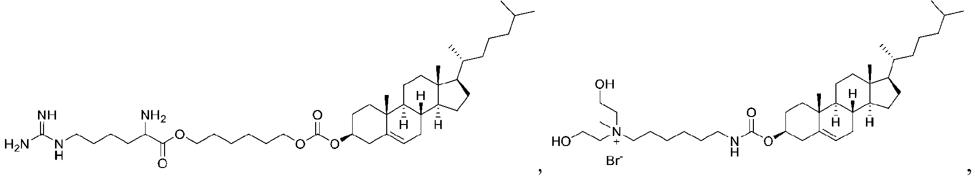 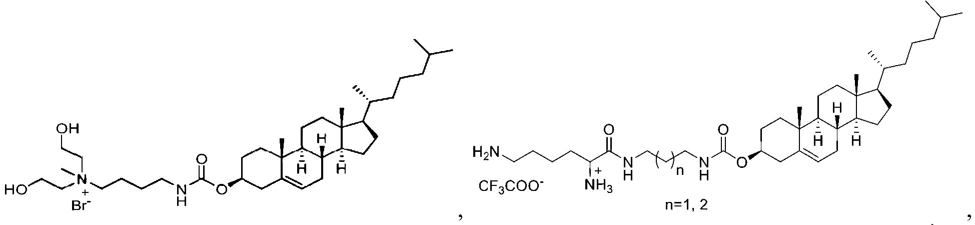 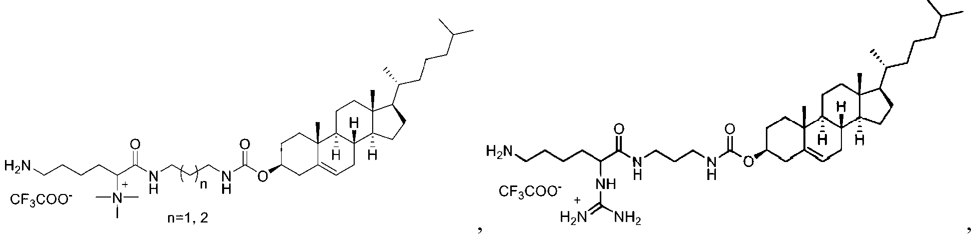 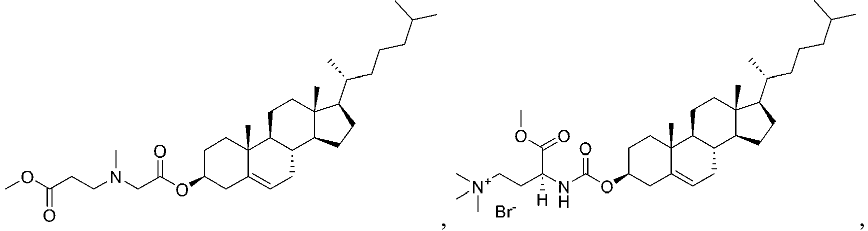 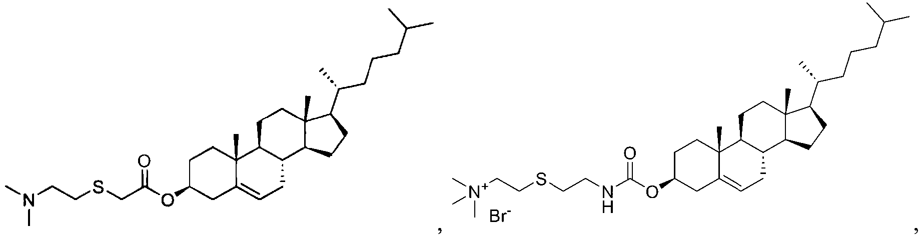 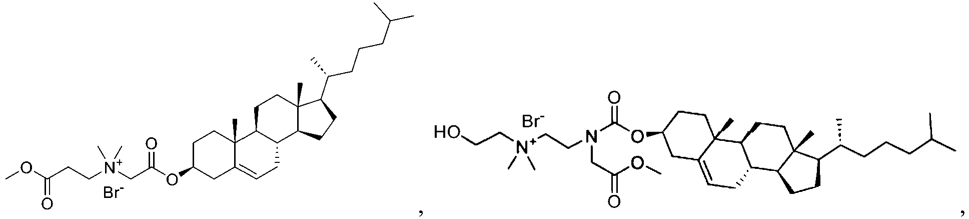 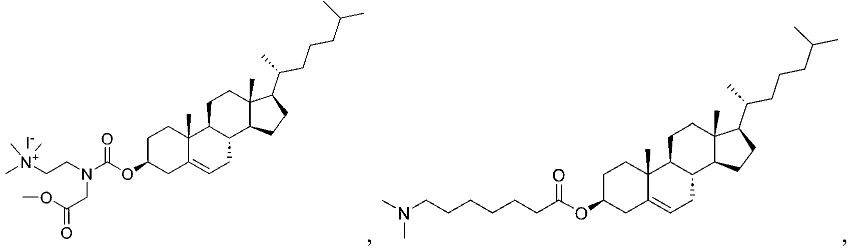 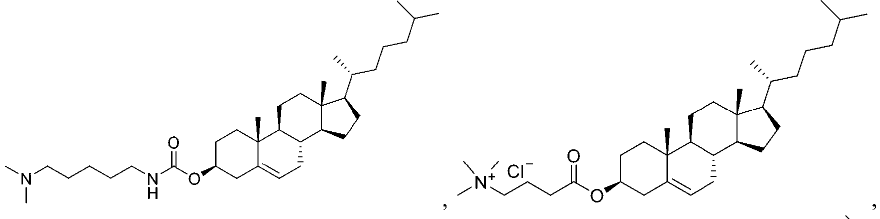 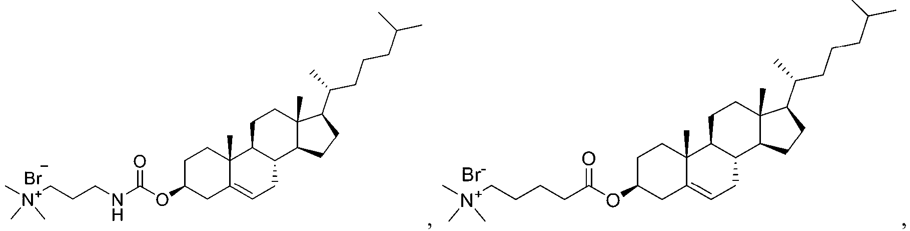 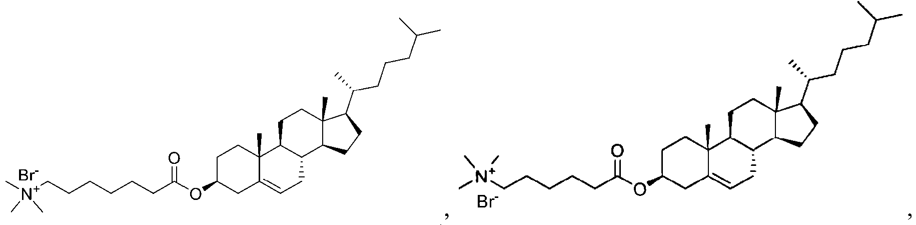 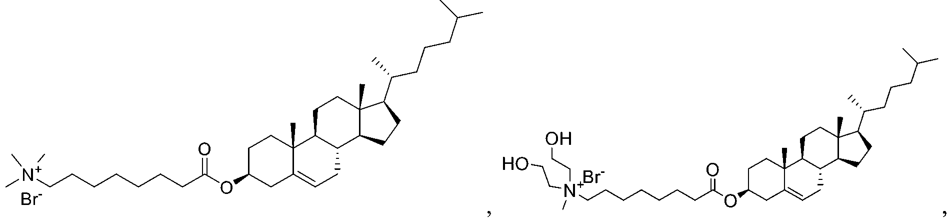 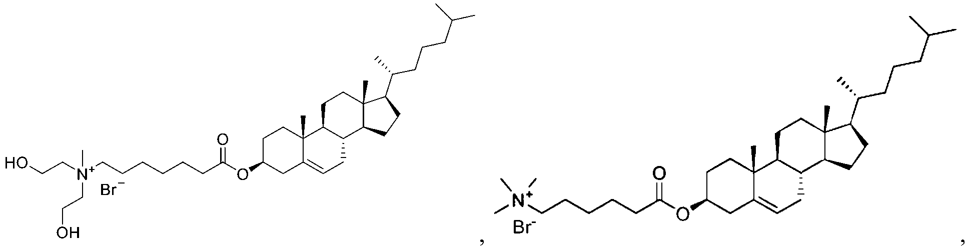 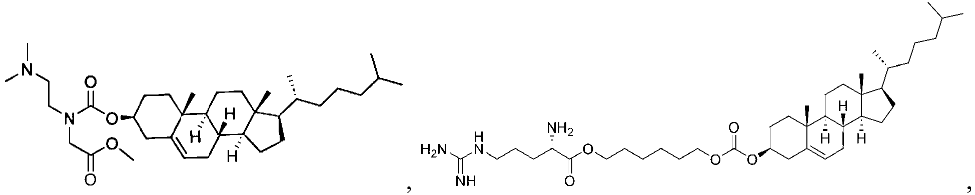 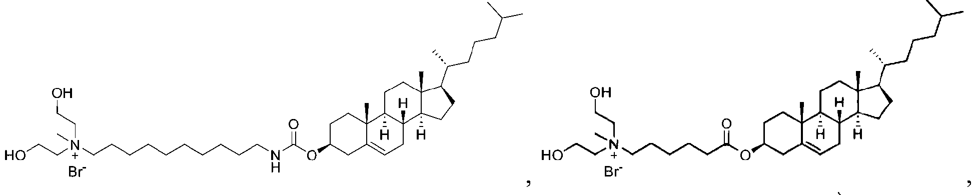 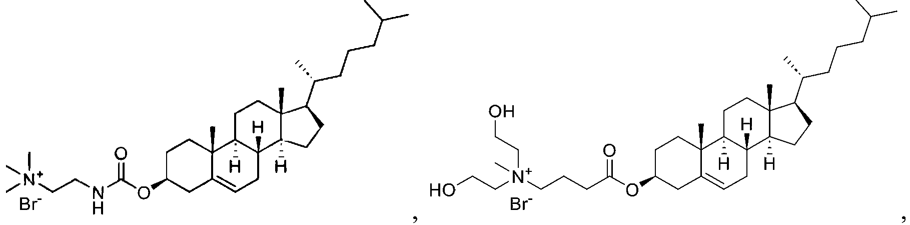 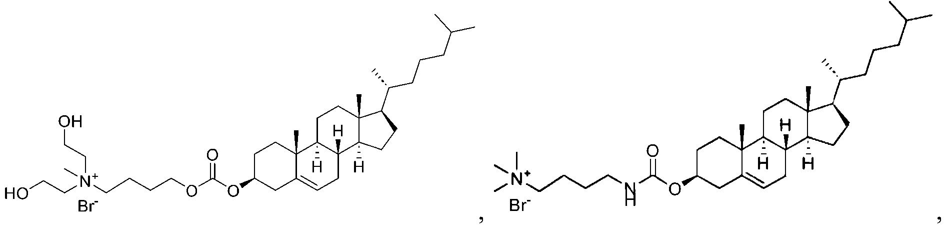 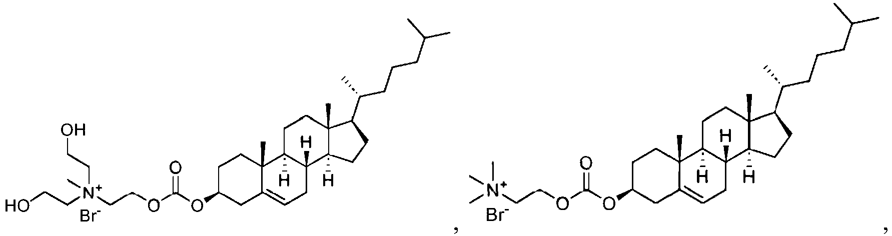 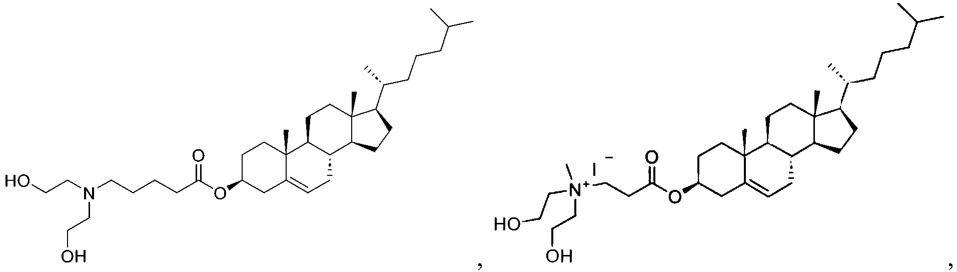 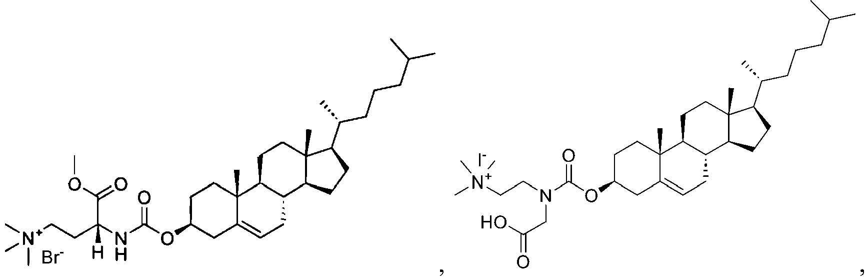 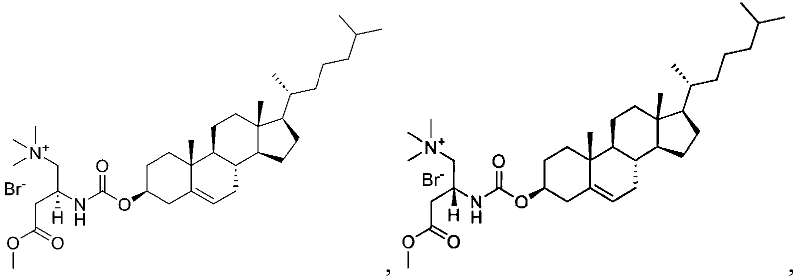 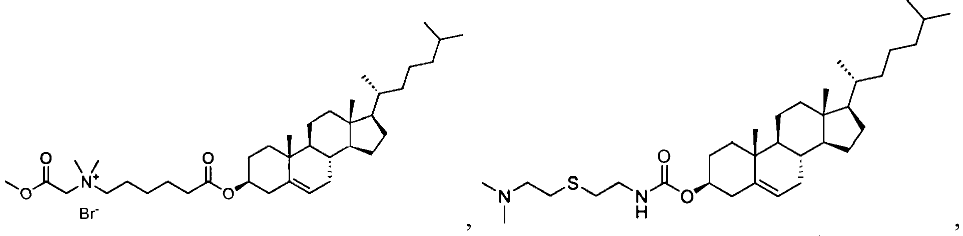 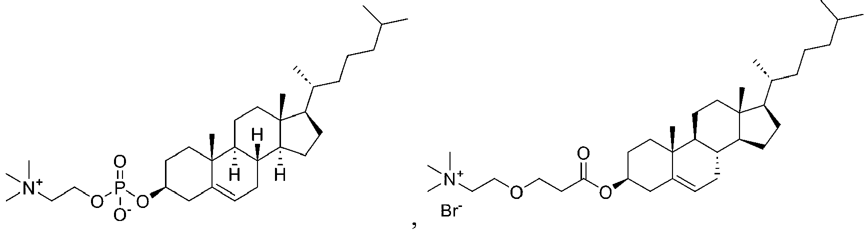 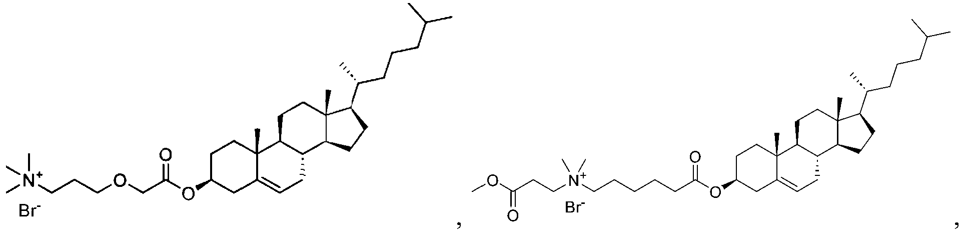 or 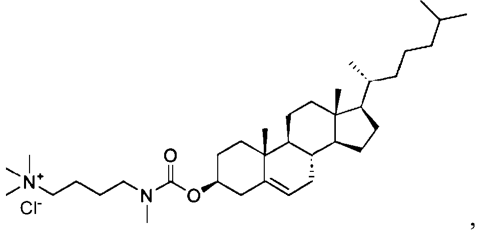 or a stereoisomer, a mixture of stereoisomers, or a pharmaceutically acceptable salt thereof.

In one embodiment, the steroid compound is: 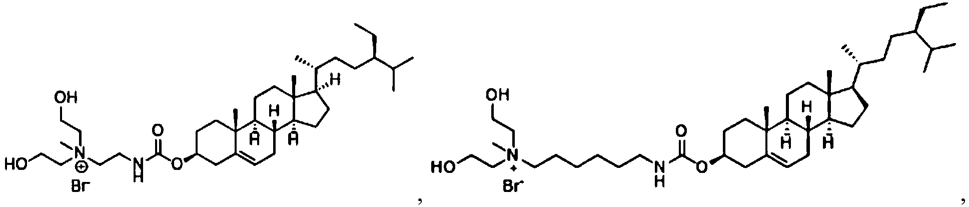 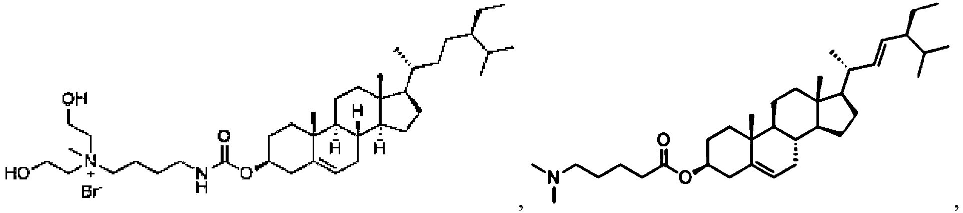 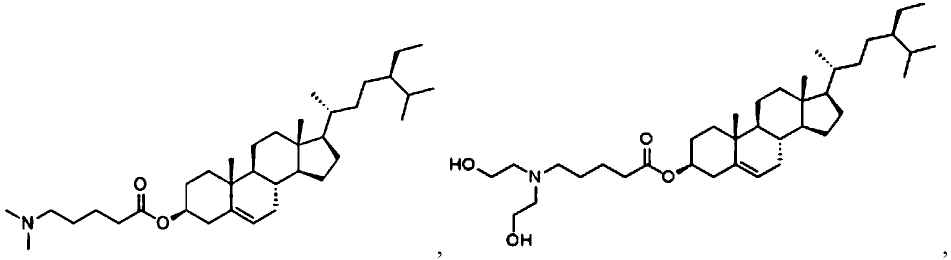 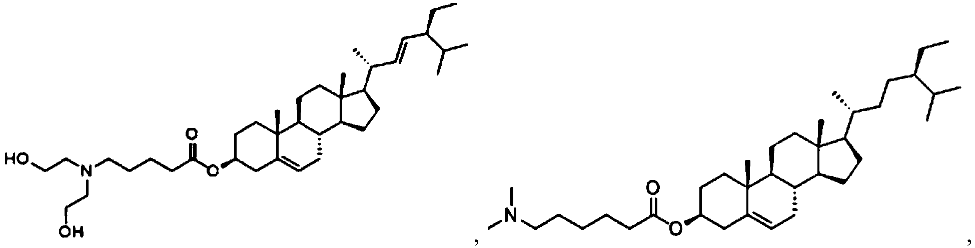 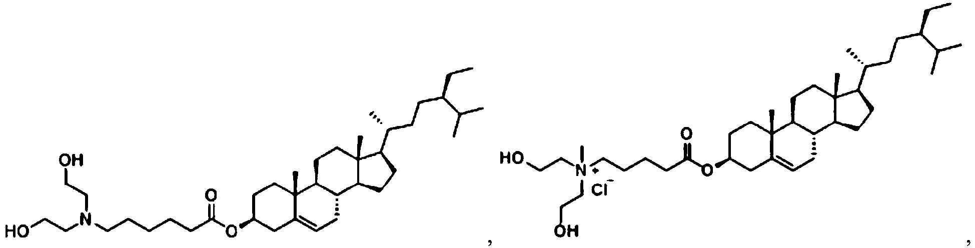 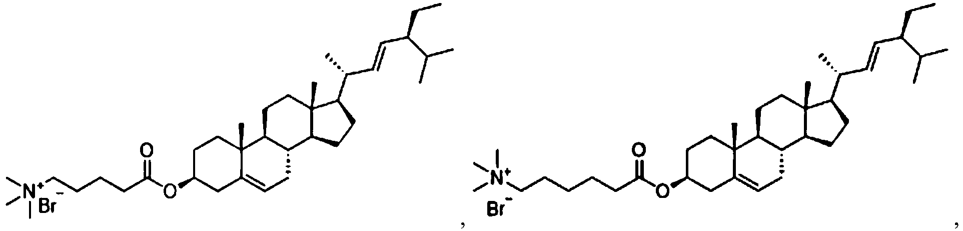 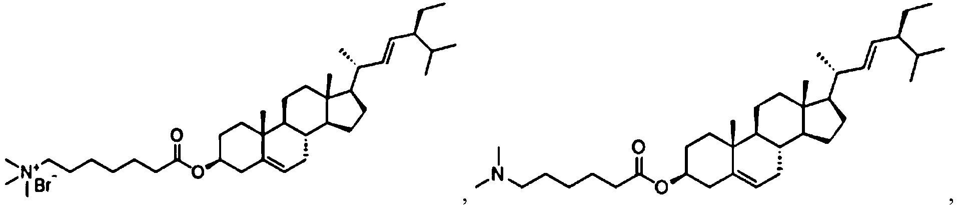 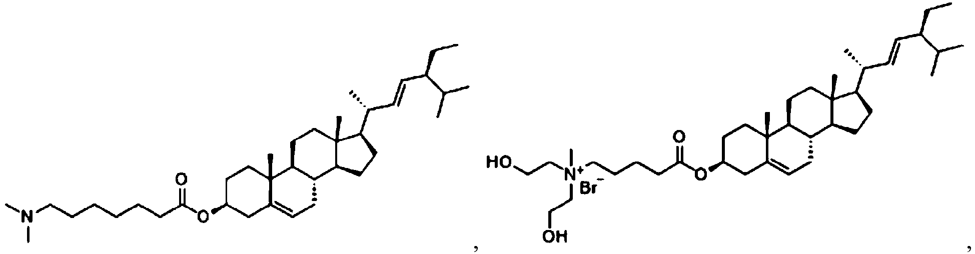 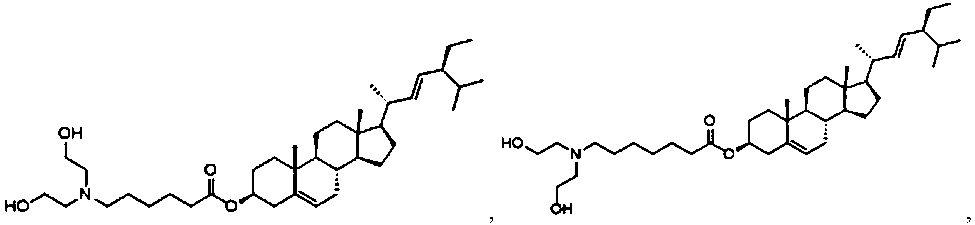 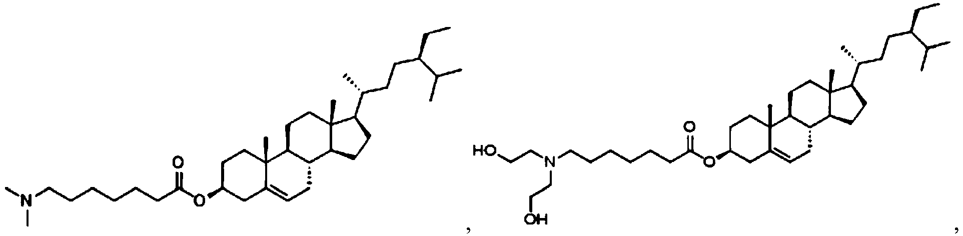 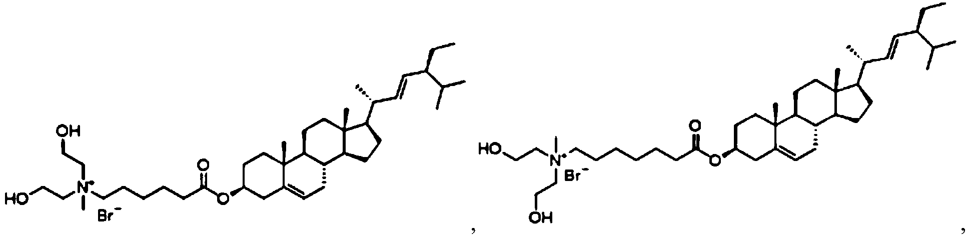 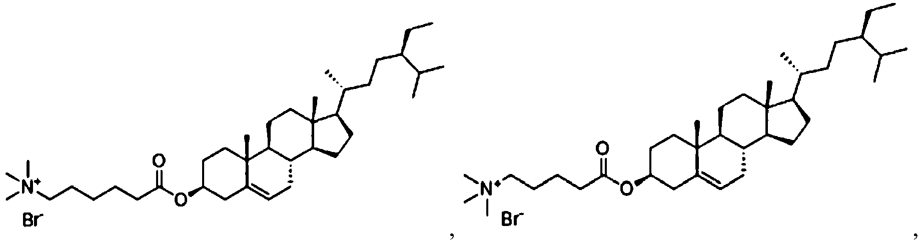 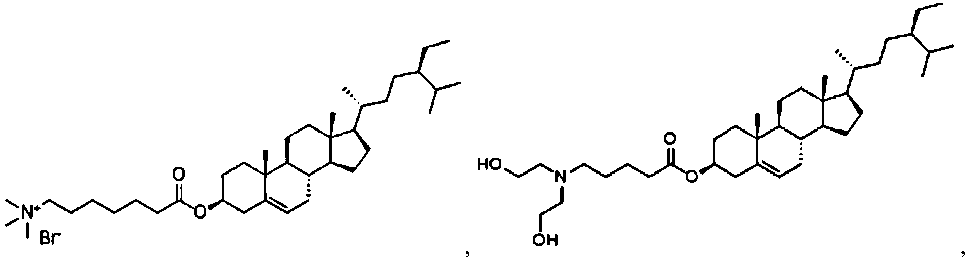 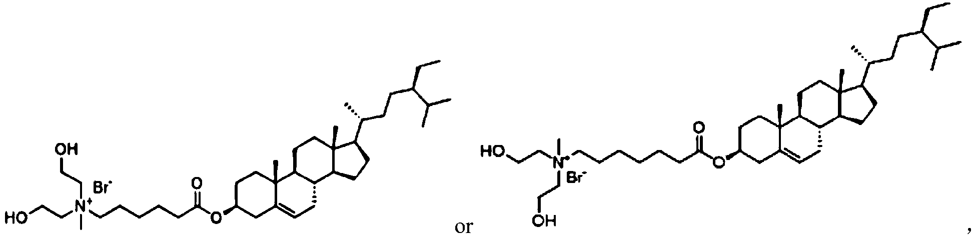 or a stereoisomer, a mixture of stereoisomers, or a pharmaceutically acceptable salt thereof.

In another aspect, provided herein the steroid compound selected form: 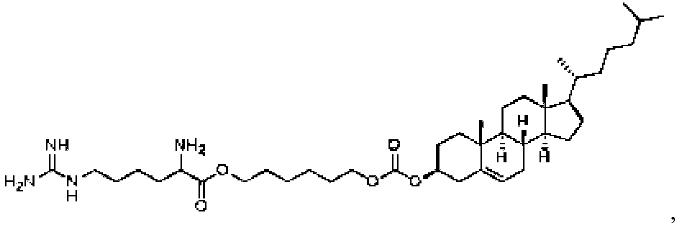 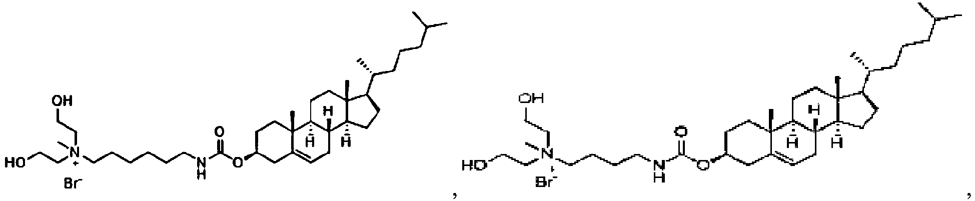 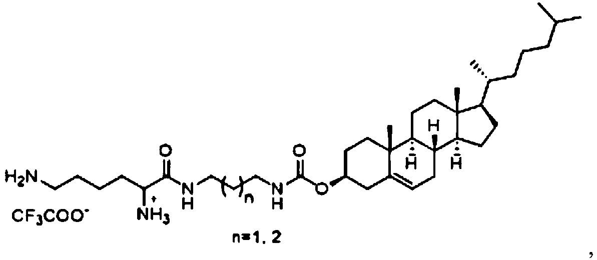 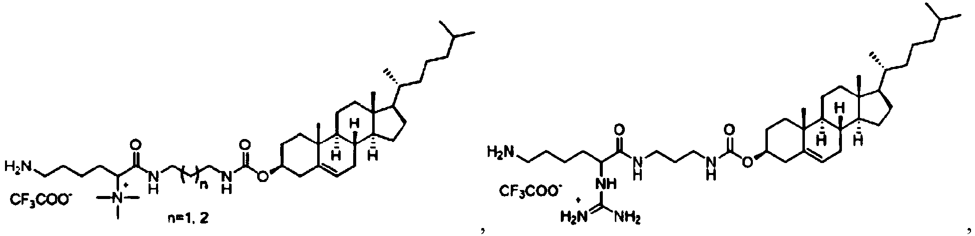 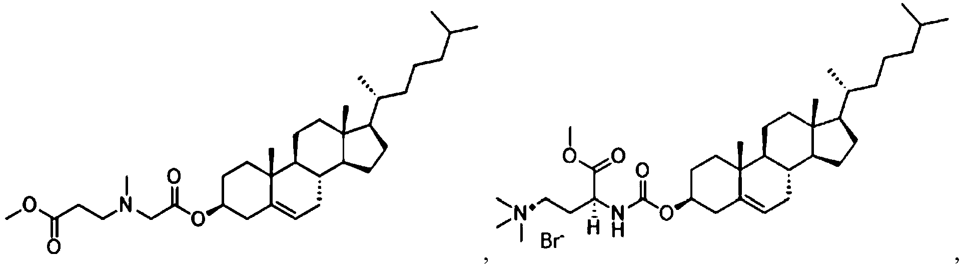 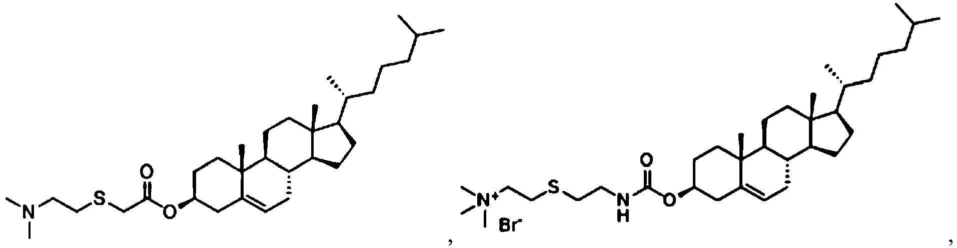 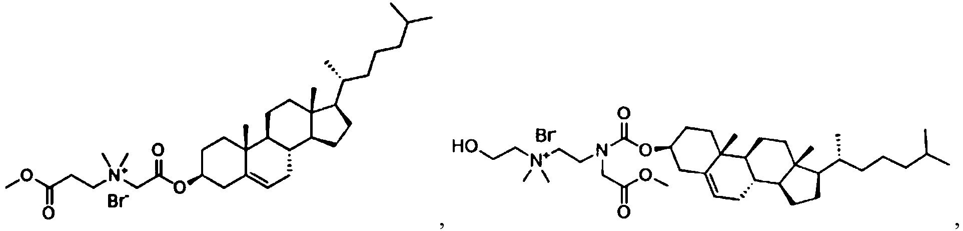 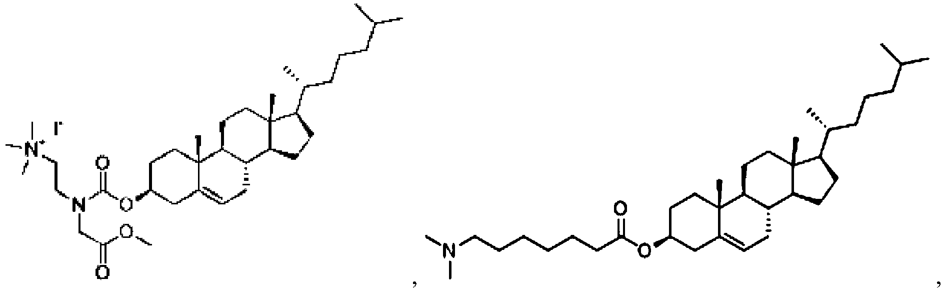 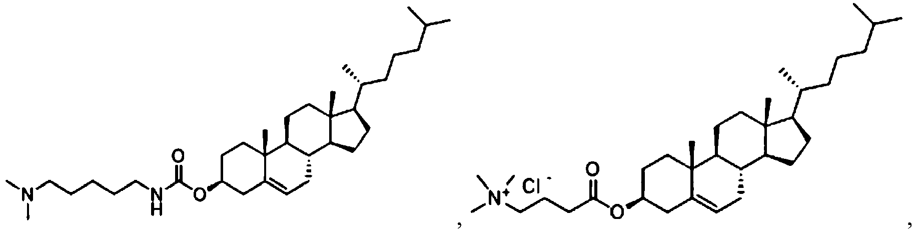 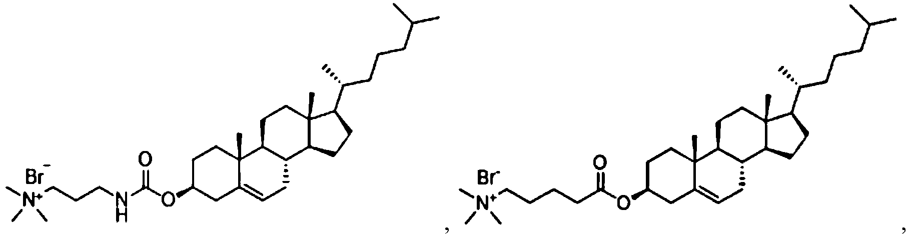 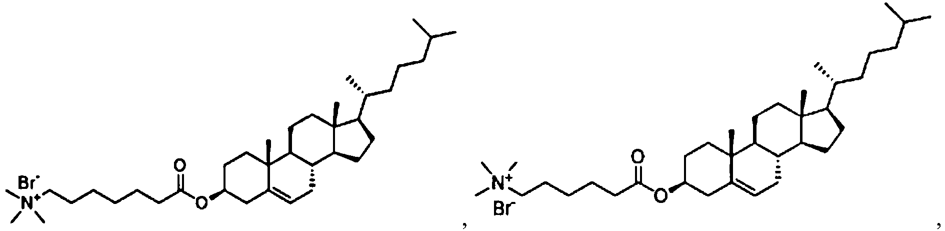 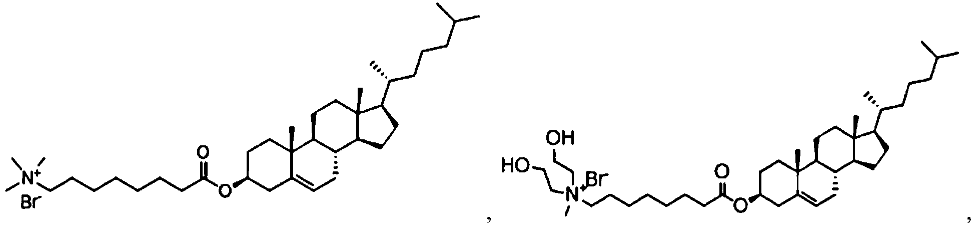 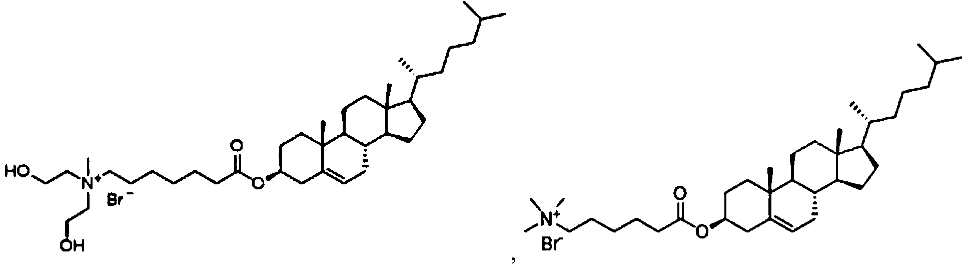 or 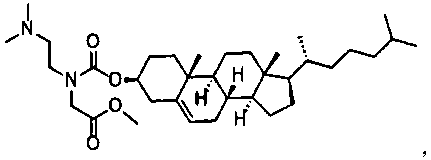 or a stereoisomer, a mixture of stereoisomers, or a pharmaceutically acceptable salt thereof.

In one embodiment, provided herein is a lipid nanoparticle comprising the steroid compound described herein.

### 4. BRIEF DESCRIPTION OF THE DRAWINGS

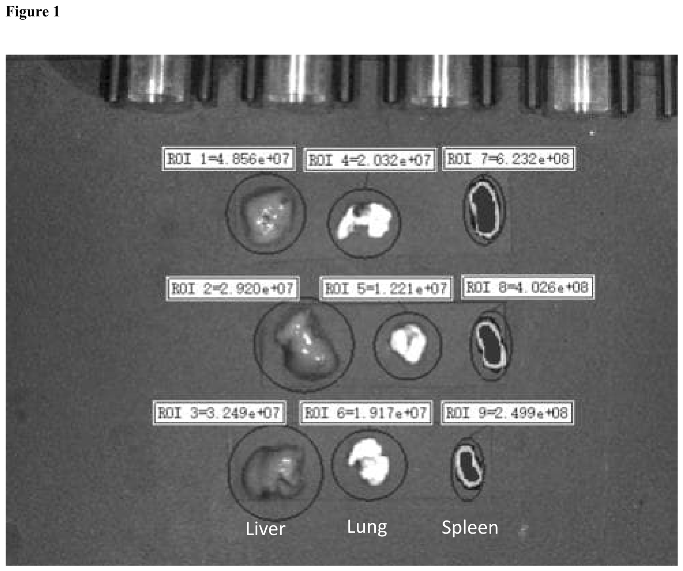

Figure 1 depicts the expression level of luciferase in the liver, lung and spleen of mice after administration of LNP5 of Example 3. The three lines in Figure 1 represent the results of three mice.

### 5. DETAILED DESCRIPTION

Provided herein are lipid nanoparticle compositions, preparations, and uses therefore.

In one aspect, provided herein is a lipid nanoparticle (LNP, such as an LNP described in Section 0). In one embodiment, the LNP comprises a steroid compound having a cationic group (cationic steroid compound, Section 0). In one embodiment, the LNP comprises an ionizable lipid (Section 0). In one embodiment, the LNP further comprises a phospholipid lipid (Section 0). In one embodiment, the LNP does not comprise phospholipid lipid. In one embodiment, the LNP further comprises a structural lipid (Section 0) In one embodiment, the LNP further comprises a polymer conjugated lipid, e.g. pegylated lipid (Section 0) In one embodiment, the LNP comprises a payload (Section 0).

In one aspect, provided herein is a lipid nanoparticle composition (Section 0).

In one embodiment, the LNP described herein preferably delivers a payload to a non-hepatic organ (e.g. spleen) when administered into a subject. In one embodiment, provided herein are LNP compositions for use in treating a splenic disease or a spleen-associated disorder.

In another aspect, provided herein is a pharmaceutical composition comprising the LNP provided herein (Section 0).

Also provided herein is a process of making the LNP compositions described herein (see Section 0). Also provided herein is a method of treating diseases using the LNP compositions described herein (Section 0). In one embodiment, provided herein is a method of treating a splenic disease or a spleen-associated disorder.

One aspect of the present application relates to the discovery that the inclusion of steroid compound (e.g., a cationic steroid compound as provided in Section 0) in an LNP significantly increases the delivery efficiency of the LNP and its payload to spleen. More specifically, the cationic steroid compound leads to higher delivery efficiency of the LNP and its payload to spleen in comparison to other cationic lipids (e.g., DOTAP). The cationic steroid compound can be effective in a relatively low amount, which may lead to reduced toxicity and side effects.

### 5.1 Definitions

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower) unless otherwise stated or otherwise evident from the context.

It should be noted that if there is a discrepancy between a depicted structure and a name for that structure, the depicted structure is to be accorded more weight.

When a range of values is listed, it is intended to encompass each value and sub-range within the range. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

As used herein and unless specified otherwise, "alkyl" refers to a radical of a linear or branched, saturated hydrocarbon group by removal of one hydrogen atom. For example, "C₁₋₂₄ alky" refers to a radical of a linear or branched, saturated hydrocarbon group having 1 to 24 carbon atoms. "C₁₋₂₀ alky" refers to a radical of a linear or branched, saturated hydrocarbon group having 1 to 20 carbon atoms. "C₆-₂₄ alkyl" refers to a radical of a linear or branched, saturated hydrocarbon group having 6 to 24 carbon atoms. "C₆-₂₀ alkyl" refers to a radical of a linear or branched, saturated hydrocarbon group having 6 to 20 carbon atoms. In some embodiments, the alkyl is a C₁₋₂₄ alkyl, C₄₋₂₀ alkyl, C₆₋₂₀ alkyl, C₈₋₂₀ alkyl, C₁₀₋₂₀ alkyl, C₆₋₁₈ alkyl, C₆₋₁₆ alkyl, C₆₋₁₄ alkyl, C₆₋₁₂ alkyl, C₈₋₁₀ alkyl, C₂₋₈ alkyl, C₇₋₉ alkyl, C₄₋₆ alkyl, C₁₋₁₆ alkyl, C₁₋₁₄ alkyl, C₂₋₁₄ alkyl, C₁₋₁₃ alkyl, C₁₋₁₂ alkyl, C₁₋₁₀ alkyl, C₁₋₈ alkyl, C₁₋₇ alkyl, C₂₋₇ alkyl, C₁₋₆ alkyl, C₁₋₅ alkyl, C₅ alkyl, C₁₋₄ alkyl, C₂₋₄ alkyl, C₁₋₃ alkyl, C₂₋₃ alkyl, C₁₋₂ alkyl, or methyl. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "alkyl" also includes heteroalkyl, wherein one or more (e.g., 1, 2, 3 or 4) carbon atoms are substituted with heteroatoms (e.g., oxygen, sulfur, nitrogen, boron, silicon, phosphorus). An alkyl can be substituted or unsubstituted. An alkyl can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃) or i-Bu (-CH₂CH(CH₃)₂).

As used herein and unless specified otherwise, "alkenyl" refers to a radical of a linear or branched, unsaturated hydrocarbon group having at least one carbon-carbon double bond. For example, "C₆₋₂₄ alkenyl" refers to a radical of a linear or branched hydrocarbon group having 6 to 24 carbon atoms and at least one carbon-carbon double bond. "C₄₋₂₈ alkenyl" refers to a radical of a linear or branched hydrocarbon group having 4 to 28 carbon atoms and at least one carbon-carbon double bond. "C₄₋₂₀ alkenyl" refers to a radical of a linear or branched hydrocarbon group having 4 to 20 carbon atoms and at least one carbon-carbon double bond. "C₂₋₁₄ alkenyl" refers to a radical of a linear or branched hydrocarbon group having 2 to 14 carbon atoms and at least one carbon-carbon double bond. In some embodiments, the alkenyl is C₄₋₂₄ alkenyl, C₆₋₂₄ alkenyl, C₈₋₂₄ alkenyl, C₁₀₋₂₄ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₂₋₁₃ alkenyl, C₂₋₁₀ alkenyl, C₂₋₆ alkenyl, or C₂₋₄ alkenyl. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). An alkenyl can be substituted or unsubstituted. An alkenyl can be optionally substituted with one or more substituents, for example, with 1 to 5 substituents, 1 to 3 substituents or 1 substituent.

As used herein and unless specified otherwise, "alkynyl" refers to a radical of a linear or branched hydrocarbon group having at least one carbon-carbon triple bond. For example, "C₄₋₂₀ alkynyl" refers to a radical of a linear or branched hydrocarbon group having 2 to 20 carbon atoms and at least one carbon-carbon triple bond. In some embodiments, the alkynyl is C₄₋₂₄ alkynyl, C₆₋₂₀ alkynyl, C₈₋₁₈ alkynyl, C₈ alkynyl, C₉ alkynyl, C₁₀ alkynyl, C₁₁ alkynyl, C₁₂ alkynyl, C₁₃ alkynyl, C₁₄ alkynyl, C₁₅ alkynyl, C₁₆ alkynyl, C₁₇ alkynyl, C₂₋₁₃ alkynyl, C₂₋₁₀ alkynyl, C₂₋₆ alkynyl, or C₂₋₄ alkynyl. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). An alkynyl can be substituted or unsubstituted. The alkynyl groups can be substituted with one or more substituents, for example, with 1 to 5 substituents, 1 to 3 substituents or 1 substituent.

As used herein and unless specified otherwise, "alkylene" refers to a divalent group formed by removing another hydrogen atom from an alkyl. For example, "C₁₋₂₄ alkylene" refers to a divalent group formed by removing another hydrogen from a C₁₋₂₄ alkyl. In some embodiments, the alkylene is a C₁₋₂₄ alkylene, C₄₋₂₄ alkylene, C₁₋₂₀ alkylene, C₈₋₂₀ alkylene, C₈ alkylene, C₉ alkylene, C₁₀ alkylene, C₁₁ alkylene, C₁₂ alkylene, C₁₃ alkylene, C₁₃ alkylene, C₁₄ alkylene, C₁₅ alkylene, C₁₆ alkylene, C₁₇ alkylene, C₁₈ alkylene, C₁₋₂₀ alkylene, C₁₋₁₄ alkylene, C₂₋₁₄ alkylene, C₁₋₁₃ alkylene, C₁₋₁₂ alkylene, C₁₋₁₀ alkylene, C₁₋₈ alkylene, C₇₋₉ alkylene, C₄₋₆ alkylene, C₁₋₇ alkylene, C₂₋₇ alkylene, C₁₋₆ alkylene, C₁₋₅ alkylene, C₅ alkylene, C₁₋₄ alkylene, C₂₋₄ alkylene, C₁₋₃ alkylene, C₂₋₃ alkylene, C₁₋₂ alkylene, or methylene. An alkylene can be substituted or unsubstituted. An unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, -CH₂CH₂C(CH₃)₂-), etc.

As used herein and unless specified otherwise, "alkenylene" refers to a divalent group formed by removing another hydrogen from alkenyl. For example, "C₂₋₂₄ alkenylene" refers to a C₂₋₂₄ alkenyl group wherein another hydrogen is removed to provide a divalent radical of alkenylene. In some embodiments, the alkenylene is C₄₋₂₄ alkenylene, C₄₋₂₀ alkenylene, C₈₋₂₀ alkenylene, C₈₋₁₈ alkenylene, C₈₋₁₆ alkenylene, C₄₋₁₄ alkenylene, C₂₋₁₃ alkenylene, C₂₋₁₀ alkenylene, C₂₋₆ alkenylene, or C₂₋₄ alkenylene. An alkenylene can be substituted or unsubstituted. Exemplary unsubstituted alkenylene groups include, but are not limited to, ethenylene (-CH=CH-) and propenylene (e.g., -CH=CHCH₂-, -CH₂-CH=CH-). Exemplary substituted alkenylene groups, e.g., substituted with one or more alkyl (methyl) groups, include but are not limited to, substituted ethenylene (-C(CH₃)=CH-, -CH=C(CH₃)-), substituted propenylene (e.g., -C(CH₃)=CHCH₂-, - CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-), and the like.

As used herein and unless specified otherwise, "alkynylene" refers to a divalent group formed by removing another hydrogen from alkynyl. For example, "C₂₋₂₄ alkynylene" refers to a C₂₋₂₄ alkynyl group wherein another hydrogen is removed to provide a divalent radical of alkynylene. "C₂₋₁₃ alkynylene" refers to a C₂₋₁₃ alkynyl group wherein another hydrogen is removed to provide a divalent radical of alkynylene. "C₄-₁₄ alkynylene" refers to a C₄₋₁₄ alkynyl group wherein another hydrogen is removed to provide a divalent radical of alkynylene. In some embodiments, the alkynylene is C₂₋₂₄ alkynylene, C₂₋₂₀ alkynylene, C₄₋₁₆ alkynylene, C₈₋₁₆ alkynylene, C₉₋₁₆ alkynylene, C₁₀₋₁₆ alkynylene, C₄₋₁₂ alkynylene, C₂₋₁₀ alkynylene, C₂₋₆ alkynylene, or C₂₋₄ alkynylene. An alkynylene can be substituted or unsubstituted. Exemplary alkynylene groups include, but are not limited to, ethynylene (-C≡C-), substituted or unsubstituted propynylene (-C≡CCH₂-), and the like.

"C₀₋₆ alkylene" refers to the chemical bond and the "C₁₋₆ alkylene" described above, "C₀₋₄ alkylene" refers to the chemical bond and the"C₁₋₄ alkylene" described above.

The term "variable A and variable B have a total length of x carbon atoms" means that the total number of carbon atoms of the main chain in the group represented by variable A and the number of carbon atoms of the main chain in the group represented by variable B is x.

"Halo" or "halogen" refers to fluorine (F), chlorine (Cl), bromine (Br), or iodine (I).

"Haloalkyl" refers to an alkyl substituted by one or more halogens. For example, "C₁₋₁₀ haloalkyl" refers to the above "C₁₋₁₀ alkyl", which is substituted by one or more halogen. In some embodiments, a haloalkyl is a C₁₋₂₄ haloalkyl, C₁₋₂₀ haloalkyl, C₁₋₁₈ haloalkyl, C₁₋₁₆ haloalkyl, C₁₋₁₄ haloalkyl, C₁₋₁₂ haloalkyl, C₆₋₁₂ haloalkyl, C₁₋₆ haloalkyl, C₁₋₄ haloalkyl, or C₁₋₂ haloalkyl, C₁ haloalkyl, C₂ haloalkyl, C₃ haloalkyl, C₄ haloalkyl, C₅ haloalkyl, C₆ haloalkyl, C₇ haloalkyl, or C₈ haloalkyl. A haloalkyl may optionally further comprises one or more non-halogen substitutions. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl can be substituted at any available point of attachment, for example, with 1 to 5 substituents, 1 to 3 substituents or 1 substituent.

As used herein and unless specified otherwise, "cycloalkyl" refers to a non-aromatic monocyclic or polycyclic hydrocarbon radical consisting solely of carbon and hydrogen atoms. For example, "C₃₋₁₄ cycloalkyl" or "3- to 14-membered cycloalkyl" refers to a radical of a non-aromatic cyclic hydrocarbon group having from 3 to 14 ring carbon atoms and zero heteroatoms, optionally wherein 1, 2 or 3 double or triple bonds are contained. In some embodiments, the cycloalkyl is a 3- to 10-membered cycloalkyl (C₃₋₁₀ cycloalkyl), 5- to 10-membered cycloalkyl (C₅₋₁₀ cycloalkyl), 3- to 8-membered cycloalkyl (C₃₋₈ cycloalkyl), 3- to 7-membered cycloalkyl(C₃₋₇ cycloalkyl), 3- to 6-membered cycloalkyl (C₃₋₆ cycloalkyl), 5- to 7-membered cycloalkyl (C₅₋₇ cycloalkyl), 4- to 6-membered cycloalkyl (C₄₋₆ cycloalkyl), 5- to 6-membered cycloalkyl(C₅₋₆ cycloalkyl), 3-membered cycloalkyl (C₃ cycloalkyl), 4-membered cycloalkyl (C₄ cycloalkyl), 5-membered cycloalkyl (C₅ cycloalkyl), and 6-membered cycloalkyl (C₆ cycloalkyl), 7-membered cycloalkyl (C₇ cycloalkyl), or 8-membered cycloalkyl (C₈ cycloalkyl). The cycloalkyl also includes a ring system in which the cycloalkyl ring described above is fused with one or more aryl or heteroaryl groups, wherein the point of attachment is on the cycloalkyl ring, and in such case, the number of carbon atoms continues to represent the number of carbon atoms in the cycloalkyl system. The cycloalkyl further comprises the cycloalkyl described above, in which the substituents on any non-adjacent carbon atoms are connected to form a bridged ring, together forming a polycyclic alkane sharing two or more carbon atoms. The cycloalkyl further comprises the cycloalkyl described above, in which the substituents on the same carbon atom are connected to form a ring, together forming a polycyclic alkane sharing one carbon atom. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. A cycloalkyl can be substituted or unsubstituted. A cycloalkyl can be substituted with one or more substituents, for example, with 1 to 5 substituents, 1 to 3 substituents or 1 substituent.

As used herein and unless specified otherwise, "cycloalkylene" refers to a divalent radical formed by removing another hydrogen from a cycloalkyl group. For example, "C₃₋₁₄ cycloalkylene" refers to a divalent radical formed by removing another hydrogen of C₃₋₁₄ cycloalkyl group. In some embodiments, the cycloalkylene is C₃₋₁₂ cycloalkylene, C₃₋₈ cycloalkylene, C₃₋₆ cycloalkylene, C₃₋₅ cycloalkylene, C₃₋₄ cycloalkylene, C₃ cycloalkylene, C₄ cycloalkylene, C₅ cycloalkylene, or C₆ cycloalkylene. A cycloalkylene can be substituted or unsubstituted. Exemplary cycloalkylene include cyclopropylene, cyclobutylene, cyclopentylene, or cyclohexylene.

As used herein and unless specified otherwise, "heterocyclyl" refers to a saturated or unsaturated radical of non-aromatic ring system having ring carbon atoms and at least one ring heteroatoms. For example, "3- to 14-membered heterocyclyl" refers to a saturated or unsaturated radical of 3- to 14-membered non-aromatic ring system having at least one ring heteroatoms (e.g. 1-5 heteroatoms), which optionally contains 1, 2, or 3 double or triple bonds. In some embodiment, the ring heteroatom in heterocyclyl is selected from one or more of nitrogen, oxygen, sulfur, boron, phosphorus, or silicon. In the heterocyclyl containing one or more nitrogen atoms, the point of attachment to the rest of a molecule can be a carbon or nitrogen atom as long as the valence permits. In some embodiments, the heterocyclyl is a 3- to 10-membered heterocyclyl, 5- to 10-membered heterocyclyl, 5- to 10-membered nitrogen-containing heterocyclyl, 3- to 8-membered heterocyclyl, 3- to 8-membered nitrogen-containing heterocyclyl, 3- to 7-membered heterocyclyl, 5- to 7-membered heterocyclyl, 3- to 6-membered heterocyclyl, 4- to 6-membered heterocyclyl, 5- or 6-membered heterocyclyl, 4-membered heterocyclyl, 5-membered heterocyclyl, 6-membered heterocyclyl, or 7-membered heterocyclyl. The heterocyclyl also includes a ring system wherein the heterocyclyl described above is fused with one or more cycloalkyl groups, wherein the point of attachment is on the heterocyclyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. The heterocyclyl may further comprises the heterocyclyl described above, in which the substituents on any non-adjacent carbon or nitrogen atoms are connected to form a bridge ring, together forming a polycyclic heteroalkane sharing two or more carbon or nitrogen atoms. The heterocyclyl may further comprises the heterocyclyl described above, in which the substituents on the same carbon atom are connected to form a ring, together forming a polycyclic heteroalkane sharing one carbon atom. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, pyrazolidyl, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc. The heterocyclyl further includes the heterocyclyl described above sharing one or two atoms with a cycloalkyl, heterocyclyl, aryl or heteroaryl to form a bridged or spiro ring, as long as the valence permits, where the shared atom may be carbon or nitrogen atoms. The heterocyclyl further includes the heterocyclyl described above, which optionally can be substituted with one or more substituents, e.g., with 1 to 5 substituents, 1 to 3 substituents or 1 substituent.

As used herein and unless specified otherwise, "aryl" refers to a radical of monocyclic or polycyclic 4n+2 aromatic ring system containing only carbon and hydrogen atoms. For example, "C₆₋₁₀ aryl" refers to a radical of monocyclic or polycyclic (e.g., bicyclic) 4n+2 aromatic ring system having 6-10 ring carbon atoms and zero heteroatoms (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. An aryl can be substituted or unsubstituted. An aryl can be substituted with one or more substituents, for example, with 1 to 5 substituents, 1 to 3 substituents or 1 substituent.

As used herein and unless specified otherwise, "heteroaryl" refers to a radical of monocyclic or polycyclic 4n+2 aromatic ring system having at least one ring heteroatoms. For example, "5- to 14-membered heteroaryl" refers to a radical of 5- to 14-membered monocyclic or bicyclic 4n+2 aromatic ring system (e.g., having 6, 10 or 14 shared π electrons in a cyclic array) having ring carbon atoms and ring heteroatoms (e.g. 1-4 ring heteroatoms). In some embodiments, each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continue to represent the number of carbon atoms in the heteroaryl ring system. In some embodiments, the heteroaryl is 5- to 10-membered heteroaryl, 5- to 8-membered heteroaryl, 5- to 6-membered heteroaryl, 5-membered heteroaryl, or 6-membered heteroaryl. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazolyl), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl or pyridonyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. A heteroaryl can be substituted or unsubstituted. A heteroaryl can be substituted with one or more substituents, for example, with 1 to 5 substituents, 1 to 3 substituents or 1 substituent.

"Hydroxyalkyl" refers to an alkyl group that is substituted with one or more hydroxyl groups.

"Alkoxy" refers to an oxyether form of a linear or branched-chain alkyl group, i.e., an -O-alkyl group. Similarly, "methoxy" refers to -O-CH₃.

The divalent groups formed by removing another hydrogen from the groups defined above such as alkyl, alkenyl, alkynyl, cycloalkyl, heterocyclyl, aryl and heteroaryl are collectively referred to as "-ylene". Ring-forming groups such as cycloalkyl, heterocyclyl, aryl and heteroaryl are collectively referred to as "cyclic groups".

As used herein and unless specified otherwise, the term "optionally substituted" means either unsubstituted or substituted. The substituents are independently selected, and substitution may be at any chemically accessible position. As used herein, the term "substituted" means that a hydrogen atom is removed and replaced by a substituent. A single divalent substituent, e.g., oxo, can replace two hydrogen atoms. It is to be understood that substitution at a given atom is limited by valency. Illustrative examples of substituents are those found in the exemplary compounds, groups, and embodiments disclosed herein, as well as halogen, deuterium, OH, CN, alkyl, haloalkyl, alkenyl, alkynyl, cycloalkyl, aryl, heterocyclyl, heteroaryl, heterocycloalkyl, cycloalkylalkyl, heterocyclylalkyl, or heteroarylalkyl. A substitution may optionally be further substituted by another substitution, such as halogen, deuterium, OH, CN, alkyl, haloalkyl, alkoxy; cycloalkyloxy, aryloxy, heterocyclyloxy, heteroaryloxy, heterocycloalkyoxy, cycloalkylalkyloxy, heterocyclylalkyloxy, heteroarylalkyloxy, oxo (=O), amino, alkylamino, cycloalkylamino, heteroarylamino, guanidino, acylamino, sulfonylamino, urea, nitrourea, oxime, hydroxylamino, alkoxyamino, hydrazine, azido, nitro, thio (-SH), sulfonyl, aminosulfonyl, acyl, formyl, carboxy, ester, carbamate, amido, cyano, or isocyanato.

All alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, -NO₂, -N₃, - SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, - CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, - NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, - OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, - NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, - C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, - SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, - OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂,-P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R^{dd} groups; or two geminal hydrogen on a carbon atom are replaced with =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc} groups;

Each of the R^{aa} is independently selected from alkyl, haloalkyl, alkenyl, alkynyl, cycloalkyl, heterocyclyl, aryl and heteroaryl, or two of the R^{aa} groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;

Each of the R^{bb} is independently selected from hydrogen, -OH, -OR^{aa}, -N(R^{cc})₂, -CN, -C(=O)R^{aa}, - C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, - SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;

Each of the R^{cc} is independently selected from hydrogen, alkyl, haloalkyl, alkenyl, alkynyl, cycloalkyl, heterocyclyl, aryl and heteroaryl, or two R^{cc} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;

Each of the R^{dd} is independently selected from halogen, -CN, -NO₂, -N₃, -SO₂H, -SO₃H, -OH, -OR^{ee}, - ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, - OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups, or two geminal R^{dd} substituents can be combined to form=O or =S;

Each of the R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 R^{gg} groups;

Each of the R^{ff} is independently selected from hydrogen, alkyl, haloalkyl, alkenyl, alkynyl, cycloalkyl, heterocyclyl, aryl and heteroaryl, or two R^{ff} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{gg} groups;

Each of the R^{gg} is independently selected from halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, - N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), - NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, - OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, C₅-C₁₀ heteroaryl; or two geminal R^{gg} substituents may combine to form =O or =S; wherein X⁻ is a counter-ion.

Exemplary substituents on nitrogen atoms include, but are not limited to, hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

"Nucleic acids" refers to single- or double-stranded deoxyribonucleic acid (DNA) or ribonucleic acid (RNA) molecules and their heterozygous molecules. Examples of nucleic acid molecules include, but are not limited to, messenger RNA (mRNA), microRNA (miRNA), small interfering RNA (siRNA), self-amplified RNA (saRNA), and antisense oligonucleotides (ASO), etc. Nucleic acids may be further chemically modified, and the chemical modifier selected from one of, or a combination of: pseudouridine, N1-methyl-pseudouridine, 5-methoxyuridine, and 5-methylcytosine. mRNA molecules contain protein coding regions and may further contain expression regulatory sequences. Typical expression regulatory sequences include, but are not limited to, 5' cap, 5' untranslated region (5' UTR), 3' untranslated region (3' UTR), polyadenylate sequence (PolyA), miRNA binding sites.

As used herein, the term "pKa" refers to the negative logarithm (p) of the acid dissociation constant (Ka) of an acid, and is equal to the pH value at which equal concentrations of the acid and its conjugate base form are present in solution. The term "pKa" as used herein can be measured using water or dimethyl sulfoxide as a solvent. Observed values previously reported as pKa in case of using water as a solvent may be employed as pKa as used herein. In some embodiments, pKa can be determined by experiments, such as titration experiments using hydrochloric acid or sodium hydroxide. In some embodiments, pKa is determine by 2-(p-toluidino) naphthalene-6-sulfonic acid (TNS) fluorescent method.

The term "ionizable" or "ionizable group" as used herein refers to a chemical group that is either ionized or capable of ionization. An ionizable group may present as a neutral group, a positively charged group (cationic group) or a negatively charged group (anionic group). As used herein, a "charged moiety" is a chemical moiety that carries a formal electronic charge, e.g., monovalent (+1, or -1), divalent (+2, or -2), trivalent (+3, or -3), etc.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, including sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., pediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease", "disorder", and "condition" can be used interchangeably herein.

Unless otherwise indicated, the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a pharmaceutical composition refers to an amount sufficient to elicit a target biological response. As understood by those skilled in the art, the effective amount of the pharmaceutical composition of the disclosure can vary depending on the following factors, such as the desired biological endpoint, the pharmacokinetics of the pharmaceutical composition, the diseases being treated, the mode of administration, and the age, health status and symptoms of the subjects. The effective amount includes therapeutically effective amount and prophylactically effective amount. Unless otherwise indicated, the "therapeutically effective amount" of the pharmaceutical composition as used herein is an amount sufficient to provide therapeutic benefits in the course of treating a disease, disorder or condition, or to delay or minimize one or more symptoms associated with the disease, disorder or condition. The therapeutically effective amount of a pharmaceutical composition refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless otherwise indicated, the term "steroid compound" or "cationic steroid compound" as used herein refers to any steroid compound comprising at least one cationic moiety. As used herein, the term "steroid" is a class of compounds with a four ring 17 carbon cyclic structure, which can further comprise one or more substitutions, including but not limited to alkyl groups, alkoxy groups, hydroxy groups, oxo groups, acyl groups, or a double bond between two or more carbon atoms. The term "steroid" as used herein also comprises steroid esters and sterols. The term "steroid" as used herein also includes compounds belonging to or related to the following illustrative families of compounds: corticosteroids, mineralicosteroids, and sex steroids (e.g. androgenic or estrogenic or anti-androgenic and anti-estrogenic molecules). Non-limiting examples of steroids in include cholesterol, cholestane, campesterol, sitosterol, sitostanol, beta-sitosterol, stigmasterol, stigmastanol, brassicasterol, avenasterol, ergosterol, ursolic acid, tocopherol, lumisterol, or a derivative thereof. In one embodiment, the cationic steroid compound is an ionizable steroid compound or a zwitterionic steroid compound that becomes positively charged at certain pH. In one embodiment, the cationic steroid compound is a permanently cationic steroid compound. In one embodiment, the cationic steroid compound is a compound described in Section 0.

Unless otherwise indicated, the term "biodegradable" as used herein refers to substances that are degraded under physiological conditions. In some embodiments, a biodegradable substance is a substance that is broken down by cellular machinery. In some embodiments of any of the aspects, a biodegradable substance is a substance that is broken down by chemical processes. As used herein, the term "biodegradable group" refers to a group that includes one or more bonds that may undergo bond breaking reactions in a biological environment, e.g., in an organism, organ, tissue, cell, or organelle. For example, the biodegradable group may be metabolizable by the body of a mammal, such as a human (e.g., by hydrolysis). Some groups that contain a biodegradable bond include, for example, but are not limited to esters, dithiols, and oximes. Non-limiting examples of biodegradable groups are -OC(O)-, -C(O)O-, -SC(O)-, -C(O)S-, - OC(S)-, -C(S)O-, -S-S-, -C(R^{cc})=N-, -N-C(R^{cc})-, -C(R^{cc})=N-O-, -O-N-C(R^{cc})-, -C(O)(NR^{cc})-, -N(R^{cc})C(O)-, -C(S)(NR^{cc})-, -N(R^{cc})C(O)-, -N(R^{cc})C(O)N(R^{cc})-, -OC(O)O-, -OSi(R^{cc})₂O-, -C(O)(CR^{cc}R^{cc})C(O)O-, or - OC(O)(CR^{cc}R^{cc})C(O)-. The term "biodegradable lipid" refers to a lipid having one or more biodegradable groups located in the mid- or distal section of a lipidic moiety (e.g., a hydrophobic chain) of the lipid. Without bound by the theory, the incorporation of the biodegradable group(s) into lipid results in faster metabolism and removal of the lipid from the body following delivery of payloads.

Unless otherwise indicated, the term "structural lipid" refers to neutral sterols and also to lipids containing neutral sterol moieties. Without bound by the theory, incorporation of structural lipids in the lipid nanoparticle may help mitigate aggregation of other lipids in the particle. Structural lipids can be selected from the group including but not limited to, cholesterol, fecosterol, sitosterol, ergosterol, campesterol, stigmasterol, brassicasterol, tomatidine, tomatine, ursolic acid, alpha-tocopherol, hopanoids, phytosterols, or a mixture thereof. In some embodiments, the structural lipid is a sterol. As defined herein, "sterols" are a subgroup of steroids consisting of steroid alcohols. In certain embodiments, the structural lipid is cholesterol. In certain embodiments, the structural lipid is an analog of cholesterol. In certain embodiments, the structural lipid is alpha-tocopherol. Unless otherwise specified, the "structural lipid" as used herein is not the "steroid compound" or "cationic steroid compound" as used herein. In one embodiment, a "structural lipid" as used herein does not comprise a cationic or ionizable moiety. In one embodiment, a "structural lipid" as used herein does not comprise a quaternary ammonium group or a tertiary amine group.

Unless otherwise indicated, the term "polymer-conjugated lipid" as used herein refers to a polymer (e.g., polyethylene glycol (PEG), polypropylene glycol, polyvinylpyrrolidone (PVP), or poly(N-(2-hydroxypropyl)methacrylamide)) conjugated with a lipid. For example, PEG can be conjugated with myristoyl diglyceride to generate DMG-PEG-2000. Alternatively, PEG can be conjugated with DSPE to generate DSPE-PEG-2000. In one embodiment, a polymer-conjugated lipid is a pegylated lipid. A pegylated lipid can incorporate various functionalized PEG terminal groups including amine, carboxylic acid, azide, aldehyde, thiol, and hydroxyl moieties. Without bound by the theory, polymer-conjugated lipids improve circulation times, drug stability, suitability of different routes of administration, and help achieve targeted drug delivery.

### 5.2 Lipid Nanoparticle

In one aspect, provided herein is a lipid nanoparticle (LNP). In one embodiment, the LNP comprises a steroid compound having a cationic group (cationic steroid compound, Section 0). In one embodiment, the LNP comprises an ionizable lipid (Section 0). In one embodiment, the LNP comprises a steroid compound having a cationic group (Section 0) and an ionizable lipid (Section 0). In one embodiment, the LNP further comprises a phospholipid lipid (Section 0). In one embodiment, the LNP does not comprises phospholipid lipid. In one embodiment, the LNP further comprises a structural lipid (Section 0). In one embodiment, the LNP further comprises a polymer-conjugated lipid (Section 0). In one embodiment, the LNP further comprises a payload (Section 0).

In one embodiment, the lipid nanoparticle described herein is not liposome. In one embodiment, the lipid nanoparticle described herein is essentially free of aqueous internal cavities, or having internal cavities less than 10% of the total volume of the nanoparticle. In one embodiment, the lipid nanoparticle described herein comprises single-layered lipid membranes. In one embodiment, the lipid nanoparticle described herein comprises smaller particles inside the lipid nanoparticle, wherein the smaller particles bind with payloads describes herein.

In one embodiment, provided herein is a lipid nanoparticle for use in delivering or expressing a payload in the spleen of a subject. In one embodiment, the amount of payload delivered or expressed in the spleen of a subject is higher than in the liver of the subject. In one embodiment, the payload is delivered by systemic administration.

In one embodiment, the lipid nanoparticle comprising a steroid compound and an ionizable lipid.

In one embodiment, the amount of the steroid compound is from 3 mol % to 15 mol % of the total lipid present in the lipid nanoparticle, preferably 5-10 mol%, such as about 3 mol %, about 4 mol %, about 5 mol %, about 6 mol %, about 7 mol %, about 8 mol %, about 9 mol %, about 10 mol %, about 11 mol %, about 12 mol %, about 13 mol %, about 14 mol % or about 15 mol %of the total lipid present in the lipid nanoparticle.

In one embodiment, the molar percentage content of said ionizable lipid is 35-70 mol%, more preferably 40-60 mol%, for example 35 mol%, 40 mol%, 45 mol%, 50 mol%, 55 mol% or 60 mol%.

In one embodiment, the lipid nanoparticle further comprising phospholipid.

In a preferred embodiment, the molar percentage content of said phospholipid is 5-30 mol%, preferably 5-20 mol%, more preferably 5-15 mol%, for example 5 mol%, 6 mol%, 7 mol%, 8 mol%, 9 mol%, 10 mol%, 11 mol%, 12 mol%, 13 mol%, 14 mol%, 15 mol%, 16 mol%, 17 mol%, 18 mol%, 19 mol%, or 20 mol%... In one embodiment, the lipid nanoparticle further comprising a structural lipid, e.g. cholesterol.

In a preferred embodiment, the molar percentage content of said structural lipid is 10-80 mol%, more preferably 15-70 mol%, still more preferably 30-50 mol%, yet more preferably 30-40 mol%, for example 30 mol%, 35 mol%, 40 mol%, 45 mol%, 50 mol%, 55 mol%, or 60 mol%..

In one embodiment, the lipid nanoparticle further comprising a polymer conjugated lipid, e.g. PEGylated lipids.

In a preferred embodiment, the molar percentage content of said polymer-conjugated lipid is 0.25-10 mol%, more preferably 0.5-5 mol%, more preferably 0.5-3 mol%, for example 0.5 mol%, 0.6 mol%, 0.7 mol%, 0.8 mol%, 0.9 mol%, 1.0 mol%, 1.1 mol%, 1.2 mol%, 1.3 mol%, 1.4 mol%, 1.5 mol%, 1.6 mol%, 1.7 mol%, 1.8 mol%, 1.9 mol%, 2.0 mol%, 2.1 mol%, 2.2 mol%, 2.3 mol%, 2.4 mol%, 2.5 mol%, 2.6 mol%, 2.7 mol%, 2.8 mol%, 2.9 mol%, 3.0 mol%, 3.1 mol%, 3.2 mol%, 3.3 mol%, 3.4 mol%, 3.5 mol%, 3.6 mol%, 3.7 mol%, 3.8 mol%, 3.9 mol%, or 4.0 mol%.

In one embodiment, the average particle size of the spleen-targeting lipid nanoparticle is from 50 nm to 800 nm, more preferably 50-400 nm, more preferably 80-200 nm, such as 80 nm, 90 nm, 100 nm, 110 nm, 120 nm, 130 nm, 140 nm, 150 nm, 160 nm, 170 nm, 180 nm, 190 nm, or 200 nm.

In one embodiment, the lipid nanoparticle comprising (1) a steroid compound; (2) an ionizable lipid; (3) phospholipid; (4) structural lipid; and (5) polymer conjugated lipid. In a more perferred embodiment, the lipid nanoparticle further comprising a payload.

In a preferred embodiment, the spleen-targeting lipid nanoparticle comprises the following components in the molar percentages:
1) Steroid compound 3-15 mol%;
2) Ionizable lipid 35-70 mol%;
3) Phospholipid 5-30 mol%;
4) Structural lipid 10-80 mol%;
5) Polymer conjugated lipid 0.25-10 mol%.

In a preferred embodiment, the spleen-targeting lipid nanoparticle comprises the following components in the molar percentages:
1) Steroid compound 5-10 mol%;
2) Ionizable lipid 40-60 mol%;
3) Phospholipid 5-20 mol%;
4) Structural lipid 15-70 mol%; and
5) Polymer conjugated lipid 0.5-5 mol%.

In a preferred embodiment, the spleen-targeting lipid nanoparticle comprises the following components in the molar percentages:
1) Steroid compound 5-10 mol%;
2) Ionizable lipid 40-60 mol%;
3) Phospholipid 5-15 mol%;
4) Structural lipid 30-50 mol%; and
5) Polymer conjugated lipid 0.5-3 mol%.

In a particularly preferred embodiment, the spleen-targeting lipid nanoparticle comprises the following components in the molar percentages:
1) Steroid compound 5 mol%;
2) Ionizable lipid 50 mol%;
3) Phospholipid 10 mol%;
4) Structural lipid 33.5 mol%;
5) Polymer conjugated lipid 1.5 mol%.

In one embodiment, the lipid nanoparticle provide herein have a polydispersity index (PDI) of not greater than 0.2, preferably not greater than 0.15.

In one embodiment, the delivery or expression level of the payload in the spleen of the subject is more than 1 times, more preferably 2 times, more preferably 3 times, more preferably 4 times, more preferably 5 times, more preferably 10 times, more preferably 20 times, more preferably 30 times, more preferably 40 times, more preferably 50 times, or more preferably 100 times higher than the delivery or expression level of the payload in the liver of the subject, after administration of the lipid nanoparticle.

In one embodiment, the delivery or expression level of the payload in the spleen of the subject is more than 1 times, more preferably 2 times, more preferably 3 times, more preferably 4 times, more preferably 5 times, more preferably 10 times, more preferably 20 times, more preferably 30 times, more preferably 40 times, more preferably 50 times, or more preferably 100 times higher than the delivery or expression level of the payload in the lung of the subject, after administration of the lipid nanoparticle.

### 5.2.1. Steroid compound

In one embodiment, the lipid nanoparticle comprises a steroid compound comprising one or more cationic groups.

In one embodiment, the cationic group of the steroid compound is a tertiary amine group, quaternary ammonium group, ammonium group (NH₃⁺), primary amine group (NH₂), imidazole group, pyridine group, pyrrole group, pyrrolidine group, pyrazole group, (iso)thiazole group, (iso)oxazole group, oxadiazine group, oxazoline group, dithiourazole group, (iso)triazole group, tetrazole group, pentazole group, indole group, dihydroindole group, pyrimidine group, pyrazine group, quinazoline group, piperazine group, piperidine group, morpholine group, pyran group, quinoxaline group, quinoline group, quinolinone group, (iso)quinoline group, amidine group, guanidine group, or urea group.

In one embodiment, the cationic group of the steroid compound is a quaternary ammonium group. In one embodiment, the cationic group is a tertiary amine group. In one embodiment, the steroid compound comprises a quaternary ammonium group and a β-Sitosterol moiety. In one embodiment, the cationic steroid compound comprises a tertiary amine group and a β-Sitosterol moiety. In one embodiment, the cationic group is an amidine group, guanidine group, or urea group.

In one embodiment, the steroid compound is a permanently cationic steroid compound (i.e. positively charged regardless of pH). In one embodiment, the steroid compound is an ionizable cationic steroid compound (e.g. positively charged at certain pH). In one embodiment, the steroid compound is a zwitterionic steroid compound.

In one embodiment, the steroid compound comprises a Z₁ moiety comprising three fused cyclohexyl rings and a fused cyclopentyl ring, wherein one or more C-C bonds in the rings are optionally replaced by a C=C double bond. In one embodiment, Z₁ comprises an optionally substituted perhydrocyclopentanophenanthrene moiety.

In one embodiment, the steroid compound comprises a Z₁ moiety, and wherein the Z₁ is 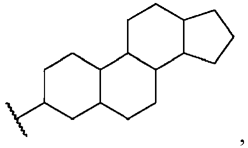 or a stereoisomer, or a mixture of stereoisomers thereof, wherein one or more ring C-C bond in Z₁ is independently optionally replaced by a C=C bond as valency permits, wherein Z₁ is optionally substituted with one or more groups selected from hydroxyl, halogen, and C₁₋₁₄ alkyl or C₁₋₁₄ alkenyl, wherein the alkyl is optionally substituted with one or more hydroxyl, nitro, cyano, halogen, C₁₋₆ alkoxy, C₂-C₆ alkenyl, C₂-C₆ alkynyl, C₃-C₈ cycloalkyl, C₆-C₁₀ aryl, 5 to 10-membered heteroaryl, or 3 to 8-membered heterocyclyl, and wherein the attachment in Z₁ is to the rest of the steroid compound.

In one embodiment, the steroid compound comprises a Z₁ moiety, wherein the Z₁ moiety is a monovalent radical of cholesterol, cholestane, campesterol, sitosterol, sitostanol, beta-sitosterol, stigmasterol, stigmastanol, brassicasterol, avenasterol, ergosterol, ursolic acid, tocopherol, lumisterol, or a stereoisomer, a mixture of stereoisomers, or a pharmaceutically acceptable salt thereof.

In one embodiment, the steroid compound has a pKa between 5 and 8. In one embodiment, the steroid compound has a pKa between 5.5 and 6.5. In one embodiment, the steroid compound has a pKa of greater than 8. In one embodiment, the steroid compound has a pKa of greater than 10. In one embodiment, the steroid compound has a pKa of greater than 10.

In one embodiment, the steroid compound is a compound of formula (C):

M⁻ V₁-[-(R₂₀)ₐ₁-Y₁]ₙ₁-Z₁ (C)

or a stereoisomer, a mixture of stereoisomers, or a pharmaceutically acceptable salt thereof, wherein:
Z₁ is an optionally substituted steroid;
each instance of Y₁ is independently absent or is -C(O)O-, -O-, -NH-, -SC(O)O-, -OC(O)NR₂ₐ-, - NR₂ₐC(O)NR₂ₐ-, -OC(O)S-, -OC(O)O-, -NR₂ₐC(O)O-, -OC(O)-, -SC(O)-, -C(O)S-, -NR₂ₐ-, -C(O)NR₂ₐ-, - NR₂ₐC(O)-, -NR₂ₐC(O)S-, -SC(O)NR₂ₐ-, -C(O)-, -OC(S)-, -C(S)O-, -OC(S)NR₂ₐ-, -NR₂ₐC(S)O-, -S-S-, or - S(O)₀₋₂-, wherein the attachment to the left is to the direction of V₁ and the attachment to the right is to the direction of Z₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, or L_{2b}N⁺R_{2b}R'_{2b}R"_{2b}
each instance of R₂₀ is independently -(CR₂₀₁R₂₀₂)-, wherein each instance of R₂₀₁ and R₂₀₂ are independently H, OH, alkyl, alkoxy, -L_{2b}-NR₂₁R₂₂, or -L_{2b}-N⁺R₂₁R₂₂R₂₃;
a1 and n1 are each independently 0, 1, 2, 3, 4, 5, 6, 7, 8, 9, or 10;
V₁ is NR₂₁R₂₂, N⁺R₂₁R₂₂R₂₃, or optionally substituted nitrogen-containing heterocyclyl selected from the group consisting of imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone and (iso)quinoline, or nitrogen-containing non-heterocyclic group selected from amidine, guanidine and urea;
M is absent or anion selected from chloride, bromide, iodide, sulfate, nitrate, perchlorate, formate, phosphate, pyrophosphates, citrate, sulfonate, methanesulfonate, triflate and trifluoroacetate;
each instance of R₂₁, R₂₂, R₂₃ are independently H, C₁₋₈ alkyl, -L_{2b}-OR_{2b}, -L_{2b}-SR_{2b}, or -L_{2c}-NR_{2b}R'_{2b}R"_{2b};
L_{2b} is absent or C₁₋₁₀ alkylene; and
each of R_{2b}, R'_{2b} and R"_{2b} are independently H or C₁₋₁₀ alkyl.

In one embodiment, a1 is 3, 4, 5, 6, 7, 8, 9, or 10.

In one embodiment, n1 is 0, 1, 2, 3, 4, 5, 6, 7, 8, 9, or 10.

In one embodiment, the steroid compound is a compound of formula (C1):

M⁻ V₁-(R₂₀)₂₋₈-Y₁-Z₁ (C1)

In one embodiment, the steroid compound is a compound of formula (C2):

M⁻ V₁-(R₂₀)₂₋₆-Y₁-(R₂₀)₂₋₆-Y₁-Z₁ (C2)

In one embodiment, R₂₀ is -(CR₂₀₁R₂₀₂)-, wherein each instance of R₂₀₁ and R₂₀₂ are independently H, OH, alkyl, alkoxy, -L_{2b}-NR₂₁R₂₂, or -L_{2b}-N⁺R₂₁R₂₂R₂₃. In one embodiment, R₂₀ is -(CR₂₀₁R₂₀₂)-, wherein each instance of R₂₀₁ and R₂₀₂ are independently H, C₁₋₆ alkyl, -NR₂₁R₂₂, or -N⁺R₂₁R₂₂R₂₃.

In one embodiment, the steroid compound is a compound of formula (I): 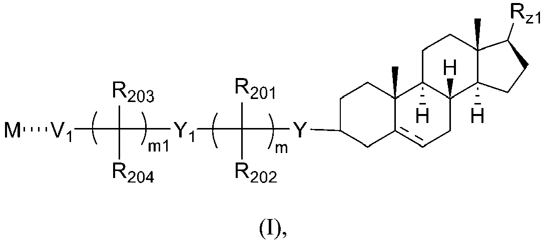 or a stereoisomer, a mixture of stereoisomers, or a pharmaceutically acceptable salt thereof, wherein:
--- is a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R''_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

In one embodiment, the steroid compound is a compound of formula (I): 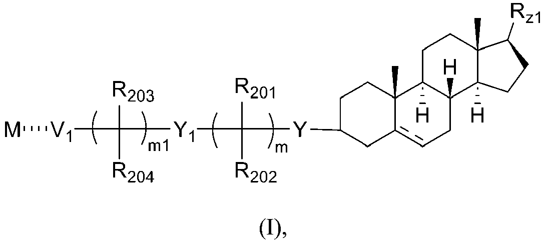 or a stereoisomer, a mixture of stereoisomers, or a pharmaceutically acceptable salt thereof, wherein:
--- is a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
m is an integer from 1 to 12;
ml is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl;
R_{z1} is C₁₋₁₄ alkyl or C₁₋₁₄ alkenyl, wherein the alkyl and alkenyl are optionally substituted; and
provided that when Y is -NHC(O)-, at least one of the following conditions are met:
   (i) m is an integer from 5 to 12; or (ii) V₁ is not N(CH₃)₂.

In one embodiment, the steroid compound is a compound of formula (I-P): 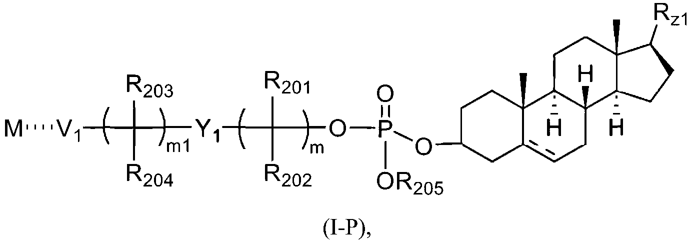 or a stereoisomer, a mixture of stereoisomers, or a pharmaceutically acceptable salt thereof, wherein:
- - - is a single bond or double bond;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, -S(O)₀₋₂-, or -OPO₃-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, -L_{2b}-N⁺R₂₁R₂₂R₂₃, L_{2b}-OC(O)R₂₁, or L_{2b}-C(O)OR₂₁;
R₂₀₅ is absent, a cation, H, C₁₋₆ alkyl, C₁₋₆ haloalkyl, C₂₋₆ alkenyl, or C₂₋₆ alkynyl, and wherein the alkyl, haloalkyl, alkenyl, and alkynyl are optionally substituted;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

In one embodiment of formula (I), V₁ is not NH₂ or NH₃⁺. In one embodiment of formula (I), when Y is -NR₂ₐC(O)-, V₁ is not NH₂ or NH₃⁺. In one embodiment of formula (I), when Y is -NHC(O)-, V₁ is not NH₂ or NH₃⁺.

The embodiments defined below are applicable to compounds represented by formula (I) and formula (I-P)

In one embodiment, Y is -C(O)O-, -OC(O)-, -O-, -NHC(O)O-, -OC(O)NH-, or -OC(O)O-. In one embodiment, Y is -C(O)O-. In one embodiment, Y is -O-. In one embodiment, Y is -NH-. In one embodiment, Y is -SC(O)O-. In one embodiment, Y is -OC(O)NR₂ₐ-. In one embodiment, Y is - NR₂ₐC(O)NR₂ₐ. In one embodiment, Y is -OC(O)S-. In one embodiment, Y is -OC(O)O-. In one embodiment, Y is -NR₂ₐC(O)O-. In one embodiment, Y is -OC(O)-. In one embodiment, Y is -SC(O)-. In one embodiment, Y is -C(O)S-. In one embodiment, Y is -NR₂ₐ-. In one embodiment, Y is -C(O)NR₂ₐ-. In one embodiment, Y is -NR₂ₐC(O)-. In one embodiment, Y is -NR₂ₐC(O)S-. In one embodiment, Y is - SC(O)NR₂ₐ-. In one embodiment, Y is -C(O)-. In one embodiment, Y is -OC(S)-. In one embodiment, Y is - C(S)O-. In one embodiment, Y is -OC(S)NR₂ₐ-. In one embodiment, Y is -NR₂ₐC(S)O-. In one embodiment, Y is -S(O)₀₋₂- (e.g. -S-). In these embodiments, the attachment to the left is to the direction of V₁.

In one embodiment, Y₁ is absent, -C(O)O-, -OC(O)-, -O-, -S-, SO, S(O)₂, -O(CO)O-, -NHC(O)-, or - C(O)NH-. In one embodiment, Y₁ is absent. In one embodiment, Y₁ is -SC(O)O-. In one embodiment, Y₁ is -OC(O)NR₂,-. In one embodiment, Y₁ is -NR₂ₐC(O)NR₂ₐ-. In one embodiment, Y₁ is -OC(O)S-. In one embodiment, Y₁ is -OC(O)O-. In one embodiment, Y₁ is -NR₂ₐC(O)O-. In one embodiment, Y₁ is - NR₂ₐC(O)S-. In one embodiment, Y₁ is -SC(O)NR₂ₐ-. In one embodiment, Y₁ is -OC(S)NR₂ₐ-. In one embodiment, Y₁ is -NR₂ₐC(S)O-. In one embodiment, Y₁ is -NHC(O)-. In one embodiment, Y₁ is - C(O)NH-. In one embodiment, Y₁ is -O(CO)O-. In one embodiment, Y₁ is -O-. In these embodiments, the attachment to the left is to the direction of V₁.

In one embodiment, the steroid compound is a compound of Formula (I-A1), (I-A2), (I-A3), (I-A4), or (I-A5): 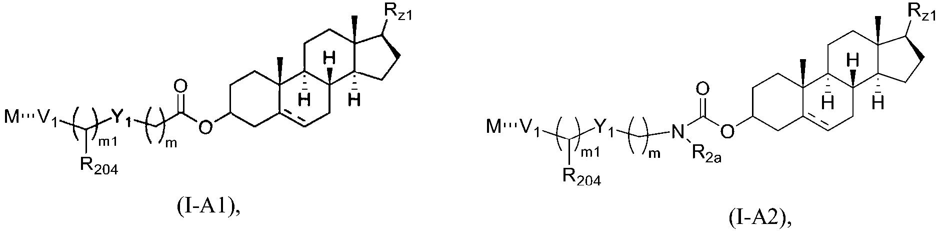 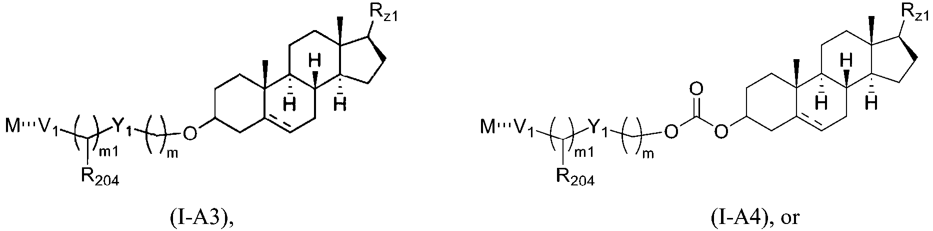 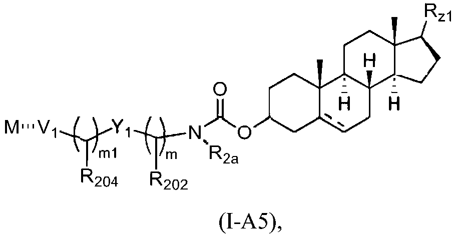 or a stereoisomer, a mixture of stereoisomers, or a pharmaceutically acceptable salt thereof,

In one embodiment, the steroid compound is a compound of Formula (I-B1), (I-B2), (I-B3), (I-B4), (I-B5), (I-B6), (I-B7), or (I-B8): 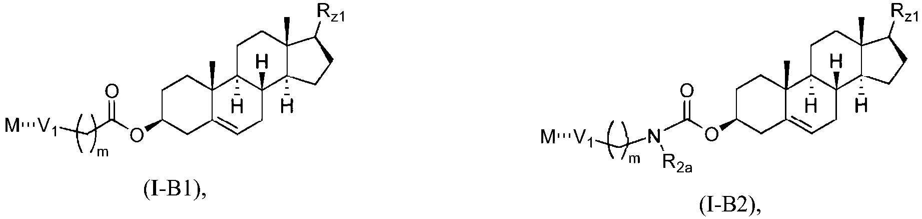 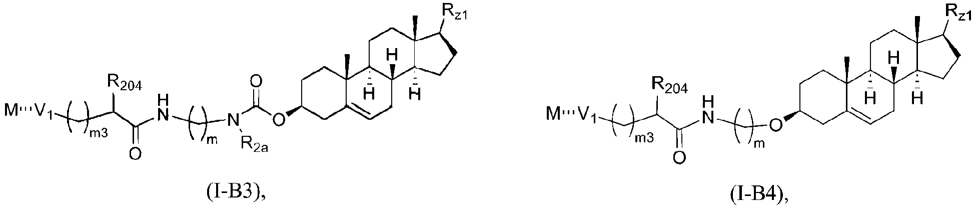 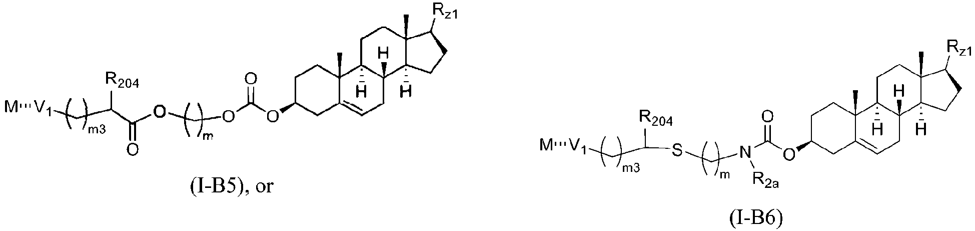 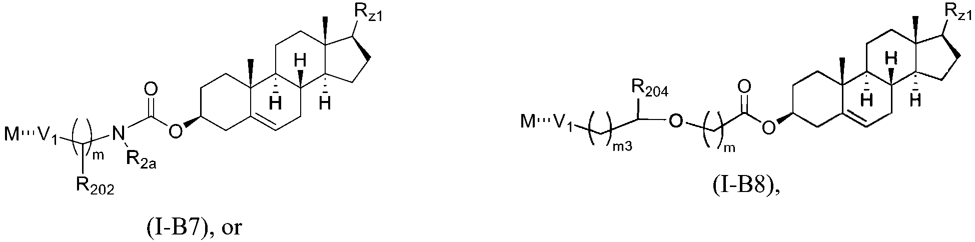 or a stereoisomer, a mixture of stereoisomers, or a pharmaceutically acceptable salt thereof, wherein m3 is an integer from 1 to 11.

In one embodiment, the steroid compound of formula (I-P) is a compound of formula (I-P1): 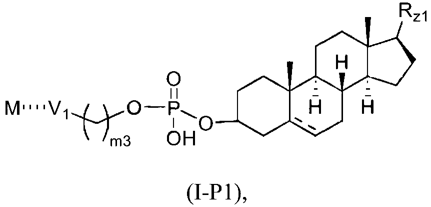 or a stereoisomer, a mixture of stereoisomers, or a pharmaceutically acceptable salt thereof, and wherein m3 is an integer from 1 to 11.

In one embodiment, m is an integer from 1 to 12. In one embodiment, m is an integer from 5 to 12. In one embodiment, m is 5. In one embodiment, m is 6. In one embodiment, m is 7. In one embodiment, m is 8. In one embodiment, m is 9. In one embodiment, m is 10. In one embodiment, m is 11. In one embodiment, m is 12.

In one embodiment, m1 is an integer from 0 to 12. In one embodiment, m1 is an integer from 0 to 6. In one embodiment, m1 is 0, 1, 2, 3, 4, 5, 6, 7, 8, 9, 10, 11, or 12.

In one embodiment, m3 is an integer from 1 to 11. In one embodiment, m3 is an integer from 1 to 6. In one embodiment, m3 is 1, 2, 3, 4, 5, 6, 7, 8, 9, 10, or 11.

In one embodiment, R₂ₐ is H.

In one embodiment, R₂ₐ is C₁₋₁₀ alkyl. In one embodiment, R₂ₐ is C₁₋₆ alkyl. In one embodiment, R₂ₐ is C₁₋₃ alkyl. In one embodiment, R₂ₐ is methyl. In one embodiment, the alkyl in R₂ₐ is substituted with L_{2b}-OR_{2b}. In one embodiment, the alkyl in R₂ₐ is unsubstituted. In one embodiment, the alkyl in R₂ₐ is substituted with L_{b}-SR_{2b}. In one embodiment, the alkyl in R₂ₐ is substituted with L_{2b}-NR_{2b}R'_{2b}. In one embodiment, the alkyl in R₂ₐ is substituted with L_{2b}N⁺R_{2b}R'_{2b}R"_{2b}.

In one embodiment, R₂ₐ is C₃₋₁₄ cycloalkyl. In one embodiment, R₂ₐ is C₃₋₈ cycloalkyl. In one embodiment, R₂ₐ is C₃₋₆ cycloalkyl. In one embodiment, R₂ₐ is C₃, C₄, C₅, or C₆ cycloalkyl. In one embodiment, the cycloalkyl in R₂ₐ is unsubstituted. In one embodiment, the cycloalkyl in R₂ₐ is substituted with C₁₋₁₀ alkyl. In one embodiment, the cycloalkyl in R₂ₐ is substituted with L_{2b}-OR_{2b}. In one embodiment, the cycloalkyl in R₂ₐ is substituted with L_{b}-SR_{2b}. In one embodiment, the cycloalkyl in R₂ₐ is substituted with L_{2b}-NR_{2b}R'_{2b}. In one embodiment, the cycloalkyl in R₂ₐ is substituted with L_{2b}N⁺R_{2b}R'_{2b}R"_{2b}.

In one embodiment, R₂ₐ is 3 to 14-membered heterocyclyl. In one embodiment, R₂ₐ is 3 to 8-membered heterocyclyl. In one embodiment, R₂ₐ is 3 to 6-membered heterocyclyl. In one embodiment, R₂ₐ is 3 to 6-membered nitrogen-containing heterocyclyl. In one embodiment, R₂ₐ is 3 to 6-membered oxygen-containing heterocyclyl. In one embodiment, the heterocyclyl in R₂ₐ is unsubstituted. In one embodiment, the heterocyclyl in R₂ₐ is substituted with C₁₋₁₀ alkyl. In one embodiment, the heterocyclyl in R₂ₐ is substituted with L_{2b}-OR_{2b}. In one embodiment, the heterocyclyl in R₂ₐ is substituted with L_{b}-SR_{2b}. In one embodiment, the heterocyclyl in R₂ₐ is substituted with L_{2b}-NR_{2b}R'_{2b}. In one embodiment, the heterocyclyl in R₂ₐ is substituted with L_{2b}N⁺R_{2b}R'_{2b}R"_{2b}.

In one embodiment, each instance of R₂₀₁, R₂₀₂, R₂₀₃, and R₂₀₄ are independently H, C₁₋₆ alkyl, -NR₂₁R₂₂, -N⁺R₂₁R₂₂R₂₃, -(CH₂)₀₋₆-OC(O)R₂₁, or -(CH₂)₀₋₆-C(O)OR₂₁.

In one embodiment, each instance of R₂₀₁, R₂₀₂, R₂₀₃, and R₂₀₄ are independently H, NH₂, NH₃⁺, - CH₂OC(O)CH₃, -CH₂C(O)OCH₃, -OC(O)CH₃, or -C(O)OCH₃.

In one embodiment, V₁ is NR₂₁R₂₂. In one embodiment, V₁ is N⁺R₂₁R₂₂R₂₃.

In one embodiment, R₂₁, R₂₂, R₂₃ are each independently H, hydroxyl, C₁₋₃ alkyl, or C₁₋₃ hydroxyalkyl.

In one embodiment, V₁ is optionally substituted nitrogen-containing heterocyclyl. In one embodiment, V₁ is optionally substituted 3 to 8-membered nitrogen-containing heterocyclyl. In one embodiment, V₁ is optionally substituted nitrogen-containing heteroaryl. In one embodiment, V₁ is optionally substituted 5- or 6-membered nitrogen-containing heteroaryl. In one embodiment, V₁ is selected from the group consisting of imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone and (iso)quinoline.

In one embodiment, V₁ is nitrogen-containing non-heterocyclic group. In one embodiment, V₁ is an optionally substituted radical of amidine. In one embodiment, V₁ is an optionally substituted radical of guanidine. In one embodiment, V₁ is an optionally substituted radical of urea.

In one embodiment, V₁ is N(CH₃)₂, N⁺(CH₃)₃, N⁺CH₃(CH₂CH₂OH)₂, N⁺(CH₃)₂(CH₂CH₂OH), NHC(=N⁺H2)NH₂, NHC(O)NH₂, CH₂C(=NH)NH₂, N(CH₃)(CH₂CH₂CO₂CH₃), N⁺(CH₃)₂(CH₂CH₂CO₂CH₃), N(CH₃)(CH₂CO₂CH₃), or N⁺(CH₃)₂(CH₂CO₂CH₃). In one embodiment, V₁ is N⁺CH₃(CH₂CH₂OH)₂, N⁺(CH₃)₂(CH₂CH₂OH), or N⁺(CH₃)₃.

In one embodiment, V₁ is N(CH₃)₂. In one embodiment, V₁ is N⁺(CH₃)₃. In one embodiment, V₁ is N⁺CH₃(CH₂CH₂OH)₂. In one embodiment, V₁ is N⁺(CH₃)₂(CH₂CH₂OH). In one embodiment, V₁ is NHC(=N⁺H2)NH₂. In one embodiment, V₁ is NHC(O)NH₂. In one embodiment, V₁ is CH₂C(=NH)NH₂.

In one embodiment, V₁ is NH₂. In one embodiment, V₁ is NH₃⁺.

In one embodiment, R₂₁, R₂₂, R₂₃ are each independently H, C₁₋₈ alkyl, -L_{2b}-OR_{2b}, -L_{2b}-SR_{2b}, or -L_{2c}-NR_{2b}R'_{2b}R"_{2b}. In one embodiment, R₂₁, R₂₂, R₂₃ are each independently H, C₁₋₆ alkyl, -OR_{2b}, -SR_{2b}, or - NR_{2b}R'_{2b}R"_{2b}. In one embodiment, R₂₁, R₂₂, R₂₃ are each independently H, hydroxyl, C₁₋₃ alkyl, or C₁₋₃ hydroxyalkyl.

In one embodiment, R₂₁ is H. In one embodiment, R₂₁ is C₁₋₈ alkyl. In one embodiment, R₂₁ is C₁₋₆ alkyl. In one embodiment, R₂₁ is C₁₋₃ alkyl. In one embodiment, R₂₁ is methyl. In one embodiment, R₂₁ is ethyl. In one embodiment, R₂₁ is propyl or isopropyl. In one embodiment, the alkyl in R₂₁ is unsubstituted. In one embodiment, the alkyl in R₂₁ is substituted with one or more halogens (i.e. F, Cl, Br, or I). In one embodiment, the alkyl in R₂₁ is substituted with L_{b}-OR_{2b}. In one embodiment, the alkyl in R₂₁ is substituted with L_{b}-SR_{2b}. In one embodiment, the alkyl in R₂₁ is substituted with L_{2b}-NR_{2b}R'_{2b}. In one embodiment, the alkyl in R₂₁ is substituted with L_{2b}N⁺R_{2b}R'_{2b}R"_{2b}.

In one embodiment, R₂₂ is -L_{2b}-OR_{2b}. In one embodiment, R₂₂ is -L_{2b}-SR_{2b}. In one embodiment, R₂₂ is - L_{2c}-NR_{2b}R'_{2b}R"_{2b}.

In one embodiment, R₂₂ is H. In one embodiment, R₂₂ is C₁₋₈ alkyl. In one embodiment, R₂₂ is C₁₋₆ alkyl. In one embodiment, R₂₂ is C₁₋₃ alkyl. In one embodiment, R₂₂ is methyl. In one embodiment, R₂₂ is ethyl. In one embodiment, R₂₂ is propyl or isopropyl. In one embodiment, the alkyl in R₂₂ is unsubstituted. In one embodiment, the alkyl in R₂₂ is substituted with one or more halogens (i.e. F, Cl, Br, or I). In one embodiment, the alkyl in R₂₂ is substituted with L_{b}-OR_{2b}. In one embodiment, the alkyl in R₂₂ is substituted with L_{b}-SR_{2b}. In one embodiment, the alkyl in R₂₂ is substituted with L_{2b}-NR_{2b}R'_{2b}. In one embodiment, the alkyl in R₂₂ is substituted with L_{2b}N⁺R_{2b}R'_{2b}R"_{2b}.

In one embodiment, R₂₂ is -L_{2b}-OR_{2b}. In one embodiment, R₂₂ is -L_{2b}-SR_{2b}. In one embodiment, R₂₂ is - L_{2c}-NR_{2b}R'_{2b}R"_{2b}.

In one embodiment, R₂₃ is H. In one embodiment, R₂₃ is C₁₋₈ alkyl. In one embodiment, R₂₃ is C₁₋₆ alkyl. In one embodiment, R₂₃ is C₁₋₃ alkyl. In one embodiment, R₂₃ is methyl. In one embodiment, R₂₃ is ethyl. In one embodiment, R₂₃ is propyl or isopropyl. In one embodiment, the alkyl in R₂₃ is unsubstituted. In one embodiment, the alkyl in R₂₃ is substituted with one or more halogens (i.e. F, Cl, Br, or I). In one embodiment, the alkyl in R₂₃ is substituted with L_{b}-OR_{2b}. In one embodiment, the alkyl in R₂₃ is substituted with L_{b}-SR_{2b}. In one embodiment, the alkyl in R₂₃ is substituted with L_{2b}-NR_{2b}R'_{2b}. In one embodiment, the alkyl in R₂₃ is substituted with L_{2b}N⁺R_{2b}R'_{2b}R"_{2b}.

In one embodiment, R₂₃ is -L_{2b}-OR_{2b}. In one embodiment, R₂₃ is -L_{2b}-SR_{2b}. In one embodiment, R₂₃ is - L_{2c}-NR₂₃R'₂₃R"₂₃.

In one embodiment, L_{2b} is absent. In one embodiment, L_{2b} is optionally substituted C₁₋₁₀ alkylene. In one embodiment, L_{2b} is optionally substituted C₁₋₆ alkylene. In one embodiment, L_{2b} is optionally substituted C₁₋₃ alkylene. In one embodiment, the alkylene in L_{2b} is unsubstituted. In one embodiment, the alkylene in L_{2b} is substituted with one or more halogen, hydroxyl, or C₁₋₃ alkoxy.

In one embodiment, R_{2b} is H. In one embodiment, R_{2b} is C₁₋₁₀ alkyl. In one embodiment, R_{2b} is C₁₋₆ alkyl. In one embodiment, R_{2b} is C₁₋₃ alkyl. In one embodiment, R_{2b} is methyl. In one embodiment, R_{2b} is ethyl. In one embodiment, R_{2b} is propyl or isopropyl. In one embodiment, the alkyl in R_{2b} is unsubstituted. In one embodiment, the alkyl in R_{2b} is substituted with one or more halogen, hydroxyl, or C₁₋₃ alkoxy.

In one embodiment, R'_{2b} is H. In one embodiment, R'_{2b} is C₁₋₁₀ alkyl. In one embodiment, R'_{2b} is C₁₋₆ alkyl. In one embodiment, R'_{2b} is C₁₋₃ alkyl. In one embodiment, R'_{2b} is methyl. In one embodiment, R'_{2b} is ethyl. In one embodiment, R'_{2b} is propyl or isopropyl. In one embodiment, the alkyl in R'_{2b} is unsubstituted. In one embodiment, the alkyl in R'_{2b} is substituted with one or more halogen, hydroxyl, or C₁₋₃ alkoxy.

In one embodiment, R"_{2b} is H. In one embodiment, R"_{2b} is C₁₋₁₀ alkyl. In one embodiment, R"_{2b} is C₁₋₆ alkyl. In one embodiment, R"_{2b} is C₁₋₃ alkyl. In one embodiment, R"_{2b} is methyl. In one embodiment, R"_{2b} is ethyl. In one embodiment, R"_{2b} is propyl or isopropyl. In one embodiment, the alkyl in R"_{2b} is unsubstituted. In one embodiment, the alkyl in R"_{2b} is substituted with one or more halogen, hydroxyl, or C₁₋₃ alkoxy.

In one embodiment, M is one or more anions. In one embodiment, M is one or more pharmaceutically acceptable anions. In one embodiment, M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate.

In one embodiment, M is chloride, bromide, iodide, sulfate, nitrate, perchlorate, formate, phosphate, pyrophosphates, citrate, sulfonate, methanesulfonate, triflate, or trifluoroacetate. In one embodiment, M is absent, or one or more of bromide, chloride, or trifluoroacetate.

In one embodiment, R_{z1} is C₁₋₁₄ alkyl or C₁₋₁₄ alkenyl, wherein the alkyl and alkenyl are optionally substituted with one or more hydroxyl, oxo, nitro, cyano, halogen, C₁₋₆ alkoxy, C₂-C₆ alkenyl, C₂-C₆ alkynyl, C₃-C₈ cycloalkyl, C₆-C₁₀ aryl, 5 to 10-membered heteroaryl, or 3 to 8-membered heterocyclyl. In one embodiment, R_{z1} is C₃₋₁₂ alkyl or C₃₋₁₂ alkenyl. In one embodiment, R_{z1} is C₇₋₁₀ alkyl or C₇₋₁₀ alkenyl. In one embodiment, R_{z1} is C₇ alkyl or C₇ alkenyl. In one embodiment, R_{z1} is C₈ alkyl or C₈ alkenyl. In one embodiment, R_{z1} is C₉ alkyl or C₉ alkenyl. In one embodiment, R_{z1} is C₁₀ alkyl or C₁₀ alkenyl.

In one embodiment, R_{z1} is C₆₋₁₂ alkyl. In one embodiment, R_{z1} is unsubstituted C₆₋₁₂ alkyl. In one embodiment, R_{z1} is C₆₋₁₂ alkyl substituted with one or more oxo or hydroxyl.

In one embodiment, R_{z1} is C₆₋₁₂ alkenyl. In one embodiment, R_{z1} is unsubstituted C₆₋₁₂ alkenyl. In one embodiment, R_{z1} is C₆₋₁₂ alkenyl substituted with one or more oxo or hydroxyl.

In one embodiment, R_{z1} is 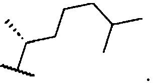 In one embodiment, R_{z1} is 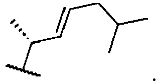 In one embodiment, R_{z1} is 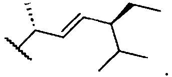 In one embodiment, R_{z1} is 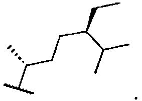 In one embodiment, R_{z1} is 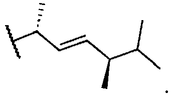 In one embodiment, R_{z1} is 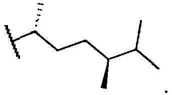 In one embodiment, R_{z1} is

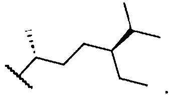

In one embodiment, the steroid compound is: 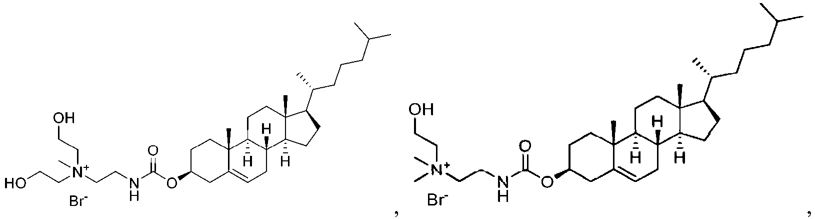 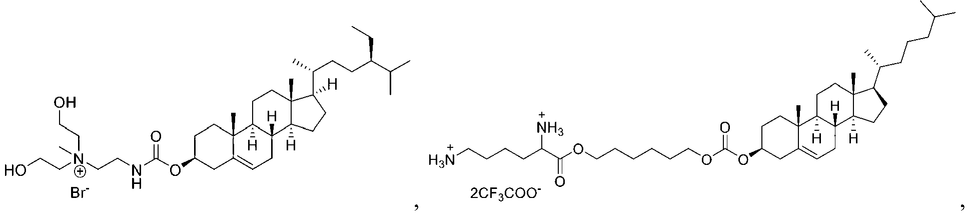 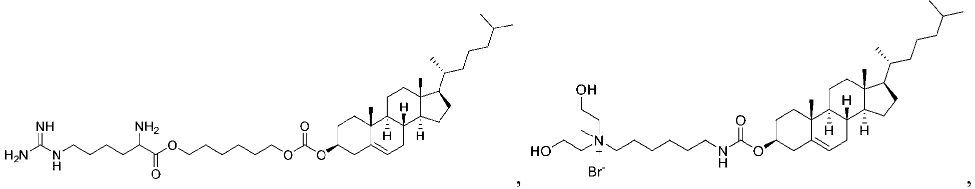 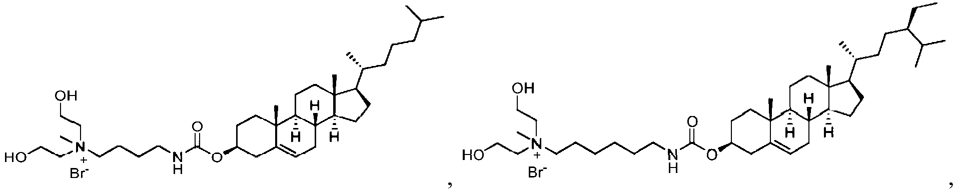 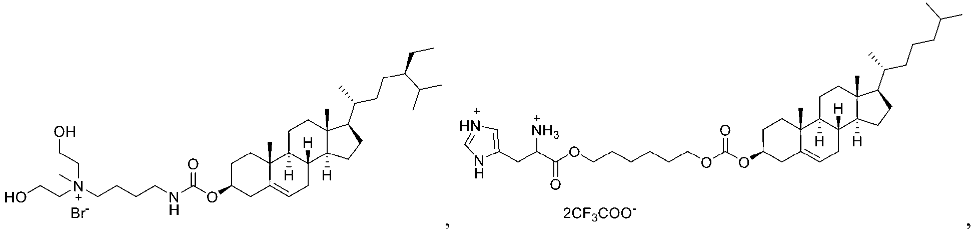 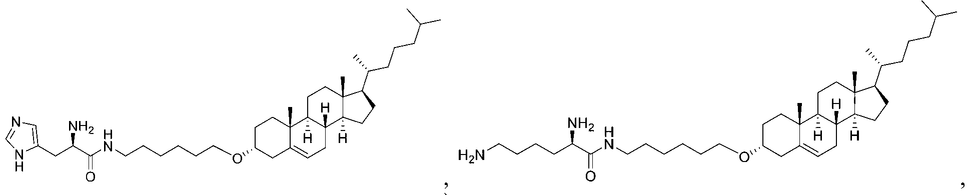 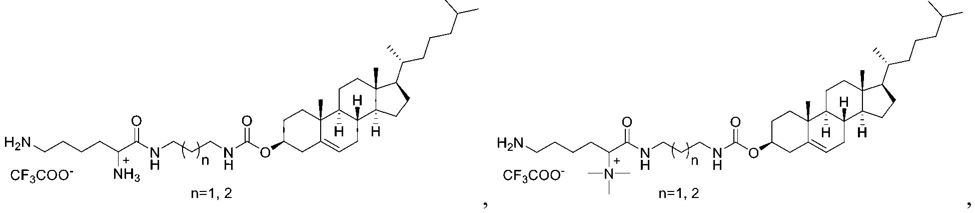 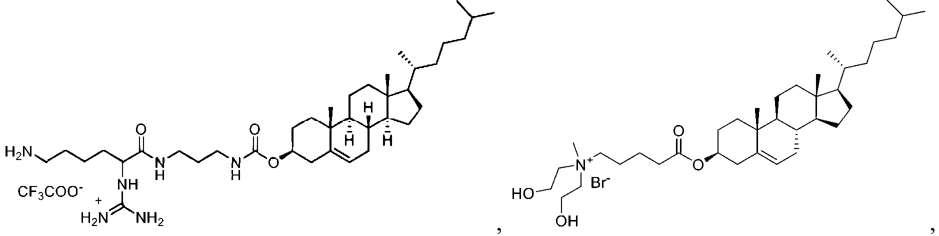 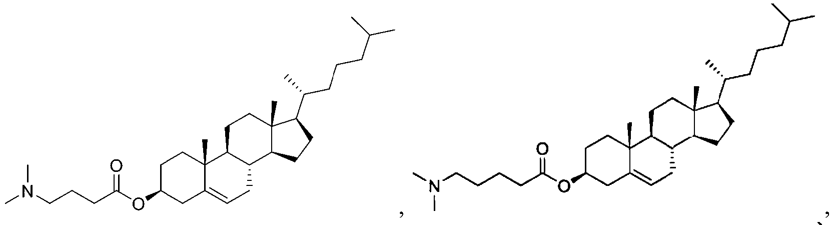 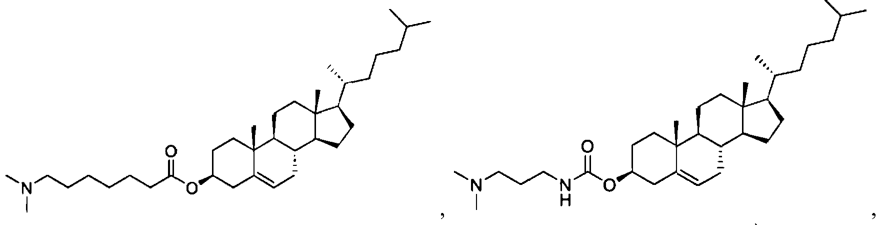 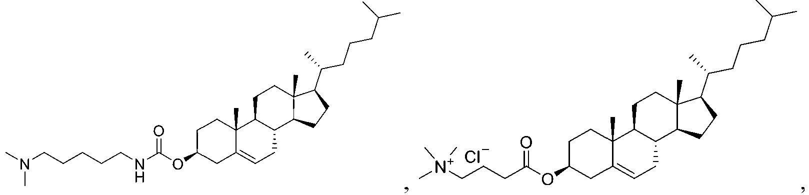 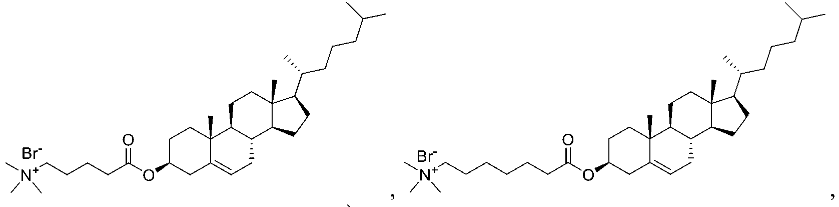 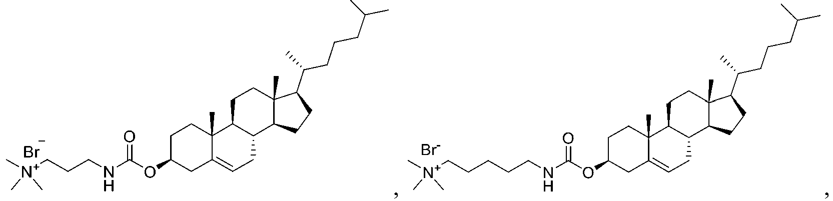 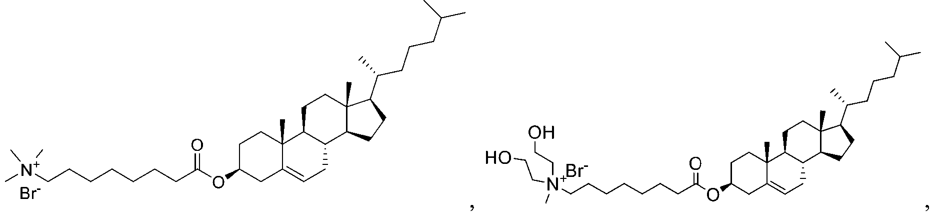 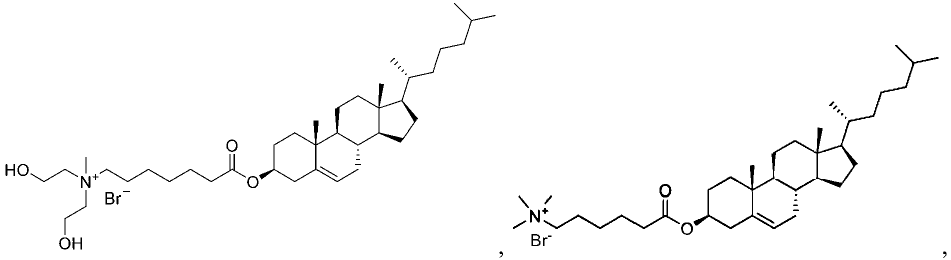 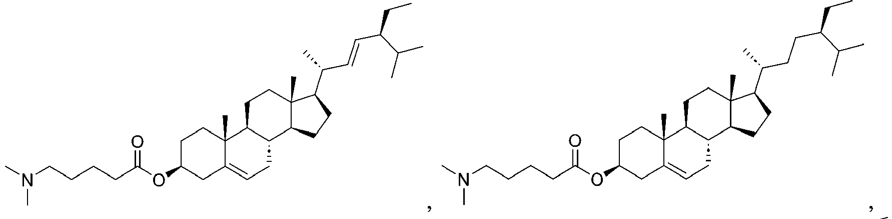 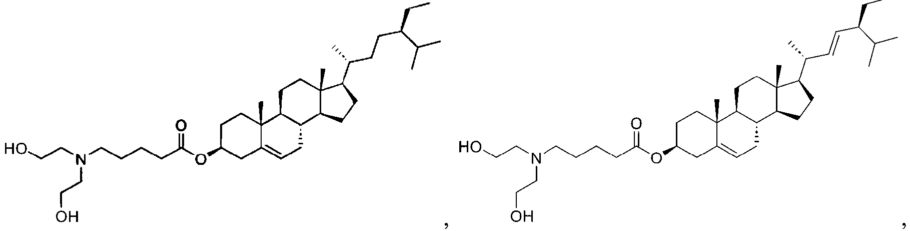 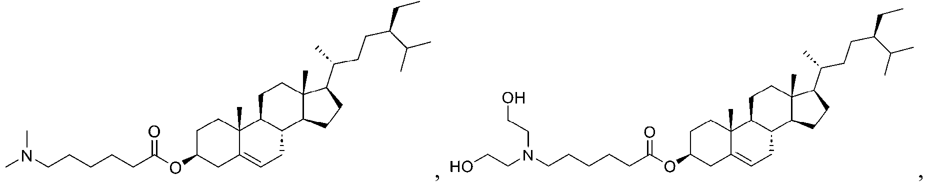 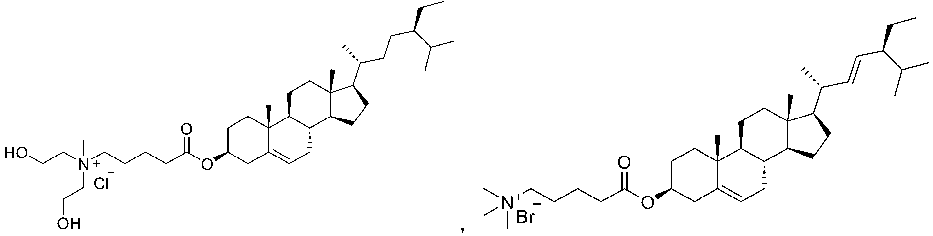 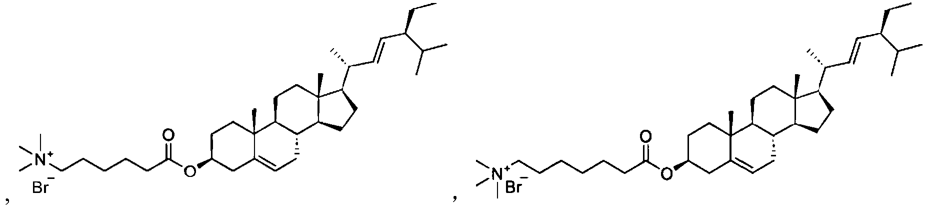 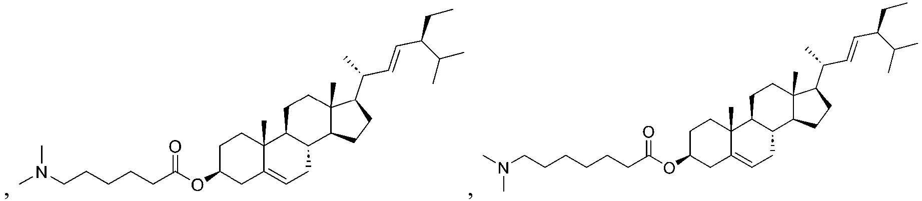 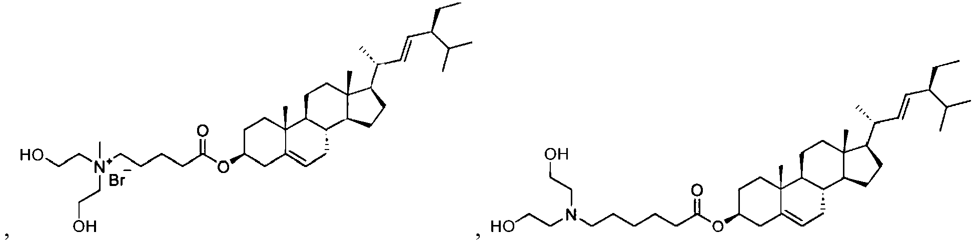 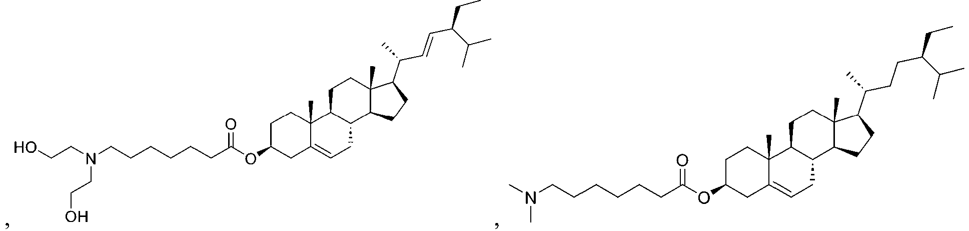 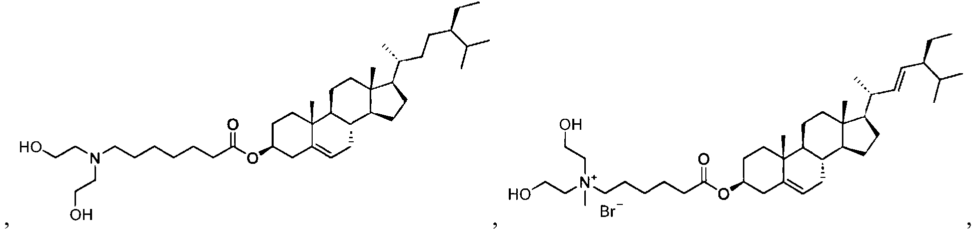 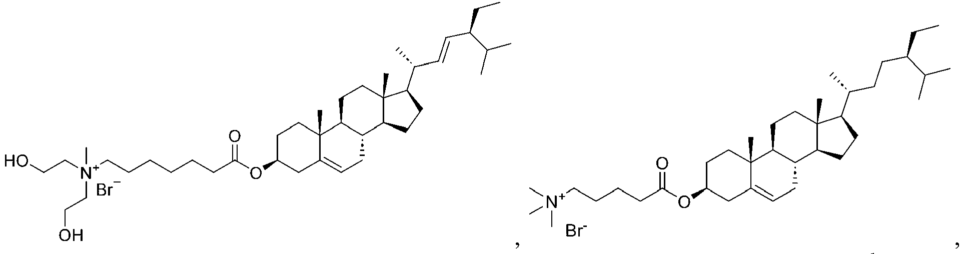 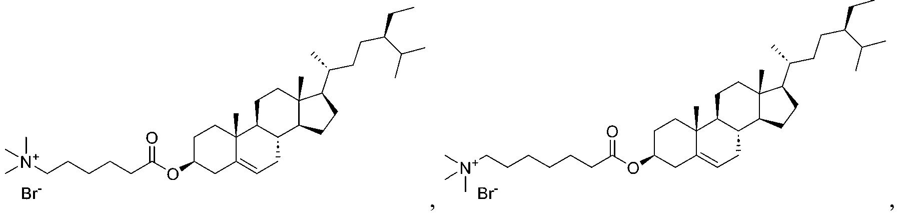 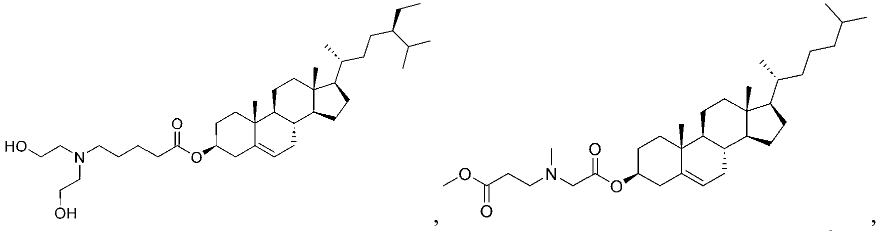 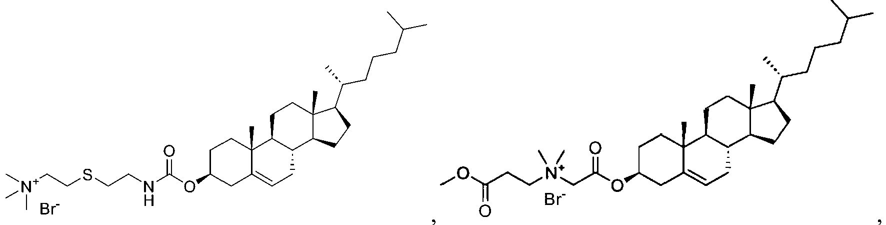 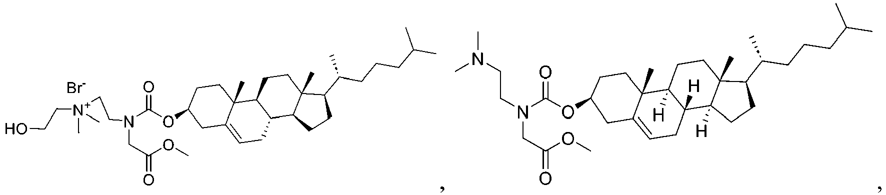 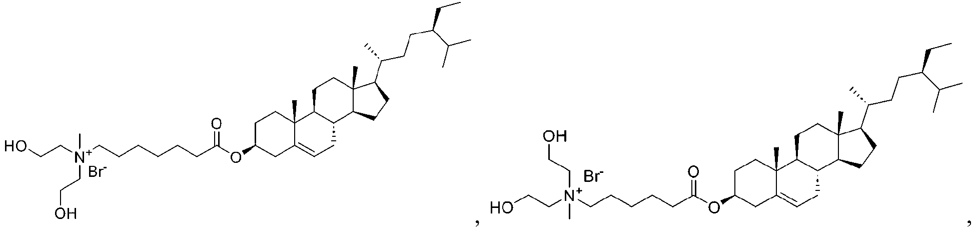 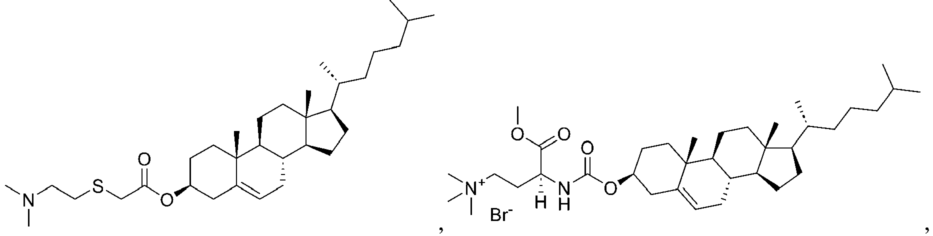 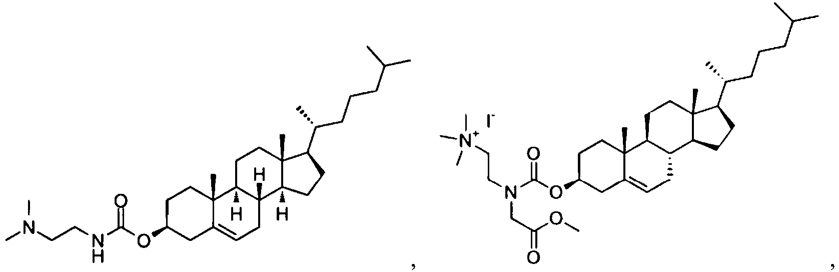 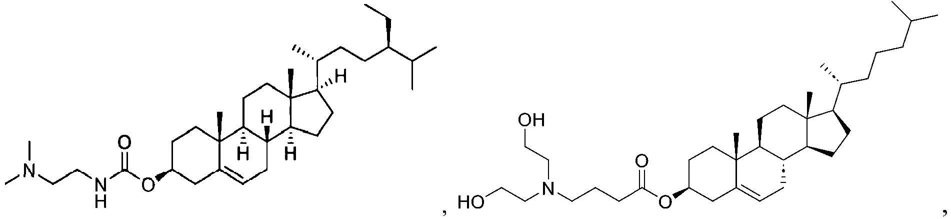 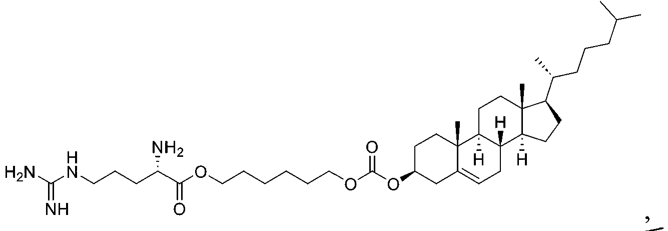 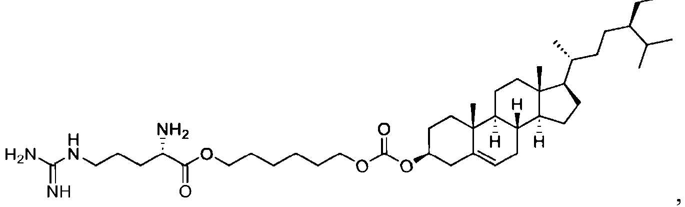 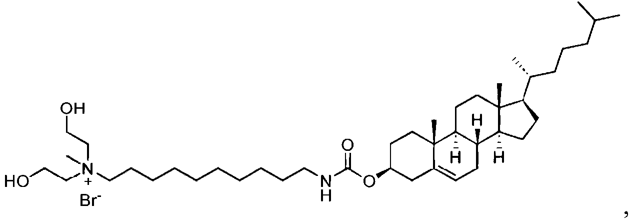 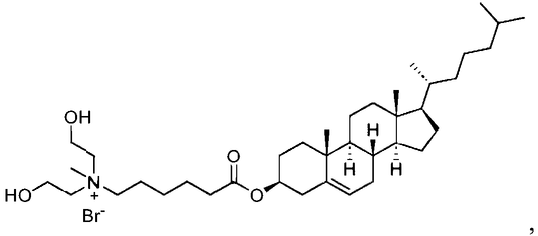 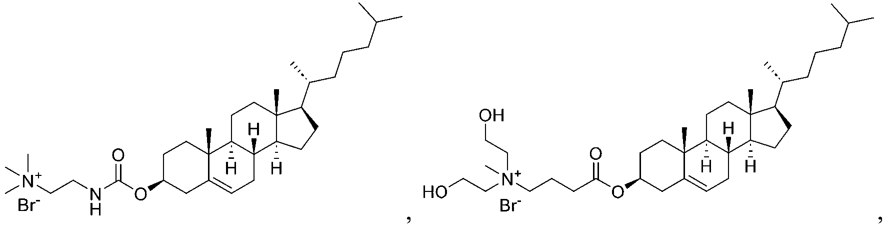 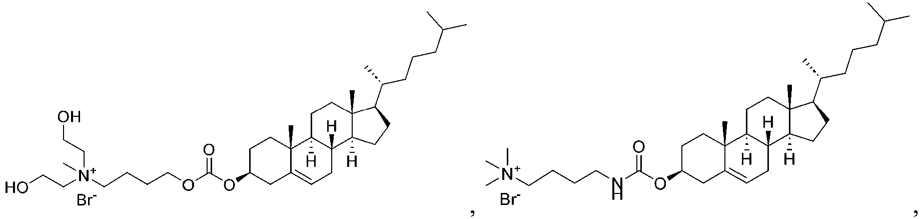 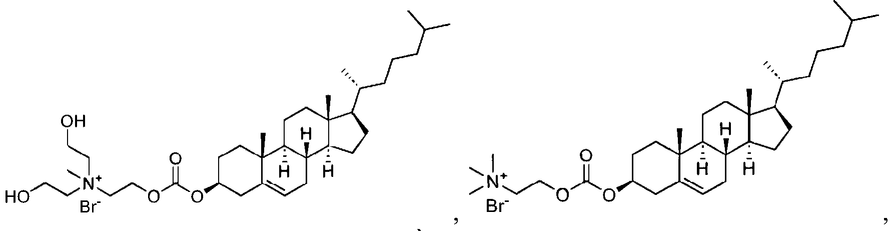 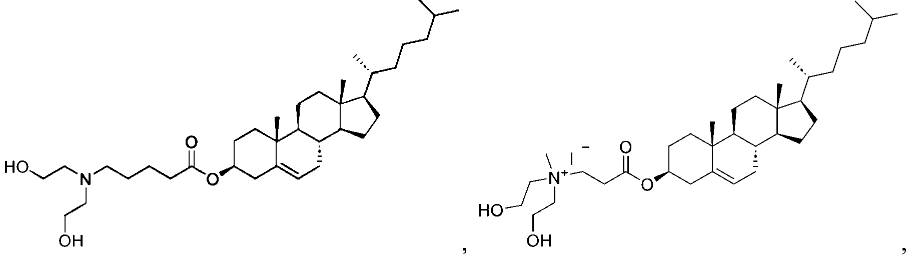 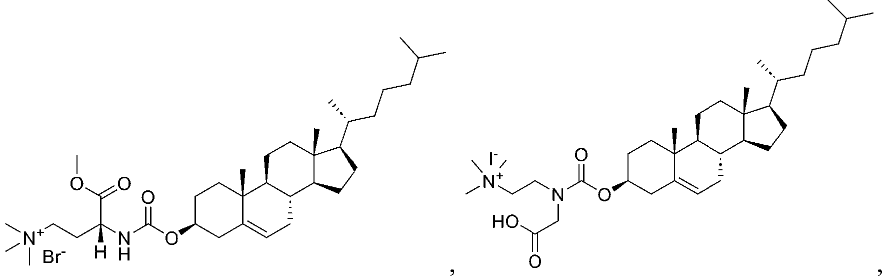 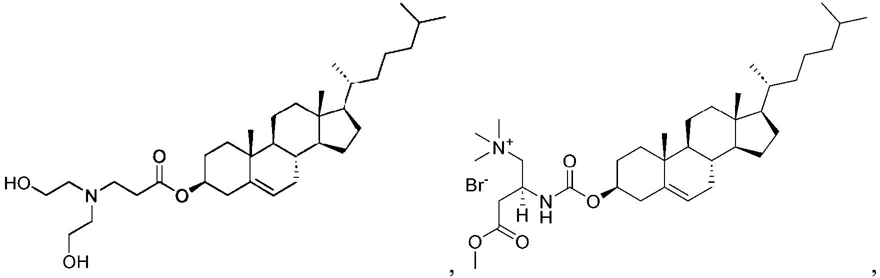 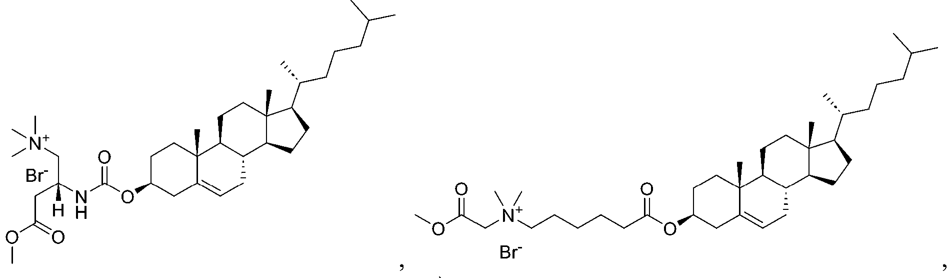 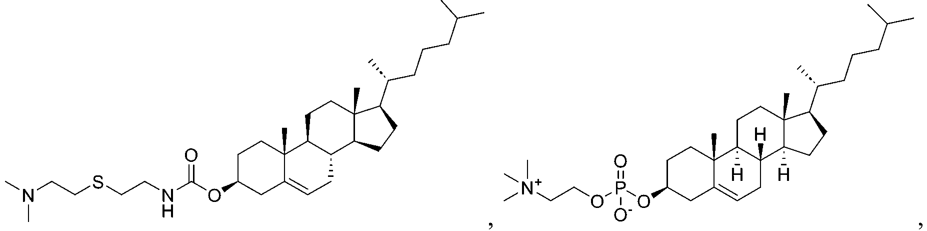 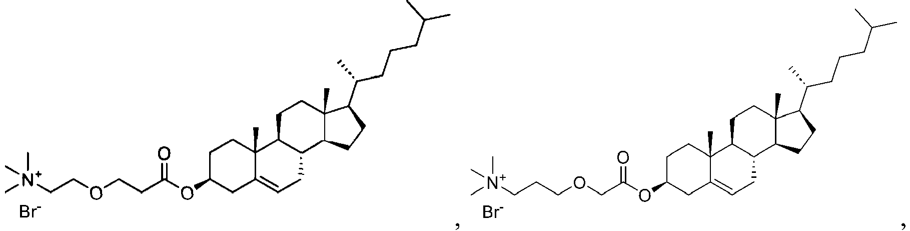 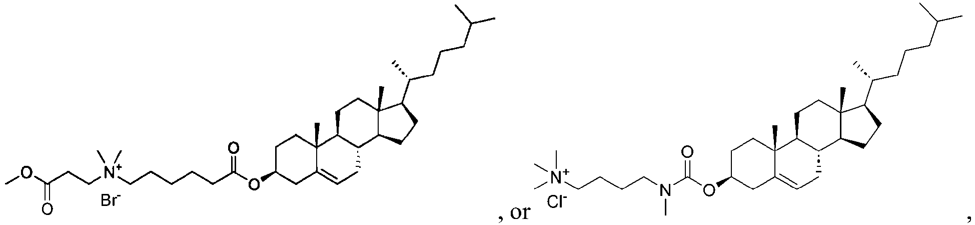 or a stereoisomer, or a mixture of stereoisomers, or a pharmaceutically acceptable salt thereof.

In one embodiment, the steroid compound is not: 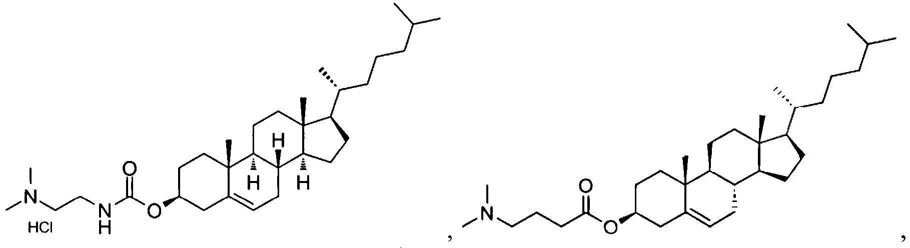 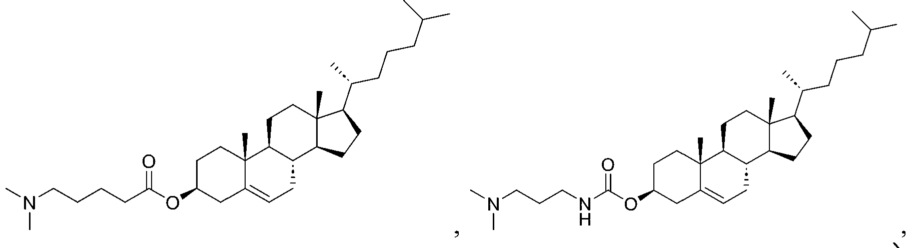 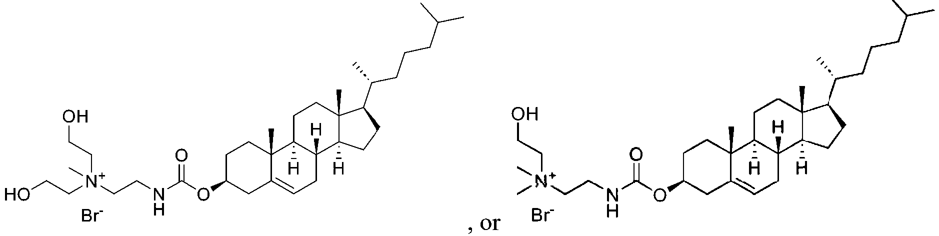 or its pharmaceutically acceptable salt thereof.

In one embodiment, provided herein is a steroid compound of formula (II-A): 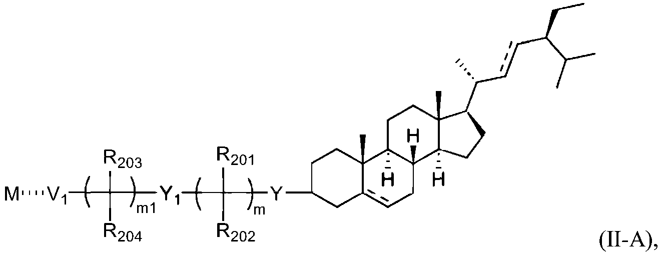 or a stereoisomer, a mixture of stereoisomers, or a pharmaceutically acceptable salt thereof, wherein:
each - - - is independently a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, or -L_{2b}-N⁺R₂₁R₂₂R₂₃, L_{2b}-OC(O)R₂₁, or L_{2b}-C(O)OR₂₁;
m is an integer from 3 to 12;
m1 is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ is independently H, optionally substituted C₁₋₈ alkyl, -L_{2b}-OR_{2b}, -L_{2b}-SR_{2b}, - L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} is independently H or C₁₋₁₀ alkyl; and
R_{z1} is C₁₋₁₄ alkyl or C₁₋₁₄ alkenyl, wherein the alkyl and alkenyl are optionally substituted; and provided that the compound is not:

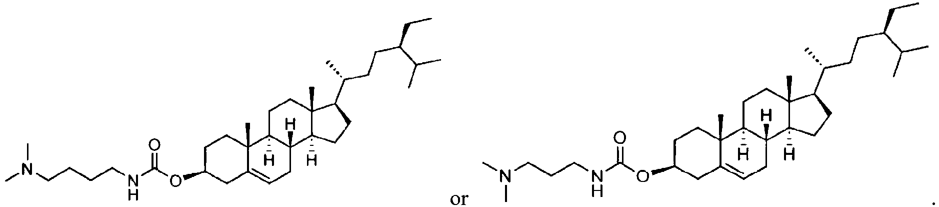

In one embodiment of formula (II-A), V₁ is not NH₂ or NH₃⁺. In one embodiment of formula (II-A), when Y is -NR₂ₐC(O)-, V₁ is not NH₂ or NH₃⁺. In one embodiment of formula (II-A), when Y is -NHC(O)-, V₁ is not NH₂ or NH₃⁺.

In one embodiment, provided herein is a steroid compound of formula (II-B): 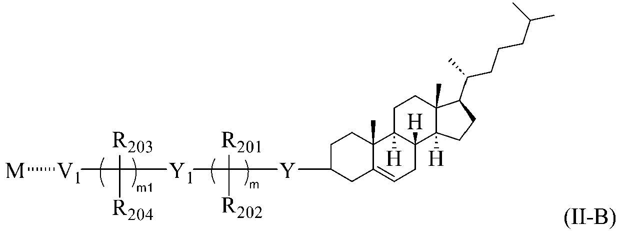 or a stereoisomer, a mixture of stereoisomers, or a pharmaceutically acceptable salt thereof, wherein:
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂,-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, or -L_{2b}-N⁺R₂₁R₂₂R₂₃, L_{2b}-OC(O)R₂₁, or L_{2b}-C(O)OR₂₁;
m is an integer from 5 to 12;
ml is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} are independently H or C₁₋₁₀ alkyl.

In one embodiment of formula (II-B), Y₁ is -C(O)O-, -OC(O)-, -O-, -S-, -NR₂ₐ-, -OC(O)NR₂ₐ-, or - NR₂ₐC(O)-. In one embodiment of formula (II-B), Y₁ is -C(O)O-, -OC(O)-, -O-, -S-, -NH-, -NCH₃-, - OC(O)NH-, or -NHC(O)-. In one embodiment of formula (II-B), Y₁ is -O-. In one embodiment of formula (II-B), Y₁ is -S-.

In one embodiment, provided herein is a steroid compound of formula (II-C1): 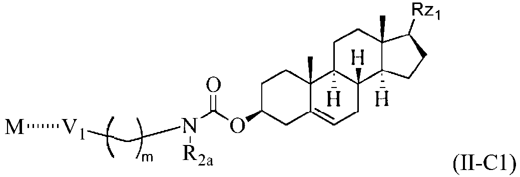 or a stereoisomer, a mixture of stereoisomers, or a pharmaceutically acceptable salt thereof, wherein:
R_{z1} is 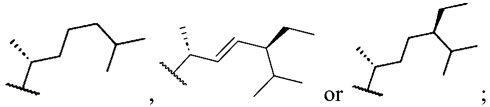
R₂ₐ is H, optionally substituted C₁₋₈ alkyl, -L_{2b}-OR_{2b}, L_{2b}-OC(O)R_{2b}, or L_{2b}-C(O)OR_{2b}, preferably H, - CH₃, -CH₂C(O)OCH₃, or -CH₂C(O)OH;
V₁ is NR₂₁R₂₂ or N⁺R₂₁R₂₂R₂₃, wherein each instance of R₂₁, R₂₂, R₂₃ is independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}; provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 12, such as 1, 2, 3, 4, 5, 6, 7, 8, 9, 10, 11 or 12.

In one embodiment, provided herein is a steroid compound of formula (II-C2): 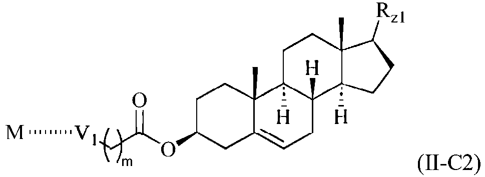 or a stereoisomer, a mixture of stereoisomers, or a pharmaceutically acceptable salt thereof, wherein:
R_{z1} is 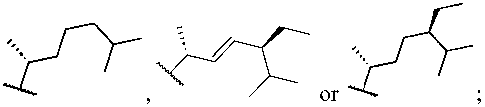
V₁ is NR₂₁R₂₂ or N⁺R₂₁R₂₂R₂₃, wherein each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}; provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 12, such as 1, 2, 3, 4, 5, 6, 7, 8, 9, 10, 11, or 12.

In one embodiment, the steroid compound is not: 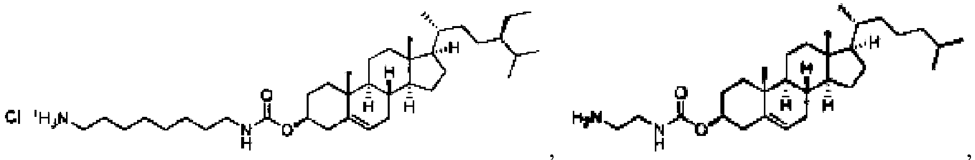 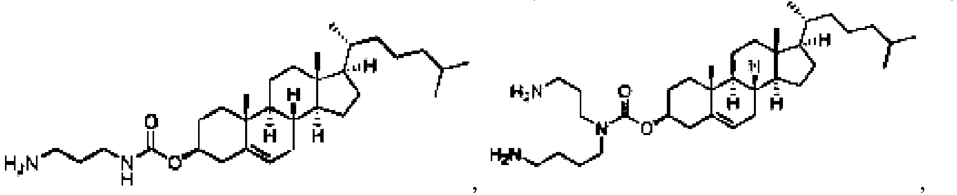 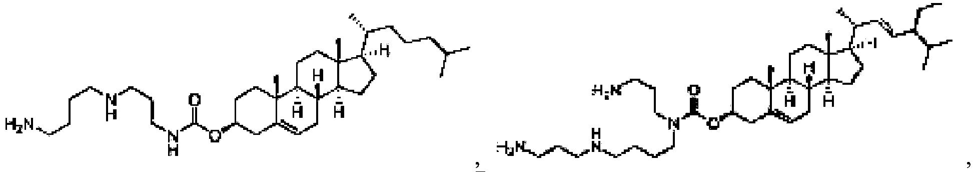 or 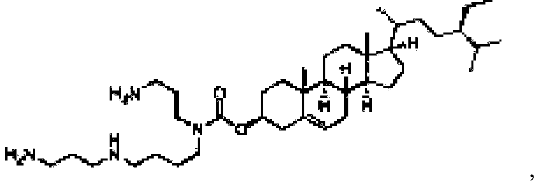 or a pharamaceutically acceptable salt thereof.

In one embodiment, provided herein are the following compounds: 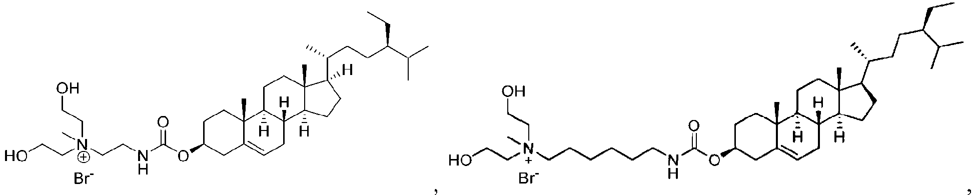 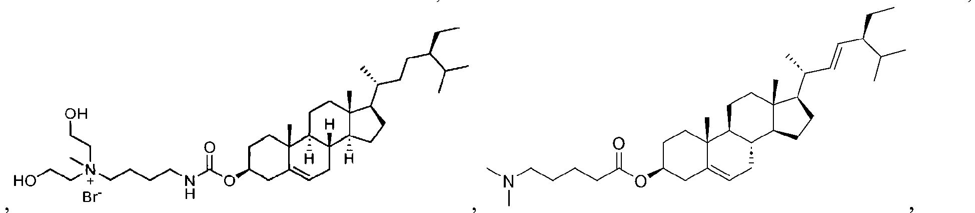 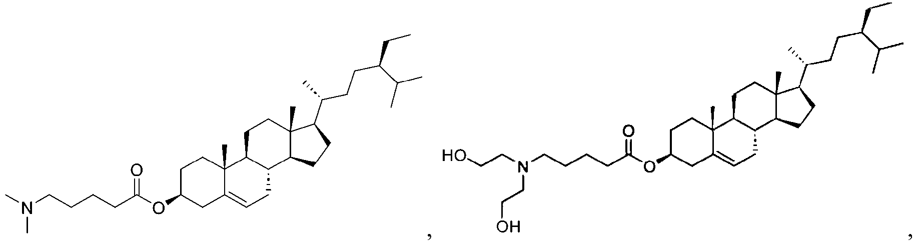 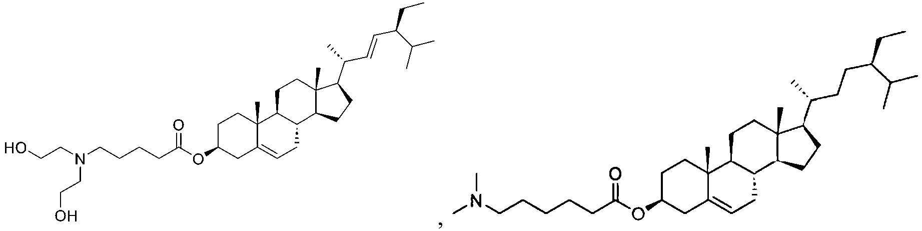 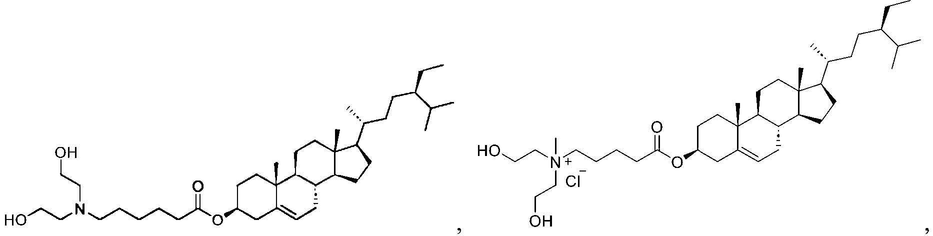 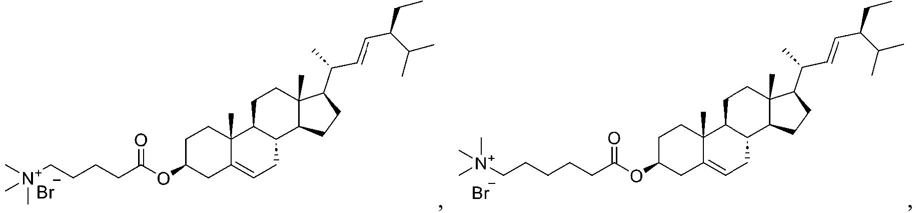 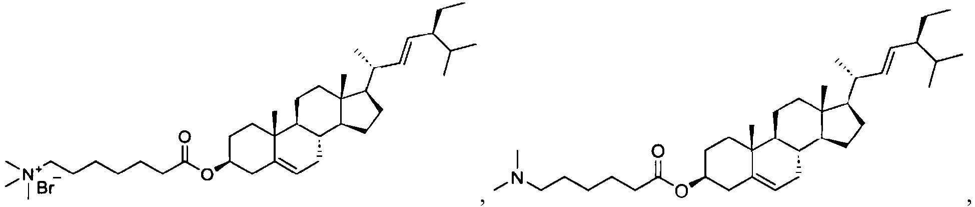 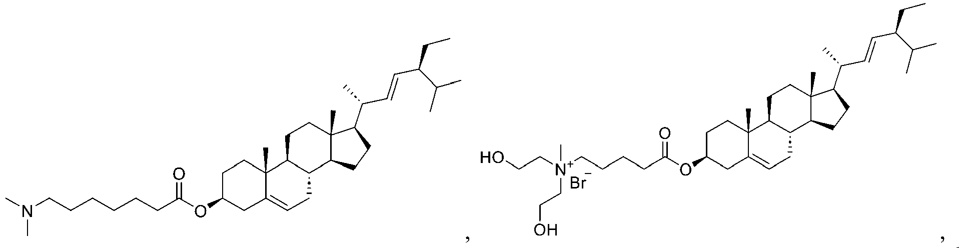 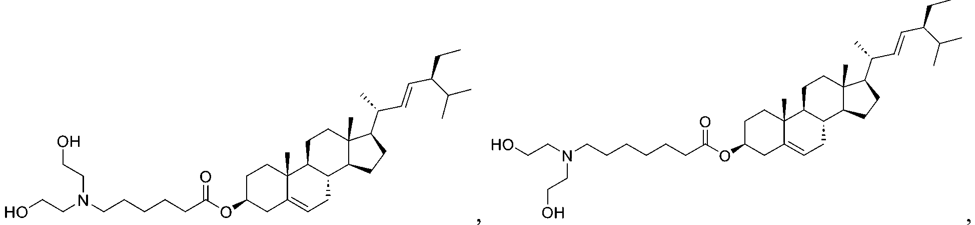 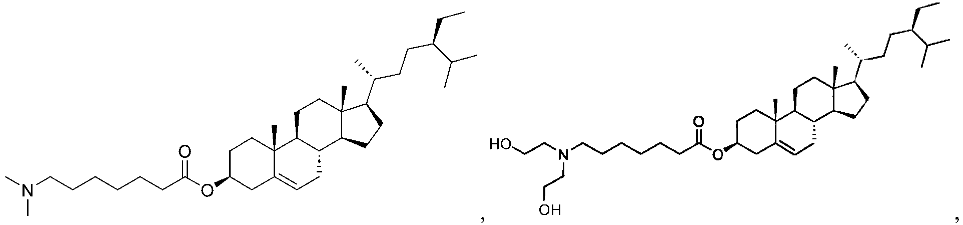 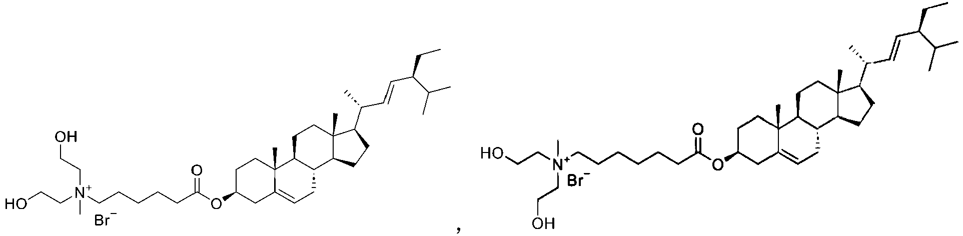 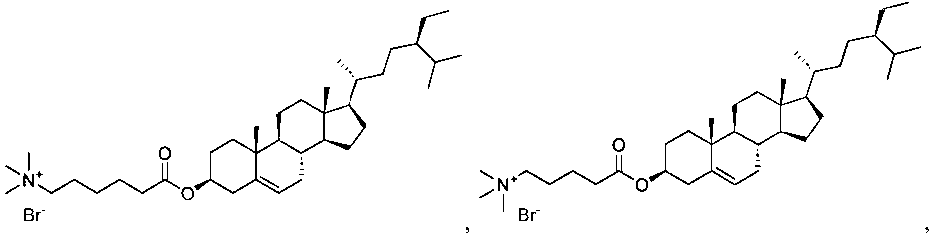 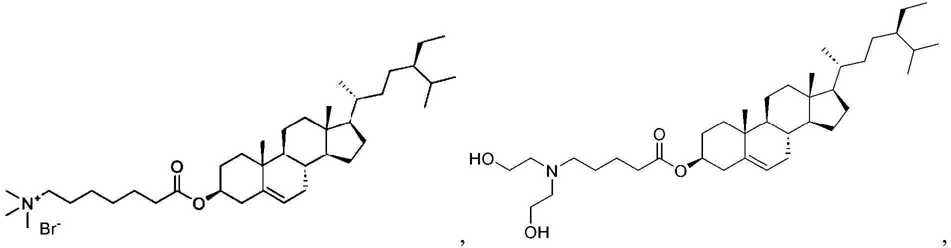 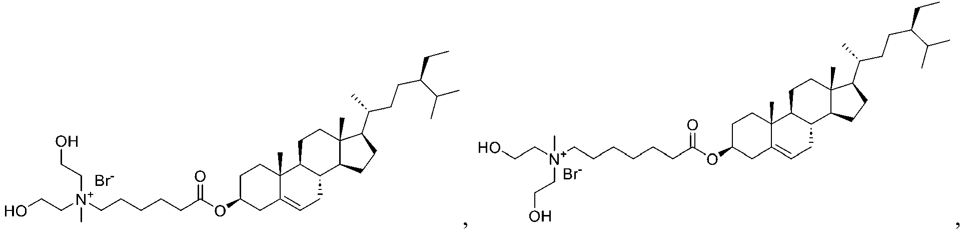 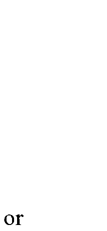 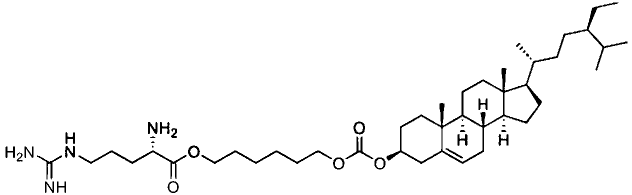 or a stereoisomer, a mixture of stereoisomers, or a pharmaceutically acceptable salt thereof.

In one embodiment, provided herein are the following compounds: 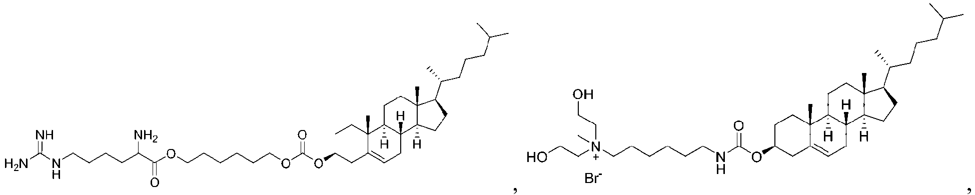 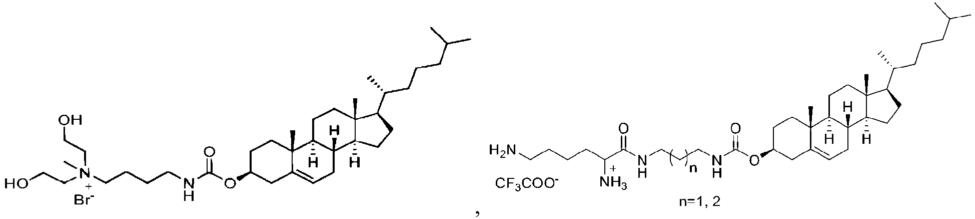 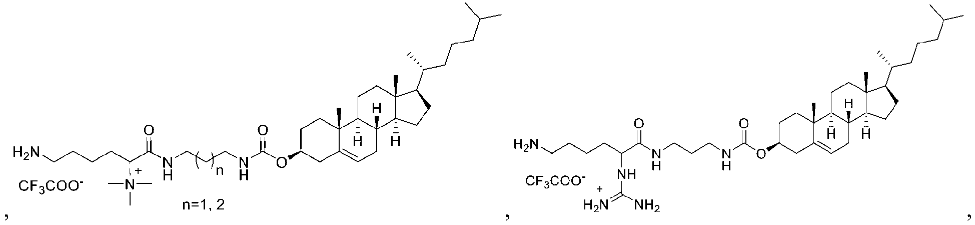 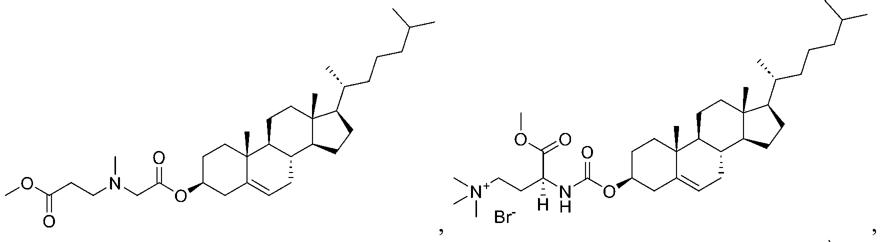 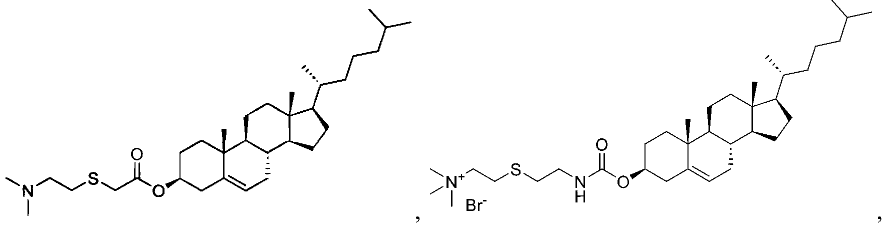 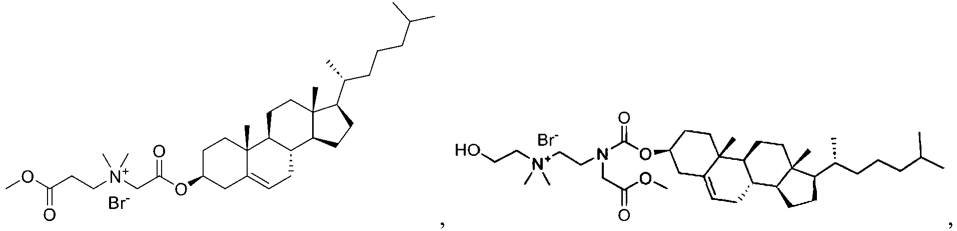 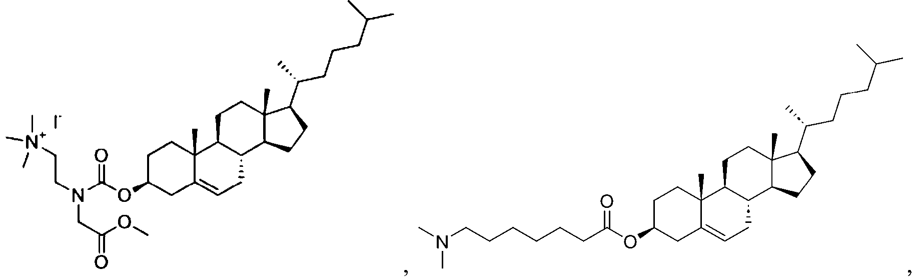 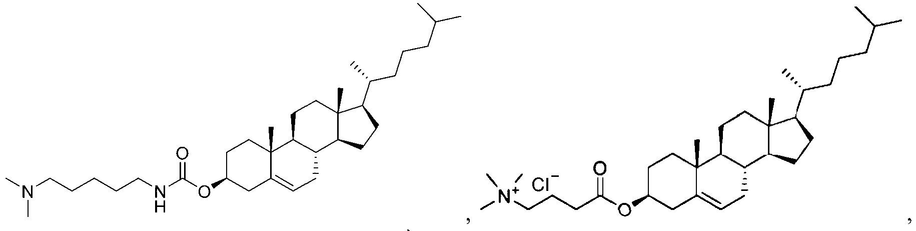 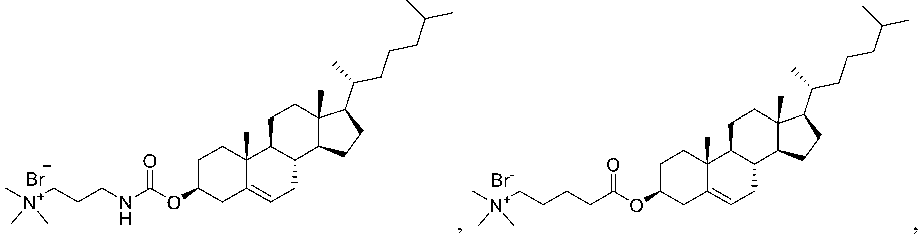 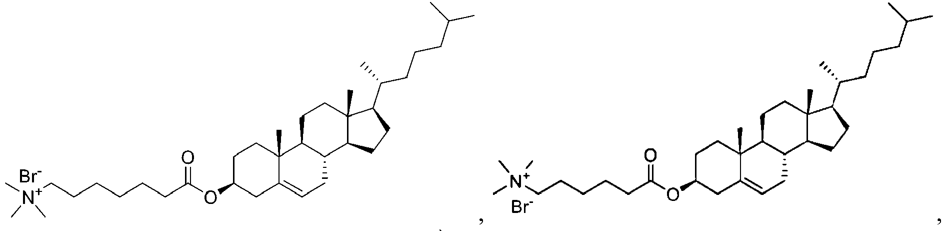 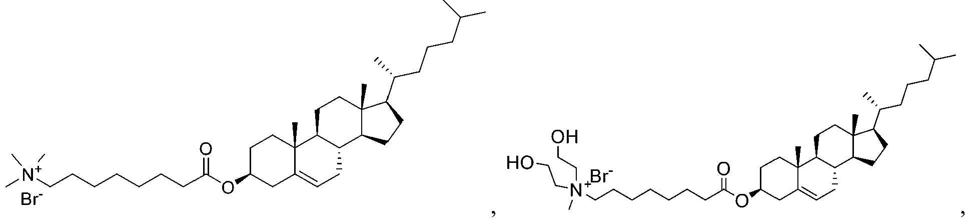 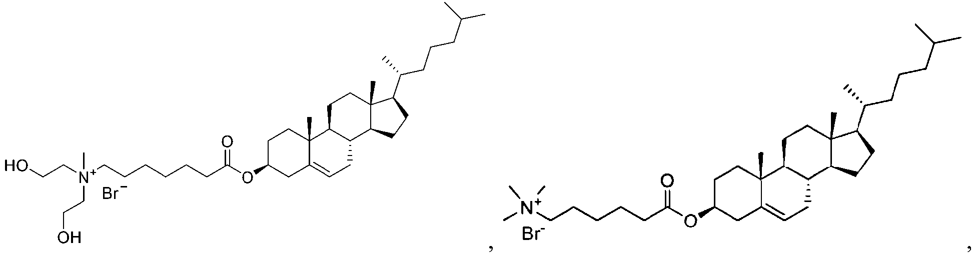 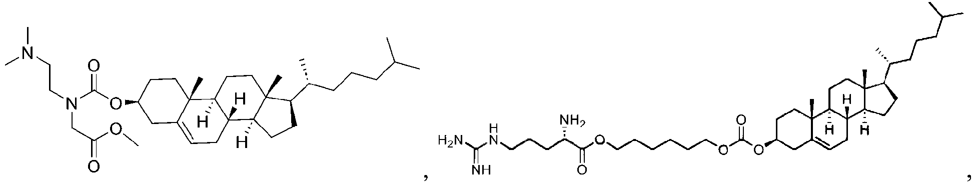 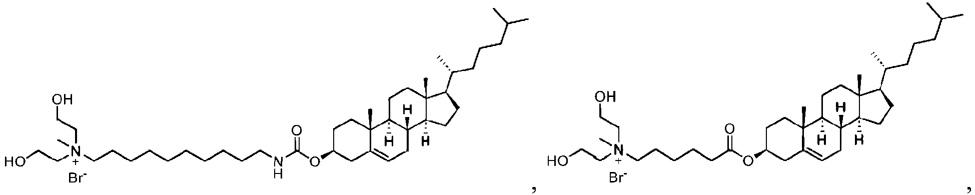 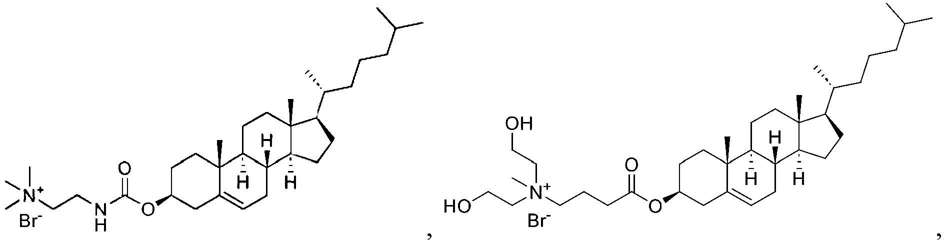 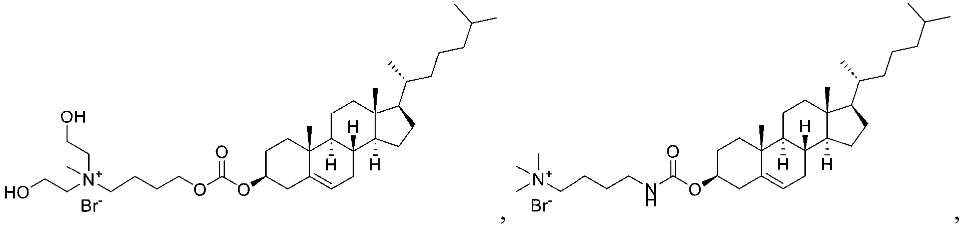 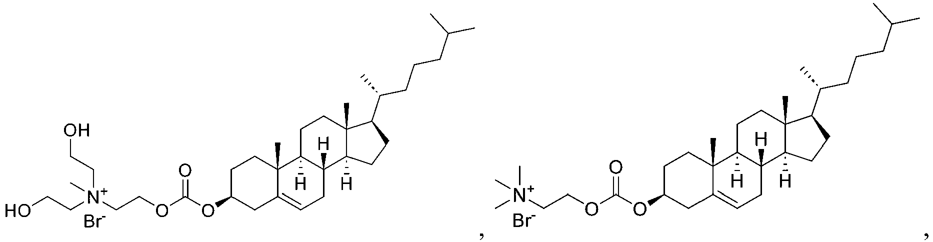 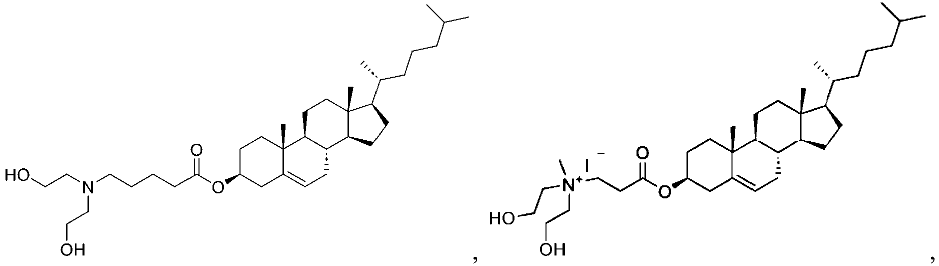 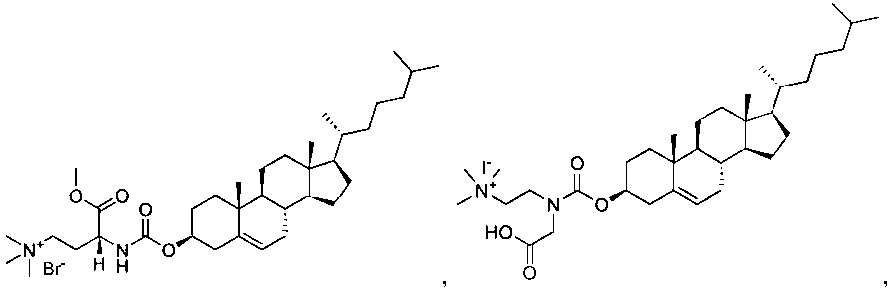 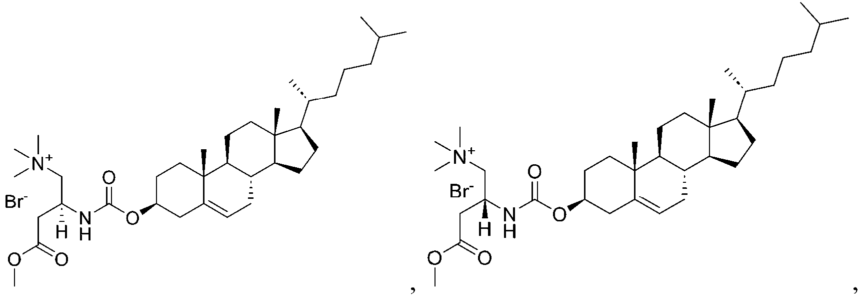 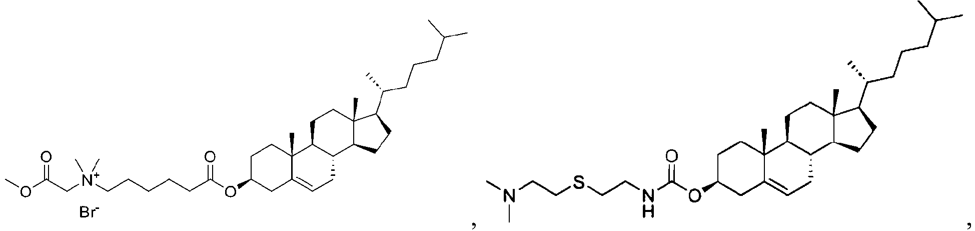 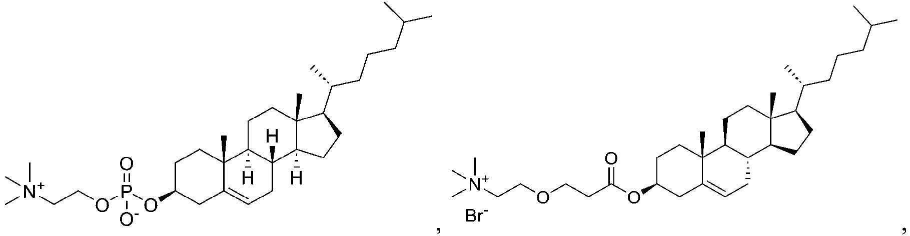 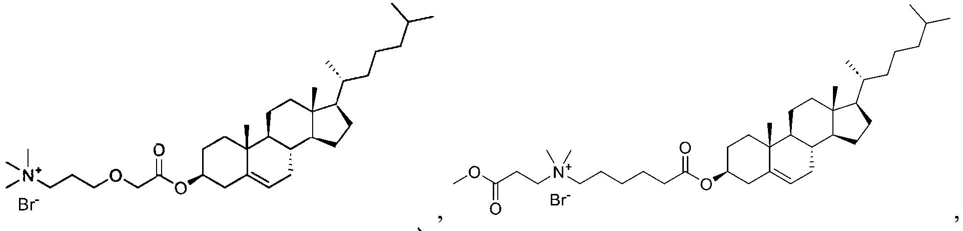 or 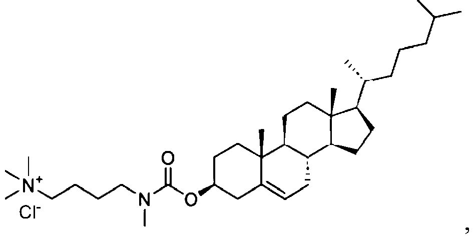 or a stereoisomer, a mixture of stereoisomers, or a pharmaceutically acceptable salt thereof.

In one embodiment, the amount of the steroid compound is from 3 mol % to 15 mol % of the total lipid present in the lipid nanoparticle, preferably 5-10 mol%, such as 3 mol%, 4 mol%, 5 mol%, 6 mol%, 7 mol%, 8 mol%, 9 mol%, 10 mol%, 11 mol%, 12 mol%, 13 mol%, 14 mol% or 15 mol% of the total lipid present in the lipid nanoparticle.

### 5.2.2. Ionizable Lipid

In one embodiment, the lipid nanoparticle further comprises an ionizable lipid. In one embodiment, the ionizable lipid is different from the steroid compound described in Section 0. In one embodiment, the lipid nanoparticle comprises an ionizable lipid, and a steroid compound having a cationic group.

In one embodiment, the ionizable lipid has a pKa of from about 4 to about 14. In one embodiment, the ionizable lipid has a pKa of from about 5 to about 7. In one embodiment, the ionizable lipid has a pKa of from about 6 to about 7. In one embodiment, the ionizable lipid has a pKa of greater than 7. In one embodiment, the ionizable lipid has a pKa of about 4, about 5, about 6, about 7, about 8, about 9, about 10, or about 11. In one embodiment, the ionizable lipid has a pKa of 5.0, 5.1, 5.2, 5.3, 5.4, 5.5, 5.6, 5.7, 5.8, 5.9, 6.0, 6.1, 6.2, 6.3, 6.4, 6.5, 7.0, 7.5, 8.0, 8.5, 9.0, 9.5, 10.0, 10.5, 11.0, or greater than 11. In one embodiment, the ionizable lipid becomes positively charged at physiological pH (i.e. pH 7.4).

In one embodiment, the ionizable lipid comprises one or more groups that is protonated at physiological pH but may deprotonated and has no charge at a pH above 8, above 9, or above 10. In one embodiment, the ionizable lipid comprises one or more tertiary amine groups. In one embodiment, the ionizable lipid comprises one, two, three, or four C₆-C₂₄ alkyl or alkenyl lipid groups. These lipid groups may be attached through a functional group (e.g. ester group or amide group) or may be further added through a Michael addition to a sulfur atom.

In one embodiment, the ionizable lipid comprises at least one ionizable headgroup and at least one biodegradable hydrophobic chain, wherein the ionizable lipid has a logP of at least about 10.1 and a pKa from about 4 to about 11; and the biodegradable hydrophobic chain has a formula of -R₁₆-M₀-R₁₇; wherein R₁₆ is a C₄-₁₄ alkylene, C₄-₁₄ alkenylene, or C₄-₁₄ alkynylene; R₁₇ is a C₆-₂₀ alkyl; and M₀ is a biodegradable group, and wherein the alkylene, alkenylene, alkynylene and alkyl are optionally substituted. In one embodiment, the ionizable head group is a tertiary amine group. In one embodiment, the tertiary amine group of the ionizable lipid comprises a -CH₂CH₂OH moiety.

In one embodiment, the ionizable lipid comprises one tertiary amine group and two biodegradable hydrophobic chains, wherein each hydrophobic chain is independently -R₁₆-C(O)O-R₁₇, and wherein R₁₆ is connected to the tertiary amine group. In one embodiment, one of the R₁₇ is a branched C₆-₂₀ alkyl, and the other R₁₇ is a linear C₆-₂₀ alkyl.

In one embodiment, M₀ is an ester group, an amide group, a thioester group, a carbonate group, a carbamate group, a carbamothioester group, a urea group, or an ether group. In one embodiment, M₀ is an ester group.

In one embodiment, the ionizable lipid is a compound of formula (III): 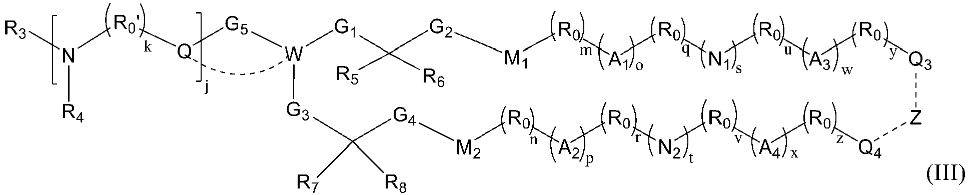 or a stereoisomer, a mixture of stereoisomers, tautomer, isotopologue, or a pharmaceutically acceptable salt thereof, wherein:
each instance of R₃ and R₄ are independently C₁₋₁₀ alkyl, C₂₋₁₀ alkenyl, C₂₋₁₀ alkynyl, C₃-C₁₄ cycloalkyl, 3 to 14-membered heterocyclyl, (C₁₋₁₀ alkylene)-(C₃-C₁₄ cycloalkyl), or (C₁₋₁₀ alkylene)-(3 to 14-membered heterocyclyl), or R₃ and R₄ together with the nitrogen atom they are attached to form a 3 to 14-membered heterocyclyl, or R₄ and one of the R₀' together with the atoms connecting them form a 4 to 10-membered heterocyclyl or 5 to 10-membered heteroaryl, and wherein the alkyl, alkenyl, alkynyl, cycloalkyl, heterocyclyl, heteroaryl, and alkylene are independently optionally substituted with one or more R*;
each instance of R₀' is an independently optionally substituted methylene, or wherein two substituents of a R₀' together with the methylene they are attached to form a 3 to 8-membered cycloalkylene, and wherein the cycloalkylene is optionally substituted with one or more R**;
k is 0, 1, 2, 3, 4, 5, or 6;
j is 0 or 1;
the dashed line connecting Q and W is absent or a bond;
W is C, CH, or N, provided that when W is N, j is 0 and the dashed line connecting Q and W is absent;
G₅ is absent, C₁₋₂₄ alkylene, C₂₋₂₄ alkenylene, C₃-C₈ cycloalkylene, C₃-C₈ cycloalkenylene, wherein the alkylene, alkenylene, cycloalkylene, cycloalkenylene are optionally substituted with one or more R**;
G₁, G₂, G₃ and G₄ are each independently absent, C₁₋₁₃ alkylene, C₂₋₁₃ alkenylene, or C₂₋₁₃ alkynylene, wherein the alkylene, alkenylene, and alkynylene are optionally substituted by one or more R⁵;
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R₅, R₆, R₇ and R₈ are each independently hydrogen or optionally substituted C₁₋₈ alkyl;
when the dashed line connecting Q and W is absent, Q is absent or is selected from -C(O)O-, -O-, -NH-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, - NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, - NR_{b}C(S)O-, -S-S- or -S(O)₀₋₂-, wherein the attachment to the right is to the direction of W;
when the dashed line connecting Q and W is a bond, G₅ is absent, and Q and W form a 5 to 10-membered single ring or fused ring, optionally wherein the ring is substituted with one or more R**;
M₁ and M₂ are each independently absent, or are -C(O)O-, -O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -NRₐ-, -C(O)NRₐ-, -NRₐC(O)-, -NRₐC(O)S-, -SC(O)NRₐ-, -C(O)-, -OC(S)-, -C(S)O-, -OC(S)NRₑ-, -NRₐC(S)O-, -S-S- or -S(O)₀₋₂-;
each instance of R₀ is independently -(CRR')-;
each instance of R and R' are independently H, C₁₋₂₀ alkyl, Lₐ-ORₐ, -Lₐ-SRₐ, or -Lₐ-NRₐR'ₐ, or R and R' together with the carbon they are attached to form a C₃₋₈ cycloalkylene;
Q₃ and Q₄ are each independently H, -(CRR')-, C₆₋₁₀ aryl, or a steroid moiety;
each instance of A₁, A₂, A₃, A₄ are independently -(CR₁₈R₁₈-CR₁₈=CR₁₈)- or -(CR₁₈R₁₈-C=C)-;
each instance of R₁₈ is independently C₁₋₂₀ alkyl;
each instance of N₁ and N₂ are independently a biodegradable group;
Z is absent, C₁₋₁₀ alkylene, or -O-P(O)(OH)-O-;
each of the dashed lines between Z-Q₃ or Z-Q₄ is independently absent or a bond, provided that when Z is absent, both dashed lines are absent;
m, n, q, r, u, v, y, and z are each independently 0, 1, 2, 3, 4, 5, 6, 7, 8, 9 or 10;
o, p, w, and x are each independently 0, 1, or 2;
s and t are each independently 0 or 1;
the number of atoms between G₁ and Q₃ or between G₃ and Q₄ is from 8 to 30;
Lₐ and Lₑ are each independently absent or C₁₋₂₀ alkylene;
L_{b} and L_{f} are each independently absent or C₁₋₁₀ alkylene;
L_{c} is absent or C₁₋₈ alkylene;
L_{d} is absent or C₁₋₁₄ alkylene;
R* is hydroxyl, halogen, C₁₋₁₀ alkyl, C₁₋₁₀ haloalkyl, C₁₋₁₀ hydroxyalkyl, -L_{b}-OR_{b}, -L_{b}-SR_{b}, or -L_{b}-NR_{b}R'_{b};
R** is hydroxyl, halogen, C₁₋₈ alkyl, C₁₋₈ haloalkyl, C₁₋₈ hydroxyalkyl, -L_{b}-OR_{b}, -L_{b}-SR_{b}, or -L_{b}-NR_{b}R'_{b};
R^{s} is C₁₋₁₄ alkyl, -L_{b}-OR_{b}, -L_{b}-SR_{b}, or -L_{b}-NR_{b}R'_{b};
Rₐ and R'ₐ are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
R_{b} and R'_{b} are each independently H, C₁₋₁₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₁₀ alkyl, -L_{f}-OR_{f}, -L_{f}-SR_{f}, or -L_{f}-NR_{f}R'_{f},
R_{c} and R'ₑ are each independently hydrogen or C₁₋₈ alkyl;
R_{d} and R'_{d} are each independently hydrogen or C₁₋₁₄ alkyl;
Rₑ and R'ₑ are each independently hydrogen or C₁₋₂₀ alkyl; and
R_{f} and R'_{f} are each independently hydrogen or C₁₋₁₀ alkyl.

In one embodiment, m is 0. In one embodiment, m is 1. In one embodiment, m is 2. In one embodiment, m is 3. In one embodiment, m is 4. In one embodiment, m is 5. In one embodiment, m is 6. In one embodiment, m is 7. In one embodiment, m is 8. In one embodiment, m is 9. In one embodiment, m is 10.

In one embodiment, n is 0. In one embodiment, n is 1. In one embodiment, n is 2. In one embodiment, n is 3. In one embodiment, n is 4. In one embodiment, n is 5. In one embodiment, n is 6. In one embodiment, n is 7. In one embodiment, n is 8. In one embodiment, n is 9. In one embodiment, n is 10.

In one embodiment, q is 0. In one embodiment, q is 1. In one embodiment, q is 2. In one embodiment, q is 3. In one embodiment, q is 4. In one embodiment, q is 5. In one embodiment, q is 6. In one embodiment, q is 7. In one embodiment, q is 8. In one embodiment, q is 9. In one embodiment, q is 10.

In one embodiment, r is 0. In one embodiment, r is 1. In one embodiment, r is 2. In one embodiment, r is 3. In one embodiment, r is 4. In one embodiment, r is 5. In one embodiment, r is 6. In one embodiment, r is 7. In one embodiment, r is 8. In one embodiment, r is 9. In one embodiment, r is 10.

In one embodiment, u is 0. In one embodiment, u is 1. In one embodiment, u is 2. In one embodiment, u is 3. In one embodiment, u is 4. In one embodiment, u is 5. In one embodiment, u is 6. In one embodiment, u is 7. In one embodiment, u is 8. In one embodiment, u is 9. In one embodiment, u is 10.

In one embodiment, v is 0. In one embodiment, v is 1. In one embodiment, v is 2. In one embodiment, v is 3. In one embodiment, v is 4. In one embodiment, v is 5. In one embodiment, v is 6. In one embodiment, v is 7. In one embodiment, v is 8. In one embodiment, v is 9. In one embodiment, v is 10.

In one embodiment, y is 0. In one embodiment, y is 1. In one embodiment, y is 2. In one embodiment, y is 3. In one embodiment, y is 4. In one embodiment, y is 5. In one embodiment, y is 6. In one embodiment, y is 7. In one embodiment, y is 8. In one embodiment, y is 9. In one embodiment, y is 10.

In one embodiment, z is 0. In one embodiment, z is 1. In one embodiment, z is 2. In one embodiment, z is 3. In one embodiment, z is 4. In one embodiment, z is 5. In one embodiment, z is 6. In one embodiment, z is 7. In one embodiment, z is 8. In one embodiment, z is 9. In one embodiment, z is 10.

In one embodiment, m+q+u+y is an integer from 3 to 25. In one embodiment, m+q+u+y is an integer from 5 to 20. In one embodiment, m+q+u+y is an integer from 5 to 20. In one embodiment, m+q+u+y is an integer from 5 to 20. In one embodiment, m+q+u+y is an integer from 5 to 18. In one embodiment, m+q+u+y is an integer from 6 to 12. In one embodiment, m+q+u+y is 5, 6, 7, 8, 9, 10, 11, 12, 13, 14, 15, 16, 17, or 18.

In one embodiment, n+r+v+z is an integer from 3 to 25. In one embodiment, n+r+v+z is an integer from 5 to 20. In one embodiment, n+r+v+z is an integer from 5 to 20. In one embodiment, n+r+v+z is an integer from 5 to 20. In one embodiment, n+r+v+z is an integer from 5 to 18. In one embodiment, n+r+v+z is an integer from 6 to 12. In one embodiment, n+r+v+z is 5, 6, 7, 8, 9, 10, 11, 12, 13, 14, 15, 16, 17, or 18.

In one embodiment, o is 0. In one embodiment, o is 1. In one embodiment, o is 2.

In one embodiment, p is 0. In one embodiment, p is 1. In one embodiment, p is 2.

In one embodiment, w is 0. In one embodiment, w is 1. In one embodiment, w is 2.

In one embodiment, x is 0. In one embodiment, x is 1. In one embodiment, x is 2.

In one embodiment, o+p is 0. In one embodiment, o+p is 1. In one embodiment, o+p is 2. In one embodiment, w+x is 0. In one embodiment, w+x is 1. In one embodiment, w+x is 2. In one embodiment, o+p+w+x is 0. In one embodiment, o+p+w+x is 1. In one embodiment, o+p+w+x is 2. In one embodiment, o+p+w+x is 3. In one embodiment, o+p+w+x is 4.

In one embodiment, R₀ is CH₂. In one embodiment, R₀ is CH(C₁₋₁₆ alkyl). In one embodiment, R₀ is CH(C₁₋₁₄ alkyl). In one embodiment, R₀ is CH(C₁₋₁₂ alkyl). In one embodiment, R₀ is CH(C₁₋₆ alkyl). In one embodiment, R₀ is CHCH₃. In one embodiment, R₀ is C(CH₃)₂. In one embodiment, R₀ is C(C₁₋₁₂ alkyl)₂. In one embodiment, R₀ is CH(Lₐ-ORₐ). In one embodiment, R₀ is CH(Lₐ-SRₐ). In one embodiment, R₀ is CH(Lₐ-NRₐR'ₐ). In one embodiment, R₀ is CH(ORₐ). In one embodiment, R₀ is CH(SRₐ). In one embodiment, R₀ is CH(NRₐR'ₐ). In one embodiment, R₀ is CHN(C₁₋₁₂ alkyl)₂. In one embodiment, R₀ is CH(CH₂)₁₋₆NRₐR'ₐ. In one embodiment, R₀ is CH(CH₂)₁₋₆N(C₁₋₁₂ alkyl)₂.

In one embodiment, Q₃ is H. In one embodiment, Q₃ is -(CRR')-. In one embodiment, Q₃ is -(CH₂)-. In one embodiment, Q₄ is H. In one embodiment, Q₄ is -(CRR')-. In one embodiment, Q₄ is -(CH₂)-.

In one embodiment A₁ is absent. In one embodiment A₁ is -(CR₁₈R₁₈-CR₁₈=CR₁₈)-. In one embodiment A₁ is -(CHR₁₈-CH=CH)-. In one embodiment A₁ is -(CH₂-CH=CH)-. In one embodiment, the C=C bond in A₁ is in Z-configuration. In one embodiment, the C=C bond in A₁ is in E-configuration. In one embodiment A₁ is -(CR₁₈R₁₈-C≡C)-. In one embodiment A₁ is -(CHR₁₈-C≡C)-. In one embodiment A₁ is -(CH₂-C≡C)-.

In one embodiment A₂ is absent. In one embodiment A₂ is -(CR₁₈R₁₈-CR₁₈=CR₁₈)-. In one embodiment A₂ is -(CHR₁₈-CH=CH)-. In one embodiment A₂ is -(CH₂-CH=CH)-. In one embodiment, the C=C bond in A₂ is in Z-configuration. In one embodiment, the C=C bond in A₂ is in E-configuration. In one embodiment A₂ is -(CR₁₈R₁₈-C≡C)-. In one embodiment A₂ is -(CHR₁₈-C=C)-. In one embodiment A₂ is -(CH₂-C≡C)-.

In one embodiment A₃ is absent. In one embodiment A₃ is -(CR₁₈R₁₈-CR₁₈=CR₁₈)-. In one embodiment A₃ is -(CHR₁₈-CH=CH)-. In one embodiment A₃ is -(CH₂-CH=CH)-. In one embodiment, the C=C bond in A₃ is in Z-configuration. In one embodiment, the C=C bond in A₃ is in E-configuration. In one embodiment A₃ is -(CR₁₈R₁₈-C≡C)-. In one embodiment A₃ is -(CHR₁₈-C=C)-. In one embodiment A₃ is -(CH₂-C≡C)-.

In one embodiment A₄ is absent. In one embodiment A₄ is -(CR₁₈R₁₈-CR₁₈=CR₁₈)-. In one embodiment A₄ is -(CHR₁₈-CH=CH)-. In one embodiment A₄ is -(CH₂-CH=CH)-. In one embodiment, the C=C bond in A₄ is in Z-configuration. In one embodiment, the C=C bond in A₄ is in E-configuration. In one embodiment A₄ is -(CR₁₈R₁₈-C≡C)-. In one embodiment A₄ is -(CHR₁₈-C=C)-. In one embodiment A₄ is -(CH₂-C≡C)-.

In one embodiment, R₁₈ is H. In one embodiment, R₁₈ is C₁₋₂₀ alkyl. In one embodiment, R₁₈ is C₁₋₁₆ alkyl. In one embodiment, R₁₈ is C₁₋₁₂ alkyl. In one embodiment, R₁₈ is C₁₋₆ alkyl.

In one embodiment, s is 0. In one embodiment, s is 1. In one embodiment, t is 0. In one embodiment, t is 1.

In one embodiment, N₁ is absent. In one embodiment, N₁ is a biodegradable group. In one embodiment, N₁ is an ester group. In one embodiment, N₁ is an amide group. In one embodiment, N₁ is a thioester group. In one embodiment, N₁ is a carbonate group. In one embodiment, N₁ is a carbamate group. In one embodiment, N₁ is a carbamothioester group. In one embodiment, N₁ is a urea group. In one embodiment, N₁ is an imine group. In one embodiment, N₁ is an oxime group. In one embodiment, N₁ is a disulfide group.

In one embodiment, N₂ is absent. In one embodiment, N₂ is a biodegradable group. In one embodiment, N₂ is an ester group. In one embodiment, N₂ is an amide group. In one embodiment, N₂ is a thioester group. In one embodiment, N₂ is a carbonate group. In one embodiment, N₂ is a carbamate group. In one embodiment, N₂ is a carbamothioester group. In one embodiment, N₂ is a urea group. In one embodiment, N₂ is an imine group. In one embodiment, N₂ is an oxime group. In one embodiment, N₂ is a disulfide group.

In one embodiment, Z is absent. In one embodiment, Z is absent, and the dashed line between Q₄ and Z and the dashed line between Q₃ and Z are both absent. In one embodiment, Z is absent, and Q₄ and Q₃ are both H.

In one embodiment, Z is C₁₋₁₀ alkylene. In one embodiment, Z is C₁₋₆ alkylene. In one embodiment, Z is C₁₋₃ alkylene. In one embodiment, Z is -O-P(O)(OH)-O-.

In one embodiment, Z is absent, and the number of carbon atoms between G₁ and Q₃ is from 8 to 30. In one embodiment, Z is absent, and the number of carbon atoms between G₁ and Q₃ is from 10 to 25. In one embodiment, Z is absent, and the number of carbon atoms between G₁ and Q₃ is from 10 to 20.

In one embodiment, Z is absent, and the number of carbon atoms between G₃ and Q₄ is from 8 to 30. In one embodiment, Z is absent, and the number of carbon atoms between G₃ and Q₄ is from 10 to 25. In one embodiment, Z is absent, and the number of carbon atoms between G₃ and Q₄ is from 10 to 20.

In one embodiment, the ionizable lipid is a compound of formula (IV'): 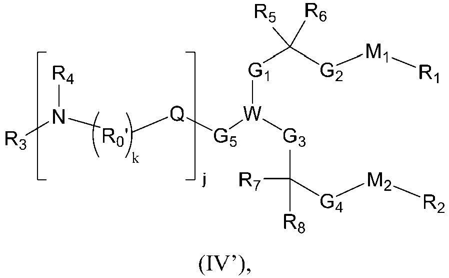 or a stereoisomer, a mixture of stereoisomers, or a pharmaceutically acceptable salt thereof, wherein
j is 0 or 1;
W is CH or N, provided that when W is N, j is 0;
k is 0, 1, 2, 3, 4, 5, 6, 7, or 8;
each instance of R₀' is an independently optionally substituted methylene, or wherein two substituents of a R₀' together with the methylene they are attached to form a 3 to 8-membered cycloalkylene, and wherein the cycloalkylene is optionally substituted with one or more R**;
M₁ and M₂ are each independently -C(O)O-, -O-, -SC(O)O-, -OC(O)NRₐ-, -NRₐC(O)NRₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, or -S(O)₀₋₂-;
Q is absent, -C(O)O-, -O-, -NH-, -SC(O)O-, -OC(O)NR_{b}-, -NR_{b}C(O)NR_{b}-, -OC(O)S-, -OC(O)O-, - NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S-, or -S(O)₀₋₂-;
Rₐ and R_{b} are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
G₅ is absent or optionally substituted C₁₋₈ alkylene;
R₁ and R₂ are each independently C₄₋₂₀ alkyl, C₄₋₂₀ alkenyl or C₄₋₂₀ alkynyl, wherein one or more methylene units in R₁ and R₂ are independently optionally replaced by -NH- or -N(C₁₋₂₀ alkyl), wherein the alkyl, alkenyl and alkynyl are optionally substituted;
each instance of R₃ and R₄ are independently H, C₁₋₁₀ alkyl, C₂₋₁₀ alkenyl, C₂₋₁₀ alkynyl, C₃-C₁₄ cycloalkyl, or 3- to 14-membered heterocyclyl, or R₃ and R₄ together with the N atom to which they are attached to form 3 to 14-membered heterocyclyl, or R₄ and one of the R₀' together with the atoms connecting them form a 4 to 10-membered heterocyclyl; and wherein the alkyl, alkenyl, alkynyl, cycloalkyl, heterocyclyl are optionally substituted with one or more R*; and
R₅, R₆, R₇ and R₈ are each independently hydrogen or optionally substituted C₁₋₈ alkyl.

In one embodiment, the ionizable lipid is a compound of formula (IV'-A): 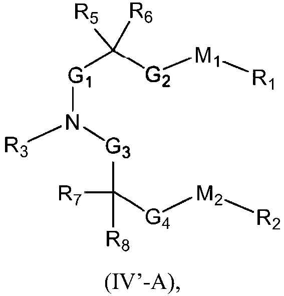 or a stereoisomer, a mixture of stereoisomers, or a pharmaceutically acceptable salt thereof.

In one embodiment, the ionizable lipid is a compound of formula (VIII): 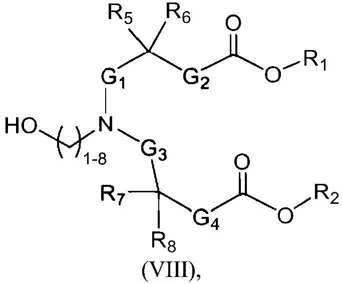 or a stereoisomer, a mixture of stereoisomers, or a pharmaceutically acceptable salt thereof.

In one embodiment, the ionizable lipid is a compound of formula (V'), (VI') or (VII'): 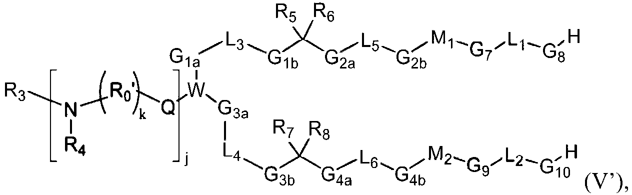 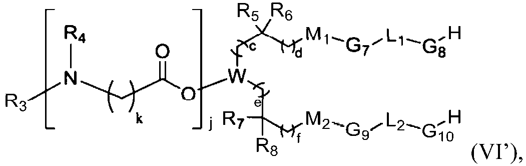 or 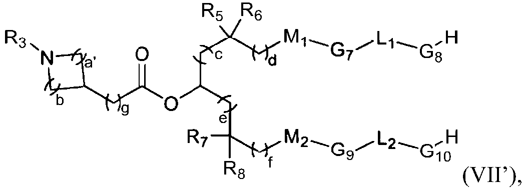 or a stereoisomer, a mixture of stereoisomers, or a pharmaceutically acceptable salt thereof, wherein
k is 0, 1, 2, 3, 4, or 5;
L₁ and L₂ are each independently -(CRR')₂-, -CH=CH-, -C≡C- or -NR"-;
one of L₃ and L₅ is -(CR^{s}R^{s}')₂-, -CH=CH-, or -C≡C-, and the other is absent;
one of L₄ and L₆ is -(CR^{s}R^{s}')₂-, -CH=CH-, or -C≡C-, and the other is absent;
each of G₁ₐ, G_{1b}, G₂ₐ, G_{2b}, G₃ₐ, G_{3b}, G₄ₐ and G_{4b} is independently absent or optionally substituted C₁₋₇ alkylene;
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms;
R₅, R₆, R₇ and R₈ are each independently hydrogen or optionally substituted C₁₋₆ alkyl;
G₇, G₈, G₉ and G₁₀ are each independently absent or optionally substituted C₁₋₁₂ alkylene, provided that G₇ and G₈ have a total length of 4, 5, 6, 7, 8, 9, 10, 11 or 12 carbon atoms, and G₉ and G₁₀ have a total length of 4, 5, 6, 7, 8, 9, 10, 11 or 12 carbon atoms;
R^{s} and R^{s}' are each independently H, C₁₋₁₀ alkyl, -L_{d}-OR_{d} or -L_{d}-NR_{d}R'_{d};
R' is H, C₁₋₁₄ alkyl, -L_{d}-OR_{d} or -Lₐ-NRₐR'ₐ;
R" is H or C₁₋₁₄ alkyl;
L_{d} is absent or C₁₋₁₀ alkylene;
Lₐ is absent or C₁₋₁₄ alkylene;
Rₐ and R'ₐ are each independently H, C₁₋₁₄ alkyl, C₃₋₁₀ cycloalkyl or 3- to 10-membered heterocyclyl, and wherein the alkyl, cycloalkyl and heterocyclyl are optionally substituted;
R_{d} and R'_{d} are each independently H or C₁₋₁₀ alkyl;
a' and b are each independently 0, 1, 3, 4, 5, provided that at least one of a' and b is not 0;
g is 0, 1, 2, 3, 4, or 5;
a'+g is 0, 1, 2, 3, 4, or 5; and
c, d, e, and f are each independently 0, 1, 2, 3, 4, 5, 6, or 7, provided that c+d is an integer between 2 and 9, and e+f is an integer between 2 and 9.

In one embodiment, R₁ is C₄₋₂₀ alkyl. In one embodiment, R₁ is C₆₋₁₈ alkyl. In one embodiment, R₁ is C₈₋₁₈ alkyl. In one embodiment, R₁ is C₈, C₉, C₁₀, C₁₁, C₁₂, C₁₃, C₁₄, C₁₅, C₁₆, C₁₇, C₁₈, C₁₉, or C₂₀ alkyl. In one embodiment, R₁ is C₄₋₂₀ alkenyl. In one embodiment, R₁ is C₆₋₁₈ alkenyl. In one embodiment, R₁ is C₈₋₁₈ alkenyl. In one embodiment, R₁ is C₈, C₉, C₁₀, C₁₁, C₁₂, C₁₃, C₁₄, C₁₅, C₁₆, C₁₇, C₁₈, C₁₉, or C₂₀ alkenyl. In one embodiment, R₁ is C₄₋₂₀ alkynyl. In one embodiment, R₁ is C₆₋₁₈ alkynyl. In one embodiment, R₁ is C₈₋₁₈ alkynyl. In one embodiment, R₁ is C₈, C₉, C₁₀, C₁₁, C₁₂, C₁₃, C₁₄, C₁₅, C₁₆, C₁₇, C₁₈, C₁₉, or C₂₀ alkynyl.

In one embodiment, one or more -CH₂- group in R₁ is replaced by -NH-. In one embodiment, one or more -CH₂- group in R₁ is replaced by -N(C₁₋₂₀ alkyl)-. In one embodiment, one or more -CH₂- group in R₁ is replaced by -N(C₁₋₁₂ alkyl)-.

In one embodiment, R₁ is unsubstituted. In one embodiment, R₁ is substituted with -Lₐ-ORₐ. In one embodiment, R₁ is substituted with -Lₐ-SRₐ. In one embodiment, R₁ is substituted with -Lₐ-NRₐR'ₐ.

In one embodiment, R₂ is C₄₋₂₀ alkyl. In one embodiment, R₂ is C₆₋₁₈ alkyl. In one embodiment, R₂ is C₈₋₁₈ alkyl. In one embodiment, R₂ is C₈, C₉, C₁₀, C₁₁, C₁₂, C₁₃, C₁₄, C₁₅, C₁₆, C₁₇, C₁₈, C₁₉, or C₂₀ alkyl. In one embodiment, R₂ is C₄₋₂₀ alkenyl. In one embodiment, R₂ is C₆₋₁₈ alkenyl. In one embodiment, R₂ is C₈₋₁₈ alkenyl. In one embodiment, R₂ is C₈, C₉, C₁₀, C₁₁, C₁₂, C₁₃, C₁₄, C₁₅, C₁₆, C₁₇, C₁₈, C₁₉, or C₂₀ alkenyl. In one embodiment, R₂ is C₄₋₂₀ alkynyl. In one embodiment, R₂ is C₆₋₁₈ alkynyl. In one embodiment, R₂ is C₈₋₁₈ alkynyl. In one embodiment, R₂ is C₈, C₉, C₁₀, C₁₁, C₁₂, C₁₃, C₁₄, C₁₅, C₁₆, C₁₇, C₁₈, C₁₉, or C₂₀ alkynyl.

In one embodiment, one or more -CH₂- group in R₂ replaced by -NH-. In one embodiment, one or more -CH₂- group in R₂ is replaced by -N(C₁₋₂₀ alkyl)-. In one embodiment, one or more -CH₂- group in R₂ is replaced by -N(C₁₋₁₂ alkyl)-.

In one embodiment, R₂ is unsubstituted. In one embodiment, R₂ is substituted with -Lₐ-ORₐ. In one embodiment, R₂ is substituted with -Lₐ-SRₐ. In one embodiment, R₂ is substituted with -Lₐ-NRₐR'ₐ.

In one embodiment, Lₐ is absent. In one embodiment, Lₐ is C₁₋₁₄ alkylene. In one embodiment, Lₐ is C₁₋₆ alkylene. In one embodiment, Lₐ is methylene. In one embodiment, Lₐ is ethylene.

In one embodiment, Rₐ is C₁₋₁₄ alkyl. In one embodiment, Rₐ is C₃₋₁₀ cycloalkyl. In one embodiment, Rₐ is 3- to 10-membered heterocyclyl. In one embodiment, Rₐ is C₁₋₁₀ alkyl; In one embodiment, Rₐ is C₈₋₁₀ alkyl; In one embodiment, Rₐ is C₈₋₁₀ linear alkyl; In one embodiment, Rₐ is -(CH₂)₈CH₃; In one embodiment, Rₐ is optionally substituted with one or more of the following substituents: H, C₁₋₂₀ alkyl, -Lₑ-ORₑ, -Lₑ-SRₑ and -Lₑ-NRₑR'ₑ.

In one embodiment, R'ₐ is C₁₋₁₄ alkyl. In one embodiment, R'ₐ is C₃₋₁₀ cycloalkyl. In one embodiment, R'ₐ is 3- to 10-membered. In one embodiment, R'ₐ is C₈₋₁₀ alkyl; In one embodiment, R'ₐ is C₈₋₁₀ linear alkyl; In one embodiment, R'ₐ is -(CH₂)₈CH₃; In one embodiment, R'ₐ is optionally substituted with one or more of the following substituents: H, C₁₋₂₀ alkyl, -Lₑ-ORₑ, -Lₑ-SRₑ and -Lₑ-NRₑR'ₑ.

In one embodiment, Rₐ and R'ₐ together with the nitrogen they are attached to form a 4 to 10-membered ring. In one embodiment, Rₐ and R'ₐ together with the nitrogen they are attached to form a 4 to 8-membered ring. In one embodiment, Rₐ and R'ₐ together with the nitrogen they are attached to form a 4 to 6-membered ring. In one embodiment, Rₐ and R'ₐ together with the nitrogen they are attached to form a 4 to 6-membered cycloalkyl. In one embodiment, Rₐ and R'ₐ together with the nitrogen they are attached to form a 4 to 6-membered heterocyclyl.

In one embodiment, R₃ is H. In one embodiment, R₃ is C₁₋₁₀ alkyl. In one embodiment, R₃ is C₁₋₁₀ haloalkyl. In one embodiment, R₃ is C₂₋₁₀ alkenyl. In one embodiment, R₃ is C₂₋₁₀ alkynyl. In one embodiment, R₃ is 3- to 14-membered cycloalkyl. In one embodiment, R₃ is 3- to 14-membered heterocyclyl. In one embodiment, R₃ is C₆₋₁₀ alkyl. In one embodiment, R₃ is 5 to 14-membered heteroaryl. In one embodiment, R₃ is C₁₋₆ alkyl. In one embodiment, R₃ is C₁₋₆ haloalkyl. In one embodiment, R₃ is 3-to 10-membered cycloalkyl. In one embodiment, R₃ is 3- to 10-membered heterocyclyl. In one embodiment, R₃ is 3- to 7-membered cycloalkyl. In one embodiment, R₃ is 3- to 7-membered heterocyclyl.

In one embodiment, R₃ is unsubstituted. In one embodiment, R₃ is substituted with one or more R*, wherein each R* is independently halogen, cyano, C₁₋₁₀ alkyl, C₁₋₁₀ haloalkyl, -L_{b}-OR_{b}, -L_{b}-SR_{b} or -L_{b}-NR_{b}R'_{b}. In one embodiment, R₃ is optionally substituted with 1, 2, 3, 4 or 5 R*, wherein each R* is independently halogen, cyano, C₁₋₁₀ alkyl, C₁₋₁₀ haloalkyl, -L_{b}-OR_{b}, -L_{b}-SR_{b} or -L_{b}-NR_{b}R'_{b}. In one embodiment, R₃ is Me. In one embodiment, R₃ is -CH₂CH₃. In one embodiment, R₃ is -CH(CH₃)₂.

In one embodiment, R₃ is C₁₋₆ alkyl optionally substituted with OH. In one embodiment, R₃ is - CH₂CH₂OH.

In one embodiment, R₄ is H. In one embodiment, R₄ is C₁₋₁₀ alkyl. In one embodiment, R₄ is C₁₋₁₀ haloalkyl. In one embodiment, R₄ is C₂₋₁₀ alkenyl. In one embodiment, R₄ is C₂₋₁₀ alkynyl. In one embodiment, R₄ is 3- to 14-membered cycloalkyl. In one embodiment, R₄ is 3- to 14-membered heterocyclyl. In one embodiment, R₄ is C₆₋₁₀ alkyl. In one embodiment, R₄ is 5 to 14-membered heteroaryl. In one embodiment, R₄ is C₁₋₆ alkyl. In one embodiment, R₄ is C₁₋₆ haloalkyl. In one embodiment, R₄ is 3-to 10-membered cycloalkyl. In one embodiment, R₄ is 3- to 10-membered heterocyclyl. In one embodiment, R₄ is 3- to 7-membered cycloalkyl. In one embodiment, R₄ is 3- to 7-membered heterocyclyl. In one embodiment, R₄ is Me. In one embodiment, R₃ is unsubstituted. In one embodiment, R₃ is substituted with one or more R*. In one embodiment, R₃ is optionally substituted with 1, 2, 3, 4 or 5 R*.

In one embodiment, R₃ and R₄ together with the N atom to which they are attached to form 3 to 14-membered heterocyclyl. In one embodiment, R₃, R₄ together with the N atom to which they are attached to form 3- to 10-membered heterocyclyl; In one embodiment, R₃, R₄ together with the N atom to which they are attached to form 3- to 7-membered heterocyclyl; In one embodiment, R₃, R₄ together with the N atom to which they are attached to form 5- to 7-membered heterocyclyl; In one embodiment, R₃, R₄ together with the N atom to which they are attached to form 4- to 6-membered heterocyclyl; In one embodiment, R₃, R₄ together with the N atom to which they are attached to form 5-membered heterocyclyl; In one embodiment, R₃, R₄ together with the N atom to which they are attached to form 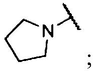 In one embodiment, R₃, R₄ together with the N atom to which they are attached to form 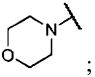 In one embodiment, R₃, R₄ together with the N atom to which they are attached to form 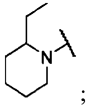 In one embodiment, R₃, R₄ together with the N atom to which they are attached to form 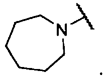 In one embodiment, the heterocyclyl formed by R₃ and R₄ together with the N atom to which they are attached is optionally substituted with one or more R*; In one embodiment, the heterocyclyl formed by R₃ and R₄ taken together with the N atom to which they are attached is optionally substituted with 1, 2, 3, 4 or 5 R*.

In one embodiment, R₄ together with the nitrogen atom to which it is attached to and one of the R₀' form a 3 to 14-membered heterocyclyl. In one embodiment, R₄ together with the nitrogen atom to which it is attached to and one of the R₀' form a 4 to 10-membered heterocyclyl. In one embodiment, R₄ together with the nitrogen atom to which it is attached to and one of the R₀' form a 4 to 6-membered heterocyclyl. In one embodiment, R₄ together with the nitrogen atom to which it is attached to and one of the R₀' form a 5 to 14-membered heteroaryl. In one embodiment, R₄ together with the nitrogen atom to which it is attached to and one of the R₀' form a 5 to 10-membered heteroaryl. In one embodiment, R₄ together with the nitrogen atom to which it is attached to and one of the R₀' form a 5 or 6-membered heteroaryl.

In one embodiment, R₅ is H; In one embodiment, R₅ is C₁₋₈ alkyl; In one embodiment, R₅ is C₁₋₆ alkyl; In one embodiment, R₅ is C₁₋₃ alkyl; In one embodiment, R₅ is Me; In one embodiment, R₅ is optionally substituted with one or more R*; In one embodiment, R₅ is optionally substituted with 1, 2, 3, 4 or 5 R*; In one embodiment, R₅ is optionally substituted with one or more R₄ₛ. In one embodiment, R₅ is optionally substituted with 1, 2, 3, 4, or 5 R₄ₛ.

In one embodiment, R₆ is H; In one embodiment, R₆ is C₁₋₈ alkyl; In one embodiment, R₆ is C₁₋₆ alkyl; In one embodiment, R₆ is C₁₋₃ alkyl; In one embodiment, R₆ is Me; In one embodiment, R₆ is optionally substituted with one or more R*; In one embodiment, R₆ is optionally substituted with 1, 2, 3, 4 or 5 R*.

In one embodiment, R₅ and R₆ together with the carbon atom they are attached to form a C₃₋₁₄ cycloalkylene or 3- to 14-membered heterocyclylene, wherein the cycloalkylene and heterocyclylene are optionally substituted by one or more R₄ₛ. In one embodiment, R₅ and R₆ together with the carbon atom they are attached to form a C₃₋₈ cycloalkylene. In one embodiment, R₅ and R₆ together with the carbon atom they are attached to form a C₃₋₆ cycloalkylene. In one embodiment, R₅ and R₆ together with the carbon atom they are attached to form a C₃ cycloalkylene, C₄ cycloalkylene, C₅ cycloalkylene, or C₆ cycloalkylene. In one embodiment, R₅ and R₆ together with the carbon atom they are attached to form a 3- to 8-membered heterocyclylene. In one embodiment, R₅ and R₆ together with the carbon atom they are attached to form a 3-to 6-membered heterocyclylene. In one embodiment, R₅ and R₆ together with the carbon atom they are attached to form a 3-membered heterocyclylene, 4-membered heterocyclylene, 5-membered heterocyclylene, or 6-membered heterocyclylene. In one embodiment, the heterocyclylene has one or more oxygen, sulfur, or nitrogen atoms on the ring. In one embodiment, the heterocyclylene or cycloalkylene formed by R₅ and R₆ is unsubstituted. In one embodiment, the heterocyclylene or cycloalkylene formed by R₅ and R₆ is substituted with 1, 2, 3, 4 or 5 R*. In one embodiment, the heterocyclylene or cycloalkylene formed by R₅ and R₆ is substituted with 1, 2, 3, 4 or 5 R₄ₛ.

In one embodiment, R₇ is H; In one embodiment, R₇ is C₁₋₈ alkyl; In one embodiment, R₇ is C₁₋₆ alkyl; In one embodiment, R₇ is C₁₋₃ alkyl; In one embodiment, R₇ is Me; In one embodiment, R₇ is optionally substituted with one or more R*; In one embodiment, R₇ is optionally substituted with 1, 2, 3, 4 or 5 R*.

In one embodiment, R₅, R₆, R₇ and R₈ are each independently hydrogen or methyl. In one embodiment, R₅ and R₆ are each independently C₁₋₆ alkyl, and R₇ and R₈ are both hydrogen. In one embodiment, R₅ and R₆ are both methyl, and R₇ and R₈ are both hydrogen. In one embodiment, R₅, R₆, R₇, R₈ are all hydrogen.

In one embodiment, R₈ is H; In one embodiment, R₈ is C₁₋₈ alkyl; In one embodiment, R₈ is C₁₋₆ alkyl; In one embodiment, R₈ is C₁₋₃ alkyl; In one embodiment, R₈ is Me; In one embodiment, R₈ is optionally substituted with one or more R*; In one embodiment, R₈ is optionally substituted with 1, 2, 3, 4 or 5 R*.

In one embodiment, R₇ and R₈ together with the carbon atom they are attached to form a C₃₋₁₄ cycloalkylene or 3- to 14-membered heterocyclylene, wherein the cycloalkylene and heterocyclylene are optionally substituted by one or more R₄ₛ. In one embodiment, R₇ and R₈ together with the carbon atom they are attached to form a C₃₋₈ cycloalkylene. In one embodiment, R₇ and R₈ together with the carbon atom they are attached to form a C₃₋₆ cycloalkylene. In one embodiment, R₇ and R₈ together with the carbon atom they are attached to form a C₃ cycloalkylene, C₄ cycloalkylene, C₅ cycloalkylene, or C₆ cycloalkylene. In one embodiment, R₇ and R₈ together with the carbon atom they are attached to form a 3- to 8-membered heterocyclylene. In one embodiment, R₇ and R₈ together with the carbon atom they are attached to form a 3-to 6-membered heterocyclylene. In one embodiment, R₇ and R₈ together with the carbon atom they are attached to form a 3-membered heterocyclylene, 4-membered heterocyclylene, 5-membered heterocyclylene, or 6-membered heterocyclylene. In one embodiment, the heterocyclylene has one or more oxygen, sulfur, or nitrogen atoms on the ring. In one embodiment, the heterocyclylene or cycloalkylene formed by R₇ and R₈ is unsubstituted. In one embodiment, the heterocyclylene or cycloalkylene formed by R₇ and R₈ is substituted with 1, 2, 3, 4 or 5 R*. In one embodiment, the heterocyclylene or cycloalkylene formed by R₇ and R₈ is substituted with 1, 2, 3, 4 or 5 R₄ₛ.

In one embodiment, R* is halogen, cyano, C₁₋₆ alkyl, C₁₋₆ haloalkyl, -L_{b}-OR_{b} or -L_{b}-NR_{b}R'_{b}; In one embodiment, R* is C₁₋₆ alkyl, C₁₋₆ haloalkyl or -OR_{b}; In one embodiment, R* is independently H, halogen, C₁₋₆ alkyl or C₁₋₆ haloalkyl; In one embodiment, R* is C₁₋₆ alkyl or C₁₋₆ haloalkyl; In one embodiment, R* is Me. In one embodiment, R* is OH. In one embodiment, R* is halogen; In one embodiment, R* is cyano; In one embodiment, R* is C₁₋₁₀ alkyl; In one embodiment, R* is C₁₋₁₀ haloalkyl; In one embodiment, R* is -L_{b}-OR_{b}; In one embodiment, R* is -L_{b}-SR_{b}; In one embodiment, R* is -L_{b}-NR_{b}R'_{b}; In one embodiment, R* is C₁₋₆ alkyl; In one embodiment, R* is C₁₋₆ haloalkyl; In one embodiment, R* is -OR_{b}.

In one embodiment, L_{b} is absent. In one embodiment, L_{b} is C₁₋₁₀ alkylene. In one embodiment, L_{b} is C₁₋₆ alkylene. In one embodiment, L_{b} is C₁₋₃ alkylene. In one embodiment, L_{b} is methylene. In one embodiment, L_{b} is ethylene.

In one embodiment, L_{f} is absent. In one embodiment, L_{f} is C₁₋₁₀ alkylene. In one embodiment, L_{f} is C₁₋₆ alkylene. In one embodiment, L_{f} is C₁₋₃ alkylene. In one embodiment, L_{f} is methylene. In one embodiment, L_{f} is ethylene.

In one embodiment, L_{c} is absent. In one embodiment, L_{c} is C₁₋₈ alkylene. In one embodiment, L_{c} is C₁₋₆ alkylene. In one embodiment, L_{c} is C₁₋₃ alkylene. In one embodiment, L_{c} is methylene. In one embodiment, L_{c} is ethylene.

In one embodiment, R_{b} is C₁₋₁₀ alkyl. In one embodiment, R_{b} is C₁₋₆ alkyl. In one embodiment, R_{b} is C₁₋₃ alkyl. In one embodiment, R_{b} is C₃₋₁₄ cycloalkyl. In one embodiment, R_{b} is C₃₋₈ cycloalkyl. In one embodiment, R_{b} is C₃₋₆ cycloalkyl. In one embodiment, R_{b} is 3- to 14-membered heterocyclyl. In one embodiment, R_{b} is 3- to 8-membered heterocyclyl. In one embodiment, R_{b} is 3- to 6-membered heterocyclyl. In one embodiment, R_{b} is 3- to 6-membered nitrogen-containing heterocyclyl. In one embodiment, R_{b} is unsubstituted. In one embodiment, R_{b} is substituted with one or more of C₁₋₁₀ alkyl, -Lₑ-ORₑ, -Lₑ-SRₑ or -L_{f}-NR_{f}R'_{f}, wherein R_{f} and R'_{f} are each independently H or C₁₋₁₀ alkyl.

In one embodiment, R'_{b} is C₁₋₁₀ alkyl. In one embodiment, R'_{b} is C₁₋₆ alkyl. In one embodiment, R'_{b} is C₁₋₃ alkyl. In one embodiment, R'_{b} is C₃₋₁₄ cycloalkyl. In one embodiment, R'_{b} is C₃₋₈ cycloalkyl. In one embodiment, R'_{b} is C₃₋₆ cycloalkyl. In one embodiment, R'_{b} is 3- to 14-membered heterocyclyl. In one embodiment, R'_{b} is 3- to 8-membered heterocyclyl. In one embodiment, R'_{b} is 3- to 6-membered heterocyclyl. In one embodiment, R'_{b} is 3- to 6-membered nitrogen-containing heterocyclyl. In one embodiment, R'_{b} is unsubstituted. In one embodiment, R'_{b} is substituted with one or more of C₁₋₁₀ alkyl, -L_{f}-OR_{f}, -Lₑ-SRₑ or -L_{f}-NR_{f}R'_{f}, wherein R_{f} and R'_{f} are each independently H or C₁₋₁₀ alkyl.

In one embodiment, j is 0. In one embodiment, j is 1.

In one embodiment, W is CH. In one embodiment, W is N.

In one embodiment, k is 0. In one embodiment, k is 1. In one embodiment, k is 2.

In one embodiment, k is 3. In one embodiment, k is 4. In one embodiment, k is 5. In one embodiment, k is 6. In one embodiment, k is 7. In one embodiment, k is 8.

In one embodiment, two substituents of R₀' together with the methylene they are attached to form a 3 to 8-membered cycloalkyl. In one embodiment, two substituents of R₀' together with the methylene they are attached to form a 3 to 6-membered cycloalkyl. In one embodiment, two substituents of R₀' together with the methylene they are attached to form a 5 or 6-membered cycloalkyl.

In one embodiment, M₁ is -C(O)O-; In one embodiment, M₁ is -O-; In one embodiment, M₁ is - SC(O)O-; In one embodiment, M₁ is -OC(O)NRₐ-; In one embodiment, M₁ is -NRₐC(O)NRₐ-; In one embodiment, M₁ is -OC(O)S-; In one embodiment, M₁ is -OC(O)O-; In one embodiment, M₁ is -NRₐC(O)O-; In one embodiment, M₁ is -OC(O)-; In one embodiment, M₁ is -SC(O)-; In one embodiment, M₁ is -C(O)S-; In one embodiment, M₁ is -NRₐ-; In one embodiment, M₁ is -C(O)NRₐ-; In one embodiment, M₁ is - NRₐC(O)-; In one embodiment, M₁ is -NRₐC(O)S-; In one embodiment, M₁ is -SC(O)NRₐ-; In one embodiment, M₁ is -C(O)-; In one embodiment, M₁ is -OC(S)-; In one embodiment, M₁ is -C(S)O-; In one embodiment, M₁ is -OC(S)NRₐ-; In one embodiment, M₁ is -NRₐC(S)O-; In one embodiment, M₁ is -S-S-; In one embodiment, M₁ is -S(O)₀₋₂-.

In one embodiment, M₂ is -C(O)O-; In one embodiment, M₂ is -O-; In one embodiment, M₂ is - SC(O)O-; In one embodiment, M₂ is -OC(O)NRₐ-; In one embodiment, M₂ is -NRₐC(O)NRₐ-; In one embodiment, M₂ is -OC(O)S-; In one embodiment, M₂ is -OC(O)O-; In one embodiment, M₂ is -NRₐC(O)O-; In one embodiment, M₂ is -OC(O)-; In one embodiment, M₂ is -SC(O)-; In one embodiment, M₂ is -C(O)S-; In one embodiment, M₂ is -NRₐ-; In one embodiment, M₂ is -C(O)NRₐ-; In one embodiment, M₂ is - NRₐC(O)-; In one embodiment, M₂ is -NRₐC(O)S-; In one embodiment, M₂ is -SC(O)NRₐ-; In one embodiment, M₂ is -C(O)-; In one embodiment, M₂ is -OC(S)-; In one embodiment, M₂ is -C(S)O-; In one embodiment, M₂ is -OC(S)NRₐ-; In one embodiment, M₂ is -NRₐC(S)O-; In one embodiment, M₂ is -S-S-; In one embodiment, M₂ is -S(O)₀₋₂-.

In one embodiment, M₁ and M₂ are each independently selected from -C(O)O-, -SC(O)O-, -OC(O)NRₐ-, -NRₐC(O)NRₐ-, -OC(O)S-, -OC(O)O-, -NRₐC(O)O-, -C(O)S-, -C(O)NRₐ-, -NRₐC(O)S-, -SC(O)NRₐ-, - C(S)O-, -OC(S)NRₐ- and -NRₐC(S)O-; In one embodiment, M₁ and M₂ are independently -C(O)O-, -C(O)S-, -C(O)NRₐ-, or -C(S)O-; In one embodiment, M₁ and M₂ are independently -C(O)O-, -C(O)S- or -C(O)NRₐ-. In one embodiment, M₁ and M₂ are both -C(O)O-.

In one embodiment, Q is absent; in another embodiment, Q is -C(O)O-; in another embodiment, Q is - O-; in another embodiment, Q is -SC(O)O-; in another embodiment, Q is -OC(O)NR_{b}-; in another embodiment, Q is -NR_{b}C(O)NR_{b}-; in another embodiment, Q is -OC(O)S-; in another embodiment, Q is - OC(O)O-; in another embodiment, Q is -NR_{b}C(O)O-; in another embodiment, Q is -OC(O)-; in another embodiment, Q is -SC(O)-; in another embodiment, Q is -C(O)S-; in another embodiment, Q is -NR_{b}-; in another embodiment, Q is -C(O)NR_{b}-; in another embodiment, Q is -NR_{b}C(O)-; in another embodiment, Q is -NR_{b}C(O)S-; in another embodiment, Q is -SC(O)NR_{b}-; in another embodiment, Q is -C(O)-; in another embodiment, Q is -OC(S)-; in another embodiment, Q is -C(S)O-; in another embodiment, Q is -OC(S)NR_{b}-; in another embodiment, Q is -NR_{b}C(S)O-; in another embodiment, Q is -S-S-; in another embodiment, Q is - S(O)₀₋₂-; in another embodiment, Q is phenylene; in another embodiment, Q is pyridylidene; in another embodiment, the phenylene or pyridylidene is optionally substituted with one or more R*.

In one embodiment, Q is selected from absent, -C(O)O-, -O-, -SC(O)O-, -OC(O)NR_{b}-, -NR_{b}C(O)NR_{b}-, - OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, - NR_{b}C(O)S-, -SC(O)NR_{b}-, -C(O)-, -OC(S)-, -C(S)O-, -OC(S)NR_{b}-, -NR_{b}C(S)O-, -S-S-, and -S(O)₀₋₂-; In one embodiment, Q is selected from -C(O)O-, -O-, -SC(O)O-, -OC(O)NH-, -NHC(O)NH-, -OC(O)S-, -OC(O)O-, -NHC(O)O-, -OC(O)-, -SC(O)-, -C(O)S-, -NH-, -C(O)NH-, -NHC(O)-, -NHC(O)S-, -SC(O)NH-, -C(O)-, - OC(S)-, -C(S)O-, -OC(S)NH- and -NHC(S)O-; In one embodiment, Q is selected from -C(O)O-, -O-, - SC(O)O-, -OC(O)NH-, -NHC(O)NH-, -OC(O)S-, -OC(O)O- and -NHC(O)O-; In one embodiment, Q is - C(O)O-.

In one embodiment, Rₐ is H; in another embodiment, Rₐ is C₁₋₂₀ alkyl; in another embodiment, Rₐ is 3- to 14-membered cycloalkyl; in another embodiment, Rₐ is 3- to 14-membered heterocyclyl; in another embodiment, Rₐ is C₁₋₁₄ alkyl; in another embodiment, Rₐ is C₁₋₁₀ alkyl; in another embodiment, Rₐ is C₈₋₁₀ alkyl; in another embodiment, Rₐ is C₈₋₁₀ linear alkyl; in another embodiment, Rₐ is -(CH₂)₈CH₃; in another embodiment, Rₐ is optionally substituted with one or more of the following substituents: H, C₁₋₂₀ alkyl, -Lₑ-ORₑ, -Lₑ-SRₑ and -Lₑ-NRₑR'ₑ, wherein Lₑ is absent or is C₁₋₂₀ alkylene, wherein Rₑ and R'ₑ are independently H or C₁₋₂₀ alkyl;

In one embodiment, R'ₐ is H; in another embodiment, R'ₐ is C₁₋₂₀ alkyl; in another embodiment, R'ₐ is 3-to 14-membered cycloalkyl; in another embodiment, R'ₐ is 3- to 14-membered heterocyclyl; in another embodiment, R'ₐ is C₁₋₁₄ alkyl; in another embodiment, R'ₐ is C₁₋₁₀ alkyl; in another embodiment, R'ₐ is C₈₋₁₀ alkyl; in another embodiment, R'ₐ is C₈₋₁₀ linear alkyl; in another embodiment, R'ₐ is -(CH₂)₈CH₃; in another embodiment, R'ₐ is optionally substituted with one or more of the following substituents: H, C₁₋₂₀ alkyl, -Lₑ-ORₑ, -Lₑ-SRₑ and -Lₑ-NRₑR'ₑ, wherein Lₑ is absent or is C₁₋₂₀ alkylene, wherein Rₑ and R'ₑ are independently H or C₁₋₂₀ alkyl.

In one embodiment, Lₑ is absent. In one embodiment, Lₑ is C₁₋₂₀ alkylene. In one embodiment, Lₑ is C₁₋₁₆ alkylene. In one embodiment, Lₑ is C₁₋₁₂ alkylene. In one embodiment, Lₑ is C₁₋₆ alkylene. In one embodiment, Lₑ is methylene. In one embodiment, Lₑ is ethylene.

In one embodiment, Rₑ is hydrogen. In one embodiment, Rₑ is C₁₋₂₀ alkyl. In one embodiment, Rₑ is C₁₋₁₆ alkyl. In one embodiment, Rₑ is C₁₋₁₂ alkyl. In one embodiment, Rₑ is C₁₋₆ alkyl. In one embodiment, R'ₑ is hydrogen. In one embodiment, R'ₑ is C₁₋₂₀ alkyl. In one embodiment, R'ₑ is C₁₋₁₆ alkyl. In one embodiment, R'ₑ is C₁₋₁₂ alkyl. In one embodiment, R'ₑ is C₁₋₆ alkyl.

In one embodiment, R_{f} is hydrogen. In one embodiment, R_{f} is C₁₋₁₀ alkyl. In one embodiment, R_{f} is C₁₋₆ alkyl. In one embodiment, R_{f} is C₁₋₃ alkyl. In one embodiment, R'_{f} is hydrogen. In one embodiment, R'_{f} is C₁₋₁₀ alkyl. In one embodiment, R'_{f} is C₁₋₆ alkyl. In one embodiment, R'_{f} is C₁₋₃ alkyl.

In one embodiment, G₁ is absent; in another embodiment, G₁ is C₁₋₁₃ alkylene; in another embodiment, G₁ is C₂₋₁₃ alkenylene; in another embodiment, G₁ is C₂₋₆ alkenylene; in another embodiment, G₁ is C₂₋₁₃ alkynylene; in another embodiment, G₁ is C₂₋₆ alkynylene; in another embodiment, G₁ is optionally substituted with one or more R^{s}, wherein R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₂ is absent; in another embodiment, G₂ is C₂₋₁₃ alkylene; in another embodiment, G₂ is C₂₋₆ alkylene; in another embodiment, G₂ is C₂₋₁₃ alkenylene; in another embodiment, G₂ is C₂₋₆ alkenylene; in another embodiment, G₂ is C₂₋₁₃ alkynylene; in another embodiment, G₂ is optionally substituted with one or more R^{s}, wherein R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₁ and G₂ have a total length of 3 carbon atoms; in another embodiment, G₁ and G₂ have a total length of 4 carbon atoms; in another embodiment, G₁ and G₂ have a total length of 5 carbon atoms; in another embodiment, G₁ and G₂ have a total length of 6 carbon atoms; in another embodiment, G₁ and G₂ have a total length of 7 carbon atoms; in another embodiment, G₁ and G₂ have a total length of 8 carbon atoms; in another embodiment, G₁ and G₂ have a total length of 9 carbon atoms; in another embodiment, G₁ and G₂ have a total length of 10 carbon atoms; in another embodiment, G₁ and G₂ have a total length of 11 carbon atoms; in another embodiment, G₁ and G₂ have a total length of 12 carbon atoms; in another embodiment, G₁ and G₂ have a total length of 13 carbon atoms.

In one embodiment, G₃ is absent; in another embodiment, G₃ is C₁₋₁₃ alkylene; in another embodiment, G₃ is C₂₋₁₃ alkenylene; in another embodiment, G₃ is C₂₋₆ alkenylene; in another embodiment, G₃ is C₂₋₁₃ alkynylene; in another embodiment, G₃ is C₂₋₆ alkynylene; in another embodiment, G₃ is optionally substituted with one or more R^{s}, wherein R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₄ is absent; in another embodiment, G₄ is C₂₋₁₃ alkylene; in another embodiment, G₄ is C₂₋₆ alkylene; in another embodiment, G₄ is C₂₋₁₃ alkenylene; in another embodiment, G₄ is C₂₋₆ alkenylene; in another embodiment, G₄ is C₂₋₁₃ alkynylene; in another embodiment, G₄ is optionally substituted with one or more R^{s}, wherein R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₁, G₂, G₃ and G₄ are each independently absent or C₁₋₈ alkylene.

In one embodiment, G₃ and G₄ have a total length of 3 carbon atoms; in another embodiment, G₃ and G₄ have a total length of 4 carbon atoms; in another embodiment, G₃ and G₄ have a total length of 5 carbon atoms; in another embodiment, G₃ and G₄ have a total length of 6 carbon atoms; in another embodiment, G₃ and G₄ have a total length of 7 carbon atoms; in another embodiment, G₃ and G₄ have a total length of 8 carbon atoms; in another embodiment, G₃ and G₄ have a total length of 9 carbon atoms; in another embodiment, G₃ and G₄ have a total length of 10 carbon atoms; in another embodiment, G₃ and G₄ have a total length of 11 carbon atoms; in another embodiment, G₃ and G₄ have a total length of 12 carbon atoms; in another embodiment, G₃ and G₄ have a total length of 13 carbon atoms.

In one embodiment, R^{s} is H; in another embodiment, R^{s} is C₁₋₁₄ alkyl; in another embodiment, R^{s} is -L_{d}-OR_{d}; in another embodiment, R^{s} is -L_{d}-SR_{d}; in another embodiment, R^{s} is -L_{d}-NR_{d}R'_{d}; in another embodiment, R^{s} is C₁₋₁₀ alkyl; in another embodiment, R^{s} is C₁₋₆ alkyl. In one embodiment, R^{s} is H, C₁₋₁₀ alkyl, -L_{d}-OR_{d} or -L_{d}-NR_{d}R'_{d}; in another more specific embodiment, R^{s} is H or C₁₋₆ alkyl.

In one embodiment, G₅ is absent. In one embodiment, G₅ is absent, j is 0, and R₃ is CH₂CH₂OH.

In another embodiment, G₅ is C₁₋₈ alkylene; in another embodiment, G₅ is C₁₋₆ alkylene; in another embodiment, G₅ is C₁₋₃ alkylene; in another embodiment, G₅ is optionally substituted with one or more R**, wherein each R** is independently C₁₋₈ alkyl, -L_{c}-OR_{c}, -L_{c}-SR_{c} or -Lₑ-NRₑR'ₑ, wherein R_{c} and R'_{c} are independently H or C₁₋₈ alkyl, and wherein L_{c} is absent or C₁₋₆ alkylene;

In one embodiment, R** is C₁₋₈ alkyl; in another embodiment, R** is -L_{c}-OR_{c}; in another embodiment, R** is -L_{c}SR_{c}; in another embodiment, R** is -L_{c}-NR_{c}R'_{c}; in another embodiment, R** is C₁₋₆ alkyl.

In one embodiment, L₁ is -(CRR')₂-. In one embodiment, L₁ is -CH=CH-. In one embodiment, L₁ is - C≡C-. In one embodiment, L₁ is -NR"-;

In one embodiment, L₂ is -(CRR')₂-. In one embodiment, L₂ is -CH=CH-. In one embodiment, L₂ is - C≡C-. In one embodiment, L₂ is -NR"-;

In one embodiment, L₃ is -(CR^{s}R^{s}')₂-, and L₅ is absent. In one embodiment, L₃ is -CH=CH-, and L₅ is absent. In one embodiment, L₃ is -C≡C-, and L₅ is absent. In one embodiment, L₅ is -(CR^{s}R^{s}')₂-, and L₃ is absent. In one embodiment, L₅ is -CH=CH-, and L₃ is absent. In one embodiment, L₅ is -C≡C-, and L₃ is absent.

In one embodiment, L₄ is -(CR^{s}R^{s'})₂-, and L₆ is absent. In one embodiment, L₄ is -CH=CH-, and L₆ is absent. In one embodiment, L₄ is -C≡C-, and L₆ is absent. In one embodiment, L₆ is -(CR^{s}R^{s'})₂-, and L₄ is absent. In one embodiment, L₆ is -CH=CH-, and L₄ is absent. In one embodiment, L₆ is -C≡C-, and L₄ is absent.

In one embodiment, G₁ₐ is absent. In one embodiment, G₁ₐ is methylene. In one embodiment, G₁ₐ is ethylene. In one embodiment, G₁ₐ is C₃ alkylene. In one embodiment, G₁ₐ is C₄ alkylene. In one embodiment, G₁ₐ is C₅ alkylene. In one embodiment, G₁ₐ is C₆ alkylene. In one embodiment, G₁ₐ is C₇ alkylene. In one embodiment, G₁ₐ is unsubstituted. In one embodiment, G₁ₐ is substituted. In one embodiment, G₁ₐ is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G_{1b} is absent. In one embodiment, G_{1b} is methylene. In one embodiment, G_{1b} is ethylene. In one embodiment, G_{1b} is C₃ alkylene. In one embodiment, G_{1b} is C₄ alkylene. In one embodiment, G_{1b} is C₅ alkylene. In one embodiment, G_{1b} is C₆ alkylene. In one embodiment, G_{1b} is C₇ alkylene. In one embodiment, G_{1b} is unsubstituted. In one embodiment, G_{1b} is substituted. In one embodiment, G_{1b} is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₂ₐ is absent. In one embodiment, G₂ₐ is methylene. In one embodiment, G₂ₐ is ethylene. In one embodiment, G₂ₐ is C₃ alkylene. In one embodiment, G₂ₐ is C₄ alkylene. In one embodiment, G₂ₐ is C₅ alkylene. In one embodiment, G₂ₐ is C₆ alkylene. In one embodiment, G₂ₐ is C₇ alkylene. In one embodiment, G₂ₐ is unsubstituted. In one embodiment, G₂ₐ is substituted. In one embodiment, G₂ₐ is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G_{2b} is absent. In one embodiment, G_{2b} is methylene. In one embodiment, G_{2b} is ethylene. In one embodiment, G_{2b} is C₃ alkylene. In one embodiment, G_{2b} is C₄ alkylene. In one embodiment, G_{2b} is C₅ alkylene. In one embodiment, G_{2b} is C₆ alkylene. In one embodiment, G_{2b} is C₇ alkylene. In one embodiment, G_{2b} is unsubstituted. In one embodiment, G_{2b} is substituted. In one embodiment, G_{2b} is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₃ₐ is absent. In one embodiment, G₃ₐ is methylene. In one embodiment, G₃ₐ is ethylene. In one embodiment, G₃ₐ is C₃ alkylene. In one embodiment, G₃ₐ is C₄ alkylene. In one embodiment, G₃ₐ is C₅ alkylene. In one embodiment, G₃ₐ is C₆ alkylene. In one embodiment, G₃ₐ is C₇ alkylene. In one embodiment, G₃ₐ is unsubstituted. In one embodiment, G₃ₐ is substituted. In one embodiment, G₃ₐ is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G_{3b} is absent. In one embodiment, G_{3b} is methylene. In one embodiment, G_{3b} is ethylene. In one embodiment, G_{3b} is C₃ alkylene. In one embodiment, G_{3b} is C₄ alkylene. In one embodiment, G_{3b} is C₅ alkylene. In one embodiment, G_{3b} is C₆ alkylene. In one embodiment, G_{3b} is C₇ alkylene. In one embodiment, G_{3b} is unsubstituted. In one embodiment, G_{3b} is substituted. In one embodiment, G_{3b} is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₄ₐ is absent. In one embodiment, G₄ₐ is methylene. In one embodiment, G₄ₐ is ethylene. In one embodiment, G₄ₐ is C₃ alkylene. In one embodiment, G₄ₐ is C₄ alkylene. In one embodiment, G₄ₐ is C₅ alkylene. In one embodiment, G₄ₐ is C₆ alkylene. In one embodiment, G₄ₐ is C₇ alkylene. In one embodiment, G₄ₐ is unsubstituted. In one embodiment, G₄ₐ is substituted. In one embodiment, G₄ₐ is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G_{4b} is absent. In one embodiment, G_{4b} is methylene. In one embodiment, G_{4b} is ethylene. In one embodiment, G_{4b} is C₃ alkylene. In one embodiment, G_{4b} is C₄ alkylene. In one embodiment, G_{4b} is C₅ alkylene. In one embodiment, G_{4b} is C₆ alkylene. In one embodiment, G_{4b} is C₇ alkylene. In one embodiment, G_{4b} is unsubstituted. In one embodiment, G_{4b} is substituted. In one embodiment, G_{4b} is substituted with one or more R^{s}, wherein each R^{s} is independently H, C₁₋₁₄ alkyl, -L_{d}-OR_{d}, -L_{d}-SR_{d} or -L_{d}-NR_{d}R'_{d}, and wherein L_{d} is absent or C₁₋₁₄ alkylene; and wherein R_{d} and R'_{d} are independently H or C₁₋₁₄ alkyl.

In one embodiment, G₁ₐ, G_{1b}, G₂ₐ and G_{2b} have a total length of 1, 2, 3, 4, 5, 6 or 7 carbon atoms; in another more specific embodiment, G₁ₐ, G_{1b}, G₂ₐ and G_{2b} have a total length of 1, 2, 3, 4, 5 or 6 carbon atoms. In one embodiment, G₁ₐ, G_{1b}, G₂ₐ and G_{2b} have a total length of 1 carbon. In one embodiment, G₁ₐ, G_{1b}, G₂ₐ and G_{2b} have a total length of 2 carbons. In one embodiment, G₁ₐ, G_{1b}, G₂ₐ and G_{2b} have a total length of 3 carbons. In one embodiment, G₁ₐ, G_{1b}, G₂ₐ and G_{2b} have a total length of 4 carbons. In one embodiment, G₁ₐ, G_{1b}, G₂ₐ and G_{2b} have a total length of 5 carbons. In one embodiment, G₁ₐ, G_{1b}, G₂ₐ and G_{2b} have a total length of 6 carbons.

In one embodiment, G₃ₐ, G_{3b}, G₄ₐ and G_{4b} have a total length of 1, 2, 3, 4, 5, 6 or 7 carbons. In one embodiment, G₃ₐ, G_{3b}, G₄ₐ and G_{4b} have a total length of 1 carbon. In one embodiment, G₃ₐ, G_{3b}, G₄ₐ and G_{4b} have a total length of 2 carbons. In one embodiment, G₃ₐ, G_{3b}, G₄ₐ and G_{4b} have a total length of 3 carbons. In one embodiment, G₃ₐ, G_{3b}, G₄ₐ and G_{4b} have a total length of 4 carbons. In one embodiment, G₃ₐ, G_{3b}, G₄ₐ and G_{4b} have a total length of 5 carbons. In one embodiment, G₃ₐ, G_{3b}, G₄ₐ and G_{4b} have a total length of 6 carbons. In one embodiment, G₃ₐ, G_{3b}, G₄ₐ and G_{4b} have a total length of 7 carbons.

In one embodiment, G₇ is absent; in another embodiment, G₇ is C₁₋₁₂ alkylene; in another embodiment, G₇ is C₁₋₆ alkylene; in another embodiment, G₇ is C₁₋₅ alkylene; in another embodiment, G₇ is C₁₋₅ linear alkylene; in another embodiment, G₇ is -CH₂-; in another embodiment, G₇ is -(CH₂)₂-; in another embodiment, G₇ is -(CH₂)₄-; in another embodiment, G₇ is -(CH₂)₅-; in another embodiment, G₇ is optionally substituted with 1, 2, 3, 4, 5 or 6 R, wherein R is C₁₋₁₀ alkyl. In another embodiment, 1, 2 or 3 methylene in G₇ are optionally and independently substituted with 1 R; in another embodiment, 1 or 2 methylene in G₇ are optionally and independently substituted with 1 R; in another embodiment, the methylene of G₇ that is connected to M₁ is not substituted with R.

In one embodiment, G₈ is absent; in another embodiment, G₈ is C₁₋₁₂ alkylene; in another embodiment, G₈ is C₁₋₁₀ alkylene; in another embodiment, G₈ is C₁₋₈ alkylene; in another embodiment, G₈ is C₁₋₈ linear alkylene; in another embodiment, G₈ is -(CH₂)₂-; in another embodiment, G₈ is -(CH₂)₄-; in another embodiment, G₈ is -(CH₂)₆-; in another embodiment, G₈ is -(CH₂)₇-; in another embodiment, G₈ is -(CH₂)₈-; in another embodiment, G₈ is optionally substituted with 1, 2, 3, 4, 5 or 6 R, wherein R is C₁₋₁₀ alkyl; in another embodiment, 1, 2 or 3 methylene in G₈ are optionally and independently substituted with 1 R; in another embodiment, 1 or 2 alkylene in G₈ are optionally and independently substituted with 1 R.

In one embodiment, G₇ and G₈ have a total length of 4 carbon atoms; in another embodiment, G₇ and G₈ have a total length of 5 carbon atoms; in another embodiment, G₇ and G₈ have a total length of 6 carbon atoms; in another embodiment, G₇ and G₈ have a total length of 7 carbon atoms; in another embodiment, G₇ and G₈ have a total length of 8 carbon atoms; in another embodiment, G₇ and G₈ have a total length of 9 carbon atoms; in another embodiment, G₇ and G₈ have a total length of 10 carbon atoms; in another embodiment, G₇ and G₈ have a total length of 11 carbon atoms; in another embodiment, G₇ and G₈ have a total length of 12 carbon atoms.

In one embodiment, G₇ and G₈ have a total length of 6, 7, 8, 9 or 10 carbon atoms. In one embodiment, G₇ and G₈ have a total length of 6, 7 or 8 carbon atoms.

In one embodiment, G₉ is absent; in another embodiment, G₉ is C₁₋₁₂ alkylene; in another embodiment, G₉ is C₁₋₆ alkylene; in another embodiment, G₉ is C₁₋₅ alkylene; in another embodiment, G₉ is C₁₋₅ linear alkylene; in another embodiment, G₉ is -CH₂-; in another embodiment, G₉ is -(CH₂)₂-; in another embodiment, G₉ is -(CH₂)₄-; in another embodiment, G₉ is -(CH₂)₅-; in another embodiment, G₉ is optionally substituted with 1, 2, 3, 4, 5 or 6 R, wherein R is C₁₋₁₀ alkyl; in another embodiment, 1, 2 or 3 methylene in G₉ are optionally and independently substituted with 1 R; in another embodiment, 1 or 2 methylene in G₉ are optionally and independently substituted with 1 R; in another embodiment, the methylene of G₉ that is connected to M₂ is not substituted with R.

In one embodiment, G₁₀ is absent; in another embodiment, G₁₀ is C₁₋₁₂ alkylene; in another embodiment, G₁₀ is C₁₋₁₀ alkylene; in another embodiment, G₁₀ is C₁₋₈ alkylene; in another embodiment, G₁₀ is C₁₋₈ linear alkylene; in another embodiment, G₁₀ is -(CH₂)₂-; in another embodiment, G₁₀ is -(CH₂)₄-; in another embodiment, G₁₀ is -(CH₂)₆-; in another embodiment, G₁₀ is -(CH₂)₇-; in another embodiment, G₁₀ is-(CH₂)₈-; in another embodiment, G₁₀ is optionally substituted with 1, 2, 3, 4, 5 or 6 R, wherein R is C₁₋₁₀ alkyl; in another embodiment, 1, 2 or 3 methylenes in G₁₀ are optionally and independently substituted with 1 R; in another embodiment, 1 or 2 methylenes in G₁₀ are optionally and independently substituted with 1 R.

In one embodiment, G₉ and G₁₀ have a total length of 4 carbon atoms; in another embodiment, G₉ and G₁₀ have a total length of 5 carbon atoms; in another embodiment, G₉ and G₁₀ have a total length of 6 carbon atoms; in another embodiment, G₉ and G₁₀ have a total length of 7 carbon atoms; in another embodiment, G₉ and G₁₀ have a total length of 8 carbon atoms; in another embodiment, G₉ and G₁₀ have a total length of 9 carbon atoms; in another embodiment, G₉ and G₁₀ have a total length of 10 carbon atoms; in another embodiment, G₉ and G₁₀ have a total length of 11 carbon atoms; in another embodiment, G₉ and G₁₀ have a total length of 12 carbon atoms.

In one embodiment, G₉ and G₁₀ have a total length of 6, 7, 8, 9 or 10 carbon atoms. In one embodiment, G₉ and G₁₀ have a total length of 6, 7 or 8 carbon atoms.

In one embodiment, R^{s} is H. In one embodiment, R^{s} is C₁₋₁₀ alkyl. In one embodiment, R^{s} is C₁₋₆ alkyl. In one embodiment, R^{s} is methyl. In one embodiment, R^{s} is ethyl. In one embodiment, R^{s} is C₃ alkyl. In one embodiment, R^{s} is C₄ alkyl. In one embodiment, R^{s} is C₅ alkyl. In one embodiment, R^{s} is C₆ alkyl. In one embodiment, R^{s} is -L_{d}-OR_{d}. In one embodiment, R^{s} is -L_{d}-NR_{d}R'_{d}. In one embodiment, R^{s} is -OR_{d}. In one embodiment, R^{s} is -NR_{d}R'_{d}. In one embodiment, R^{s} is -CH₂-OR_{d}. In one embodiment, R^{s} is -CH₂-NR_{d}R'_{d}.

In one embodiment, R^{s'} is H. In one embodiment, R^{s'} is C₁₋₁₀ alkyl. In one embodiment, R^{s'} is C₁₋₆ alkyl. In one embodiment, R^{s'} is methyl. In one embodiment, R^{s'} is ethyl. In one embodiment, R^{s'} is C₃ alkyl. In one embodiment, R^{s'} is C₄ alkyl. In one embodiment, R^{s'} is C₅ alkyl. In one embodiment, R^{s'} is C₆ alkyl. In one embodiment, R^{s'} is -L_{d}-OR_{d}. In one embodiment, R^{s'} is -L_{d}-NR_{d}R'_{d}. In one embodiment, R^{s'} is -OR_{d}. In one embodiment, R^{s'} is -NR_{d}R'_{d}. In one embodiment, R^{s'} is -CH₂-OR_{d}. In one embodiment, R^{s'} is -CH₂-NR_{d}R'_{d}.

Each instance of R and R' are independently H, C₁₋₂₀ alkyl, Lₐ-ORₐ, -Lₐ-SRₐ, or -L_{d}-NR_{d}R'_{d}, or R and R' together with the carbon they are attached to form a C₃₋₈ cycloalkylene;

In one embodiment, R is H. In one embodiment, R is C₁₋₂₀ alkyl. In one embodiment, R is C₁₋₁₄ alkyl. In one embodiment, R is C₁₋₈ alkyl. In one embodiment, R is C₁₋₆ alkyl. In one embodiment, R is -L_{d}-OR_{d}. In one embodiment, R is -Lₐ-NRₐR'ₐ. In one embodiment, R is -ORₐ. In one embodiment, R is -NRₐR'ₐ. In one embodiment, R is -CH₂-ORₐ. In one embodiment, R is -CH₂-NRₐR'ₐ.

In one embodiment, R' is H. In one embodiment, R' is C₁₋₂₀ alkyl. In one embodiment, R' is C₁₋₁₄ alkyl. In one embodiment, R' is C₁₋₈ alkyl. In one embodiment, R' is C₁₋₆ alkyl. In one embodiment, R' is -L_{d}-OR_{d}. In one embodiment, R' is -Lₐ-NRₐR'ₐ. In one embodiment, R' is -ORₐ. In one embodiment, R' is -NRₐR'ₐ. In one embodiment, R' is -CH₂-ORₐ. In one embodiment, R' is -CH₂-NRₐR' ₐ.

In one embodiment, R and R' together with the carbon atom they are attached to form a C₃₋₈ cycloalkylene. In one embodiment, R and R' together with the carbon atom they are attached to form a C₃₋₆ cycloalkylene. In one embodiment, R and R' together with the carbon atom they are attached to form a C₃ cycloalkylene, C₄ cycloalkylene, C₅ cycloalkylene, or C₆ cycloalkylene,

In one embodiment, R" is H. In one embodiment, R" is C₁₋₁₄ alkyl. In one embodiment, R" is C₁₋₈ alkyl. In one embodiment, R" is C₁₋₆ alkyl. In one embodiment, R" is methyl. In one embodiment, R" is ethyl. In one embodiment, R" is C₃ alkyl. In one embodiment, R" is C₄ alkyl. In one embodiment, R" is C₅ alkyl. In one embodiment, R" is C₆ alkyl.

In one embodiment, L_{d} is absent. In one embodiment, L_{d} is C₁₋₁₀ alkylene. In one embodiment, L_{d} is C₁₋₆ alkylene. In one embodiment, L_{d} is methylene. In one embodiment, L_{d} is ethylene. In one embodiment, L_{d} is C₃ alkylene. In one embodiment, L_{d} is C₄ alkylene. In one embodiment, L_{d} is C₅ alkylene. In one embodiment, L_{d} is C₆ alkylene.

In one embodiment, R_{c} is H. In one embodiment, R_{c} is C₁₋₈ alkyl. In one embodiment, R_{c} is C₁₋₆ alkyl. In one embodiment, R_{c} is methyl. In one embodiment, R_{c} is ethyl. In one embodiment, R_{c} is C₃ alkyl. In one embodiment, R_{c} is C₄ alkyl. In one embodiment, R_{c} is C₅ alkyl. In one embodiment, R_{c} is C₆ alkyl. In one embodiment, R_{c} is C₇ alkyl. In one embodiment, R_{c} is C₈ alkyl.

In one embodiment, R'_{c} is H. In one embodiment, R'_{c} is C₁₋₈ alkyl. In one embodiment, R'_{c} is C₁₋₆ alkyl. In one embodiment, R'_{c} is methyl. In one embodiment, R'_{c} is ethyl. In one embodiment, R'_{c} is C₃ alkyl. In one embodiment, R'_{c} is C₄ alkyl. In one embodiment, R'_{c} is C₅ alkyl. In one embodiment, R'_{c} is C₆ alkyl. In one embodiment, R'_{c} is C₇ alkyl. In one embodiment, R'_{c} is C₈ alkyl.

In one embodiment, R_{d} is H. In one embodiment, R_{d} is C₁₋₁₀ alkyl. In one embodiment, R_{d} is C₁₋₈ alkyl. In one embodiment, R_{d} is C₁₋₆ alkyl. In one embodiment, R_{d} is methyl. In one embodiment, R_{d} is ethyl. In one embodiment, R_{d} is C₃ alkyl. In one embodiment, R_{d} is C₄ alkyl. In one embodiment, R_{d} is C₅ alkyl. In one embodiment, R_{d} is C₆ alkyl. In one embodiment, R_{d} is C₇ alkyl. In one embodiment, R_{d} is Cs alkyl. In one embodiment, R_{d} is C₉ alkyl. In one embodiment, R_{d} is C₁₀ alkyl.

In one embodiment, R'_{d} is H. In one embodiment, R'_{d} is C₁₋₁₀ alkyl. In one embodiment, R'_{d} is C₁₋₈ alkyl. In one embodiment, R'_{d} is C₁₋₆ alkyl. In one embodiment, R'_{d} is methyl. In one embodiment, R'_{d} is ethyl. In one embodiment, R'_{d} is C₃ alkyl. In one embodiment, R'_{d} is C₄ alkyl. In one embodiment, R'_{d} is C₅ alkyl. In one embodiment, R'_{d} is C₆ alkyl. In one embodiment, R'_{d} is C₇ alkyl. In one embodiment, R'_{d} is C₈ alkyl. In one embodiment, R'_{d} is C₉ alkyl. In one embodiment, R'_{d} is C₁₀ alkyl.

In one embodiment, a' is 0. In one embodiment, a' is 1. In one embodiment, a' is 2. In one embodiment, a' is 3. In one embodiment, a' is 4. In one embodiment, a' is 5.

In one embodiment, b is 0. In one embodiment, b is 1. In one embodiment, b is 2. In one embodiment, b is 3. In one embodiment, b is 4. In one embodiment, b is 5.

In one embodiment, g is 0. In one embodiment, g is 1. In one embodiment, g is 2. In one embodiment, g is 3. In one embodiment, g is 4. In one embodiment, g is 5.

In one embodiment, a' is 2 and b is 2. In one embodiment, a' is 0 and b is 2. In one embodiment, a' is 2 and b is 0. In one embodiment, a' is 1 and b is 2. In one embodiment, a' is 2 and b is 1.

In one embodiment, a'+g equals 2. In one embodiment, a'+g equals 3. In one embodiment, a'+g equals 0. In one embodiment, a'+g equals 1. In one embodiment, a'+g equals 4. In one embodiment, a'+g equals 5.

In one embodiment, c is 0. In one embodiment, c is 1. In one embodiment, c is 2. In one embodiment, c is 3. In one embodiment, c is 4. In one embodiment, c is 5. In one embodiment, c is 6. In one embodiment, c is 7.

In one embodiment, d is 0. In one embodiment, d is 1. In one embodiment, d is 2. In one embodiment, d is 3. In one embodiment, d is 4. In one embodiment, d is 5. In one embodiment, d is 6. In one embodiment, d is 7.

In one embodiment, e is 0. In one embodiment, e is 1. In one embodiment, e is 2. In one embodiment, e is 3. In one embodiment, e is 4. In one embodiment, e is 5. In one embodiment, e is 6. In one embodiment, e is 7.

In one embodiment, f is 0. In one embodiment, f is 1. In one embodiment, f is 2. In one embodiment, f is 3. In one embodiment, f is 4. In one embodiment, f is 5. In one embodiment, f is 6. In one embodiment, f is 7.

In one embodiment, c+d equals 2. In one embodiment, c+d equals 3. In one embodiment, c+d equals 0. In one embodiment, c+d equals 1. In one embodiment, c+d equals 4. In one embodiment, c+d equals 5. In one embodiment, c+d equals 6. In one embodiment, c+d equals 7. In one embodiment, c+d equals 8. In one embodiment, c+d equals 9.

In one embodiment, e+f equals 2. In one embodiment, e+f equals 3. In one embodiment, e+f equals 0. In one embodiment, e+f equals 1. In one embodiment, e+f equals 4. In one embodiment, e+f equals 5. In one embodiment, e+f equals 6. In one embodiment, e+f equals 7. In one embodiment, e+f equals 8. In one embodiment, e+f equals 9.

In one embodiment, 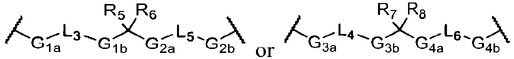 have a total length of 4, 5, 6, 7, 8 or 9 carbon atoms.

In one embodiment, 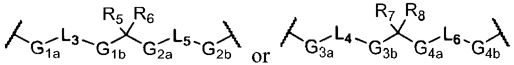 is independently selected from: -(CH₂)₃-C(CH₃)₂-, -(CH₂)₄-C(CH₃)₂-, -(CH₂)₅-C(CH₃)₂-, -(CH₂)₆-C(CH₃)₂-, -(CH₂)₇-C(CH₃)₂-, -(CH₂)₈-C(CH₃)₂-, -(CH₂)₃-CH=CH-C(CH₃)₂-, -(CH₂)₃-C≡C-C(CH₃)₂-, -(CH₂)₄-C(CH₃)₂-CH₂-, -(CH₂)₃-C(CH₃)₂-(CH₂)₂-, -(CH₂)₂-C(CH₃)₂-(CH₂)₃-, -(CH₂)₂-CH=CH-C(CH₃)₂-CH₂-, -(CH₂)₂-C(CH₃)₂-C≡C-CH₂-, -(CH₂)₂-C(CH₃)₂-CH=CH-CH₂-, -(CH₂)₂-C≡C-C(CH₃)₂-CH₂- and -(CH₂)₃-C(CH₃)₂-C≡C-; In one embodiment, 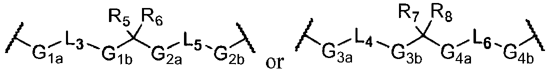 is independently -(CH₂)₄-C(CH₃)₂-, -(CH₂)₅-C(CH₃)₂- or -(CH₂)₆-C(CH₃)₂-; In one embodiment, 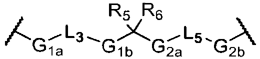 or 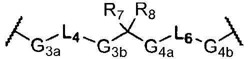 is -(CH₂)₅-C(CH₃)₂-. In one embodiment, 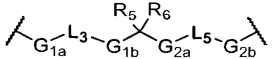 is - (CH₂)₆-C(CH₃)₂-, and 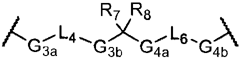 is -(CH₂)₇-. In one embodiment, 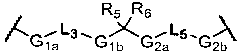 is -(CH₂)₇-, and 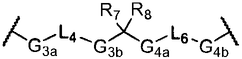 is -(CH₂)₇-.

In one embodiment, -G₇-L₁-G₈-H or -G₉-L₂-G₁₀-H is independently selected from: -(CH₂)₅CH₃,-(CH₂)₆CH₃, -(CH₂)₇CH₃, -(CH₂)₈CH₃, -(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

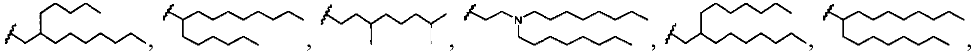

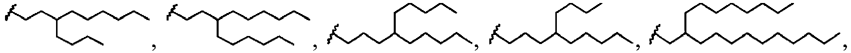

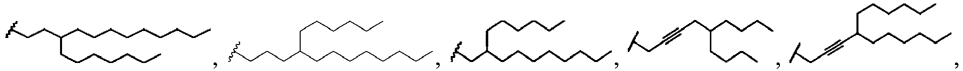

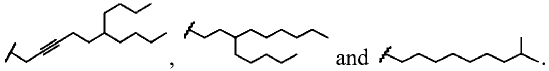

In one embodiment, -G₇-L₁-G₈-H or -G₉-L₂-G₁₀-H is independently selected from the following groups: -(CH₂)₅CH₃, -(CH₂)₆CH₃, -(CH₂)₇CH₃, -(CH₂)₈CH₃, -(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

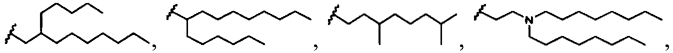

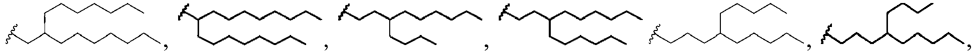

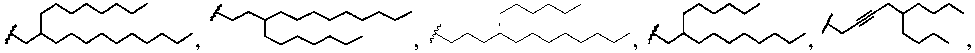

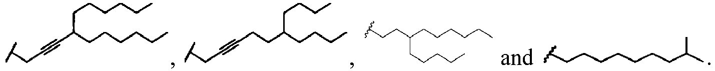

In one embodiment, -G₇-L₁-G₈-H or -G₉-L₂-G₁₀-H is 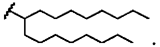 In one embodiment, -G₇-L₁-G₈-H or -G₉-L₂-G₁₀-H is -(CH₂)₈CH₃. In one embodiment, one of -G₇-L₁-G₈-H and -G₉-L₂-G₁₀-H is 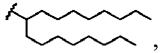 and the other is -(CH₂)₈CH₃.

In one embodiment, the ionizable lipid is a compound of formula (IX): 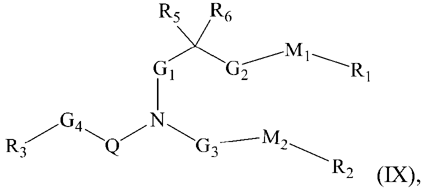 or a stereoisomer, a mixture of stereoisomers, or a pharmaceutically acceptable salt thereof, wherein
G₁ and G₂ are each independently a bond, C₁₋₁₃ linear alkylene, C₂₋₁₃ linear alkenylene, or C₂₋₁₃ linear alkynylene, wherein the alkylene, alkenylene, and alkynylene are optionally substituted by one or more R_{G1};
G₁ and G₂ have a total length of 3, 4, 5, 6, 7, 8, 9, 10, 11, 12 or 13 carbon atoms;
each instance of R_{G1} is independently C₁₋₁₄ alkyl, -Lₐ-ORₐ, -Lₐ-SRₐ, or -Lₐ-NRₐR'ₐ;
G₃ is C₄₋₁₄ linear alkylene, C₄₋₁₄ linear alkenylene, or C₄₋₁₄ linear alkynylene, wherein the alkylene, alkenylene, and alkynylene are optionally substituted by one or more R_{G3};
each instance of R_{G3} is independently H, -Lₐ-ORₐ, -L_{d}-SR_{d} or -Lₐ-NRₐR'ₐ;
Lₐ is independently a bond or C₁₋₁₄ alkylene;
Rₐ and R'ₐ are each independently H, C₁₋₁₄ alkyl, C₃₋₁₄ cycloalkyl, or 3- to 14-membered heterocyclyl;
G₄ is a bond, C₁₋₆ alkylene, C₂₋₆ alkenylene, or C₂₋₆ alkynylene, and wherein the alkylene, alkenylene, and alkynylene are optionally substituted by one or more R_{G4};
each of instance of R_{G4} is independently H, C₁₋₆ alkyl, -L_{b}-OR_{b}, -L_{b}-SR_{b} or -L_{b}-NR_{b}R'_{b};
L_{b} is independently a bond or C₁₋₆ alkylene;
R_{b} and R'_{b} are independently H, C₁₋₆ alkyl, C₃₋₁₀ cycloalkyl or 3- to 10-membered heterocyclyl;
or two R_{G4} together with the same carbon atom they are attached to form a C₃₋₁₄ cycloalkylene or 3- to 14-membered heterocyclylene, wherein the cycloalkylene and heterocyclylene are optionally substituted by one or more R_{4g};
each of instance of R_{4g} is independently H, halogen, cyano, C₁₋₈ alkyl, C₁₋₈ haloalkyl, -Lₑ-ORₑ, -Lₑ-SRₑ, or -Lₑ-NRₑR'ₑ;
Lₑ is independently a bond or C₁₋₈ alkylene;
Rₑ and R'ₑ are independently H, C₁₋₈ alkyl, C₃₋₁₄ cycloalkyl, or 3- to 14-membered heterocyclyl;
M₁ and M₂ are each independently -C(O)O-, -OC(O)-, -O-, -SC(O)O-, -OC(O)NR-, -NRC(O)NR-,-OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S- or -S(O)₀₋₂-;
Q is a bond, -C(O)O-, -O-, -SC(O)O-, -OC(O)NR_{f}-, -NR_{f}C(O)NR_{f}-, -OC(O)S-, -OC(O)O-, -NR_{f}C(O)O-, -OC(O)-, -SC(O)-, -C(O)S-, -NR_{f}-, -C(O)NR_{f}-, -NR_{f}C(O)-, -NR_{f}C(O)S-, -SC(O)NR_{f}-, -C(O)-, -OC(S)-,-C(S)O-, -OC(S)NR_{f}-, -NR_{f}C(S)O-, -S-S-, -S(O)₀₋₂-, phenylene, or pyridylidene, wherein the phenylene and pyridylidene are optionally substituted with one or more R*;
R* is independently H, halogen, cyano, C₁₋₁₀ alkyl, C₁₋₁₀ haloalkyl, -L_{f}-OR_{f}, -L_{f}-SR_{f}, or -L_{f}-NR_{f}R'_{f},
L_{f} is independently a bond or C₁₋₈ alkylene;
R_{f} and R'_{f} are independently H, C₁₋₁₀ alkyl, C₃₋₁₄ cycloalkyl, or 3- to 14-membered heterocyclyl;
R₁ and R₂ are independently C₄₋₂₀ alkyl, C₄₋₂₀ alkenyl, or C₄₋₂₀ alkynyl, which is optionally substituted with one or more R₁ₛ, and wherein one or more methylene units are optionally and independently replaced with -NR'-;
R₁ₛ is independently H, C₁₋₂₀ alkyl, -L_{c}-OR_{c}, -L_{c}-SR_{c}, or -L_{c}-NR_{c}R'_{c};
R are R' are each independently H or C₁₋₂₀ alkyl;
L_{c} is independently a bond or C₁₋₂₀ alkylene;
R_{c} and R'_{c} are each independently H, C₁₋₂₀ alkyl, C₃₋₁₄ cycloalkyl, or 3- to 14-membered heterocyclyl;
R₃ is -CN, -OR_{g}, -C(O)R_{g}, -OC(O)R_{g}, -NR"C(O)R_{g}, -NR_{g}R'_{g}, -NR"C(O)NR_{g}R'_{g}, -NR"C(O)R_{g},-NR"S(O)₂R_{g}, -OC(O)NR_{g}R'_{g}, -NR"C(O)OR_{g}, -N(OR_{g})C(O)R_{g}, -N(OR_{g})S(O)₂R_{g}, -N(OR_{g})C(O)OR_{g},-N(OR_{g})C(O)R_{g}R'_{g}, 3- to 14-membered heterocyclyl, or 5- to 14-membered heteroaryl;
R_{g} and R'_{g} are each independently H, C₁₋₁₀ alkyl, C₃₋₁₀ cycloalkyl, or 3- to 10-membered heterocyclyl;
R" is independently H or C₁₋₆ alkyl;
R₅ and R₆ are independently C₁₋₈ alkyl, wherein the alkyl is optionally substituted by one or more R₄ₛ;
or R₅ and R₆ together with the carbon atom they are attached to form a C₃₋₁₄ cycloalkylene or 3- to 14-membered heterocyclylene, wherein the cycloalkylene and heterocyclylene are optionally substituted by one or more R₄ₛ;
each instance of R₄ₛ is independently H, halogen, cyano, C₁₋₈ alkyl, C₁₋₈ haloalkyl, -L_{d}-OR_{d}, -L_{d}-SR_{d}, or-L_{d}-NR_{d}R'_{d};
L_{d} is independently a bond or C₁₋₈ alkylene;
R_{d} and R'_{d} are independently H, C₁₋₈ alkyl, C₃₋₁₄ cycloalkyl or 3- to-14-membered heterocyclyl.

In one embodiment, G₁ and G₂ are independently a bond, C₁₋₉ linear alkylene, C₂₋₉ linear alkenylene, or C₂₋₉ linear alkynylene.

In one embodiment, G₁ is C₁₋₆ linear alkylene, C₂₋₆ linear alkenylene, or C₂₋₆ linear alkynylene. In one embodiment, G₁ is C₁₋₆ linear alkylene. In one embodiment, G₁ is C₂₋₆ linear alkylene.

In one embodiment, G₂ is a bond, C₁₋₆ linear alkylene, C₂₋₆ linear alkenylene, or C₂₋₆ linear alkynylene. In one embodiment, G₂ is a bond or C₁₋₆ linear alkylene. In one embodiment, G₂ is a bond or C₁₋₄ linear alkylene.

In one embodiment, G₁ and G₂ have a total length of 3, 4, 5, 6, 7, 8 or 9 carbon atoms. In one embodiment, G₁ and G₂ have a total length of 4, 5 or 6 carbon atoms. In one embodiment, G₁ and G₂ have a total length of 5 or 6 carbon atoms. In one embodiment, G₁ and G₂ have a total length of 5, 6 or 7 carbon atoms. In one embodiment, G₁ and G₂ have a total length of 6 or 7 carbon atoms. In one embodiment, G₁ and G₂ are optionally substituted by 1, 2, 3, or 4 R_{G1}.

In one embodiment, each instance of R_{G1} is independently H or C₁₋₁₀ alkyl. In one embodiment, each instance of R_{G1} is independently H or C₁₋₆ alkyl.

In one embodiment, G₃ is C₄₋₁₀ linear alkylene, C₄₋₁₀ linear alkenylene, or C₄₋₁₀ linear alkynylene. In one embodiment, G₃ is C₄₋₉ linear alkylene. In one embodiment, G₃ is C₅₋₈ linear alkylene. In one embodiment, G₃ is optionally substituted by 1, 2, 3 or 4 R_{G3}.

In one embodiment, Lₐ is independently a bond or C₁₋₁₀ alkylene. In one embodiment, Lₐ is independently a bond or C₁₋₆ alkylene.

In one embodiment, Rₐ and R'ₐ are each independently H, C₁₋₁₀ alkyl, C₃₋₁₀ cycloalkyl, or 3- to 10-membered heterocyclyl. In one embodiment, Rₐ and R'ₐ are each independently H or C₁₋₆ alkyl.

In one embodiment, G₄ is C₁₋₄ alkylene, C₂₋₄ alkenylene, or C₂₋₄ alkynylene. In one embodiment, G₄ is C₂₋₄ alkylene. In one embodiment, G₄ is C₂₋₃ alkylene. In one embodiment, G₄ is optionally substituted by 1, 2, 3 or 4 R_{G4}.

In one embodiment, R_{G4} is independently H or C₁₋₆ alkyl. In one embodiment, R_{G4} is independently C₁₋₄ alkyl.

In one embodiment, two R_{G4} together with the same carbon atom they are attached to form a C₃₋₁₀ cycloalkylene or 3- to 10-membered heterocyclylene. In one embodiment, two R_{G4} together with the same carbon atom they are attached to form a C₃₋₇ cycloalkylene or 3- to 7-membered heterocyclylene. In one embodiment, the cycloalkylene and heterocyclylene are optionally substituted by 1, 2, or 3 R_{4g};

In one embodiment, R_{4g} is independently H, halogen, cyano, C₁₋₆ alkyl or C₁₋₆ haloalkyl.

In one embodiment, L_{b} is independently a bond or C₁₋₄ alkylene.

In one embodiment, R_{b} and R'_{b} are independently H, C₁₋₆ alkyl, C₃₋₇ cycloalkyl or 3-to 7-membered heterocyclyl. In one embodiment, R_{b} and R'_{b} are independently H or C₁₋₆ alkyl. In one embodiment, R_{b} and R'_{b} are independently H or C₁₋₄ alkyl.

In one embodiment, M₁ and M₂ are independently -C(O)O-, -OC(O)-, -OC(O)O-, -SC(O)-, -C(O)S-,-C(O)NR- or -NRC(O)-. In one embodiment, M₁ and M₂ are independently -C(O)O-, -OC(O)O-, -OC(O)-,-SC(O)- or -C(O)S-. In one embodiment, M₁ and M₂ are independently -C(O)O-, -OC(O)-, -SC(O)- or-C(O)S-. In one embodiment, M₁ and M₂ are independently -C(O)O- or -OC(O)-.

In one embodiment, R₁ and R₂ are independently C₆₋₁₄ alkyl, C₆₋₁₄ alkenyl, or C₆₋₁₄ alkynyl. In one embodiment, R₁ and R₂ are independently C₆₋₁₄ alkyl. In one embodiment, R₁ and R₂ are independently C₇₋₁₂ alkyl. In one embodiment, R₁ and R₂ are independently C₈₋₁₂ alkyl. In one embodiment, R₁ and R₂ are independently C₇₋₁₂ alkyl, C₇₋₁₂ alkenyl, or C₇₋₁₂ alkynyl.

In one embodiment, R₁ and R₂ are optionally substituted by 1, 2, 3 or 4 R₁ₛ. In one embodiment, R₁ and R₂ are optionally substituted by one R₁ₛ.

In one embodiment, R₁ and R₂ are independently -(CH₂)₅CH₃, -(CH₂)₆CH₃, -(CH₂)₇CH₃, -(CH₂)₈CH₃,-(CH₂)₉CH₃, -(CH₂)₁₀CH₃, -(CH₂)₁₁CH₃, -CH₂-C≡C-(CH₂)₅CH₃, -CH₂-C≡C-(CH₂)₆CH₃, -(CH₂)₂-C≡C-(CH₂)₅CH₃, -(CH₂)₂-C≡C-(CH₂)₄CH₃, -(CH₂)₃-C≡C-(CH₂)₃CH₃, -(CH₂)₄-C≡C-(CH₂)₃CH₃, -CH₂-CH=CH-(CH₂)₅CH₃, -CH₂-CH=CH-(CH₂)₆CH₃, -(CH₂)₂-CH=CH-(CH₂)₅CH₃, -(CH₂)₄-CH=CH-(CH₂)₃CH₃, -(CH₂)₅-CH=CH-CH₂CH₃,

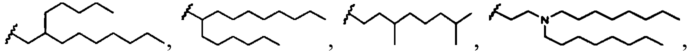

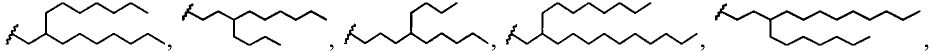

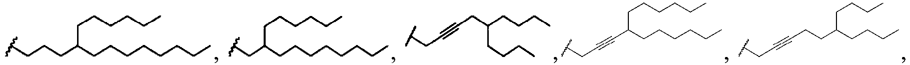

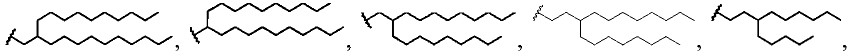

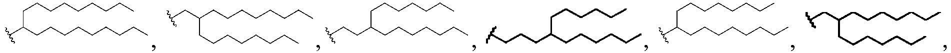

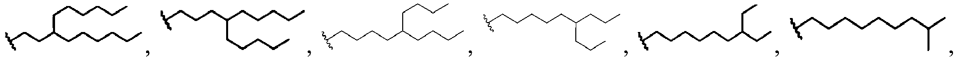

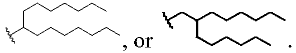

In one embodiment, R₁ₛ is independently H, C₁₋₁₄ alkyl, -L_{c}-OR_{c}, or -L_{c}-NR_{c}R'_{c}. In one embodiment, R₁ₛ is independently H or C₁₋₁₄ alkyl. In one embodiment, R₁ₛ is independently H or C₁₋₁₀ alkyl. In one embodiment, R₁ₛ is independently H or C₁₋₉ alkyl. In one embodiment, R₁ₛ is independently H or C₁₋₆ alkyl. In one embodiment, R₁ₛ is independently H or C₁₋ₐ alkyl.

In one embodiment, R and R' are independently H or C₁₋₂₀ alkyl. In one embodiment, R and R' are independently H or C₁₋₁₄ alkyl. In one embodiment, R and R' are independently H or C₁₋₉ alkyl. In one embodiment, R and R' are independently H or C₁₋₆ alkyl. In one embodiment, R is H.

In one embodiment, L_{c} is independently a bond or C₁₋₁₄ alkylene. In one embodiment, L_{c} is independently a bond or C₁₋₁₀ alkylene. In one embodiment, L_{c} is independently a bond or C₁₋₆ alkylene.

In one embodiment, R_{c} and R'_{c} are independently H or C₁₋₁₄ alkyl. In one embodiment, R_{c} and R'_{c} are independently H or C₁₋₁₀ alkyl. In one embodiment, R_{c} and R'_{c} are independently H or C₁₋₆ alkyl.

In one embodiment, R₃ is cyano, -OR_{g} or -NR_{g}R'_{g}. In one embodiment, R₃ is -OR_{g} or -NR_{g}R'_{g}. In one embodiment, R₃ is -OR_{g∘} In one embodiment, R₃ is -OH.

In one embodiment, R₃ is -OH or -N(CH₃)₂.

In one embodiment, R_{g} and R'_{g} are independently H, C₁₋₆ alkyl, C₃₋₇ cycloalkyl, or 3- to 7-membered heterocyclyl. In one embodiment, R_{g} and R'_{g} are independently H or C₁₋₆ alkyl. In one embodiment, R_{g} and R'_{g} are independently H or C₁₋₄ alkyl. In one embodiment, R_{g} and R'_{g} are independently H or -CH₃.

In one embodiment, R₅ and R₆ are independently C₁₋₆ alkyl. In one embodiment, R₅ and R₆ are independently C₁₋₃ alkyl. In one embodiment, R₅ and R₆ are independently -CH₃. In one embodiment, R₅ and R₆ are independently and optionally substituted by 1, 2, or 3 R₄ₛ.

In one embodiment, R₅ and R₆ together with the carbon atom they are attached to form a C₃₋₁₀ cycloalkylene or 3- to 10-membered heterocyclylene. In one embodiment, the cycloalkylene or heterocyclylene is optionally substituted by 1, 2, or 3 R₄ₛ.

In one embodiment, R₄ₛ is independently H, halogen, cyano, C₁₋₆ alkyl, C₁₋₆ haloalkyl, -L_{d}-OR_{d}, -L_{d}-SR_{d}, or -L_{d}-NR_{d}R'_{d}. In one embodiment, R₄ₛ is independently H, halogen, cyano, C₁₋₆ alkyl, or C₁₋₆ haloalkyl. In one embodiment, R₄ₛ is independently H, C₁₋₃ alkyl, or C₁₋₃ haloalkyl.

In one embodiment, L_{d} is independently a bond or C₁₋₆ alkylene. In one embodiment, L_{d} is independently a bond or C₁₋₃ alkylene.

In one embodiment, R_{d} and R'_{d} are independently H, C₁₋₆ alkyl, C₃₋₁₀ cycloalkyl, or 3- to 10-membered heterocyclyl. In one embodiment, R_{d} and R'_{d} are independently H or C₁₋₆ alkyl. In one embodiment, R_{d} and R'_{d} are independently H or C₁₋₆ alkyl.

In one embodiment, Lₑ is independently a bond or C₁₋₆ alkylene. In one embodiment, Lₑ is independently a bond or C₁₋₄ alkylene.

In one embodiment, Rₑ and R'ₑ are independently H, C₁₋₆ alkyl, C₃₋₁₀ cycloalkyl, or 3- to 10-membered heterocyclyl. In one embodiment, Rₑ and R'ₑ are independently H or C₁₋₆ alkyl. In one embodiment, Rₑ and R'ₑ are independently H or C₁₋₄ alkyl.

In one embodiment, L_{f} is independently a bond or C₁₋₆ alkylene. In one embodiment, L_{f} is independently a bond or C₁₋₄ alkylene.

In one embodiment, R_{f} and R'_{f} are independently H, C₁₋₆ alkyl, C₃₋₁₀ cycloalkyl or 3- to 10-membered heterocyclyl. In one embodiment, R_{f} and R'_{f} are independently H or C₁₋₆ alkyl. In one embodiment, R_{f} and R'_{f} are independently H or C₁₋₄ alkyl.

In one embodiment, the compound of formula (IX) is a compound of formula (X): 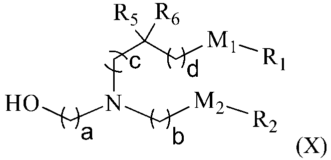
or a stereoisomer, a mixture of stereoisomers, or a pharmaceutically acceptable salt thereof,
wherein:
   a is 1, 2, 3, 4, 5, or 6;
   b is 4, 5, 6, 7, 8, 9, or 10;
   c is 1, 2, 3, 4, 5, or 6;
   d is 0, 1, 2, 3, or 4; and
   c+d is 3, 4, 5, 6, 7, 8, or 9.

In one embodiment, the M₁ or M₂ is separated from the substitution site of R₁ₛ on R₁ or R₂ by from 0 to 10 carbon atoms. In one embodiment, the M₁ or M₂ is separated from the substitution site of R₁ₛ on R₁ or R₂ by from 0 to 6 carbon atoms. In one embodiment, the M₁ or M₂ is separated from the substitution site of R₁ₛ on R₁ or R₂ by from 0 to 4 carbon atoms. In one embodiment, the M₁ or M₂ is separated from the substitution site of R₁ₛ on R₁ or R₂ by from 0 to 2 carbon atoms. In one embodiment, the M₁ or M₂ is separated from the substitution site of R₁ₛ on R₁ or R₂ by 0 carbon atoms.

In one embodiment, R₂ is 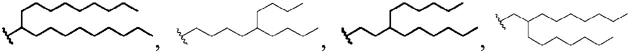 or 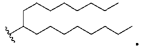 In one embodiment, R₂ is 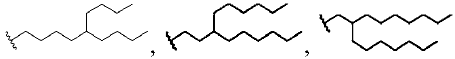 or 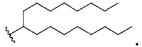 In one embodiment, R₂ is 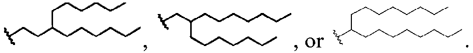 In one embodiment, R₂ is

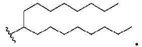

In one embodiment, R₁ is 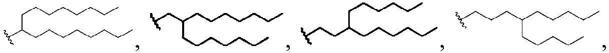 or

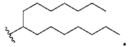

In one embodiment, the ionizable lipid is: 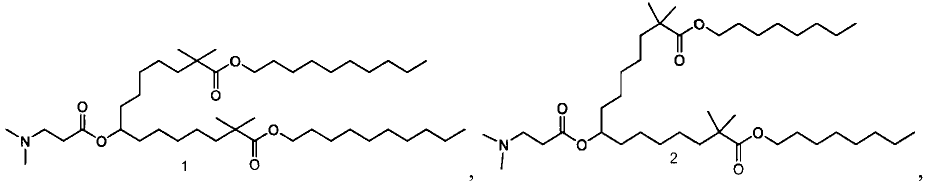 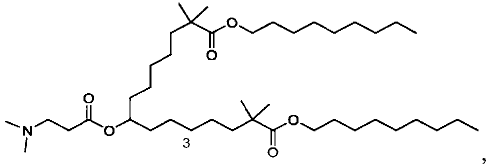 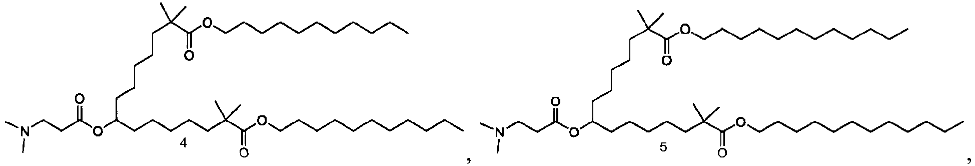 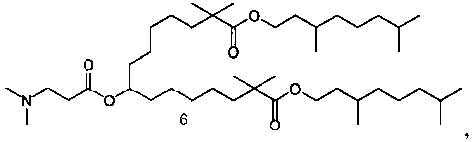 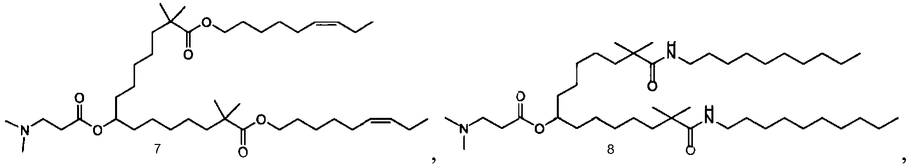 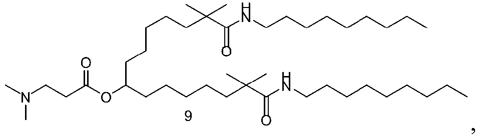 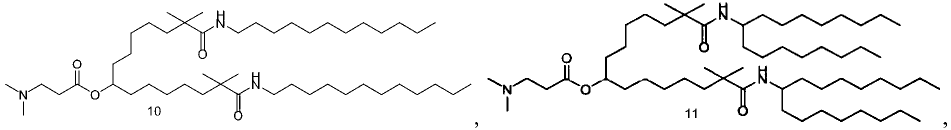 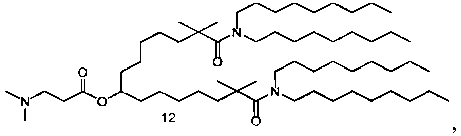 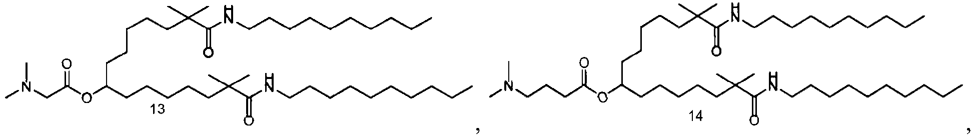 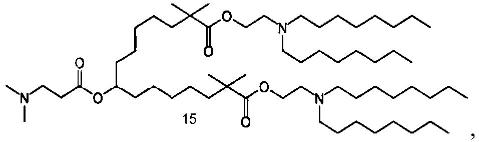 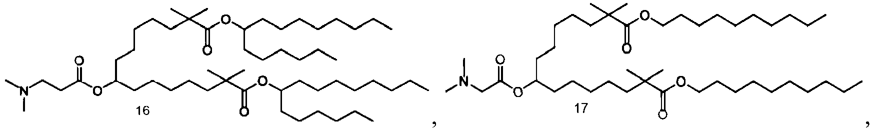 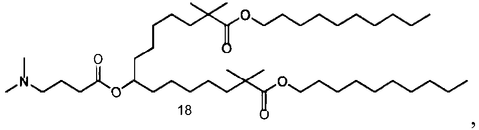 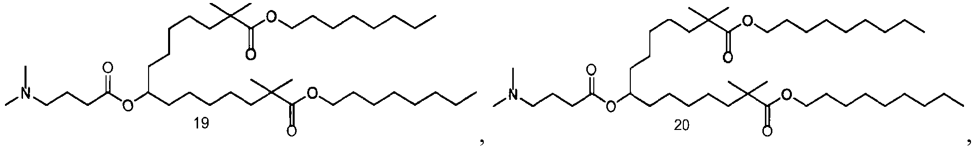 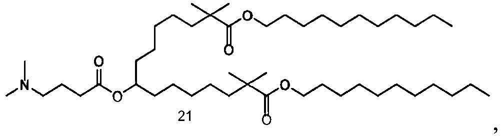 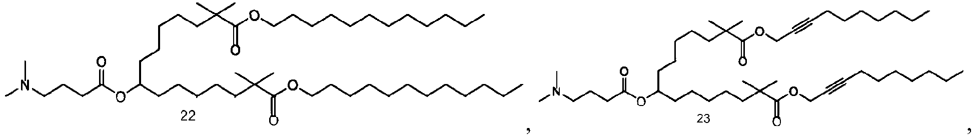 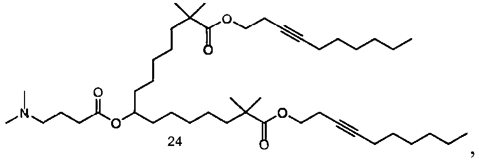 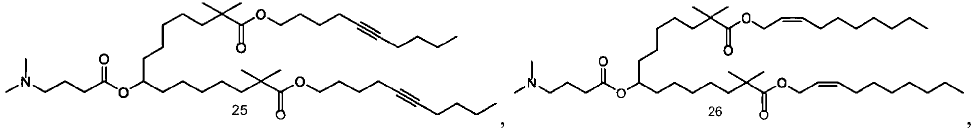 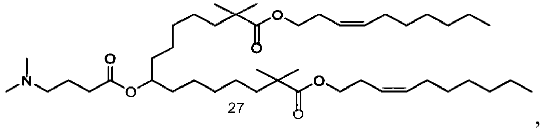 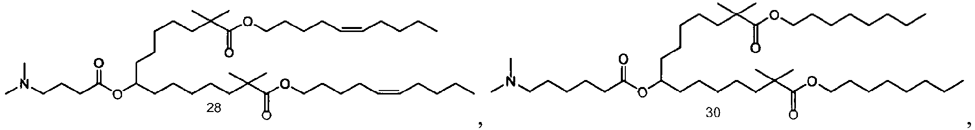 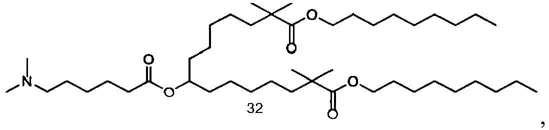 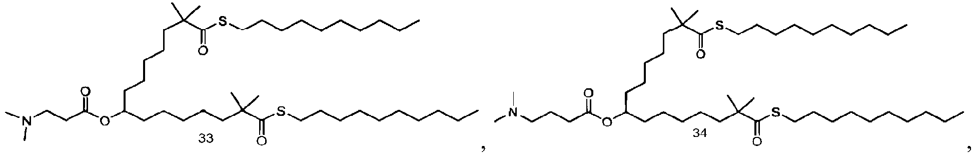 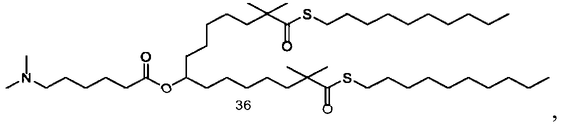 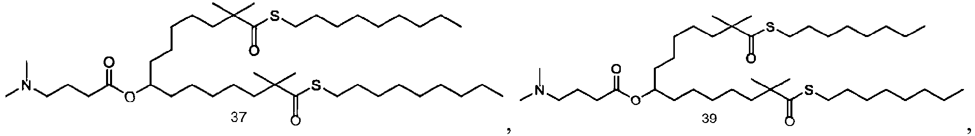 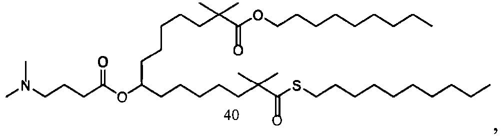 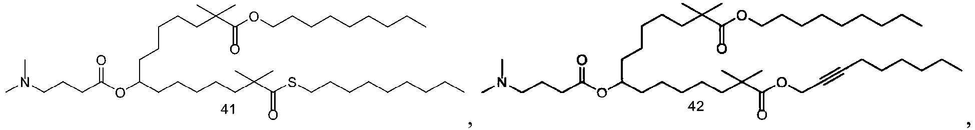 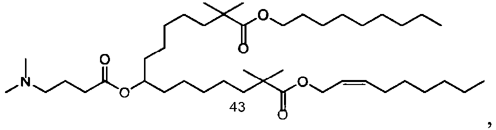 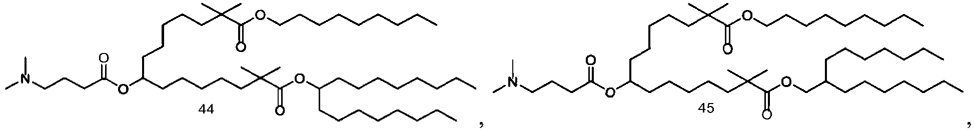 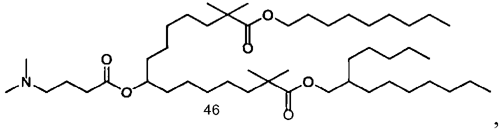 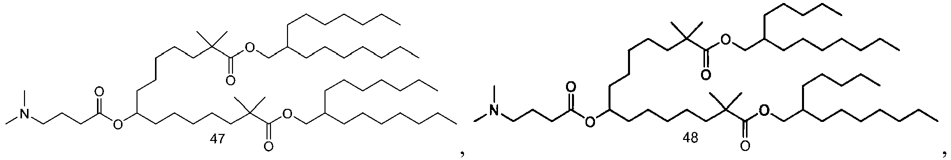 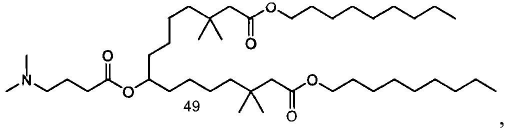 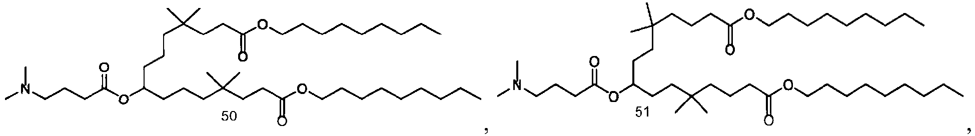 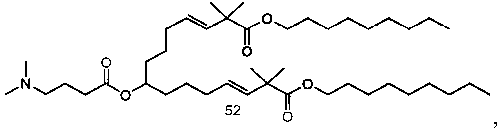 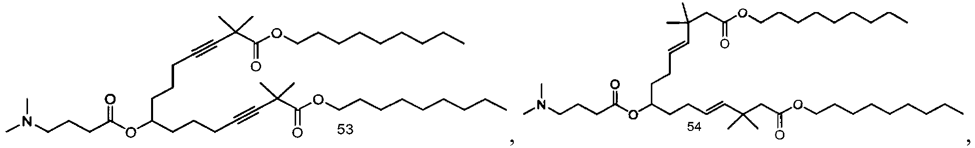 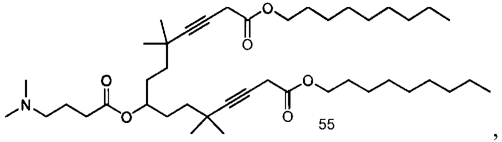 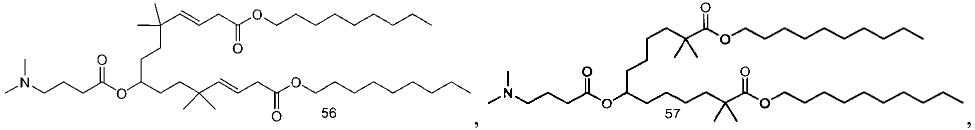 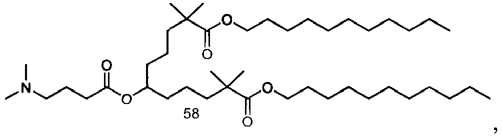 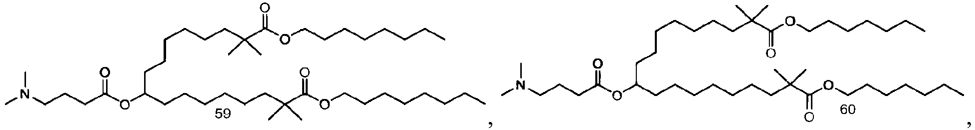 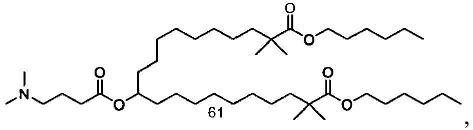 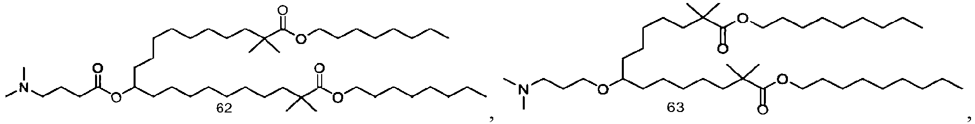 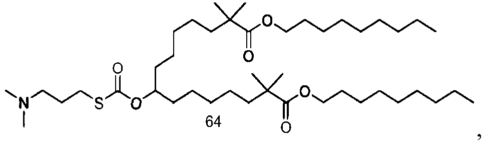 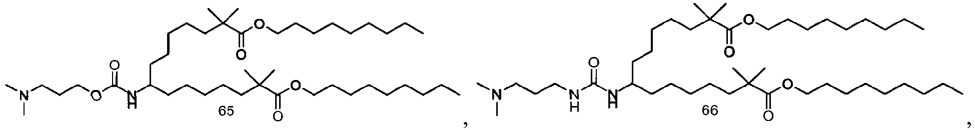 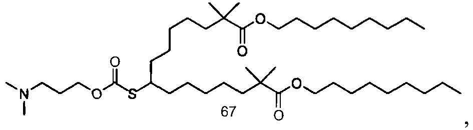 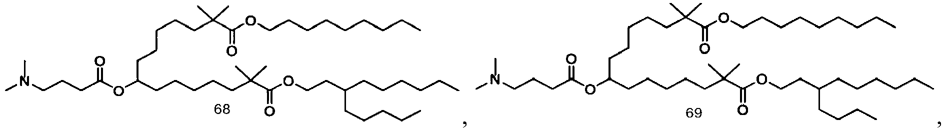 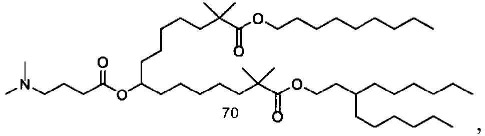 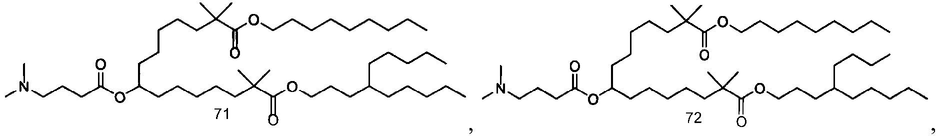 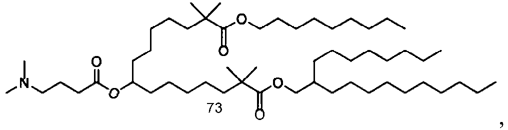 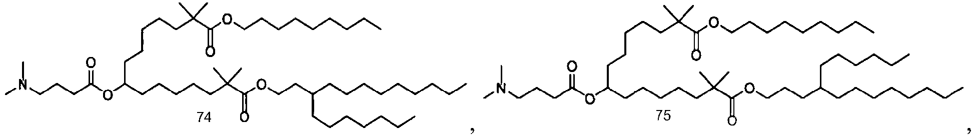 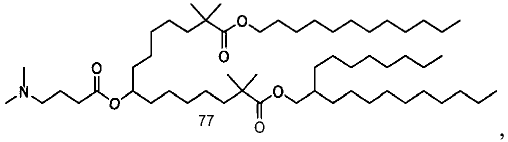 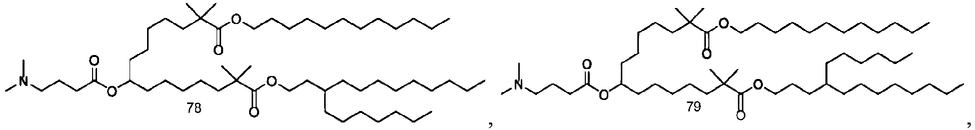 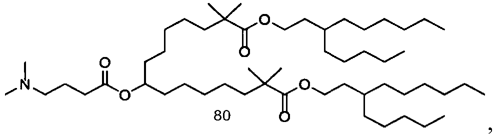 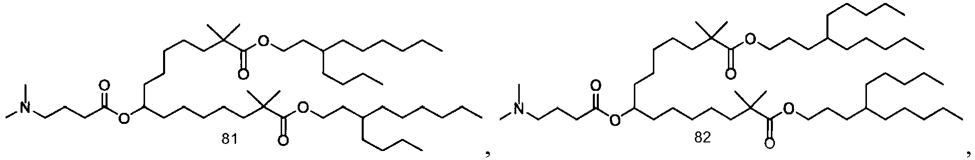 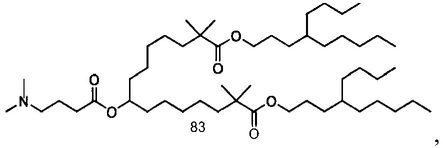 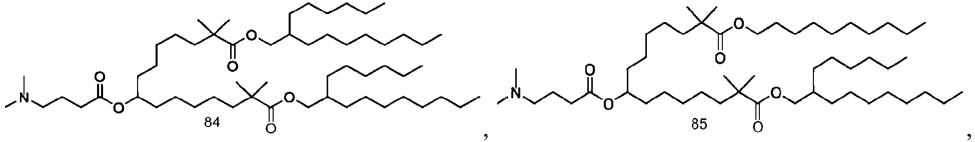 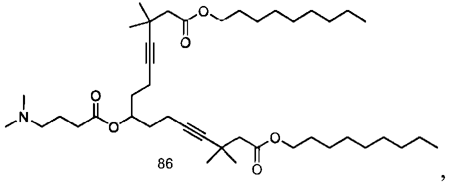 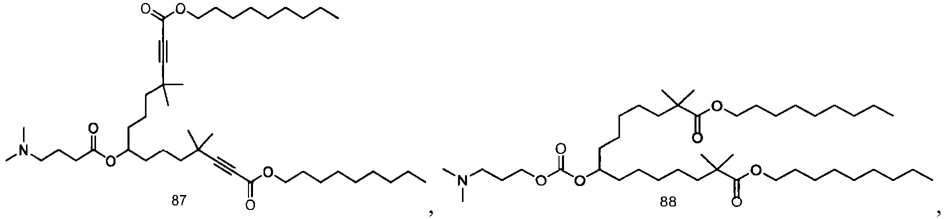 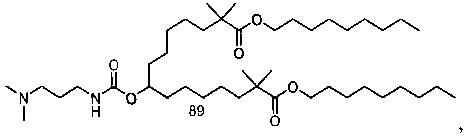 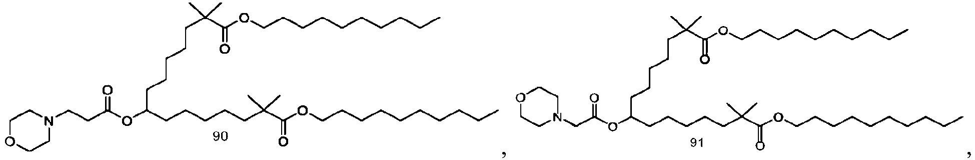 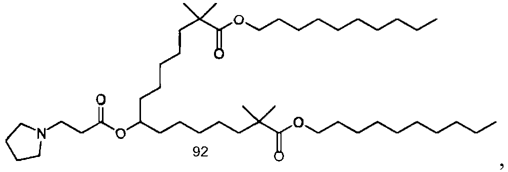 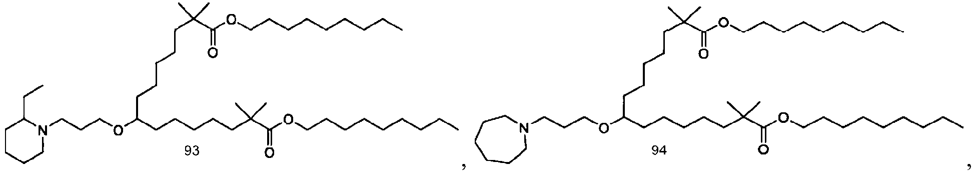 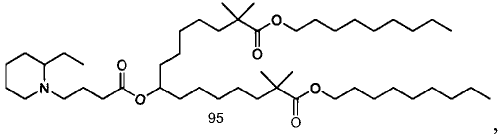 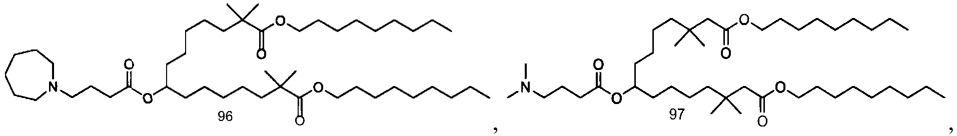 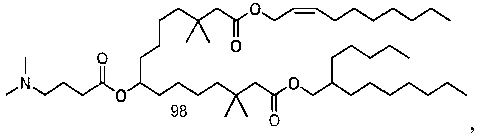 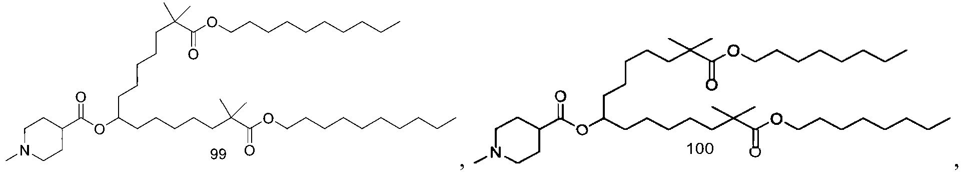 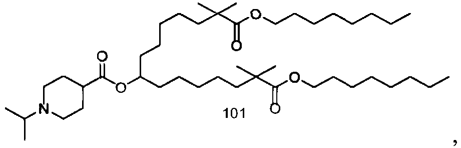 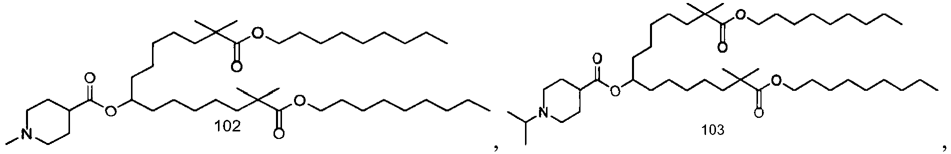 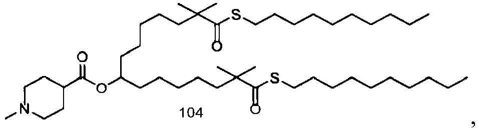 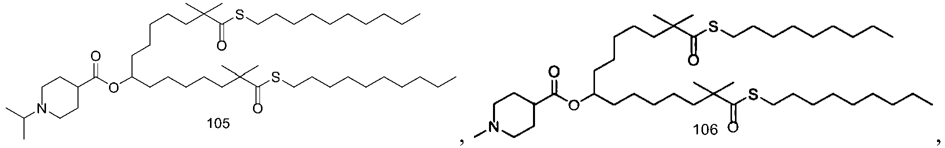 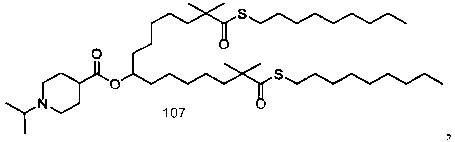 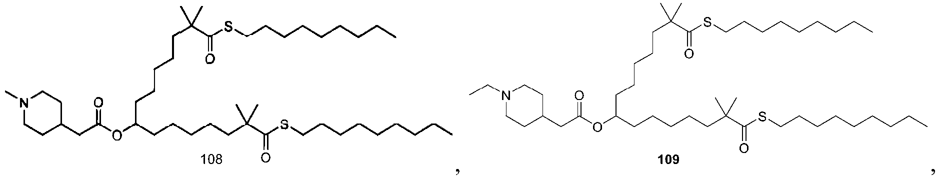 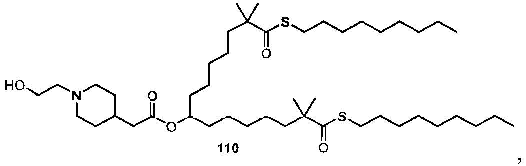 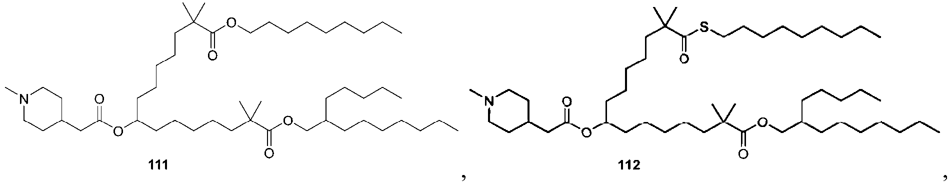 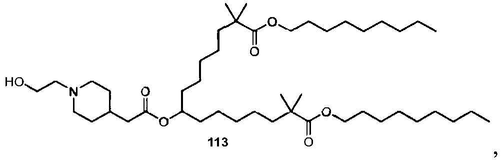 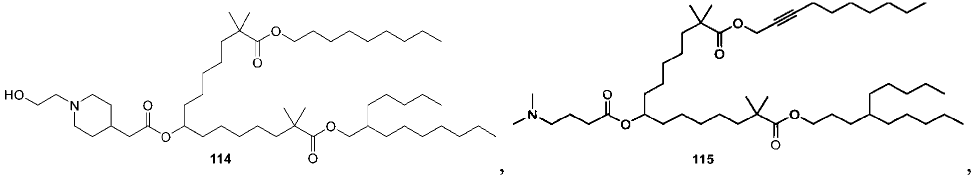 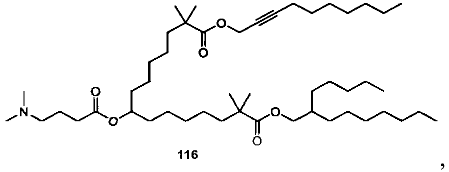 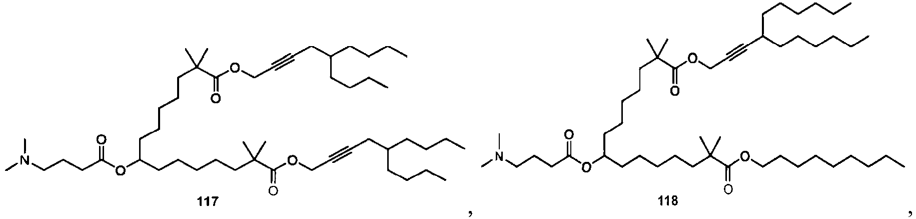 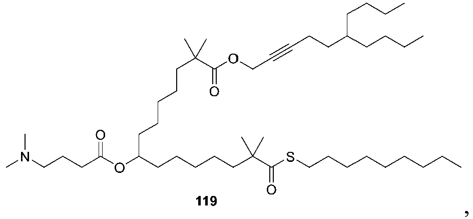 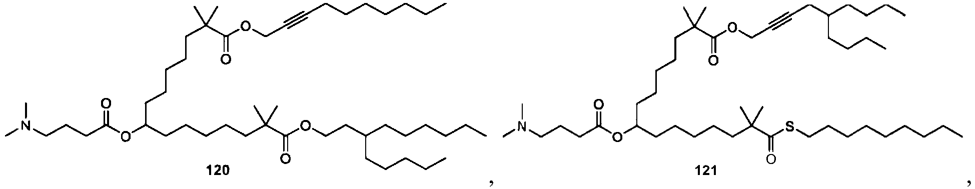 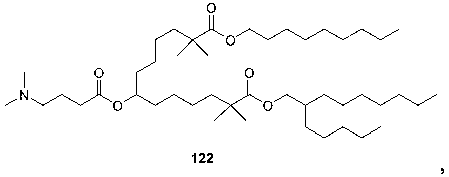 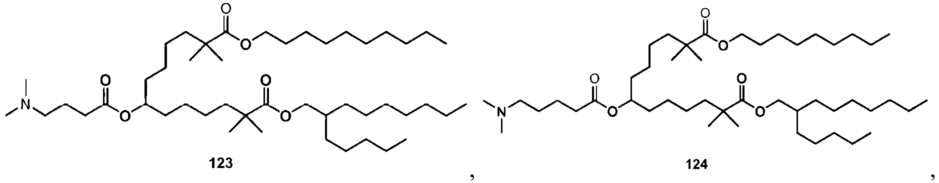 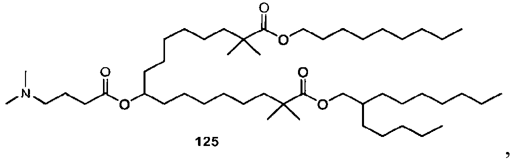 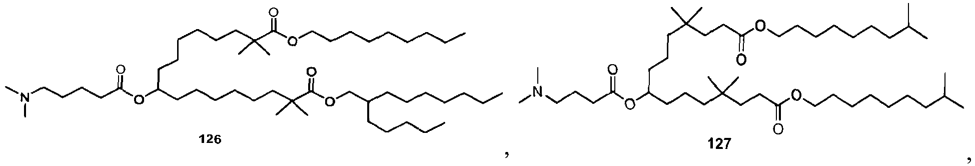 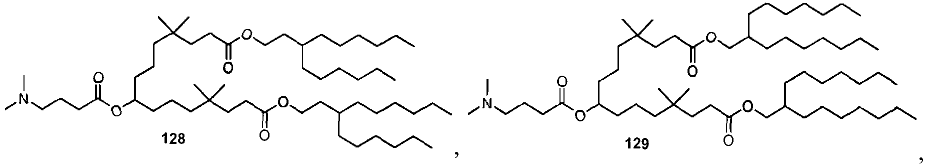 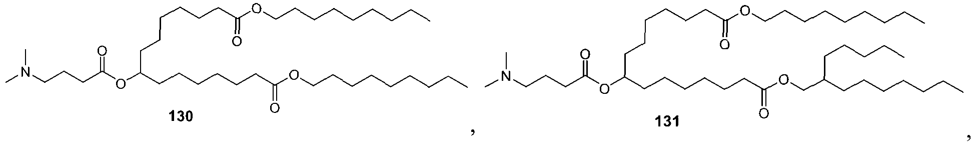 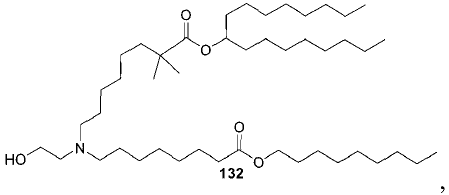 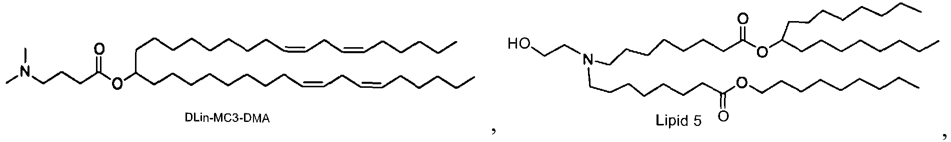 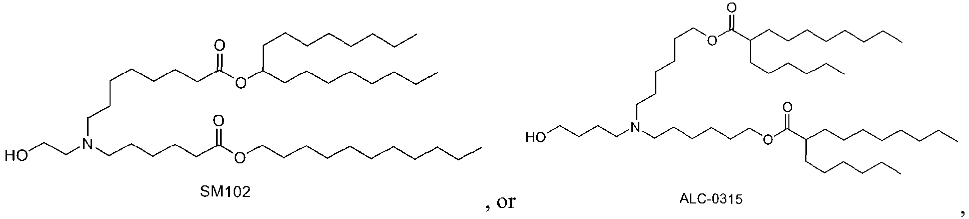 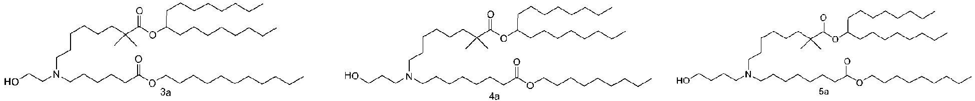 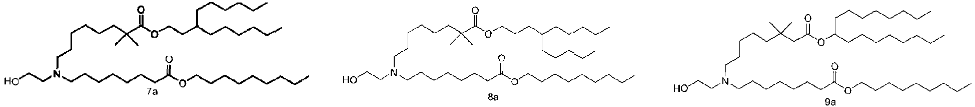 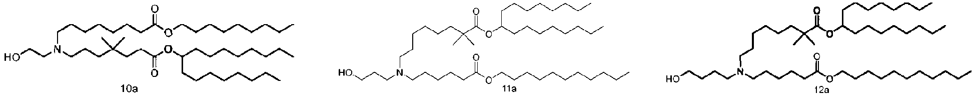 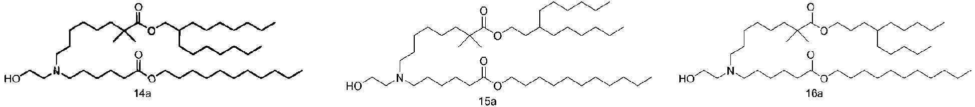 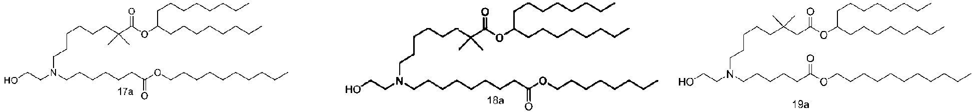 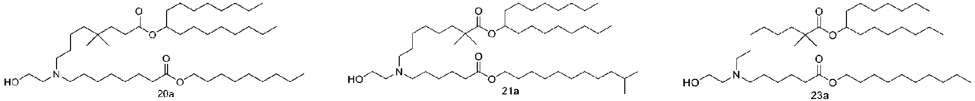 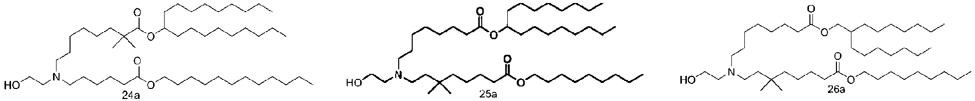 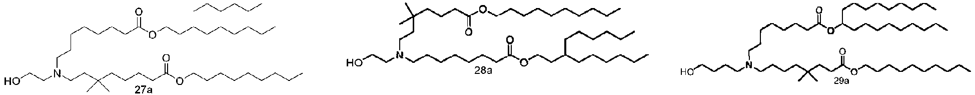 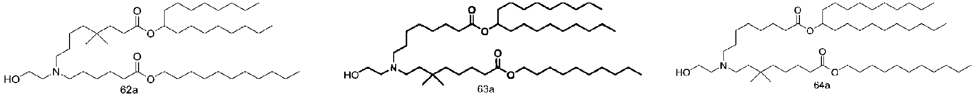 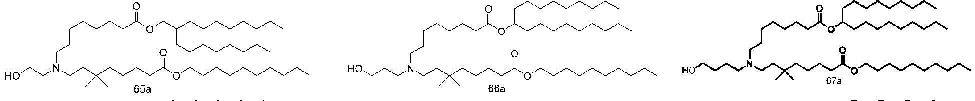 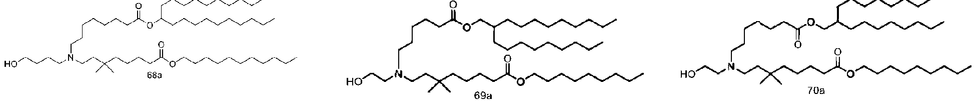 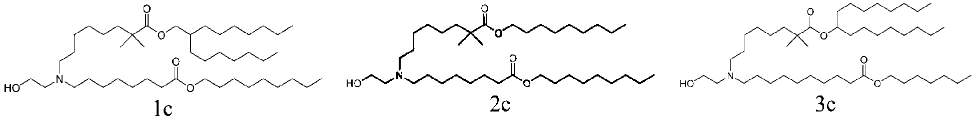 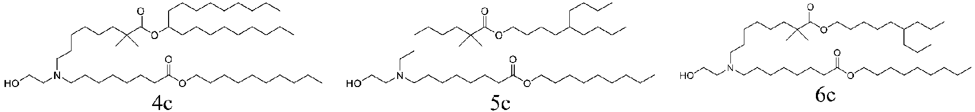 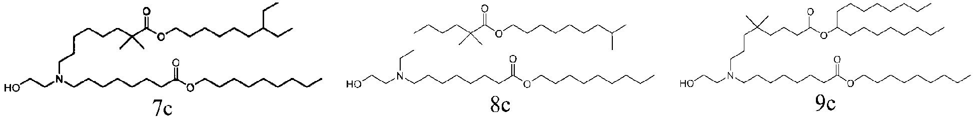 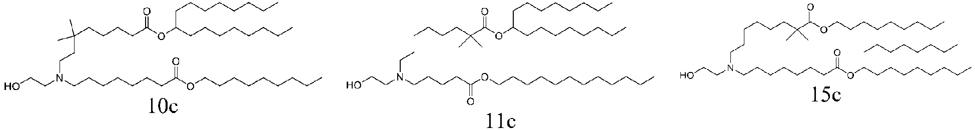 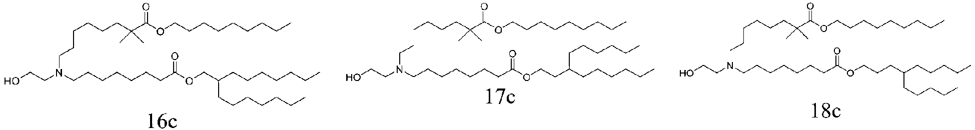 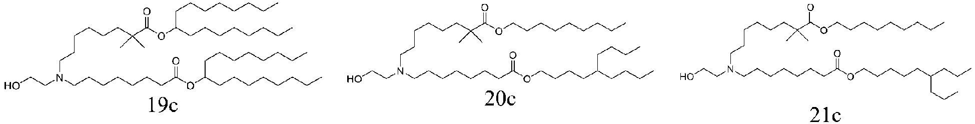 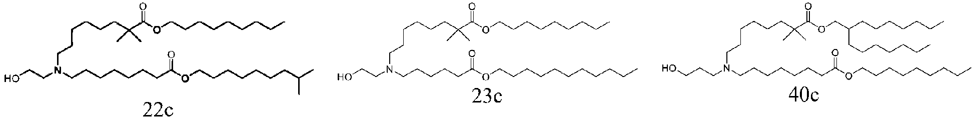 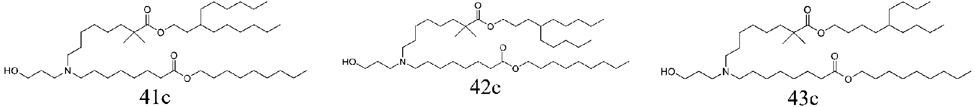 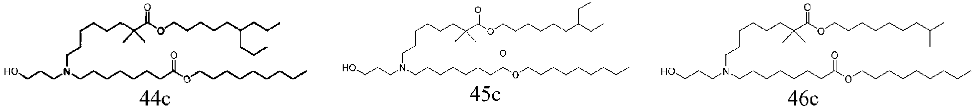 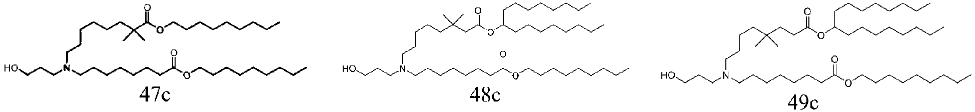 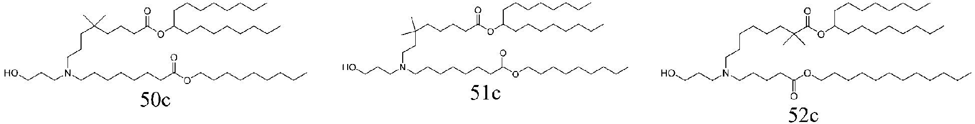 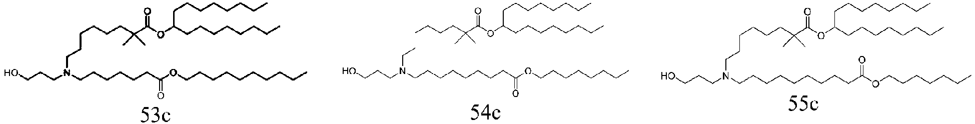 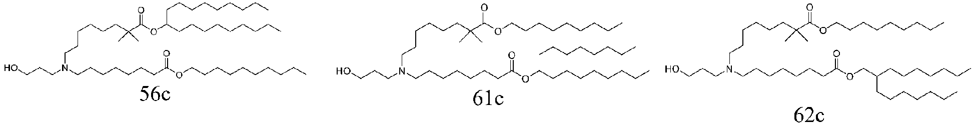 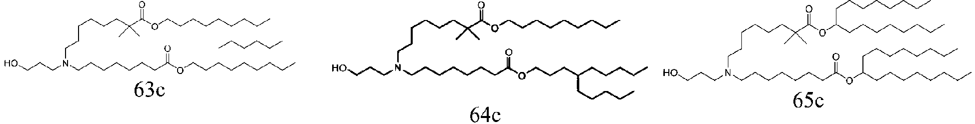 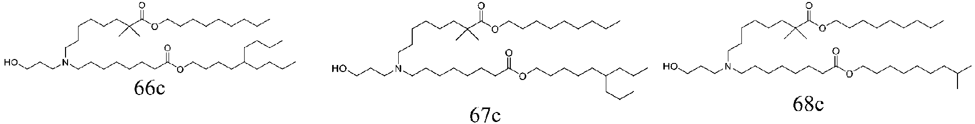 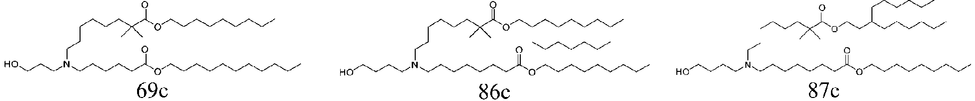 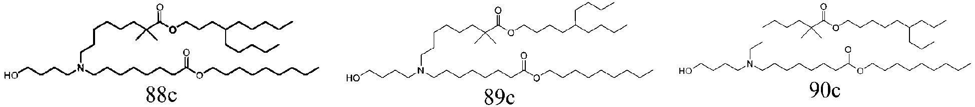 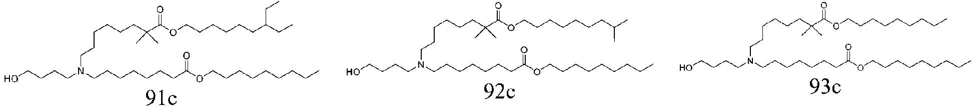 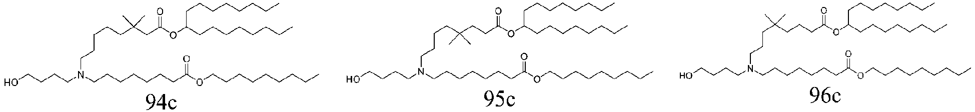 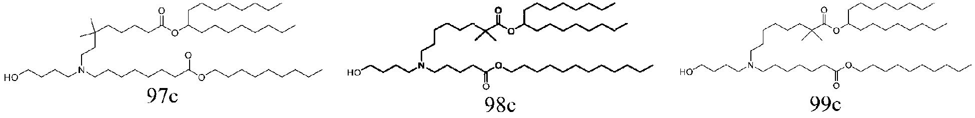 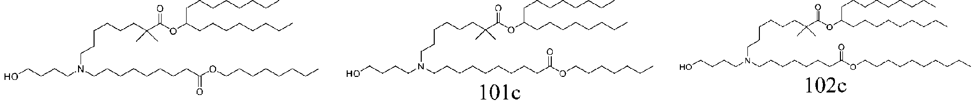 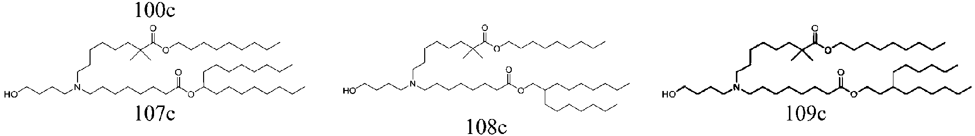 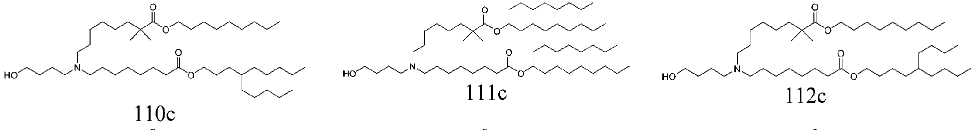 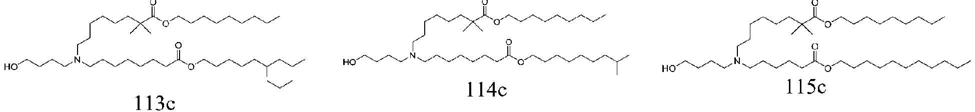 or a stereoisomer, a mixture of stereoisomers, or a pharmaceutically acceptable salt thereof.

Descriptions related to the ionizable lipids, including the preparation processes thereof, can be found in CN 115850104 A, the entirely of which is incorporated herein by reference.

In one embodiment, the ionizable lipid is DODAP, DODMA, DLinDMA, DLin-KC2-DMA, DLin-MC3-DMA, SM-102, or ALC-0315, or a combination thereof, or a stereoisomer, a mixture of stereoisomers, or a pharmaceutically acceptable salt thereof.

In one embodiment, the ionizable lipid is Compound 46 of formula: 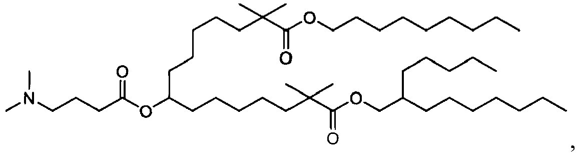 or a stereoisomer, a mixture of stereoisomers, or a pharmaceutically acceptable salt thereof.

In one embodiment, the ionizable lipid is Compound 132 of formula: 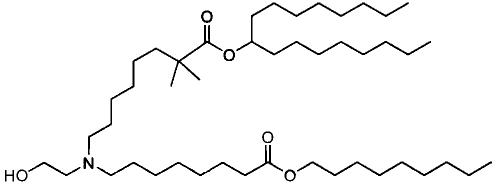 or a stereoisomer, a mixture of stereoisomers, or a pharmaceutically acceptable salt thereof.

In one embodiment, the ionizable lipid is: 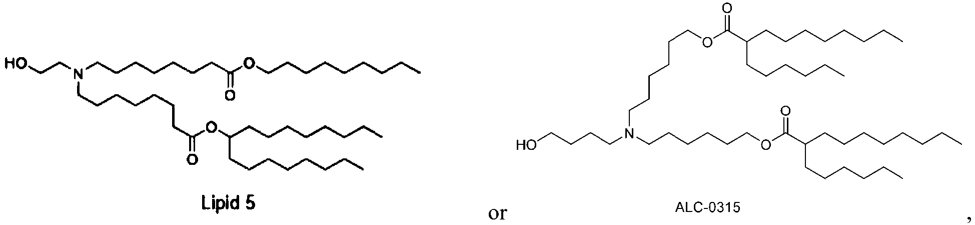 or a stereoisomer, a mixture of stereoisomers, or a pharmaceutically acceptable salt thereof.

In one embodiment, the amount of the ionizable lipid is from 35 mol % to 70 mol % of the total lipid present in the lipid nanoparticle, preferably 40 mol%-60 mol%, such as 35 mol%, 40 mol%, 45 mol%, 50% mol%, 55 mol% or 60%.

### 5.2.3. Phospholipid

As used herein, phospholipid refers to an amphiphilic lipid having a negatively charged phosphate group and one or more hydrophobic moieties. Phospholipids may assemble into one or more lipid bilayers. Phospholipids are made up of two fatty acid tails (hydrophobic moiety) and a phosphate group head, connected via glycerol, and may be natural or synthetic. Non-limiting examples of phospholipids include soybean lecithin, egg lecithin, phosphatidylglycerols, phosphatidylinositols, phosphatidylethanolamines, phosphatidic acids, sphingomyelin, diphosphatidylglycerols, phosphatidylserine, phosphatidylcholines, dimyristoylphosphatidylcholine, dimyristoyl phosphatidylglycerol, distearoyl phosphatidylglycerol, dipalmitoylphosphatidylcholine, and hydrogenated or partially hydrogenated lecithins. In some embodiments, the phospholipid is a phosphatidic acid (DMPA, DPPA, DSPA), a phosphatidylcholine (DDPC, DLPC, DMPC, DPPC, DSPC, DOPC, POPC, DEPC), a phosphatidylglycerol (DMPG, DPPG, DSPG, POPG), a phosphatidylethanolamine (DMPE, DPPE, DSPE, DOPE), or a phosphatidylserine (DOPS). A phospholipid may be a natural phospholipid, non-natural phospholipid, or synthetic phospholipid. A phospholipid may be modified or functionalized by various chemical reactions, such as being connected to alkynes for conjugation to an azide group. A phospholipid may be functionalized for targeting or imaging purposes.

In one embodiment, the lipid nanoparticle comprises a phospholipid. In one embodiment, phospholipid is distearoylphosphatidylcholine (DSPC). In one embodiment, phospholipid is dioleoylphosphatidylethanolamine (DOPE). In one embodiment, phospholipid is dimyristoylphosphatidylcholine (DMPC). In one embodiment, phospholipid is 1,2-Dioleoyl-sn-glycero-3-phosphocholine (DOPC). In one embodiment, phospholipid is dipalmitoylphosphatidylcholine (DPPC). In one embodiment, phospholipid is 1-palmitoyl-2-oleoyl-sn-glycero-3-phosphocholine (POPC). In one embodiment, phospholipid is 1,2-Dimyristoyl-sn-glycero-3-phosphoethanolamine (DMPE). In one embodiment, phospholipid is 1,2-dihexadecanoyl-sn-glycero-3-phosphoethanolamine (DPPE). In one embodiment, phospholipid is dipalmitoylphosphatidylcholine (DPPC). In one embodiment, phospholipid is hexadecanoyl-2-(9Z-Octadecanoyl)-sn-Glycero-3-Phosphoethanolamine (POPE).

In one embodiment, the lipid nanoparticle does not comprise a phospholipid, or comprises a phospholipid in an amount less than about 50 mol %, about 40 mol %, about 30 mol %, about 20 mol %, about 15 mol %, about 10 mol %, or about 5 mol % of the total lipid present in the lipid nanoparticle.

In one embodiment, the amount of the phospholipid is from 5 mol% to 30 mol% of the total lipid present in the lipid nanoparticle, more preferably 5-20 mol%, more preferably 5-15 mol%, such as 5 mol%, 6 mol%, 7 mol%, 8 mol%, 9 mol%, 10 mol%, 11 mol%, 12 mol%, 13 mol%, 14 mol%, 15 mol%, 16 mol%, 17 mol%, 18 mol%, 19 mol%, or 20 mol% of the total lipid present in the lipid nanoparticle.

### 5.2.4. Structural Lipid

In one embodiment, the lipid nanoparticle further comprises a structural lipid. Unless specified otherwise, the structural lipid is a neutral lipid that is different from the steroid compound described in Section 0. In one embodiment, the structural lipid does not comprise a cationic group. In one embodiment, the structural lipid does not comprise a tertiary amine group or a quaternary ammonium group.

In one embodiment, the structural lipid is a sterol. In one embodiment, the structural lipid is a neutral sterol. In one embodiment, the structural lipid is cholesterol. In certain embodiments, the structural lipid is an analog of cholesterol. In one embodiment, the structural lipid is sitosterol or beta-sitosterol. In one embodiment, the structural lipid is coprosterol. In one embodiment, the structural lipid is fucosterol. In one embodiment, the structural lipid is brassicasterol. In one embodiment, the structural lipid is ergosterol. In one embodiment, the structural lipid is tomatine. In one embodiment, the structural lipid is ursolic acid. In one embodiment, the structural lipid is α-tocopherol. In one embodiment, the structural lipid is stigmasterol. In one embodiment, the structural lipid is avenasterol. In one embodiment, the structural lipid is campesterol. In one embodiment, the structural lipid is solanine.

In one embodiment, the amount of the structural lipid is from 10 mol% to 80 mol% of the total lipid in the lipid nanoparticle, more preferably 15-70 mol%, more preferably 30-50 mol%, more preferably 30-40 mol%, such as 30 mol%, 35 mol%, 40 mol%, 45 mol%, 50 mol%, 55 mol% or 60 mol% of the total lipid in the lipid nanoparticle.

### 5.2.5. Polymer-Conjugated Lipid

In one embodiment, the lipid nanoparticle comprises a polymer-conjugated lipid. In one embodiment, the polymer-conjugated lipid is a pegylated lipid (PEG lipid). In one embodiment, the PEG lipid is a diglyceride which also comprises a PEG chain attached to the glycerol group. In one embodiment, the PEG lipid is a compound which contains one or more C₆-C₂₄ long chain alkyl or alkenyl group or a C₆-C₂₄ fatty acid group attached to a linker group with a PEG chain. Non-limiting examples of a PEG lipid includes a PEG modified phosphatidylethanolamine and phosphatidic acid, a PEG ceramide conjugated, PEG modified dialkylamines and PEG modified 1,2-diacyloxy propan-3-amines, PEG modified diacylglycerols and dialkylglycerols. In one embodiment, PEG modified distearoylphosphatidylethanolamine or PEG modified dimyristoyl-sn-glycerol is used. In one embodiment, the PEG modification is measured by the molecular weight of PEG component of the lipid. In one embodiment, the pegylated lipid has a molecule weight of from about 1000 Da to about 10,000 Da. In one embodiment, the pegylated lipid has a molecule weight of from about 1000 Da to about 5000 Da. In one embodiment, the pegylated lipid has a molecule weight of from about 1000 Da to about 2000 Da.

In one embodiment, the pegylated lipid is methoxypolyethyleneglycoloxy(2000)-N,N-ditetradecylacetamide (ALC-0159). In one embodiment, the pegylated lipid is 1,2-dimyristoyl-rac-glycero-3-methoxypolyethylene glycol-2000 (DMG-PEG2000). In one embodiment, the pegylated lipid is 1,2-Dimyristoyl-sn-glycero-3-phosphoethanolamine-polyethylene glycol 1000 (DMPE-PEG1000). In one embodiment, the pegylated lipid is 1,2-dipalmitoyl-sn-glycero-3-phosphoethanolamine-polyethylene glycol 1000 (DPPE-PEG1000). In one embodiment, the pegylated lipid is 1,2-distearoyl-sn-glycero-3-phosphoethanolamine-polyethylene glycol 1000 (DSPE-PEG1000). In one embodiment, the pegylated lipid is 1,2-dioleoyl-sn-glycero-3-phosphoethanolamine-polyethylene glycol 1000 (DOPE-PEG1000).

In one embodiment, the pegylated lipid is 1,2-dimyristoyl-rac-glycero-3-methoxypolyethylene glycol-2000 (Ceramide-PEG2000). In one embodiment, the pegylated lipid is 1,2-Dimyristoyl-sn-glycero-3-phosphoethanolamine-polyethylene glycol 2000 (DMPE-PEG2000). In one embodiment, the pegylated lipid is 1,2-dipalmitoyl-sn-glycero-3-phosphoethanolamine-polyethylene glycol 2000 (DPPE-PEG2000). In one embodiment, the pegylated lipid is 1,2-distearoyl-sn-glycero-3-phosphoethanolamine-polyethylene glycol 2000 (DSPE-PEG2000). In one embodiment, the pegylated lipid is 1,2-distearoyl-sn-glycero-3-phosphoethanolamine-N-(polyethylene glycol 2000)-Mannose (DSPE-PEG2000-Mannose). In one embodiment, the pegylated lipid is 1,2-dimyristoyl-rac-glycero-3-methoxypolyethylene glycol-5000 (Ceramide-PEG5000). In one embodiment, the pegylated lipid is 1,2-distearoyl-sn-glycero-3-phosphoethanolamine-polyethylene glycol 5000 (DSPE-PEG5000). In one embodiment, the pegylated lipid is 1,2-distearoyl-sn-glycero-3-phosphoethanolamine-N-[amino(polyethylene glycol)-2000 (DSPE-PEG2000 amine).

In one embodiment, the amount of the polymer conjugated lipid is from 0.25 mol% to 10 mol% of the total lipid in the lipid nanoparticle, more preferably 0.5-5 mol%, more preferably 0.5-3 mol%, such as 0.5 mol%, 0.6 mol%, 0.7 mol%, 0.8 mol%, 0.9 mol%, 1.0 mol%, 1.1 mol%, 1.2 mol%, 1.3 mol%, 1.4 mol%, 1.5 mol%, 1.6 mol%, 1.7 mol%, 1.8 mol%, 1.9 mol%, 2.0 mol%, 2.1 mol%, 2.2 mol%, 2.3 mol%, 2.4 mol%, 2.5 mol%, 2.6 mol%, 2.7 mol%, 2.8 mol%, 2.9 mol%, 3.0 mol%, 3.1 mol%, 3.2 mol%, 3.3 mol%, 3.4 mol%, 3.5 mol%, 3.6 mol%, 3.7 mol%, 3.8 mol%, 3.9 mol%, 4.0 mol% of the total lipid in the lipid nanoparticle.

### 5.2.6. Payload

In one embodiment, the lipid nanoparticle comprises a payload.

In one embodiment, the payload is a small molecule.

In one embodiment, the payload is a nucleic acid.

In one embodiment, the payload is an anticancer agents, antifungal agents, psychiatric agents such as analgesics, consciousness level-altering agents such as anesthetic agents or hypnotics, nonsteroidal antiinflammatory drugs (NSAIDS), anthelminthics, antiacne agents, antianginal agents, antiarrhythmic agents, anti-asthma agents, antibacterial agents, anti-benign prostate hypertrophy agents, anticoagulants, antidepressants, antidiabetics, antiemetics, antiepileptics, antigout agents, antihypertensive agents, antiinflammatory agents, antimalarials, antimigraine agents, antimuscarinic agents, antineoplastic agents, antiobesity agents, antiosteoporosis agents, antiparkinsonian agents, antiproliferative agents, antiprotozoal agents, antithyroid agents, antitussive agent, anti-urinary incontinence agents, antiviral agents, anxiolytic agents, appetite suppressants, beta-blockers, cardiac inotropic agents, chemotherapeutic drugs, cognition enhancers, contraceptives, corticosteroids, Cox-2 inhibitors, diuretics, erectile dysfunction improvement agents, expectorants, gastrointestinal agents, histamine receptor antagonists, immunosuppressants, keratolytics, lipid regulating agents, leukotriene inhibitors, macrolides, muscle relaxants, neuroleptics, nutritional agents, opioid analgesics, protease inhibitors, or sedatives.

In one embodiment, the payload is a protein. In one embodiment, the payload is a peptide. In one embodiment, the payload is an antibody. In one embodiment, the payload is a monoclonal antibody. In one embodiment, the payload is a bispecific antibody.

In one embodiment, the payload is antisense oligonucleotide (ASO). In one embodiment, the ASO comprises a naturally-occurring nucleoside. In one embodiment, the ASO comprises a modified nucleoside. In one embodiment, the ASO is capable of modulating expression of a target gene by hybridizing to a target nucleic acid, particularly a contiguous sequence on a target nucleic acid. In one embodiment, the ASO is singled stranded.

In one embodiment, the payload is deoxyribonucleic acid (DNA). In one embodiment, the payload is plasmid DNA (pDNA). In one embodiment, the payload is double stranded DNA (dsDNA). In one embodiment, the payload is single stranded DNA (ssDNA).

In one embodiment, the payload is ribonucleic acid (RNA). In one embodiment, the payload is RNA interference (RNAi). In one embodiment, the payload is small interfering RNA (siRNA). In one embodiment, the payload is short hairpin RNA (shRNA). In one embodiment, the payload is antisense RNA (aRNA). In one embodiment, the payload is messenger RNA (mRNA). In one embodiment, the payload is modified messenger RNA (mmRNA). In one embodiment, the payload is long noncoding RNA (lncRNA). In one embodiment, the payload is microRNA (miRNA). In one embodiment, the payload is small activating RNA (saRNA). In one embodiment, the payload is multicoding nucleic acid (MCNA). In one embodiment, the payload is polymer-coded nucleic acid (PCNA). In one embodiment, the payload is any RNA in the ribozyme.

In one embodiment, the payload is a clustered regularly interspaced short palindromic repeats (CRISPR) related nucleic acid. In one embodiment, the payload is guide RNA (gRNA). In one embodiment, the payload is CRISPR RNA (crRNA). In one embodiment, the payload comprises a first nucleic acid and a second nucleic acid. In one embodiment, the first nucleic acid is a messenger RNA. In one embodiment, the second nucleic acid is a single guide RNA. In one embodiment, the first nucleic acid is a messenger RNA (mRNA) and the second nucleic acid is a single guide RNA (sgRNA).

In one embodiment, the ratio of (total number of nitrogen atoms in the cationic steroid compound and ionizable lipid) and (total number of phosphorus atoms in the nucleic acid) is from about 1:1 to about 15:1 (N:P ratio). In one embodiment, the N:P ratio is from about 3:1 to about 12:1. In one embodiment, the N:P ratio is from about 4:1 to about 9:1. In one embodiment, the N:P ratio is about 4:1, about 5:1, about 6:1, about 7:1, about 8:1, or about 9:1. In one embodiment, the N:P ratio is about 6:1. In one embodiment, the N:P ratio is 6:1. In one embodiment, the N:P ratio is about 8:1. In one embodiment, the N:P ratio is 8:1. In one embodiment, the N:P ratio is about 4:1. In one embodiment, the N:P ratio is 4:1. In one embodiment, the N:P ratio is about 5.5:1. In one embodiment, the N:P ratio is 5.5:1.

In one embodiment, the amount of the payload delivered or expressed in a non-hepatic tissued of a subject by the lipid nanoparticle is higher than the amount of the payload delivered or expressed in the liver of the subject by the lipid nanoparticle, when the lipid nanoparticle is administered to the subject. In one embodiment, the amount of the payload delivered or expressed in the spleen of a subject by the lipid nanoparticle is higher than the amount of the payload delivered or expressed in the liver of the subject by the lipid nanoparticle, when the lipid nanoparticle is administered to the subject.

In one embodiment, the amount of the payload delivered or expressed in the spleen of the subject is higher than the amount of the payload delivered or expressed in the liver of the subject. In one embodiment, the amount of the payload delivered or expressed in the spleen of the subject is at least 2 times higher than the amount of the payload delivered or expressed in the liver of the subject. In one embodiment, the amount of the payload delivered or expressed in the spleen of the subject is at least 5 times higher than the amount of the payload delivered or expressed in the liver of the subject. In one embodiment, the amount of the payload delivered or expressed in the spleen of the subject is at least 10 times higher than the amount of the payload delivered or expressed in the liver of the subject. In one embodiment, the amount of the payload delivered or expressed in the spleen of the subject is at least 20 times higher than the amount of the payload delivered or expressed in the liver of the subject. In one embodiment, the amount of the payload delivered or expressed in the spleen of the subject is at least 40 times higher than the amount of the payload delivered or expressed in the liver of the subject. In one embodiment, the amount of the payload delivered or expressed in the spleen of the subject is at least 60 times higher than the amount of the payload delivered or expressed in the liver of the subject. In one embodiment, the amount of the payload delivered or expressed in the spleen of the subject is at least 100 times higher than the amount of the payload delivered or expressed in the liver of the subject.

In one embodiment, the amount of protein expressed by a nucleic acid in the spleen of a subject is higher than the amount of the protein expressed by the nucleic acid in the liver of the subject, when a lipid nanoparticle comprising the nucleic acid is administered to the subject. In one embodiment, the amount of protein expressed by an mRNA in the spleen of a subject is higher than the amount of the protein expressed by the mRNA in the liver of the subject, when a lipid nanoparticle comprising the mRNA is administered to the subject.

In one embodiment, the amount of protein expressed in the spleen of the subject is higher than the amount of protein expressed in the liver of the subject. In one embodiment, the amount of protein expressed in the spleen of the subject is at least 2 times higher than the amount of protein expressed in the liver of the subject. In one embodiment, the amount of protein expressed in the spleen of the subject is at least 5 times higher than the amount of protein expressed in the liver of the subject. In one embodiment, the amount of protein expressed in the spleen of the subject is at least 10 times higher than the amount of protein expressed in the liver of the subject. In one embodiment, the amount of protein expressed in the spleen of the subject is at least 20 times higher than the amount of protein expressed in the liver of the subject. In one embodiment, the amount of protein expressed in the spleen of the subject is at least 40 times higher than the amount of protein expressed in the liver of the subject. In one embodiment, the amount of protein expressed in the spleen of the subject is at least 60 times higher than the amount of protein expressed in the liver of the subject. In one embodiment, the amount of protein expressed in the spleen of the subject is at least 100 times higher than the amount of protein expressed in the liver of the subject.

### 5.3 A lipid nanoparticle composition

In one embodiment, provided herein is a lipid nanoparticle composition comprising the lipid nanoparticle described in Section 0. In one embodiment, the lipid nanoparticle composition described herein comprises the steroid compound described in Section 0 and the ionizable lipid described in Section 0. In one embodiment, the lipid nanoparticle composition described herein comprises the phospholipid described in Section 0. In one embodiment, the lipid nanoparticle composition described herein does not comprises phospholipid. In one embodiment, the lipid nanoparticle composition described herein comprises the structural lipid described in Section 0. In one embodiment, the lipid nanoparticle composition described herein comprises a polymer-conjugated lipid described in Section 0. In one embodiment, the lipid nanoparticle composition described herein comprises a payload described in Section 0.

In one embodiment, the lipid nanoparticle composition has a polydispersity index (PDI) of less than 0.2. In one embodiment, the lipid nanoparticle composition has a PDI of less than 0.15. In one embodiment, the lipid nanoparticle composition has a PDI of about 0.01, about 0.02, about 0.03, about 0.04, about 0.05, about 0.06, about 0.07, about 0.08, about 0.09, or about 0.10.

In one embodiment, the apparent pKa of the lipid nanoparticle composition is between 2 and 7. In one embodiment, the apparent pKa of the lipid nanoparticle composition is 2, 3, 4, 5, 6, or 7. In one embodiment, the apparent pKa of the lipid nanoparticle composition is less than 6. In one embodiment, the apparent pKa of the lipid nanoparticle composition is between 3 and 6.

In one embodiment, the apparent pKa is determined by 2-(p-toluidino)-6-naphthalene sulfonic acid (TNS) fluorescent methods. The TNS fluorescent method has been described in the art, such as Jayaraman M, et al., Maximizing the potency of siRNA lipid nanoparticles for hepatic gene silencing in vivo. Angew Chem Int Ed, 2012. 51, 8529-8533, which is incorporated herein by reference.

### 5.4 Pharmaceutical Composition

In one embodiment, provided herein is a pharmaceutical composition comprising the lipid nanoparticle described in Section 0 or the lipid nanoparticle composition described in Section 0. In one embodiment, the pharmaceutical composition further comprises a pharmaceutically acceptable carrier.

A pharmaceutically acceptable carrier for use in the present application includes a non-toxic carrier, adjuvant or vehicle which does not destroy the pharmacological activity of the compound formulated together. Pharmaceutically acceptable carriers that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

In one embodiment, the pharmaceutical compositions are formulated for oral administration. In one embodiment, the pharmaceutical compositions are formulated for intravenous administration. In one embodiment, the pharmaceutical compositions are formulated for intramuscular administration. In one embodiment, the pharmaceutical compositions are formulated for inhalation administration. In one embodiment, the administration is intraarterial administration. In one embodiment, the administration is intraperitoneal administration.

Generally, the pharmaceutical compositions provided herein are administered in an effective amount. The amount of the pharmaceutical composition actually administered will typically be determined by a physician, in the light of the relevant circumstances, including the condition to be treated or prevented, the chosen route of administration, the actual pharmaceutical composition administered, the age, weight, and response of the individual patient, the severity of the patient's symptoms, and the like.

When used to prevent the disorder of the present disclosure, the pharmaceutical compositions provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc.,* or may be continued indefinitely, for example, for the rest of the subject's life. In certain embodiments, the chronic administration is intended to provide a constant level of the compound in the blood, *e*.*g*., within the therapeutic window over the extended period of time.

The pharmaceutical compositions of the present disclosure may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g*., through an IV drip) allows a much faster delivery which quickly raises the concentration of the active ingredient in the blood to an effective level. In other embodiments, the pharmaceutical composition may be administered as a continuous infusion, *e*.*g*., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the active substance is usually a minor component (from about 0.1 to about 50% by weight or alternatively from about 1 to about 40% by weight) with the remainder being various vehicles or excipients and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

Also provided herein is a kit (e.g., pharmaceutical packs) comprising the pharmaceutical composition described herein. A kit described herein may include the pharmaceutical composition and other therapeutic, or diagnostic, or prophylactic agents, and a first and a second containers (e.g., vials, ampoules, bottles, syringes, and/or dispersible packages or other materials) containing the pharmaceutical composition or other therapeutic, or diagnostic, or prophylactic agents. In some embodiments, kits provided can also optionally include a third container containing a pharmaceutically acceptable excipient for diluting or suspending the nanoparticle composition of the present disclosure and/or other therapeutic, or diagnostic, or prophylactic agent. In some embodiments, the nanoparticle composition of the present application provided in the first container and the other therapeutic, or diagnostic, or prophylactic agents provided in the second container is combined to form a unit dosage form.

### 5.5 Methods of Making or Producing a Lipid Nanoparticle

Also provided herein is a method of producing a lipid nanoparticle comprising the steps of:
(i) dissolving in a first solution a mixture comprising the lipids described herein, wherein the lipid solution is formed in an organic solvent;
(ii) dissolving in a second solution a payload to form a payload solution; and
(iii) mixing the lipid solution and the payload solution to form the lipid nanoparticles.

In one embodiment, the organic solvent is a water-miscible organic solvent. In one embodiment, the organic solvent is an alcohol. In one embodiment, the organic solvent is ethanol.

In one embodiment, the second solution is an aqueous solution. In one embodiment, the second solution is an aqueous solution of pH below 7. In one embodiment, the second solution is an aqueous solution of pH between 3 and 6. In one embodiment, the second solution is an aqueous solution of pH about 3.5, about 4, about 4.5, about 5, about 5.5, or about 6. In one embodiment, the second solution is a sodium acetate buffer solution having a pH of about 4.5.

### 5.6 Methods of Treatment

In one embodiment, provided herein is a method of treating or preventing a disease or disorder in a subject. In one embodiment, the method comprises administering to a subject the lipid nanoparticle described in Section 0. In one embodiment, the method comprises administering to a subject the lipid nanoparticle composition described in Section 0. In one embodiment, the method comprises administering to a subject the pharmaceutical composition described in Section 0.

In one embodiment, provided herein is a method of treating or preventing a disease or disorder in a subject, comprising administering to the subject a therapeutically effective amount of a lipid nanoparticle comprising (i) a steroid compound (see Section0) and (ii) an ionizable lipid (Section 0).

In one embodiment, provided herein is a lipid nanoparticle for use in delivering or expressing a payload in the spleen of a subject, wherein the lipid nanoparticle is administered to the subject via a systemic administration route, wherein the lipid nanoparticle comprises an ionizable lipid and a steroid compound, and wherein the steroid compound comprises one or more cationic groups (e.g. steroid compound in Section 0).

In one embodiment, the administration is systemic administration. In one embodiment, the administration is intravenous administration. In one embodiment, the administration is intraarterial administration. In one embodiment, the administration is intraperitoneal administration. In one embodiment, the administration is oral administration. In one embodiment, the administration is intramuscular administration.

In one embodiment, provided herein is a method of treating a splenic disease in a subject. In one embodiment, provided herein is a method of treating spleen-related disease in a subject.

In one embodiment, the splenic disease or spleen related disease includes hematologic disorders, infectious diseases, or cancer.

In one embodiment, said splenic disease or spleen-related disease includes hemolytic anemia; thrombocytopenic purpura; chronic leukemia; lymphoma such as Hodgkin lymphoma or non-Hodgkin lymphoma; myelodysplastic syndrome; myelofibrosis; splenomegaly; lipid metabolism disorders such as Gaucher disease and sphingomyelinosis; splenomegaly and hypersplenism associated with sepsis, typhoid, infectious mononucleosis or subacute bacterial endocarditis; splenomegaly and hypersplenism associated with malaria, tuberculosis, Kala-azar or other related diseases; congestive splenomegaly and hypersplenism due to cirrhosis of the liver and portal hypertension; splenic cysts such as parasitic splenic cysts or pseudosplenic cysts; splenic abscesses; primary splenic tumors; splenic metastases; splenic artery aneurysms; splenic infarction; splenic purpura; splenic rupture; and the like.

In one embodiment, provided herein is a method of delivering or expressing a payload described in Section 0 in a subject, comprising administration to the subject a therapeutically effective amount of the lipid nanoparticle described in Section 0 or the population of lipid nanoparticle described in Section 0.

In one embodiment, the amount of payload delivered or expressed in the spleen of the subject is higher than the amount of the payload delivered or expressed in the liver of the subject. In one embodiment, the amount of payload delivered or expressed in the spleen of the subject is at least 2 times higher than the amount of payload delivered or expressed in the liver of the subject. In one embodiment, the amount of payload delivered or expressed in the spleen of the subject is at least 5 times higher than the amount of payload delivered or expressed in the liver of the subject. In one embodiment, the amount of payload delivered or expressed in the spleen of the subject is at least 10 times higher than the amount of payload delivered or expressed in the liver of the subject. In one embodiment, the amount of payload delivered or expressed in the spleen of the subject is at least 20 times higher than the amount of payload delivered or expressed in the liver of the subject. In one embodiment, the amount of payload delivered or expressed in the spleen of the subject is at least 40 times higher than the amount of payload delivered or expressed in the liver of the subject. In one embodiment, the amount of payload delivered or expressed in the spleen of the subject is at least 60 times higher than the amount of payload delivered or expressed in the liver of the subject. In one embodiment, the amount of payload delivered or expressed in the spleen of the subject is at least 100 times higher than the amount of payload delivered or expressed in the liver of the subject.

In one embodiment, the amount of protein expressed in the spleen of the subject by an mRNA encapsulated in the LNP is higher than the amount of the protein expressed in the liver of the subject by the mRNA encapsulated in the LNP. In one embodiment, the amount of protein expressed in the spleen of the subject by an mRNA encapsulated in the LNP is at least 5 times, at least 10 times, at least 20 times, at least 30 times, at least 40 times, at least 50 times, at least 60 times, at least 70 times, at least 80 times, at least 90 times, or at least 100 times higher than the amount the protein expressed in the liver of the subject by the mRNA encapsulated in the LNP.

In one embodiment, the ratio (spleen/liver ratio) of the amount of payload delivered or expressed in the spleen of the subject and the amount of the payload delivered or expressed in the liver of the subject is greater than 1. In one embodiment, the spleen/liver ratio is greater than 2. In one embodiment, the spleen/liver ratio is greater than 5. In one embodiment, the spleen/liver ratio is greater than 10. In one embodiment, the spleen/liver ratio is greater than 15. In one embodiment, the spleen/liver ratio is greater than 20. In one embodiment, the spleen/liver ratio is greater than 25. In one embodiment, the spleen/liver ratio is greater than 30. In one embodiment, the spleen/liver ratio is greater than 35. In one embodiment, the spleen/liver ratio is greater than 40. In one embodiment, the spleen/liver ratio is greater than 50. In one embodiment, the spleen/liver ratio is greater than 60. In one embodiment, the spleen/liver ratio is greater than 70. In one embodiment, the spleen/liver ratio is greater than 80. In one embodiment, the spleen/liver ratio is greater than 90. In one embodiment, the spleen/liver ratio is greater than 100.

### 6. EXAMPLES

In order to make the technical solutions of the present disclosure clearer and more explicit, the present disclosure is further elaborated through the following examples. The following examples are used only to illustrate specific embodiments of the present disclosure so that a person skilled in the art can understand the present application, but are not intended to limit the scope of protection of the application. The technical means or methods, etc. not specifically described in the specific embodiments of the present disclosure are conventional technical means or methods, etc. in the art. The materials, reagents, etc. used in examples are commercially available if not otherwise specified.

**Table 1 Abbreviations**

| **Abbreviation** | **Full name** |
|---|---|
| THF | Tetrahydrofuran |
| DCM | dichloromethane |
| MeOH | methanol |
| DMF | N, N-Dimethylformamide |
| DMSO | Dimethyl sulfoxide |
| DCE | 1,2-Dichloroethane |
| CDCl₃ | Deuterated chloroform |
| TBAI | Tetrabutylammonium iodide |
| TsCH₂CN | 4-Toluenesulfonylacetonitrile |
| TMSOK | Potassium trimethylsiloxide |
| TBDMSCl | tert-Butyldimethylsilyl chloride |
| LDA | Lithium diisopropylamide |
| DMAP | 4-Dimethylaminopyridine |
| (COCl)₂ | Oxalyl chloride |
| SOCl₂ | Thionyl dichloride |
| NaBH₄ | Sodium borohydride |
| NaH | Sodium hydride |
| K₂CO₃ | Potassium carbonate |
| EDCI | 1-(3-Dimethylaminopropyl)-3-ethylcarbodiimide |
| DIPEA | N,N-Diisopropylethylamine |
| Et₃N | Triethylamine |
| AcOH | Acetic acid |
| NaBH₃CN | Sodium cyanoborohydride |
| Imidazole | Imidazole |
| NMO | 4-Methylmorpholine N-oxide |
| BDMEP | 2,6-di-tert-Butylpyridine |

### Example 1.1: Synthesis of compound 46

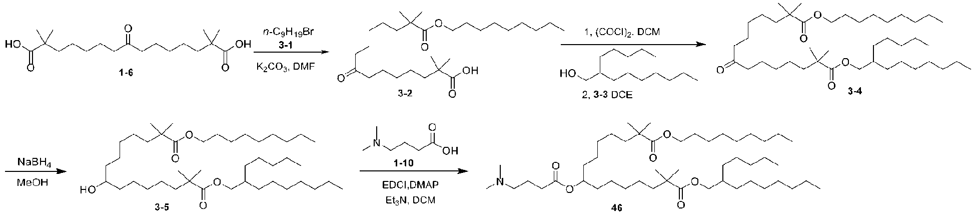

To a solution of compound 1-6 (959 mg, 2.8 mmol, 1.0 eq.) and 3-1 (638 mg, 3.08 mmol, 1.1 eq.) in DMF was added potassium carbonate (1.55 g, 11.2 mmol, 4.0 eq.). The mixture was heated at 60 °C for 4 hours, and then cooled to room temperature and quenched with a saturated sodium chloride aqueous solution. The resulting solution was extracted with ethyl acetate, and the organic phase was combined, dried over anhydrous sodium sulfate, filtered and concentrated to obtain a crude product, which was further purified by silica gel column chromatography to yield compound 3-2 (682 mg).

Compound 3-2 (324 mg, 0.69 mmol, 1.0 eq.) was dissolved in 5.0 mL of dichloromethane, and the reaction system was cooled to 0 °C in an ice bath. 2 drops of DMF were added and oxalyl chloride (0.24 mL, 2.8 mmol, 4.0 eq.) was then added dropwise to the reaction solution. The ice bath was removed after the dropwise addition was complete, and the mixture was stirred for 1 h at room temperature. The solvent was removed using a rotary-evaporator to give acyl chloride crude product (309 mg) as an oil, which was used directly in the next reaction step.

Compound 3-3 (407 mg, 1.9 mmol, 3.0 eq) was added to a solution of crude acyl chloride (309 mg) in DCE (3.0 mL), and the reaction was heated to 70 °C to react overnight. The reaction solution was cooled to room temperature and the solvent was removed using a rotary-evaporator to give the crude product, which was purified by silica gel column to give compound 3-4 (325 mg).

Compound 3-4 was dissolved in 4.0 mL methanol, and NaBH₄ (28 mg, 0.73 mmol) was added at room temperature. TLC indicates the disappearance of the starting materials. The mixture was quenched by saturated NaCl aqueous solution, extracted with dichloromethane (x3), which was combined and dried through anhydrous sodium sulfate, filtered and concentrated under vacuum to obtain the crude product 3-5 (260 mg), which was used in the next step without purification.

Crude compound 3-5 (260 mg, 0.39 mmol, 1.0 eq.) was dissolved in 5.0 mL of dichloromethane, and Compound 1-10 (77.4 mg, 0.59 mmol, 1.5 eq.), EDCI (224 mg, 1.17 mmol, 3.0 eq.), triethylamine (0.16 mL, 1.17 mmol, 3.0 eq.) and DMAP (48 mg, 0.39 mmol, 1.0 eq.) were added to the reaction system. The reaction solution was stirred at room temperature for 12 h. The reaction solution was then quenched by adding saturated sodium chloride solution, and extracted with dichloromethane. The organic phases were combined, dried over anhydrous sodium sulfate, and filtered. The filtrate was collected and concentrated to give the crude product, which was passed through preparative high performance liquid chromatography to give compound 46 (32.6 mg) as an oily product. **¹H NMR** (400 MHz, CDCl₃): δ ppm 0.88 (t, *J* = 7.2 Hz, 9H), 1.14 (s, 12H), 1.15-1.28 (m, 37H), 1.47-1.59 (m, 18H), 1.75-1.84 (m, 2H), 2.24-2.35 (m, 10H), 3.95 (d,*J* = 5.6 Hz, 2H), 4.03 (t, *J* = 6.8 Hz, 2H), 4.80-4.87 (m, 1H); **MS m/z [M+H]⁺ (ESI):** 780.7.

### Example 1.2: Synthesis of compound 132

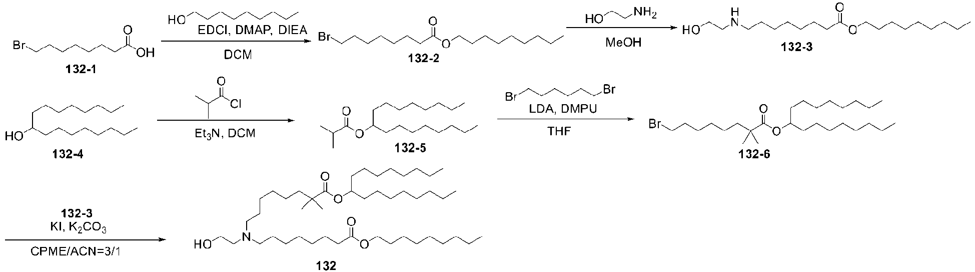

1-nonanol (15 g, 104.0 mmol, 1.0 eq.), 8-Bromooctanoic acid (25.5 g, 114.0 mmol, 1.1 eq.), DMAP (2.54 g, 20.8 mmol, 0.2 eq.), DIEA (40.3 g, 312.0 mmol, 3.0 eq.), and EDCI (25.9 g, 135 mmol, 1.3 eq.) were dissolved in 250 mL of DCM at room temperature. The reaction solution was stirred at room temperature for 4 hours, and then quenched with 200 mL of saturated sodium chloride aqueous solution. The resulting solution was extracted with 3 x 100 mL DCM, and the organic phase was combined, dried over anhydrous sodium sulfate, filtered and concentrated to obtain a crude product, which was further purified by silica gel column chromatography to yield 21 g of yellow oily compound 132-2 (682 mg).

Compounds 132-2 (21 g, 60.1 mmol, 1.0 eq.) and 2-hydroxyethylamine (110 g, 1.80 mol, 30.0 eq.) were dissolved in 100 mL of methanol at room temperature. The mixture was heated to 60 °C and stirred for 18 hours. The solvent was removed to obtain the crude product. The crude product was dissolved in saturated ammonium chloride aqueous solution and ethyl acetate, extracted, and the organic phase was combined, dried over anhydrous sodium sulfate, filtered and concentrated and further purified by silica gel column chromatography to obtain yellow oily compound **132-3** (14 g).

9-Heptadecanol (9.8 g, 38.2 mmol, 1.0 eq.) and Triethylamine(15.5 g, 152.8 mmol, 4.0 eq.) were dissolved in 100 mL DCM at room temperature. The reaction system was cooled by an ice bath, isobutyryl chloride (9.8 g, 91.7 mmol, 2.4 eq.) was added slowly, the resulting solution was stirred at room temperature overnight, and then quenched with saturated ammonium chloride aqueous solution. The resulting solution was extracted with DCM, and the organic phase was combined, dried over anhydrous sodium sulfate, filtered and concentrated to obtain a crude product, which was further purified by silica gel column chromatography to obtain yellow oily compound **132-5** (8.7 g).

Compound **132-5** (8.7 g, 26.6 mmol, 1.0 eq.) was dissolved in THF, then cooled to -45 °C, LDA (13.1 mL, 26.2 mmol, 0.98 eq.) was added dropwise slowly, and then the mixture was stirred 1h, then 1,6-dibromohexane (9.03 g, 37.0 mmol, 1.39 eq.) and DMPU (0.48 g, 3.73 mmol, 0.14 eq.) were added, The reaction solution was stirred at room temperature overnight, and then quenched with saturated ammonium chloride aqueous solution, the resulting solution was extracted with ethyl acetate, and the organic phase was combined, dried over anhydrous sodium sulfate, filtered and concentrated to obtain a crude product, which was further purified by silica gel column chromatography to obtain yellow oily compound **132-6** (10.1 g).

Compounds **132-6** (1.48 g, 3.04 mmol, 2.0 eq.) and **132-3** (500 mg, 1.52 mmol, 1.0 eq.), K₂CO₃ (628.2 mg, 4.55 mmol, 3.0 eq.), KI (302.3 mg, 1.82 mmol, 1.2 eq.) were dissolved in cyclopentyl methyl ether (7.5 mL) and acetonitrile (2.5 mL). The resulting mixture was heated to 80 °C. When the reaction finished, the reaction was quenched with saturated ammonium chloride ice water solution, extracted with DCM. The organic phase was combined, dried over anhydrous sodium sulfate, filtered, and concentrated to obtain the crude product, which was further purified by silica gel column chromatography to obtain oily compound **132** (96.98 mg). **¹H NMR** (400 MHz, CDCl₃) δ: 4.86-4.80 (m, 1H), 4.04 (t, *J* = 7.2 Hz, 2H), 3.57 (m, 2H), 2.62 (t, *J* = 7.2 Hz, 2H), 2.49 (t, *J* = 7.2 Hz, 4H), 2.29 (t, *J* = 7.2 Hz, 2H), 1.62 (m, 4H), 1.51-1.45 (m, 10H), 1.38-1.23 (m, 49H), 1.15 (s, 6H), 0.88 (t, *J* = 7.2 Hz, 9H); **ESI-MS** m/z: 738.60 [M+H]⁺.

### Example 2: Synthesis of compound Lys-es-Cholesterol

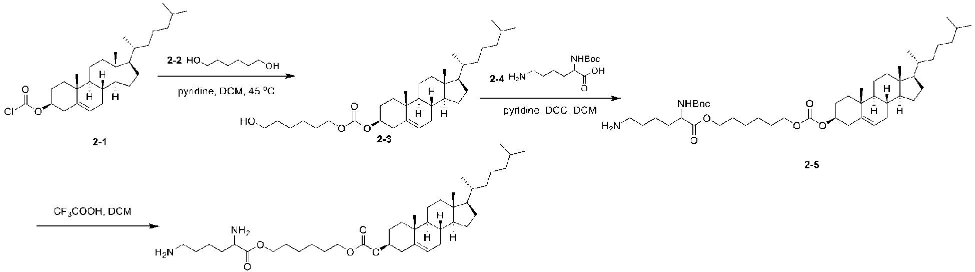

### (pyridine: pyridine)

The Cholesteryl chloroformate 2-1 (1.0 g, 2.2 mmol, 1.0 eq.) and pyridine (18 µL, 0.22 mmol, 0.1 eq.) were dissolved in 20 mL of dichloromethane. Then 1,6-hexanediol 2-2 (1.1 mL, 8.8 mmol, 4.0 eq.) was added to the reaction mixture, which was then heated at 45 °C for 12 hours. After the reaction, the reaction mixture was cooled to room temperature, quenched with saturated sodium chloride solution, extracted with dichloromethane, which was then combined and dried over anhydrous sodium sulfate. The solution was filtered and dried to obtain the crude product, which was purified by silica gel column chromatography to obtain the intermediate compound 2-3 (879 mg).

Compound 2-4 (0.49 g, 2.0 mmol, 1.0 eq.), pyridine (0.24 mL, 3.0 mmol, 1.5 eq.), and DCC (0.62 g, 3.0 mmol, 1.5 eq.) were dissolved in dichloromethane (20 mL). Then, compound 2-3 (879 mg, 1.7 mmol, 0.85 eq.) prepared above was added to the reaction mixture. The reaction was carried out at room temperature until complete conversion is observed by TLC. The reaction mixture was quenched by saturated sodium chloride solution, extracted with dichloromethane, and the organic phases were combined and dried over anhydrous sodium sulfate. The mixture was filtered, concentrated to obtain the crude product, which was then purified by silica gel column chromatography to yield intermediate compound 2-5 (641 mg).

Compound 2-5 (200 mg) was dissolved in a mixture of dichloromethane (4.0 mL) and trifluoroacetic acid (2.0 mL). The mixture was stirred at room temperature until the complete reaction of compound 2-5 was achieved. The organic solvent and trifluoroacetic acid were then removed by rotary evaporation, resulting in a solid crude product, which was dispersed in a mixture of methanol and n-hexane. Then the slurry was filtered and dried to yield the final product Lys-es-Cholesterol (62.2 mg). **¹H NMR** (400 MHz, CD₃OD): *δ* 0.74 (s, 3H), 0.88 (d, *J* = 6.8 Hz, 6H), 0.90-1.68 (m, 38H), 1.75-2.18 (m, 8H), 2.28-2.38 (m, 2H), 2.96 (t, *J* = 7.6 Hz, 2H), 4.08 (t, *J* = 6.8 Hz, 1H), 4.12 (t, *J* = 6.8 Hz, 4H), 4.28-4.30 (m, 2H), 4.30-4.47 (m, 1H), 5.41-5.43 (m, 1H); **MS m/z [M+H]⁺ (ESI):** 659.5.

### Example 3: Synthesis of compound Arg-es-Cholesterol

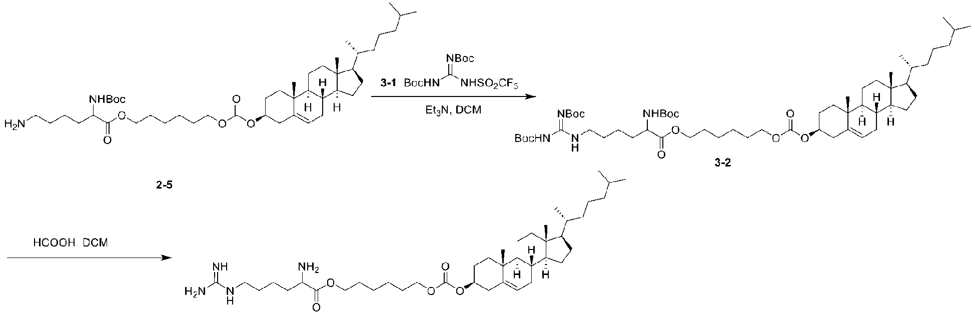

Compound 2-5 (200 mg, 0.26 mmol, 1.0 eq.) prepared in Example 2 and compound 3-1 (113 mg, 0.29 mmol, 1.1 eq.) were dissolved in dichloromethane (5.0 mL). Triethylamine (72 µL, 0.52 mmol, 2.0 eq.) was added to the reaction solution, which was then allowed to react until complete consumption of the substrate was confirmed by TLC analysis. The reaction solution was cooled to room temperature, and the reaction was quenched with saturated sodium chloride solution, extracted with dichloromethane and the organic phases were combined and dried over anhydrous sodium sulfate. The mixture was filtered and concentrated to obtain the crude product, which was further purified by silica gel column chromatography to yield intermediate compound 3-2 (174 mg).

Compound 3-2 (174 mg) was dissolved in dichloromethane (4.0 mL) and formic acid (2.0 mL). The reaction mixture was stirred at room temperature until compound 3-2 mostly disappeared. The solvent was removed using a rotary evaporator to obtain the crude product. The obtained crude product was dispersed in a mixture of methanol and n-hexane, and then the slurry was filtered and dried to obtain the final product Arg-es-Cholesterol (60.2 mg). **¹H NMR** (400 MHz, CD₃OD): *δ* 0.73 (s, 3H), 0.88 (d, *J* = 6.8 Hz, 3H), 0.94 (d, *J* = 6.8 Hz, 3H), 1.05-1.44 (m, 14H), 1.53-1.72 (m, 24H), 1.74-2.08 (m, 8H), 2.37 (m, 2H), 3.21 (t, *J* = 6.8 Hz, 2H), 3.82 (t, *J* = 6.4 Hz, 1H), 4.11 (t, *J* = 6.4 Hz, 2H), 4.22 (t, *J* = 6.4 Hz, 2H), 4.20-4.40 (m, 1H), 5.40-5.42 (m, 1H); **MS m/z [M+H]⁺ (ESI):** 701.5.

### Example 4: Synthesis of compound MHEM-Cholesterol

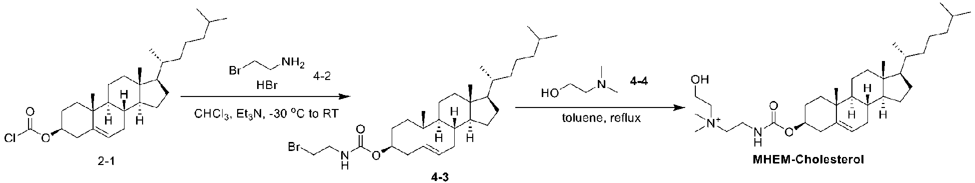

### (RT: room temperature; toluene: toluene; reflux: reflux; MHEM-Cholesterol: MHEM-Cholesterol)

Compound 2-1 (450 mg, 1.0 mmol, 1.0 eq.) and 2-bromoethylamine hydrobromide (Compound 4-2, 225 mg, 1.1 mmol, 1.1 eq.) were dissolved in 10 mL of chloroform. The reaction mixture was cooled to -30 °C, and then triethylamine (0.21 mL, 1.5 mmol, 1.5 eq.) was added to the reaction solution. After 30 minutes, the temperature was raised to room temperature, and the reaction was continued with stirring until compound 2-1 was completely consumed. The reaction solution was quenched with saturated sodium chloride solution and extracted with dichloromethane. The organic phases were combined, dried over anhydrous sodium sulfate, and then the organic solvent was removed by rotary evaporation to obtain the crude intermediate compound 4-3 (452 mg), which was directly used for the next step without purification.

Compound 4-3 (452 mg, 0.84 mmol, 1.0 eq.) prepared above and compound 4-4 (89 mg, 1.0 mmol, 1.2 eq.) were added to 5.0 mL of anhydrous toluene and heated under reflux overnight. The solvent was removed by rotary evaporation, and the obtained crude product was added to ether and stirred. The white solid obtained after filtration was then recrystallized with ethanol to yield the final product MHEM-Cholesterol (65 mg). **¹H NMR** (400 MHz, CDCl₃): *δ* 0.68 (s, 3H), 0.85 (d, *J* = 6.8 Hz, 3H), 0.90 (d, *J* = 6.8 Hz, 3H), 1.05-1.62 (m, 28H), 1.76 (m, 3H), 1.92-2.07 (m, 2H), 2.30 (m, 2H), 3.41 (s, 6H), 3.62-3.83 (m, 6H), 4.14 (m, 2H), 4.43 (m, 1H), 5.37 (m, 1H), 6.38 (s, 1H); **MS m/z [M]⁺ (ESI):** 545.5.

### Example 5: Synthesis of compound BHEM-β-Sitosterol

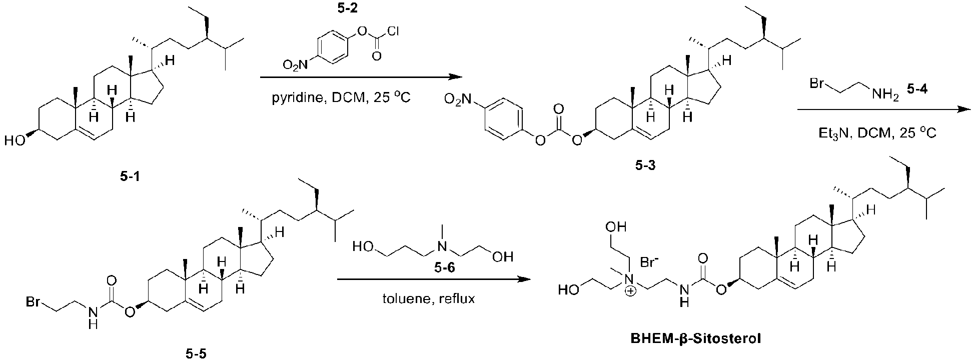

### (pyridine: pyridine; toluene: toluene; reflux: reflux; BHEM-β-Sitosterol: BHEM-β-Sitosterol)

Compound 5-1 (830 mg, 2.0 mmol, 1.0 eq.) was dissolved in 15 mL of dichloromethane. The reaction mixture was cooled to 0 °C, and then pyridine (0.32 mL, 4.0 mmol, 2.0 eq.) was added. After 10 minutes, p-nitrophenyl chloroformate (484 mg, 2.4 mmol, 1.2 eq.) was added to the reaction mixture, and the temperature was raised to room temperature for further stirring until the starting material (compound 5-1) was mostly consumed. The reaction solution was quenched with saturated sodium chloride solution and extracted with dichloromethane. The organic phases were combined, dried over anhydrous sodium sulfate, and then the organic solvent was removed by rotary evaporation to obtain the crude product, which was further purified by silica gel column chromatography to yield compound 5-3 (927 mg).

Compound 5-3 (300 mg, 0.52 mmol, 1.0 eq.), 2-bromoethylamine hydrobromide (127 mg, 0.62 mmol, 1.2 eq.), and triethylamine (0.14 mL, 1.04 mmol) were added to 5.0 mL of dichloromethane. The reaction was carried out at room temperature until compound 5-3 was completely consumed. The reaction solution was quenched with saturated sodium chloride solution and extracted with dichloromethane. The organic phases were combined, dried over anhydrous sodium sulfate, and then the organic solvent was removed by rotary evaporation to obtain the crude product, which was further purified by silica gel column chromatography to yield compound 5-5 (237 mg).

Referring to the method of Example 4, the final product was prepared using compound 5-5 and 5-6 to obtain the BHEM-β-Sitosterol (56 mg). **¹H NMR** (400 MHz, CDCl₃): *δ* 0.58 (s, 3H), 0.84 (d, *J* = 6.8 Hz, 3H), 0.88 (d, *J* = 6.8 Hz, 3H), 1.04-2.05 (m, 35H), 1.76 (m, 3H), 1.92-2.07 (m, 2H), 2.30 (m, 2H), 3.40 (s, 3H), 3.45-3.74 (m, 6H), 4.04 (m, 4H), 4.45 (m, 1H), 5.32 (m, 1H), 6.38 (s, 1H); **MS m/z [M]⁺ (ESI):** 603.5.

### Example 6: Synthesis of compound BHEM-4-Chol

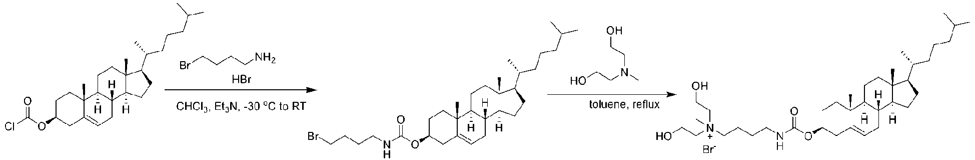

### (RT: room teperature; toluene: toluene; reflux: reflux)

Compound BHEM-4-Chol (452 mg) was prepared using a similar method as described in Example 4. **¹H NMR** (400 MHz, CDCl₃): *δ* 0.67 (s, 3H), 0.84-0.88 (m, 6H), 0.90-0.92 (m, 3H), 0.95-1.05 (m, 4H), 1.09-1.22 (m, 6H), 1.30-1.65 (m, 10H), 1.75-2.04 (m, 6H), 2.23-2.33 (m, 2H), 3.09 (d, J = 6.0 Hz, 3H), 3.14-3.26 (m, 2H), 3.33 (s, 3H), 3.40-3.51 (m, 2H), 3.55-3.76 (m, 8H), 4.02-4.14 (m, 8H), 4.36-4.72 (m, 4H), 5.35 (m, 1H), 8.78 (s, 1H); **MS m/z [M]⁺ (ESI):** 603.4.

### Example 7: Synthesis of compound BHEM-6-Chol

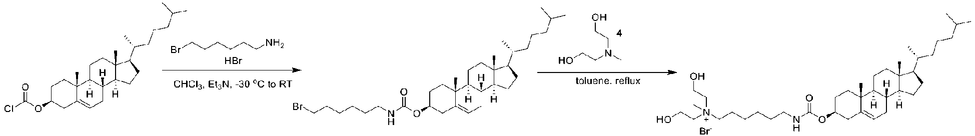

### (RT: room teperature; toluene: toluene; reflux: reflux)

Compound BHEM-6-Chol (172 mg) was prepared using a similar method as described in Example 4. **¹H NMR** (400 MHz, CDCl₃): *δ* 0.67 (s, 3H), 0.83-0.94 (m, 10H), 0.98-1.03 (m, 6H), 1.05-1.18 (m, 8H), 1.30-1.63 (m, 16H), 1.72-2.06 (m, 6H), 2.21-2.36 (m, 2H), 3.08-3.16 (m, 2H), 3.33 (s, 3H), 3.41-3.46 (m, 1H), 3.54-3.64 (m, 2H), 3.66-3.83 (m, 4H), 3.99-4.04 (m, 1H), 4.06-4.16 (m, 4H), 4.36-4.49 (m, 1H), 4.91-5.08 (m, 1H), 5.33-5.38 (m, 1H); **MS m/z [M]⁺ (ESI):** 631.5..

### Example 8: Synthesis of compound BHEM-4-Sito

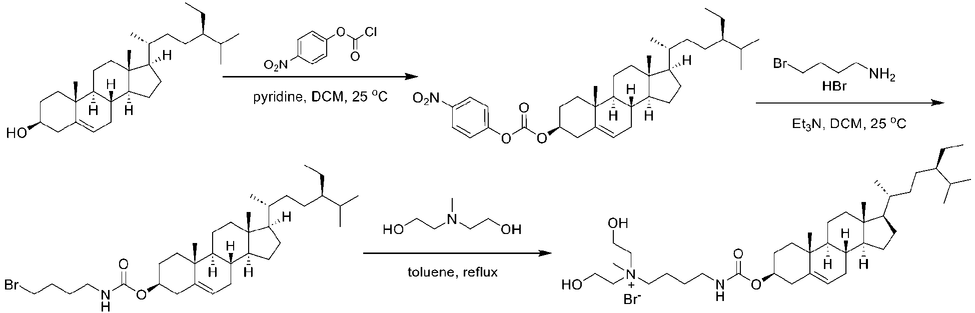

### (pyridine: pyridine; toluene: toluene; reflux: reflux)

Compound BHEM-4-Sito (62 mg) was prepared using a similar method as described in Example 5. **¹H NMR** (400 MHz, CDCl₃): *δ* 0.68 (s, 3H), 0.73-0.88 (m, 9H), 0.89-1.04 (m, 8H), 1.09-1.70 (m, 16H), 1.76-2.04 (m, 5H), 2.22-2.37 (m, 2H), 3.03-3.24 (m, 2H), 3.33 (s, 3H), 3.45-3.82 (m, 5H), 3.95-4.18 (m, 12H), 4.26-4.47 (m, 1H), 4.58-4.93 (m, 4H), 5.36 (m, 1H); **MS m/z [M]⁺ (ESI):** 631.7.

### Example 9: Synthesis of compound BHEM-6-Sito

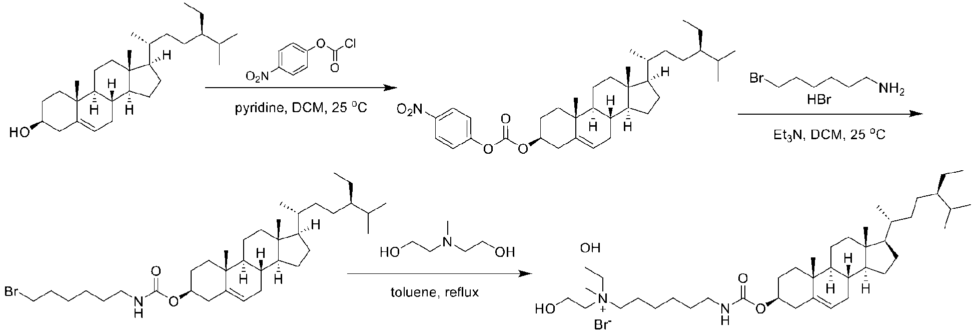

### (pyridine: pyridine; toluene: toluene; reflux: reflux)

Compound BHEM-6-Sito (40 mg) was prepared using a similar method as described in Example 5. **¹H NMR** (400 MHz, CDCl₃): *δ* 0.67 (s, 3H), 0.80-0.87 (m, 9H), 0.89-1.04 (m, 10H), 1.09-1.32 (m, 10H), 1.36-1.55 (m, 14H), 1.75-2.05 (m, 6H), 2.22-2.37 (m, 2H), 3.04-3.17 (m, 2H), 3.33 (s, 3H), 3.40-3.48 (m, 1H), 3.54-3.66 (m, 2H), 3.68-3.84 (m, 4H), 3.98-4.14 (m, 5H), 4.37-4.54 (m, 1H), 4.74-5.04 (m, 3H), 5.36 (m, 1H); **MS m/z [M]⁺ (ESI):** 659.7.

### Example 10: Synthesis of compound S1

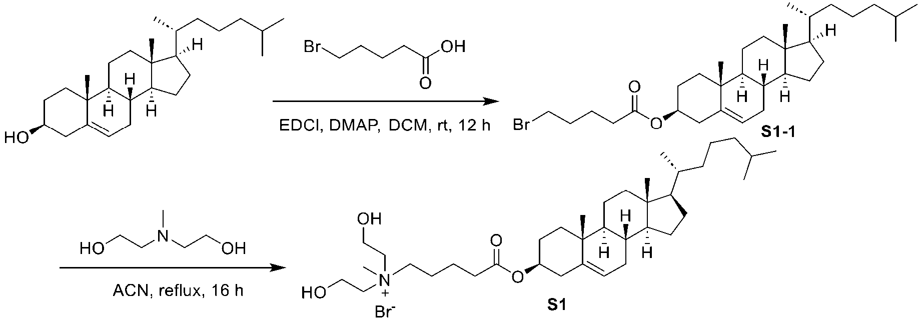

### (rt: room temperature; 12h: 12 hours; reflux: reflux; 16h: 16 hours)

A mixture of cholesterol (1.9 g, 4.91 mmol, 1.0 eq.), 5-bromovaleric acid (1.33 g, 7.35 mmol, 1.5 eq.), EDCI (1.9 g, 9.91 mmol, 2.0 eq.), and DMAP (57 mg, 0.49 mmol, 0.1 eq.) in DCM was stirred at room temperature overnight. The resulting solution was extracted with DCM (3 x 15 mL), and the combined organic layers were washed with water (3 x 15 mL), dried over anhydrous Na₂SO₄, filtered, and concentrated under reduced pressure. The residue was purified by silica gel column chromatography, eluting with a PE/EA (10:1) solution to obtain compound S1-1 as a pale white solid (2.5 g) with a yield of 92.56%. ¹H NMR (300 MHz, Chloroform-d) δ 5.40-5.35 (m, 1H), 4.68-4.56 (m, 1H), 3.45-3.39 (m, 2H), 2.37-2.26 (m, 4H), 2.07-1.72 (m, 9H), 1.64-1.05 (m, 21H), 1.03-1.01 (m, 3H), 0.88-0.84 (m, 9H), 0.68 (s, 3H).

A mixture of Compound S1-1 (480 mg, 0.87 mmol, 1.0 eq.) and N-methyldiethanolamine (156 mg, 1.31 mmol, 1.5 eq.) in MeCN (8 mL) was stirred at 80°C overnight. The precipitated solid was collected by filtration, washed with MeCN (3 x 5 mL), and recrystallized from MeOH/EtOH (90:1, 15 mL) to give compound S1 as a white solid (132.9 mg) in a yield of 22.45%. **¹H NMR** (400 MHz, Chloroform-d): δ 5.43-5.32 (m, 1H), 4.56 (m, 1H), 4.10 (m, 4H), 3.74 (s, 7H), 3.60 (m, 2H), 3.32 (s, 3H), 2.39 (m, 2H), 2.30 (m, 2H), 2.06-1.93 (m, 2H), 1.92-1.76 (m, 5H), 1.63-1.21 (m, 13H), 1.12-0.79 (m, 19H), 0.68 (s, 3H); **MS m/z [M]⁺ (ESI):** 588.4.

### Example 11: Synthesis of compound S8

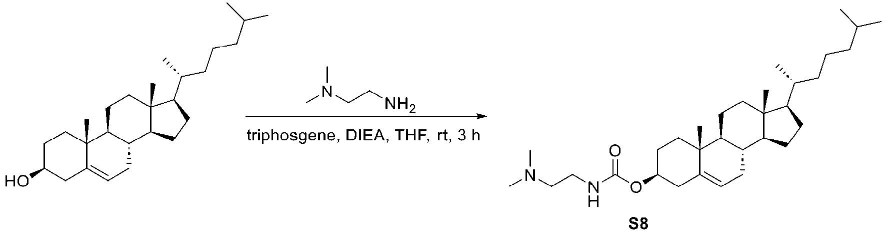

### (triphosgene: triphosgene; rt: room temperature; 3h: 3 hours)

Cholesterol (500 mg, 1.29 mmol, 1.0 eq.), DIEA (1.13 mL, 6.45 mmol, 5.0 eq.) and triphosgene (154 mg, 0.52 mmol, 0.4 eq.) were dissolved in 8 mL THF at room temperature, and the mixture was purged with nitrogen gas for four times. Then (2-aminoethyl)dimethylamine (114 mg, 1.29 mmol, 2.0 eq.) was added, and the reaction mixture was stirred at room temperature under a nitrogen atmosphere for 3 hours. The resulting mixture was extracted with DCM (3 x 10 mL), the organic layers were combined, washed with water (30 mL) and saturated sodium chloride solution (30 mL). The solution was dried over anhydrous Na₂SO₄, filtered, and concentrated under reduced pressure to give the crude product, which was purified by preparative liquid chromatography under the following conditions: chromatography column UniHybrid 8-200 C8 OBD Column 30x150 mm, 8 µm; mobile phase A: water (100 mmol/L NH4HCO3)/ACN=6:4, mobile phase B: ACN/IPA=1:9; flow rate: 60 mL/min; elution gradient: 65% B to 85% B in 12 min; detection wavelength: 220 nm; RT1(min): 9.5. Compound S8 was obtained as white solid (125.8 mg). **¹H NMR** (400 MHz, CDCl₃): δ 5.39-5.35 (m, 1H), 5.21-5.16 (m, 1H), 4.54-4.44 (m, 1H), 3.30-3.23 (m, 2H), 2.51-2.34 (m, 3H), 2.25 (s, 6H), 2.11-1.75 (m, 6H), 1.63-1.41 (m, 7H), 1.41-1.23 (m, 4H), 1.23-0.94 (m, 13H), 0.91 (d, J = 6.5 Hz, 3H), 0.88-0.84 (m, 6H), 0.67 (s, 3H); **MS m/z [M+H]⁺ (ESI):** 501.40.

### Example 12: Synthesis of compound S2

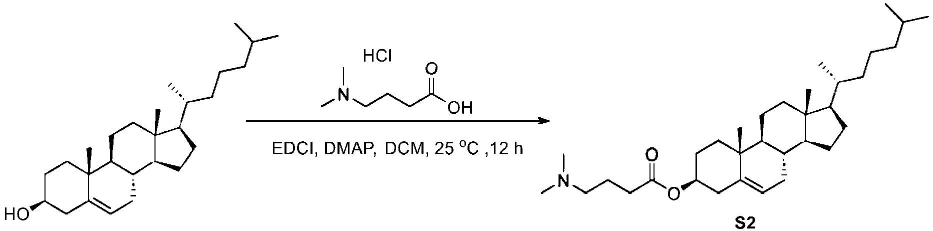

### (12h: 12hours)

DMAP (158 mg, 1.29 mmol, 2 eq.) and EDCI (372 mg, 1.94 mmol, 1.5 eq.) were added to a DCM solution (8 mL) of cholesterol (500 mg, 1.29 mmol, 1.0 eq.) and 4-(dimethylamino)butyric acid hydrochloride (260 mg, 1.55 mmol, 1.2 eq.) under stirring. The resulting mixture was stirred at 25 °C under a nitrogen atmosphere for 12 hours. The mixed solution was extracted with DCM (3 x 10 mL), and the combined organic layers were washed with water (30 mL) and saturated sodium chloride solution (30 mL), dried over anhydrous Na₂SO₄, filtered, and concentrated under reduced pressure to obtain a crude product. The crude product was purified by preparative liquid chromatography with the following chromatographic conditions: column UniHybrid 8-200 C8 OBD Column 30x150 mm, 8 µm; mobile phase A: water (100 mmol/L NH₄HCO₃)/ACN=6:4, mobile phase B: ACN/IPA=1:9; flow rate: 60 mL/min; elution gradient: 50% B to 80% B in 12 min; detection wavelength: 220nm; RT1(min): 9.5. Compound S2 was obtained as a white solid (349.7 mg) in a yield of 51.40%. **¹H NMR** (400 MHz, CDCl3): δ 5.49-5.28 (m, 1H), 4.72-4.51 (m, 1H), 2.33-2.27 (m, 6H), 2.23 (s, 6H), 2.06-1.93 (m, 2H), 1.91-1.73 (m, 5H), 1.67-1.42 (m, 7H), 1.42-1.29 (m, 3H), 1.28-1.23 (m, 1H), 1.22-0.94 (m, 13H), 0.91 (d, *J =* 6.5 Hz, 3H), 0.89-0.84 (m, 6H), 0.68 (s, 3H); **MS m/z [M+H]+ (ESI):**500.4.

### Example 13: Synthesis of compound S26

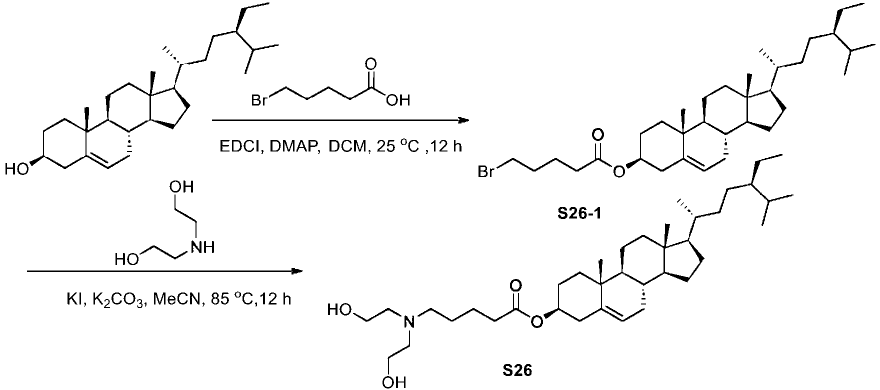

### (12h: 12hours)

To a solution of Sitosterol (1.55 g, 4.0 mmol, 1.0 eq.) and 5-bromovaleric acid (0.87 g, 4.8 mmol, 1.2 eq.) in DCM (20 mL) was added DMAP (0.98 g, 8.0 mmol, 2.0 eq.) and EDCI (1.15 g, 6.0 mmol, 1.5 eq.). The mixture was stirred at room temperature under nitrogen atmosphere for 12 hours. The resulting mixture was extracted with DCM (3 x 20 mL), and the combined organic layers were washed with water (50 mL) and saturated sodium chloride solution (50 mL), dried over anhydrous Na₂SO₄, filtered, and concentrated under reduced pressure to give a crude product. The crude product was purified by silica gel column chromatography to yield compound S26-1 as a white solid (1.78 g).

Compound S26-1 (500 mg, 0.82 mmol, 1.0 eq.) was dissolved in 10 mL ACN, and diethanolamine (91 mg, 0.82 mmol, 1.0 eq.), potassium carbonate (479 mg, 3.29 mmol, 4.0 eq.), and potassium iodide (144 mg, 0.82 mmol, 1.0 eq.) were added sequentially under nitrogen. The mixture was heated to 85 °C and stirred for 12 hours. The mixture was then cooled to 25 °C, diluted with water (20 mL), and extracted with DCM (3 x 20 mL). The combined organic extract were washed with saturated NaCl solution (50 mL), dried over anhydrous Na₂SO₄, and filtered. The organic solvent was evaporated using a rotary evaporator to obtain the crude product, which was purified by HPLC under the following conditions: chromatography column UniHybrid 8-200 C8 OBD Column 30x150 mm, 8µm; mobile phase A: acetonitrile/water (10 mmol/L ammonium bicarbonate + 0.5% ammonia) = 4:6, mobile phase B: acetonitrile/isopropanol = 1:9; flow rate: 60 mL/min; gradient: 55% B to 70% B, 12 min; retention time: 9 min. Compound S26 (105 mg) was obtained after purification. **¹H NMR** (400 MHz, CDCl₃): δ 5.37-5.27 (m, 1H), 4.63-4.56 (m, 1H), 3.70-3.61 (m, 3H), 2.77-2.68 (m, 3H), 2.67-2.58 (m, 2H), 2.36-2.28 (m, 5H), 2.05-1.93 (m, 2H), 1.90-1.80 (m, 3H), 1.71-1.40 (m, 12H), 1.37-1.05 (m, 10H), 1.04-0.89 (m, 10H), 0.88-0.77 (m, 10H), 0.68 (s, 3H); **MS m/z [M+H]⁺ (ESI):** 602.50.

### Example 14: Synthesis of compound S46

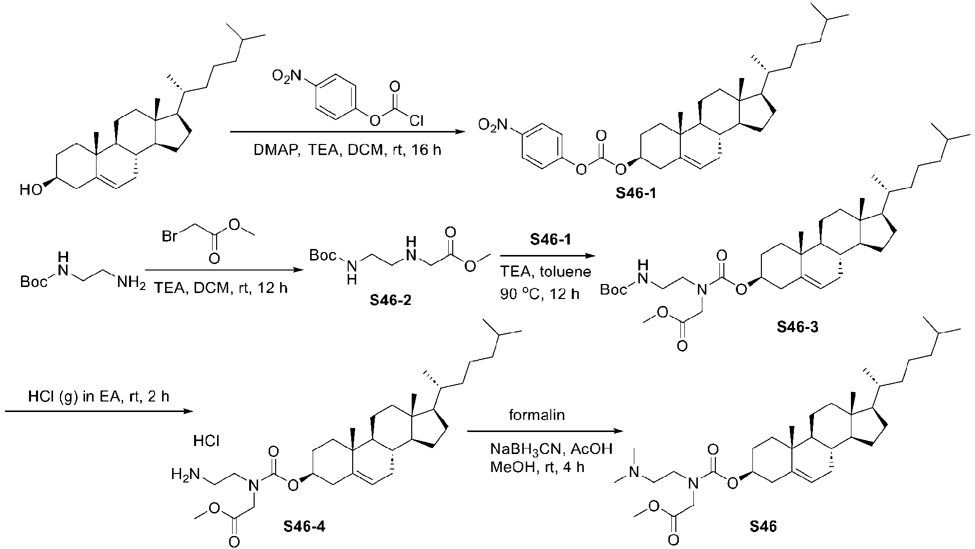

### (rt: room temperature; 16h: 16 hours; 12h: 12 hours; toluene: toluene; HCl(g) in EA: a solution of HCl (g) in EA; 2h: 2 hours; formalin: formalin; 4h: 4 hours)

To a solution of Cholesterol (7.0 g, 17.20 mmol, 1.0 eq.) in 50 mL was added triethylamine (3.66 g, 34.40 mmol, 2.0 eq.), 4-dimethylaminopyridine (0.22 g, 1.72 mmol, 0.1 eq.), and p-nitrophenyl chloroformate (5.47 g, 25.80 mmol, 1.5 eq.) sequentially at 0 °C under nitrogen. The reaction was stirred at 0 °C for 2 hours, then left to react overnight at room temperature. After the reaction was complete, the organic solvent was removed using a rotary evaporator to obtain the crude product, which was washed with methanol to obtain compound S46-1.

N-Boc-ethylenediamine (5.22 g, 32.61 mmol, 1.0 eq.) was dissolved in 50 mL DCM, and triethylamine (4.17 g, 39.13 mmol, 1.2 eq.) and methyl bromoacetate (5.25 g, 32.61 mmol, 1.0 eq.) were added sequentially under nitrogen. The mixture was stirred at room temperature for 12 hours. After the reaction was complete, water (30 mL) was added, and extracted with DCM (3 x 30 mL). The combined organic layers were washed with saturated NaCl solution (3 x 30 mL) and dried over anhydrous Na₂SO₄. The organic phase was collected by filtration and the solvent was removed using a rotary evaporator to obtain a crude product. The crude product was purified by silica gel column chromatography to obtain compound S46-2 (3.3 g) as a light-yellow oily product.

Compound S46-2 (3.3 g, 12.08 mmol, 1.57 eq.) was dissolved in 20 mL toluene, and compound S46-1 (5.0 g, 7.70 mmol, 0.9 eq.) and triethylamine (2.37 g, 22.25 mmol, 2.6 eq) were added sequentially under nitrogen. The mixture was reacted at 90 °C for 12 hours under stirring. The organic solvent was removed to obtain the crude product, which was purified on silica gel chromatography to obtain compound S46-3 (750 mg) as a light-yellow oily product.

Compound S46-3 (750 mg, 0.99 mmol, 1.0 eq.) was added to a 10 mL solution of HCl in ethyl acetate (4 M), and stirred at room temperature for 2 hours. After the reaction was complete, the organic solvent was removed to obtain S46-4 (700 mg) as an off-white solid, which was used directly in the next step without purification.

Compound S46-4 (700 mg, 1.02 mmol, 1.0 eq.) was dissolved in 20 mL methanol, and then formaldehyde (250 mg, 3.06 mmol, 3.0 eq., 37% aqueous solution), acetic acid (0.14 mL, 2.32 mmol, 2.26 eq.) and NaBH₃CN (195 mg, 3.06 mmol, 3.0 eq.) were added sequentially. The mixture was stirred at room temperature for 12 hours. After the reaction was complete, the organic solvent was removed, diluted with 30 mL water. The mixture was extracted with DCM (3 x 30 mL), and the combined organic phases were washed with saturated NaCl solution (3 x 30 mL), dried over anhydrous Na₂SO₄, and filtered to collect the organic phase. The organic solvent was removed to obtain a crude product, which was purified by silica gel column chromatography to obtain compound S46 (400 mg) as a pale yellow oily compound. **¹H NMR** (400 MHz, CDCl₃): δ 5.43-5.31 (m, 1H), 4.63-4.42 (m, 1H), 4.05 (d, *J =* 16.8 Hz, 2H), 3.73 (s, 3H), 3.56-3.37 (m, 2H), 2.59-2.12 (m, 10H), 2.09-1.74 (m, 5H), 1.74-1.23 (m, 11H), 1.22-1.07 (m, 7H), 1.07-0.95 (m, 6H), 0.95-0.77 (m, 9H), 0.67 (s, 3H); **MS m/z [M+H]⁺ (ESI):** 573.50.

### Example 15: Synthesis of compound S47

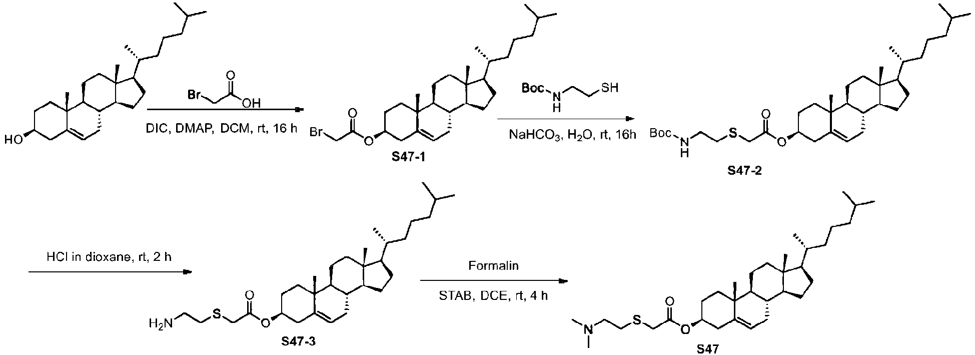

### (rt: room temperature; 16h: 16 hours; HCl in dioxane: a solution of HCl in dioxane; 2h: 2 hours; formalin: formalin; 4h: 4 hours)

To a solution of cholesterol (5.0 g, 12.93 mmol, 1.0 eq.) and bromoacetic acid (2.0 g, 14.35 mmol, 1.1 eq.) in 100 mL DCM was added DMAP (80 mg, 0.65 mmol, 0.05 eq.) and DIC (2.0 g, 15.91 mmol, 1.2 eq.) sequentially under nitrogen. The mixture was stirred at room temperature for 16 hours. The organic solvent was removed to obtain the crude product, which was purified by silica gel column chromatography to obtain compound S47-1 as a white solid (4.3 g).

Compound S47-1 (4.0 g, 7.88 mmol, 1.0 eq.) and 2-(Boc-amino)ethanethiol (1.54 g, 8.67 mmol, 1.1 eq.) were added to 40 mL water, and sodium bicarbonate (0.99 g, 11.82 mmol, 1.5 eq.) was added at room temperature. The mixture was stirred for 16 hours at room temperature. After the reaction was complete, ethyl acetate was added for extraction, and the organic phase was washed with saturated NaCl, dried over anhydrous Na₂SO₄, and filtered. The organic solvent was removed to obtain the crude product, which was purified by silica gel column chromatography to obtain compound S47-2 as a white solid (4.8 g).

Compound S47-2 (4.8 g, 7.95 mmol, 1.0 *eq*) was added to a 20 mL solution ofHCl in dioxane (4 M), and stirred at room temperature for 2 hours. After the reaction was complete, the organic solvent was removed to obtain S47-3 (4.1 g) as a white solid, which was used directly in the next step without purification.

Compound S47-3 (500 mg, 0.99 mmol, 1.0 eq.) and formaldehyde (1.49 g, 14.88 mmol, 15.0 eq., 37% aqueous solution) were added to 5 mL of 1,2-dichloroethane and stirred at room temperature for 1 hour. Sodium triacetoxyborohydride (STAB, 421 mg, 1.98 mmol, 2.0 eq.) was added in portions to the mixture at room temperature and stirred for an additional 4 hours. The reaction was quenched with 20 mL water at room temperature, extracted with ethyl acetate (2 x 30 mL), and the organic phase was washed with saturated NaCl. The organic phase was dried over anhydrous Na₂SO₄, and filtered. The organic solvent was removed to obtain the crude product, which was purified by HPLC with the following conditions: chromatography column YMC Triart C18 ExRs, 30x150 mm, 5 µm; mobile phase A: water (0.1% formic acid), mobile phase B: acetonitrile; flow rate: 60 mL/min; gradient: 70% B to 90% B, 12 min. Compound S47 was obtained as a yellow oily compound (71.0 mg). **¹H NMR** (400 MHz, CDCl₃): δ 5.50-5.30 (m, 1H), 4.80-4.50 (m, 1H), 3.23 (s, 2H), 2.90-2.70 (m, 2H), 2.64-2.54 (m, 2H), 2.45-2.18 (m, 8H), 2.15-1.75 (m, 4H), 1.72-1.41 (m, 7H), 1.40-1.24 (m, 5H), 1.24-1.06 (m, 7H), 1.06-0.90 (m, 9H), 0.90-0.80 (m, 6H), 0.68 (s, 3H); **MS m/z [M+H]⁺ (ESI):** 532.40.

### Example 16: Synthesis of compound S42

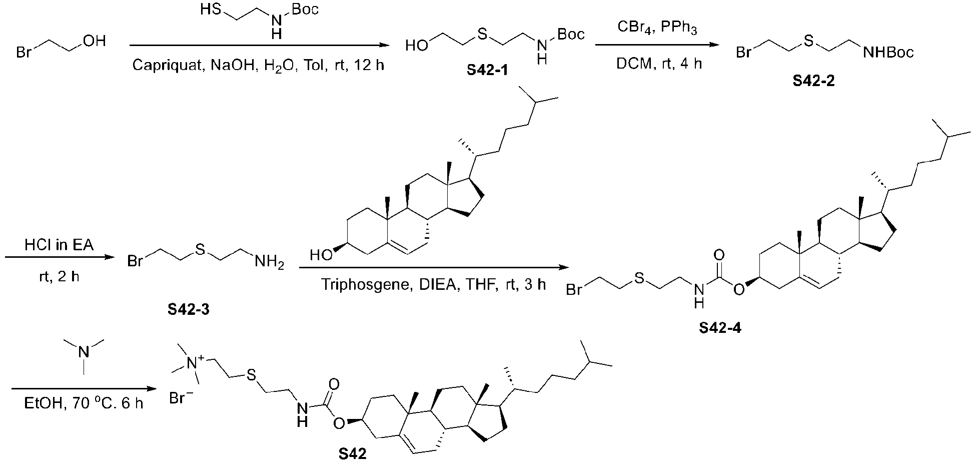

### (rt: room temperature; 12h: 12 hours; 4h: 4 hours; HCl in EA: a solution of HCl in EA; 2h: 2 hours; Triphosgene: Triphosgene; 3h: 3 hours; 6h: 6 hours)

To a solution of 2-Bromoethanol (8.1 g, 64.82 mmol, 2.0 eq.) and 2-(Boc-amino)ethanethiol (5.7 g, 32.41 mmol, 1.0 eq.) in 27 mL toluene was added trioctylmethylammonium chloride (Capriquat, 262 mg, 0.65 mmol, 0.01 eq.), sodium hydroxide (1.56 g, 38.89 mmol, 1.2 eq.) and water (36 mL) sequentially. The mixture was stirred at room temperature for 12 hours. The organic phase was separated and washed with water, dried over anhydrous sodium sulfate, and concentrated. The crude product was purified by silica gel column chromatography to obtain compound S42-1 as a yellow oily compound (6.6 g).

To a solution of compound S42-1 (6.6 g, 29.82 mmol, 1.0 eq.) in 60 mL DCM was added triphenylphosphine (11.73 g, 44.73 mmol, 1.5 eq.) and carbon tetrabromide (14.8 g, 44.73 mmol, 1.5 eq.) sequentially at 0 °C under nitrogen atmosphere. The reaction was stirred under nitrogen at room temperature for 4 hours. After the reaction was complete, it was diluted with water (30 mL), extracted with DCM (3 x 30 mL), and the combined organic phases were washed with saturated NaCl solution (3 x 30 mL), dried over anhydrous sodium sulfate. The organic phase was collected by filtration, and the organic solvent was removed to obtain the crude product, which was purified by silica gel chromatography to obtain compound S42-2 as yellow oily compound (3.3 g)

Compound S42-2 (3.3 g, 11.61 mmol, 1.0 *eq*.) was added to a 20 mL solution of HCl in ethyl acetate (4 M), and stirred at room temperature for 2 hours. After the reaction was complete, the organic solvent was removed to obtain S42-3 (2.6 g) as a white solid, which was used directly in the next step without purification.

To a solution of compound 42-3 (1.3 g, 0.89 mmol, 1.5 eq.) and cholesterol (1.52 g, 3.93 mmol, 1.0 eq.) in 10 mL THF was added triphosgene (2.33 g, 7.86 mmol, 2.0 eq.) and N, N-diisopropylethylamine (1.02 g, 7.86 mmol, 2.0 eq.) sequentially at room temperature under nitrogen. The mixture was stirred at room temperature for 3 hours. After the reaction was complete, it was diluted with 30 mL water and extracted with DCM (3 x 30 mL). The combined organic phases were washed with saturated NaCl solution (3 x 20 mL), dried over anhydrous sodium sulfate, filtered to collect the organic phase. The organic solvent was removed to obtain the crude product, which was purified by silica gel column chromatography to obtain compound S42-4 as a white solid (800 mg).

Compound S42-4 (500 mg, 0.84 mmol, 1.0 eq.) and trimethylamine (2M solution in THF, 0.84 mL, 1.68 mmol, 2.0 eq.) were added to 5 mL acetonitrile, which was sealed and stirred at 70 °C for 6 hours. After the reaction was complete, it was cooled to room temperature. The solid was collected by filtration to obtain the crude product, which was recrystallized from 10 mL acetonitrile/ethanol (90:1) to obtain compound S42 as a white solid (129.8 mg). **¹H NMR** (400 MHz, CDCl₃) δ 7.40-7.20 (m, 1H), 5.50-5.25 (m, 1H), 4.50-4.20 (m, 1H), 3.56-3.47 (m, 2H), 3.25-3.15 (m, 2H), 3.15-3.05 (m, 9H), 2.94-2.86 (m, 2H), 2.69-2.59 (m, 2H), 2.32-2.15 (m, 2H), 2.05-1.70 (m, 5H), 1.65-1.46 (m, 5H), 1.45-1.20 (m, 6H), 1.20-1.00 (m, 8H), 1.00-0.97 (m, 4H), 0.97-0.75 (m, 10H), 0.70-0.55 (m, 3H); **MS m/z [M]⁺ (ESI):** 575.50.

### Example 17: Synthesis of compound S43

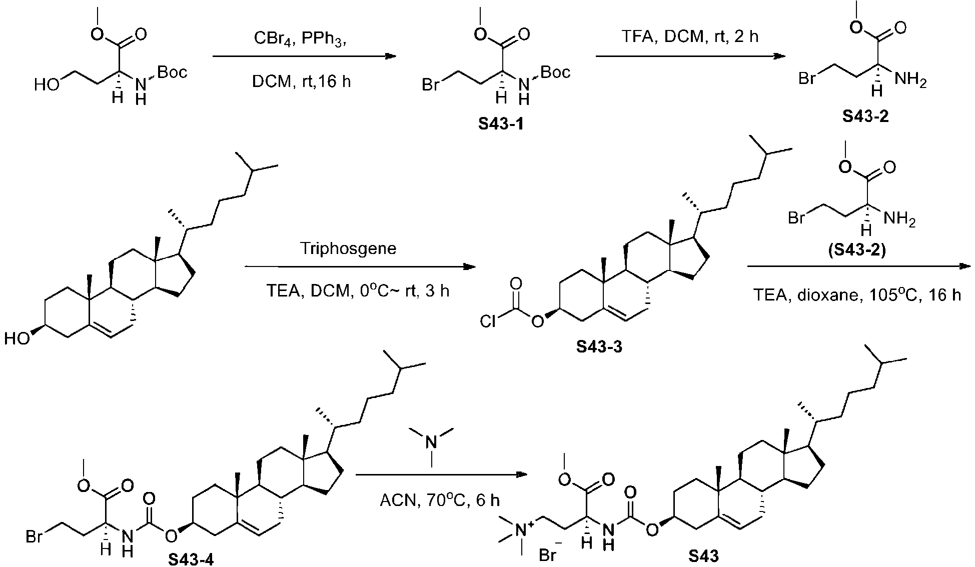

### (rt: room temperature; 16h: 16 hours; 2h: 2 hours; Triphosgene: Triphosgene; 3h: 3 hours; dioxane: dioxane; 6h: 6 hours)

To a solution of N-Boc-L-serine methyl ester (0.9 g, 3.86 mmol, 1.0 eq.) and carbon tetrabromide (3.84 g, 11.57 mmol, 3.0 eq.) in DCM (12 mL) was added triphenylphosphine (2.02 g, 7.72 mmol, 2.0 eq.) under nitrogen. The mixture was stirred at 25 °C for 16 hours. After the reaction was complete, petroleum ether (30 mL) was added, and the solid was filtered off. The filtrate was dried to obtain the crude product, which was purified by silica gel column chromatography to obtain S43-1 as a white solid (1.0 g).

Compound S43-1 (900 mg, 3.04 mmol, 1.0 *eq*.) was added to a 10 mL solution of HCl in ethyl acetate (4 M), and stirred at room temperature for 2 hours. After the reaction was complete, the organic solvent was removed to obtain crude compound S43-2 (660 mg), which was used directly in the next step without purification.

To a solution of cholesterol (5.0 g, 12.93 mmol, 1.0 eq.) and triethylamine (2.70 mL, 19.4 mmol, 1.5 eq.) in 50 mL DCM was added triphosgene (1.92 g, 6.465 mmol, 0.5 eq.) slowly at 0°C under nitrogen. The mixture was stirred at room temperature for 3 hours. After the reaction was complete, it was washed with water three times, and the organic solvent was removed to obtain the crude product. The crude product was slurried with ethyl acetate/methanol (15:1) (20 mL), filtered and vacuum dried to obtain compound S43-3 as white solid (2.7 g).

To a solution of compound S43-3 (1.80 g, 4.00 mmol, 1.5 eq.) and S43-2 (620 mg, 2.67 mmol, 1.0 eq.) in 15 mL dioxane was added triethylamine (0.48 mL, 3.47 mmol, 1.3 eq.) at room temperature. The mixture was stirred under nitrogen at 105°C for 16 hours. After the reaction was complete, the organic solvent was removed to obtain the crude product, which was purified by silica gel column chromatography to obtain compound S43-4 as a white solid (1.15 g).

To a solution of compound S43-4 (600 mg, 0.99 mmol, 1.0 eq.) in acetonitrile (10 mL) was added trimethylamine (2M solution in THF, 2mL, 4.0 eq.). The mixture was stirred under nitrogen at 80 °C for 16 hours. After the reaction was complete, the organic solvent was removed to obtain the crude product, which was purified by silica gel column chromatography to yield compound S43 as a white solid (126.9 mg). **¹H NMR** (400 MHz, CDCl₃): δ 6.81-6.75 (m, 1H), 5.38-5.32 (m, 1H), 4.48-4.33 (m, 2H), 3.79 (s, 5H), 3.43 (s, 9H), 2.46-2.21 (m, 4H), 2.05-1.76 (m, 5H), 1.64-1.23 (m, 11H), 1.23-1.10 (m, 7H), 1.08-0.93 (m, 5H), 0.96-0.89 (m, 3H), 0.89-0.83 (m, 6H), 0.68 (s, 3H); **MS m/z [M]⁺ (ESI):** 587.50.

### Example 18: Synthesis of compound S45

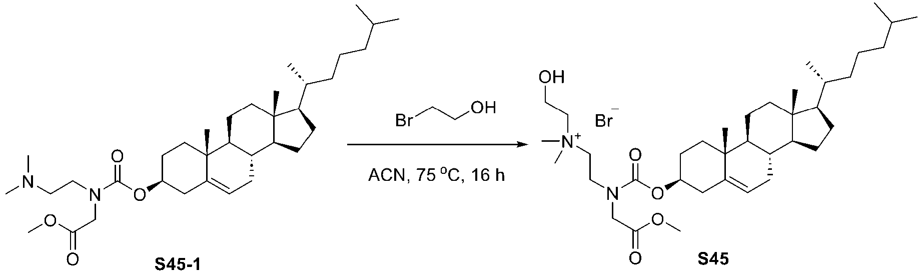

### (16h: 16 hours)

A mixture of compound S45-1 (500 mg, 0.87 mmol, 1.0 eq.) and 2-bromoethanol (873 mg, 7.0 mmol, 8.0 eq.) were dissolved in 20 mL acetonitrile, which was sealed and stirred at 75 °C for 16 hours. After the reaction was complete, the organic solvent was removed to obtain the crude product, which was purified by silica gel column chromatography to obtain compound S45 as a white solid (127.1 mg). **¹H NMR** (400 MHz, CDCl₃) δ 5.47-5.33 (m, 1H), 4.50 (s, 4H), 4.23 (s, 4H), 4.06-3.67 (m, 9H), 3.42 (s, 6H), 2.41-2.12 (m, 2H), 2.08-1.90 (m, 2H), 1.90-1.74 (m, 3H), 1.68-1.22 (m, 10H), 1.22-1.06 (m, 6H), 1.06-0.96 (m, 5H), 0.96-0.80 (m, 10H), 0.67 (s, 3H); **MS m/z [M]⁺ (ESI):** 617.50.

### Example 19: Synthesis of compound S48

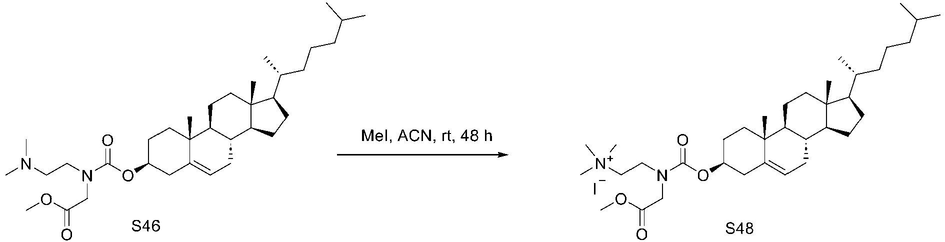

### (rt: room temperature; 48h: 48 hours)

To a solution of compound S46 (300 mg, 0.445 mmol, 1.0 eq.) in acetonitrile (10 mL) was added MeI (1.33 g, 8.9 mmol, 20 eq.). The mixture was stirred at room temperature for 48 hours. After the reaction was complete, the organic solvent was removed to obtain the crude product, which was purified by silica gel column chromatography and then recrystallized from 5 mL acetonitrile to obtain compound S48 as a light yellow solid (41.8 mg mg). **¹H NMR** (400 MHz, CDCl₃): δ 5.45-5.31 (m, 1H), 4.51-4.41 (m, 1H), 4.26 (s, 2H), 4.12-3.94 (m, 2H), 3.93-3.84 (m, 2H), 3.77 (s, 3H), 3.46 (s, 9H), 2.37-2.14 (m, 2H), 2.03-1.96 (m, 2H), 1.94-1.78 (m, 2H), 1.59-1.45 (m, 7H), 1.39-1.22 (m, 4H), 1.18-0.97 (m, 14H), 0.93-0.89 (m, 3H), 0.88-0.81 (m, 6H), 0.67 (s, 3H); **MS m/z [M]⁺ (ESI):** 587.45.

### Example 20: Synthesis of compound S72

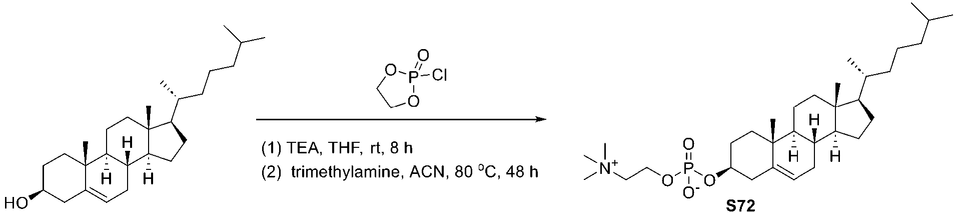

A solution of cholesterol (500 mg, 1.29 mmol, 1.0 eq.) and Et₃N (0.54 mL, 3.88 mmol, 3.0 eq.) in THF (10 mL) was cooled to 0 °C, then 2-chloro-1,3,2-dioxaphospholane-2-oxide (0.55 g, 3.88 mmol, 3.0 eq.) was added into the reaction mixture over 20 min under N₂ atmosphere. The mixture was stirred for another 8 hours. After the reaction was complete, reaction mixture was filtered, the filtrate was added into a sealed tube and added Me₃N (2M in ACN, 10 mL). The mixture was heated to 80 °C and stirred for 48 hours. After the reaction was completed, the solvent was removed, and the crude product was purified to afford compound **S72** (66.4 mg). **¹H NMR** (400 MHz, Methanol-d₄) δ 5.42-5.34 (m, 1H), 4.31-4.20 (m, 2H), 4.03-3.90 (m, 1H), 3.66-3.58 (m, 2H), 3.22 (s, 9H), 2.50-2.29 (m, 2H), 2.09-1.79 (m, 5H), 1.73-0.97 (m, 24H), 0.97-0.92 (m, 3H), 0.91-0.82 (m, 6H), 0.72 (s, 3H); **MS m/z [M+H]⁺ (ESI):** 552.45.

The following compounds are prepared analogously using the methods described in the above examples.

| Compound | Characterization | Preparation Method |
|---|---|---|
| 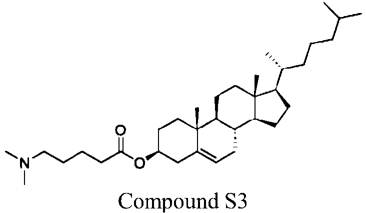 | **¹H NMR:** (400 MHz, CDCl₃) δ 5.39-5.35 (m, 1H), 4.79-4.48 (m, 1H), 2.37-2.26 (m, 6H), 2.23 (s, 6H), 2.07-1.91 (m, 2H), 1.89-1.77 (m, 3H), 1.69-1.39 (m, 10H), 1.39-1.30 (m, 3H), 1.29-1.20 (m, 1H), 1.19-0.94 (m, 13H), 0.91 (d, *J* = 6.6 Hz, 3H), 0.88-0.84 (m, 6H), 0.68 (s, 3H); **MS m/z [M+H]⁺ (ESI):** 514.50. | Example 12 |
| 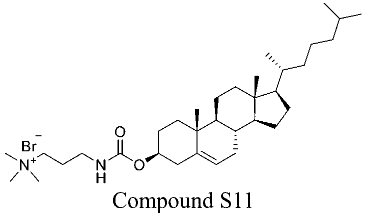 | **¹H NMR** (400 MHz, CDCl₃): δ 6.70-5.80 (m, 1H), 5.80-5.00 (m, 1H), 4.60-4.30 (m, 1H), 3.88-3.70 (m, 1H), 3.68- 3.60 (m, 2H), 3.40-3.27 (m, 10H), 2.30 (m, 2H), 2.18-1.78 (m, 7H), 1.85-0.70 (m, 33H), 0.67 (s, 3H); **MS m/z [M]⁺ (ESI):** 529.45. | Example 5 |
| 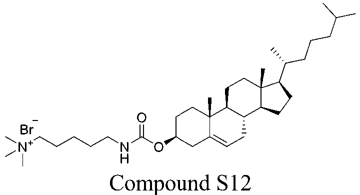 | **¹H NMR** (400 MHz, CDCl₃): δ 5.36 (m, 1H), 5.10 (s, 1H), 4.46 (m, 2H), 3.44 (m, 9H), 3.19 (m, 2H), 2.32 (m, 2H), 2.12-1.84 (m, 7H), 1.63-1.23 (m, 15H), 1.23-0.83 (m, 22H), 0.68 (s, 3H); **MS m/z [M]⁺ (ESI):** 557.50. | Example 5 |
| 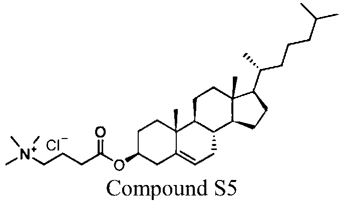 | **¹H NMR** (400 MHz, CDCl₃): δ 5.41-5.35 (m, 1H), 3.77-3.67 (m, 1H), 3.81-3.70 (m, 2H), 3.60-3.40 (m, 9H), 2.55-2.45 (m, 2H), 2.40-2.26 (m, 2H), 2.08-2.00 (m, 2H), 2.00-1.95 (m, 1H), 1.95-1.79 (m, 3H), 1.70-0.81 (m, 34H), 0.72-0.65 (m, 3H); **MS m/z [M]⁺ (ESI):** 514.50. | Example 10 |
| 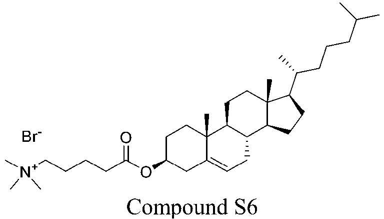 | **¹H NMR** (400 MHz, CDCl₃): δ 5.40-5.34 (m, 1H), 4.65-4.52 (m, 1H), 3.75-3.65 (m, 2H), 3.47 (s, 9H), 2.44-2.25 (m, 4H), 2.06-1.92 (m, 2H), 1.91-1.78 (m, 5H), 1.76-1.66 (m, 2H), 1.65-0.82 (m, 33H), 0.68 (s, 3H); **MS m/z [M]⁺ (ESI):** 528.50. | Example 10 |
| 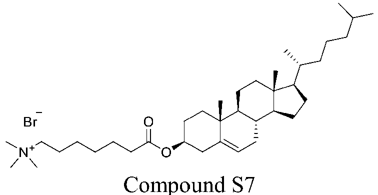 | **¹H NMR:** (400 MHz, CDCl₃) δ 5.37 (d, *J* = 5.0 Hz, 1H), 4.65-4.55 (m, 1H), 3.62 (d, *J* = 8.7 Hz, 2H), 3.48 (s, 9H), 2.40-2.22 (m, 4H), 2.09-1.91 (m, 3H), 1.90-1.73 (m, 4H), 1.67-1.23 (m, 17H), 1.25-0.94 (m, 13H), 0.91 (d, *J* = 6.5 Hz, 3H), 0.88-0.85 (m, 6H), 0.68 (s, 3H); **MS m/z [M]⁺ (ESI):** 556.50. | Example 10 |
| 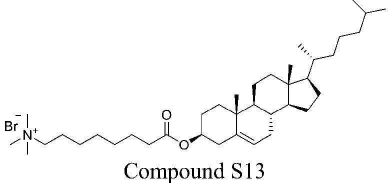 | **¹H NMR** (400 MHz, CDCl₃): δ 5.40-5.34 (m, 1H), 4.66-4.53 (m, 1H), 3.62-3.54 (m, 2H), 3.45 (s, 9H), 2.34-2.23 (m, 4H), 2.06-1.91 (m, 2H), 1.87-1.73 (m, 5H), 1.67-1.51 (m, 5H), 1.50-1.42 (m, 1H), 1.42-1.29 (m, 7H), 1.29-1.07 (m, 6H), 1.06-0.94 (m, 13H), 0.93-0.89 (m, 3H), 0.89-0.80(m, 6H), 0.68 (s, 3H); **MS m/z [M]⁺ (ESI):** 570.50. | Example 10 |
| 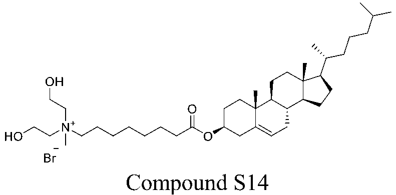 | **¹H NMR** (400 MHz, CDCl₃): δ 5.40-5.34 (m, 1H), 4.65-4.52 (m, 1H), 4.24-4.03 (m, 7H), 3.83-3.67 (m, 4H), 3.61-3.52 (m, 2H), 3.33 (s, 3H), 2.34-2.23 (m, 4H), 2.06-1.91 (m, 2H), 1.87-1.44 (m, 5H), 1.39-1.30 (m, 9H), 1.29-1.15 (m, 10H), 1.14-1.07 (m, 6H), 1.14-1.07 (m, 6H), 0.93-0.89 (m, 3H), 0.89-0.83 (m, 6H), 0.68 (s, 3H); **MS m/z [M]⁺ (ESI):** 630.50. | Example 10 |
| 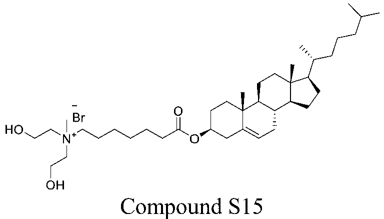 | **¹H NMR** (400 MHz, CDCl₃): δ 5.39-5.35 (m, 1H), 4.64-4.53 (m, 1H), 4.12 (s, 3H), 3.74 (s, 4H), 3.64-3.49 (m, 6H), 3.33 (s, 3H), 2.36-2.23 (m, 4H), 2.05-1.93 (m, 2H), 1.90-1.73 (m, 5H), 1.67-1.51 (m, 3H), 1.51-1.23 (m, 13H), 1.23-1.05 (m, 7H), 1.03-0.94 (m, 6H), 0.91 (d, *J* = 6.4 Hz, 3H), 0.88-0.85 (m, 6H), 0.68 (s, 3H); **MS m/z [M]⁺ (ESI):** 616.60. | Example 15 |
| 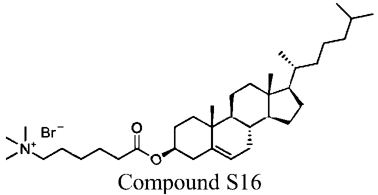 | **¹H NMR** (400 MHz, CDCl₃): δ 5.43-5.31 (m, 1H), 4.48-4.83 (m, 1H), 3.72-3.35 (m, 9H), 2.39-2.17 (m, 8H), 2.05-1.91 (m, 2H), 1.91-1.75 (m, 5H), 1.75-0.79 (m, 35H), 0.75-0.61 (m, 3H); **MS m/z [M]⁺ (ESI):** 542.50. | Example 10 |
| 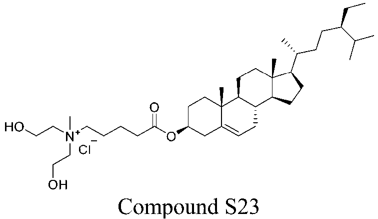 | **¹H NMR** (400 MHz, CDCl₃): δ 5.37-5.27 (m, 1H), 4.63-4.56 (m, 1H), 4.10 (s, 7H), 3.67-3.57 (m, 5H), 3.32 (s, 3H), 2.42-2.28 (m, 4H), 1.99-1.92 (m, 2H), 1.90-1.82 (m, 4H), 1.72-1.41 (m, 8H), 1.36-1.08 (m, 11H), 1.04-0.90 (m, 10H), 0.89-0.78 (m, 9H), 0.68 (s, 3H); **MS m/z [M]⁺ (ESI):** 616.55. | Example 10 |
| 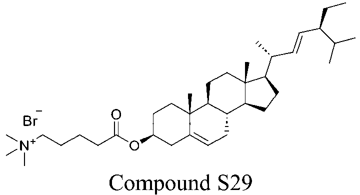 | **¹H NMR** (400 MHz, CDCl₃): δ 5.50-5.28 (m, 1H), 5.20-4.96 (m, 2H), 4.62-4.56 (m, 1H), 3.71-3.65 (m, 2H), 3.45 (s, 9H), 2.44 -2.38 (m, 4H), 2.35-2.26 (m, 3H), 2.03-1.97 (m, 4H), 1.88-1.82 (m, 5H), 1.75-1.69 (m, 3H), 1.63-1.35 (m, 2H), 1.32-1.09 (m, 6H), 1.06-0.98 (m, 7H), 0.88-0.82 (m, 4H), 0.80 (d, J = 6.3 Hz, 6H), 0.70 (s, 3H); **MS m/z [M]⁺ (ESI):** 554.50. | Example 10 |
| 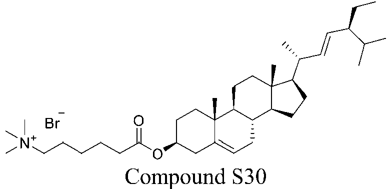 | **¹H NMR** (400 MHz, CDCl₃): δ 5.43-5.33 (m, 1H), 5.23-5.10 (m, 1H), 5.08-4.95 (m, 1H), 4.68-4.50 (m, 1H), 3.72-3.58 (m, 2H), 3.46 (s, 9H), 2.39-2.24 (m, 4H), 2.12-1.93 (m, 3H), 1.93-1.77 (m, 4H), 1.77-1.64 (m, 4H), 1.63-1.33 (m, 11H), 1.33-0.89 (m, 13H), 0.89-0.75 (m, 9H), 0.70 (s, 3H); **MS m/z [M]⁺ (ESI):** 568.50. | Example 10 |
| 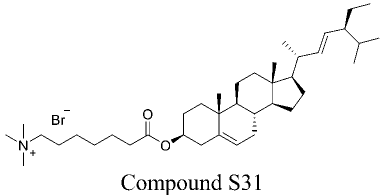 | **¹H NMR** (400 MHz, CDCl₃): δ 5.43-5.32 (m, 1H), 5.23-5.07 (m, 1H), 5.07-4.93 (m, 1H), 4.70-4.52 (m, 1H), 3.68-3.54 (m, 2H), 3.48 (d, *J* = 9.3 Hz, 9H), 2.38-2.23 (m, 4H), 2.10-1.93 (m, 3H), 1.92-1.66 (m, 8H), 1.66-1.35 (m, 13H), 1.34-0.89 (m, 13H), 0.88-0.75 (m, 9H), 0.70 (s, 3H); **MS m/z [M]⁺ (ESI):** 582.50. | Example 10 |
| 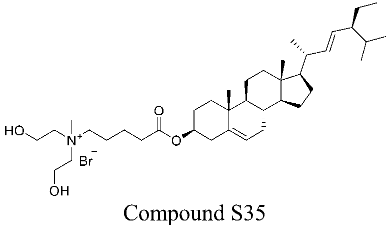 | **¹H NMR** (400 MHz, CDCl₃): δ 5.40-5.34 (m, 1H), 5.20-5.09 (m, 1H), 5.06-4.96 (m, 1H), 4.63-4.52 (m, 1H), 4.14-4.10 (m, 4H), 3.76-3.72 (m, 4H), 3.69-3.59 (m, 5H), 3.33 (s, 3H), 2.43-2.36 (m, 2H), 2.34-2.27 (m, 2H), 2.08-1.91 (m, 2H), 1.91-1.80 (m, 4H), 1.74-1.65 (m, 3H), 1.62-1.37 (m, 5H), 1.32-1.09 (m, 4H), 1.06-0.95 (m, 12H), 0.88-0.76 (m, 9H), 0.70 (s, 3H); **MS m/z [M]⁺ (ESI):** 614.50. | Example 10 |
| 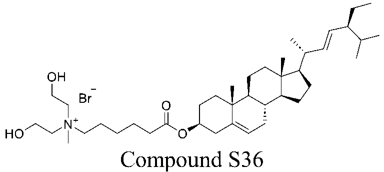 | **¹H NMR** (400 MHz, CDCl₃): δ 5.41-5.30 (m, 1H), 5.25-5.10 (m, 1H), 5.08-4.94 (m, 1H), 4.70-4.49 (m, 1H), 4.18 (s, 4H), 3.95-3.71 (m, 4H), 3.71-3.49 (m, 2H), 3.34 (s, 3H), 2.41-2.22 (m, 4H), 2.22-1.93 (m, 3H), 1.93-1.78 (m, 4H), 1.78-1.64 (m, 3H), 1.64-1.35 (m, 11H), 1.35-0.90 (m, 14H), 0.90-0.74 (m, 9H), 0.70 (s, 3H); **MS m/z [M]⁺ (ESI):** 628.50. | Example 10 |
| 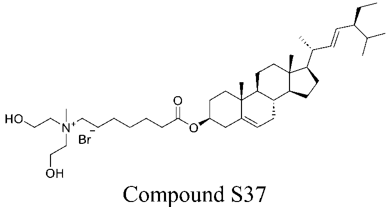 | **¹H NMR** (400 MHz, DMSO-*d₆*): δ 5.40-5.31 (m, 1H), 5.31-5.22 (m, 2H), 5.22-5.10 (m, 1H), 5.10-4.96 (m, 1H), 4.55-4.41 (m, 1H), 3.91-3.72 (m, 4H), 3.53-3.40 (m, 4H), 3.07 (s, 3H), 2.39-2.18 (m, 4H), 2.10-1.60 (m, 8H), 1.60-1.46 (m, 8H), 1.46-1.36 (m, 3H), 1.36-1.09 (m, 8H), 1.09-0.87 (m, 10H), 0.87-0.73 (m, 9H), 0.67 (s, 3H); **MS m/z [M]⁺ (ESI):** 642.50. | Example 10 |
| 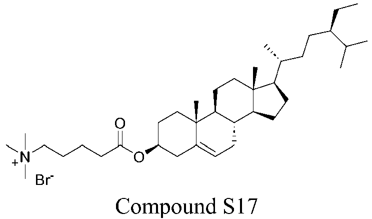 | **¹H NMR** (400 MHz, CDCl₃): δ 5.38-5.29 (m, 1H), 4.59-4.49 (m, 1H), 3.62-3.54 (m, 2H), 3.44 (s, 9H), 2.40-2.34 (m, 2H), 2.30-2.21 (m, 2H), 1.99-1.89 (m, 2H), 1.90-1.79 (m, 5H), 1.73-1.54 (m, 6H), 1.52-1.41 (m, 3H), 1.38-1.08 (m, 9H), 1.02 (s, 5H), 0.96-0.89 (m, 6H), 0.89-0.76 (m, 10H), 0.68 (s, 3H); **MS m/z [M]⁺ (ESI):** 556.50. | Example 10 |
| 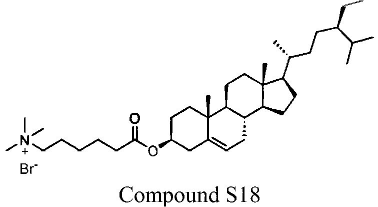 | **¹H NMR** (400 MHz, CDCl₃): δ 5.37-5.27 (m, 1H), 4.63-4.56 (m, 1H), 3.58-3.54 (m, 2H), 3.44 (s, 9H), 2.31-2.21 (m, 4H), 1.99-1.94 (m, 2H), 1.91-1.79 (m, 5H), 1.73-1.41 (m, 11H), 1.36-1.07 (m, 10H), 1.04-0.90 (m, 10H), 0.89-0.76 (m, 10H), 0.68 (s, 3H); **MS m/z [M]⁺ (ESI):** 570.55. | Example 10 |
| 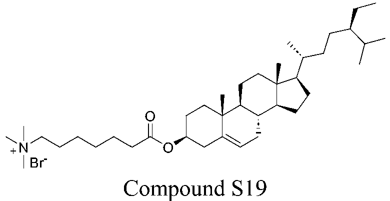 | **¹H NMR** (400 MHz, CDCl₃): δ 5.37-5.27 (m, 1H), 4.63-4.56 (m, 1H), 3.61-3.40 (m, 11H), 2.34-2.25 (m, 4H), 2.05-1.93 (m, 2H), 1.89-1.74 (m, 5H), 1.71-1.53 (m, 6H), 1.52-1.38 (m, 7H), 1.34-0.90 (m, 20H), 0.89-0.74 (m, 10H), 0.68 (s, 3H); **MS m/z [M]⁺ (ESI):** 584.55. | Example 10 |
| 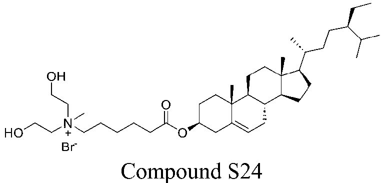 | **¹H NMR** (400 MHz, CD₃OD): δ 5.42-5.32 (m, 1H), 4.60-4.47 (m, 1H), 4.03-3.93 (m, 4H), 3.62-3.45 (m, 6H), 3.19 (s, 3H), 2.39-2.28 (m, 4H), 2.09-1.76 (m, 7H), 1.75-1.44 (m, 9H), 1.43-1.10 (m, 12H), 1.08-1.02 (m, 6H), 0.98-0.80 (m, 14H), 0.72 (s, 3H); **MS m/z [M]⁺ (ESI):** 630.55. | Example 10 |
| 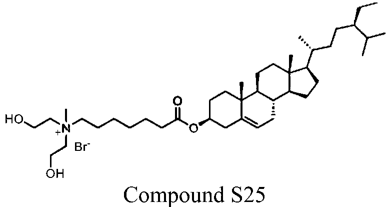 | **¹H NMR** (400 MHz, CD₃OD): δ 5.39 (d, *J* = 5.1 Hz, 1H), 4.59-4.47 (m, 1H), 3.97 (d, *J* = 5.6 Hz, 4H), 3.59-3.42 (m, 6H), 3.18 (s, 3H), 2.35-2.27 (m, 4H), 2.09-1.76 (m, 7H), 1.75-1.34 (m, 16H), 1.32-1.09 (m, 9H), 1.07-1.02 (m, 4H), 0.98-0.92 (m, 4H), 0.90-0.77 (m, 10H), 0.72 (s, 3H); **MS m/z [M]⁺ (ESI):** 644.60. | Example 10 |
| 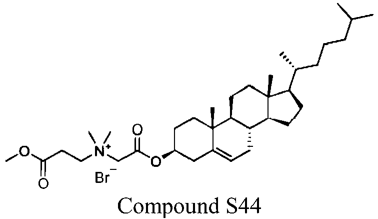 | **¹H NMR** (400 MHz, CDCl₃): δ 5.42-5.36 (m, 1H), 4.66-4.62 (m, 1H), 4.43 (s, 2H), 3.78 (t, *J* = 7.7 Hz, 2H), 3.66 (s, 3H), 3.20 (s, 6H), 2.98 (t, *J* = 7.7 Hz, 2H), 2.40-2.32 (m, 2H), 2.01-1.75 (m, 5H), 1.69-1.45 (m, 11H), 1.44-1.21 (m, 4H), 1.19-0.93 (m, 9H), 0.90 (d, *J* = 6.4 Hz, 3H), 0.87-0.81 (m, 6H), 0.66 (s, 3H); **MS m/z [M]⁺ (ESI):** 558.50. | Example 10 |
| 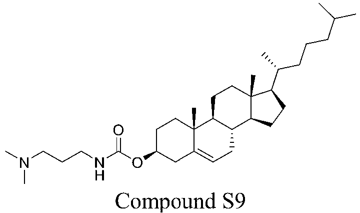 | **¹H NMR** (400 MHz, CDCl₃): δ 5.38-5.31 (m, 2H), 4.54-4.44 (m, 1H), 3.28-3.20 (m, 2H), 2.41-2.27 (m, 4H), 2.26 (s, 6H), 2.06-1.92 (m, 3H), 1.91-1.75 (m, 3H), 1.71-1.64 (m, 2H), 1.63-1.41 (m, 7H), 1.42-1.05 (m, 10H), 1.05-0.93 (m, 6H), 0.91 (d, *J* = 6.5 Hz, 3H), 0.88-0.85 (m, 6H), 0.67 (s, 3H); **MS m/z [M+H]⁺ (ESI):** 515.50. | Example 11 |
| 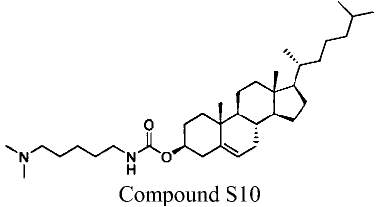 | **¹H NMR** (400 MHz, CDCl₃): δ 5.38-5.35 (m, 1H), 4.69-4.65 (m, 1H), 4.53-4.44 (m, 1H), 3.21-3.12 (m, 2H), 2.40-2.20 (m, 10H), 2.09-1.92 (m, 2H), 1.92-1.75 (m, 3H), 1.63-1.23 (m, 17H), 1.22-0.94 (m, 13H), 0.91 (d, J = 6.5 Hz, 3H), 0.88-0.84 (m, 6H), 0.67 (s, 3H); **MS m/z [M+H]⁺ (ESI):** 543.50. | Example 11 |
| 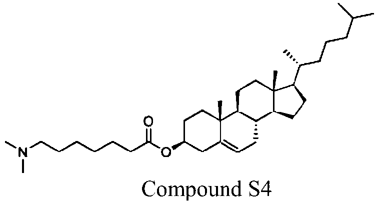 | **¹H NMR** (400 MHz, CDCl₃): δ 5.38-5.35 (m, 1H), 4.65-4.57 (m, 1H), 2.35-2.17 (m, 12H), 2.07-1.93 (m, 2H), 1.89-1.78 (m, 3H), 1.68-1.41 (m, 11H), 1.39-1.24 (m, 8H), 1.23-0.93 (m, 13H), 0.91 (d, *J* = 6.6 Hz, 3H), 0.88-0.85 (m, 6H), 0.68 (s, 3H); **MS m/z [M+H]⁺ (ESI):** 542.50. | Example 12 |
| 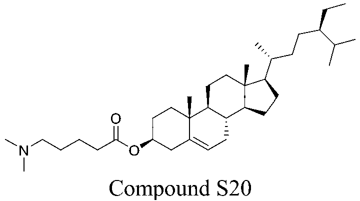 | **¹H NMR** (400 MHz, CDCl₃): δ 5.40-5.34 (m, 1H), 4.66-4.56 (m, 1H), 2.37-2.23 (m, 12H), 2.06-1.91 (m, 2H), 1.91-1.80 (m, 3H), 1.73-1.42 (m, 11H), 1.39-1.08 (m, 11H), 1.06-0.90 (m, 10H), 0.88-0.77 (m, 9H), 0.68 (s, 3H); **MS m/z [M+H]⁺ (ESI):** 542.35. | Example 12 |
| 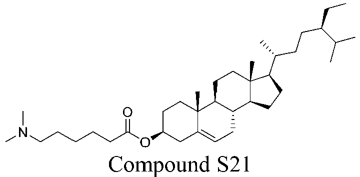 | **¹H NMR** (400 MHz, CDCl₃): δ 5.37-5.29 (m, 1H), 4.68-4.54 (m, 1H), 2.42-2.24 (m, 12H), 2.06-1.91 (m, 2H), 1.90-1.80 (m, 3H), 1.76-1.42 (m, 11H), 1.40-1.08 (m, 12H), 1.07-0.89 (m, 11H), 0.85-0.76 (m, 9H), 0.71-0.64 (m, 3H); **MS m/z [M+H]⁺ (ESI):** 556.45. | Example 12 |
| 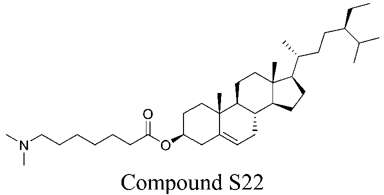 | **¹H NMR** (400 MHz, CDCl₃): δ 5.37-5.29 (m, 1H), 4.64-4.57 (m, 1H), 2.41-2.24 (m, 12H), 2.05-1.93 (m, 2H), 1.90-1.80 (m, 3H), 1.73-1.41 (m, 12H), 1.37-1.31 (m, 6H), 1.29-1.06 (m, 8H), 1.03-1.00 (m, 5H), 0.98-0.90 (m, 5H), 0.89-0.78 (m, 9H), 0.68 (s, 3H); **MS m/z [M+H]⁺ (ESI):** 570.50. | |
| 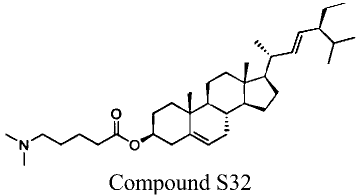 | **¹H NMR** (400 MHz, CDCl₃): δ 5.44-5.32 (m, 1H), 5.29-5.12 (m, 1H), 5.10-4.95 (m, 1H), 4.70-4.55 (m, 1H), 2.40-2.26 (m, 12H), 2.12-1.91 (m, 4H), 1.90-1.79 (m, 2H), 1.75-1.57 (m, 4H), 1.56-1.40 (m, 9H), 1.31-1.20 (m, 2H), 1.20-1.15 (m, 3H), 1.10-0.90 (m, 9H), 0.89-0.75 (m, 9H), 0.74-0.60 (m, 3H); **MS m/z [M+H]⁺ (ESI):** 540.45. | Example 12 |
| 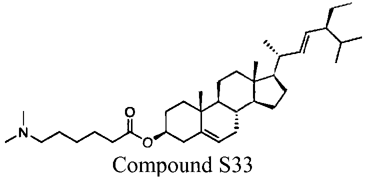 | **¹H NMR** (400 MHz, CDCl₃): δ 5.42-5.33 (m, 1H), 5.25-5.10 (m, 1H), 5.08-4.94 (m, 1H), 4.69 -4.55 (m, 1H), 2.52-2.19 (m, 11H), 2.11-1.92 (m, 3H), 1.92-1.79 (m, 2H), 1.77-0.88 (m, 32H), 0.89-0.74 (m, 9H), 0.70 (s, 3H); **MS m/z [M+H]⁺ (ESI):** 554.50. | Example 12 |
| 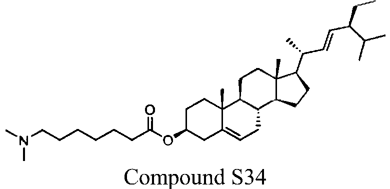 | **¹H NMR** (400 MHz, CDCl₃): δ 5.43-5.31 (m, 1H), 5.22-5.09 (m, 1H), 5.09-4.95 (m, 1H), 4.69-4.53 (m, 1H), 2.43-2.17 (m, 11H), 2.12-1.91 (m, 3H), 1.91-1.79 (m, 2H), 1.77-1.38 (m, 15H), 1.37-1.30 (m, 4H), 1.30-1.07 (m, 6H), 1.07-0.90 (m, 8H), 0.88-0.76 (m, 9H), 0.70 (s, 3H); **MS m/z [M+H]⁺ (ESI):** 568.50. | Example 12 |
| 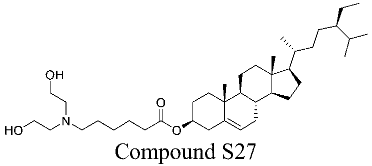 | **¹H NMR:** (400 MHz, CDCl₃) δ 5.38-5.29 (m, 1H), 4.70-4.54 (m, 1H), 3.78 (t, *J* = 5.1 Hz, 4H), 2.92-2.25 (m, 9H), 2.05-1.93 (m, 2H), 1.87-1.82 (m, 3H), 1.73-1.53 (m, 8H), 1.51-1.21 (m, 11H), 1.20-1.07 (m, 6H), 1.05-0.89 (m, 10H), 0.87-0.76 (m, 9H), 0.68 (s, 3H); **MS m/z [M+H]⁺ (ESI):** 616.50. | Example 13 |
| 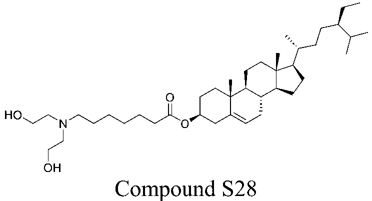 | **¹H NMR** (400 MHz, CDCl₃): δ 5.38-5.29 (m, 1H), 4.66-4.56 (m, 1H), 3.66 (t, *J* = 5.3 Hz, 4H), 2.72 (t, *J* = 5.3 Hz, 4H), 2.63-2.55 (m, 2H), 2.34-2.23 (m, 6H), 2.05-1.93 (m, 3H), 1.90-1.80 (m, 3H), 1.71-1.42 (m, 10H), 1.39-1.09 (m, 12H), 1.04-0.90 (m, 10H), 0.88-0.76 (m, 10H), 0.68 (s, 3H); **MS m/z [M+H]⁺ (ESI):** 630.50. | Example 13 |
| 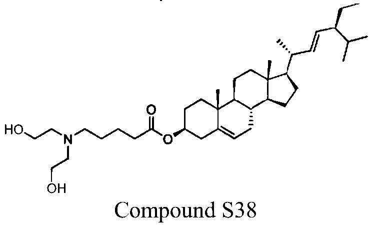 | **¹H NMR** (400 MHz, CDCl₃): δ 5.43-5.30 (m, 1H), 5.21-5.12 (m, 1H), 5.09-4.95 (m, 1H), 4.70-4.50 (m, 1H), 3.96-3.83 (m, 1H), 3.64-3.41 (m, 2H), 3.10-2.90 (m, 1H), 2.90-2.75 (m, 1H), 2.74-2.62 (m, 2H), 2.60-2.57 (m, 1H), 2.39-2.21 (m, 6H), 2.11-1.91 (m,4H), 1.90-1.80 (m, 2H), 1.75-1.35 (m, 12H), 1.35-1.05(m, 6H), 1.05-0.88 (m, 9H), 0.90-0.77 (m, 9H), 0.70 (s, 3H); **MS m/z [M+H]⁺ (ESI):** 600.45. | Example 13 |
| 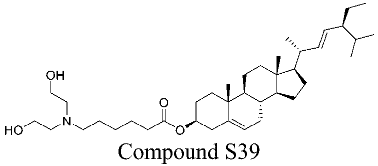 | **¹H NMR** (400 MHz, CDCl₃): δ 5.43-5.30 (m, 1H), 5.22-5.08 (m, 1H), 5.07-4.95 (m, 1H), 4.70-4.57 (m, 1H), 3.98-3.82 (m, 1H), 3.64 (t, *J* = 5.3 Hz, 2H), 3.08-2.75 (m, 2H), 2.73-2.61 (m, 2H), 2.61-2.52 (m, 1H), 2.40-2.22 (m, 4H), 2.16-1.93 (m, 4H), 1.93-1.80 (m, 3H), 1.80-1.31 (m, 6H), 1.31-0.90 (m, 14H), 0.90-0.75 (m, 9H), 0.70 (s, 3H); **MS m/z [M+H]⁺ (ESI):** 614.50. | Example 13 |
| 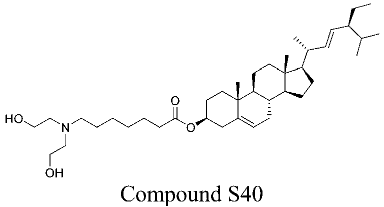 | **¹H NMR** (400 MHz, CDCl₃): δ 5.42-5.33 (m, 1H), 5.22-5.08 (m, 1H), 5.09-4.95 (m, 1H), 4.70-4.51 (m, 1H), 4.00-3.83 (m, 1H), 3.76-3.60 (m, 2H), 3.12-2.88 (m, 1H), 2.88-2.68 (m, 3H), 2.65-2.52 (m, 1H), 2.39-2.22 (m, 4H), 2.16-1.80 (m, 7H), 1.76-1.08 (m, 23H), 1.05-0.89 (m, 9H), 0.89-0.77 (m, 9H), 0.70 (s, 3H); **MS m/z [M+H]⁺ (ESI):** 628.50. | Example 13 |
| 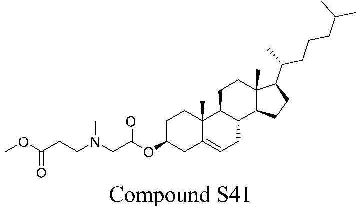 | **¹H NMR** (400 MHz, CD₃OD): δ 5.40-5.30 (m, 1H), 4.64-4.53 (m, 1H), 3.66 (s, 3H), 3.27 (s, 2H), 2.84 (t, *J* = 7.2 Hz, 2H), 2.51 (t, *J* = 7.2 Hz, 2H), 2.35 (s, 5H), 2.09-1.80 (m, 5H), 1.71-1.44 (m, 7H), 1.42-1.27 (m, 4H), 1.27-1.08 (m, 7H), 1.06-0.91 (m, 9H), 0.90-0.83 (m, 6H), 0.72 (s, 3H); **MS m/z [M+H]⁺ (ESI):** 544.45. | Example 13 |
| 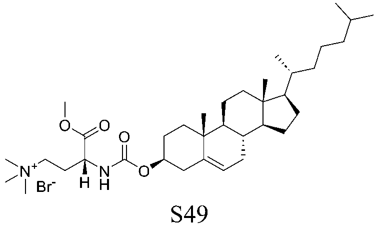 | **¹H NMR** (400 MHz, DMSO-d₆): δ 7.76-7.69 (m, 1H), 5.38-5.31 (m, 1H), 4.38-4.26 (m, 1H), 4.14-4.04 (m, 1H), 3.70-3.65 (m, 3H), 3.52-3.40 (m, 1H), 3.32-3.23 (m, 1H), 3.07 (s, 9H), 2.33-1.73 (m, 9H), 1.60-1.45 (m, 5H), 1.44-1.01 (m, 14H), 1.00-0.96 (m, 4H), 0.96-0.87 (m, 4H), 0.87-0.81 (m, 6H), 0.65 (s, 3H); **MS m/z [M+H]⁺ (ESI):** 587.45. | Example 17 |
| 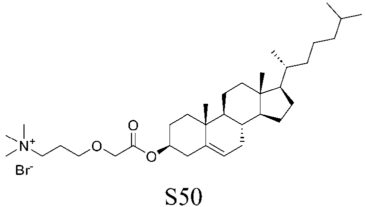 | **¹H NMR** (400 MHz, DMSO-d₆): δ 5.39-5.33 (m, 1H), 4.57-4.50 (m, 1H), 4.13-4.02 (m, 2H), 3.58-3.50 (m, 2H), 3.43-3.35 (m, 2H), 3.07 (s, 9H), 2.33-2.26 (m, 2H), 2.03-1.71 (m, 7H), 1.61-1.20 (m, 11H), 1.18-0.81 (m, 22H), 0.65 (s, 3H); **MS m/z [M+H]⁺ (ESI):** 544.45. | Example 10 |
| 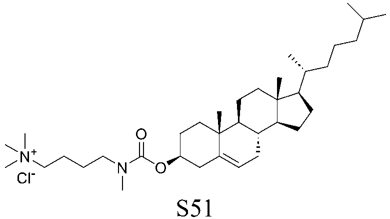 | **¹H NMR** (400 MHz, MeOD): δ 5.39 (d, *J* = 5.2 Hz, 1H), 4.41 (s, 1H), 3.45-3.35 (m, 4H), 3.13 (s, 9H), 2.91 (s, 3H), 2.34 (d, *J* = 8.0 Hz, 2H), 2.10-1.70 (m, 7H), 1.70-1.50 (m, 9H), 1.50-1.25 (m, 4H), 1.25-1.10 (m, 7H), 1.10-0.98 (m, 6H), 0.94 (d, *J* = 6.4 Hz, 3H), 0.92-0.80 (m, 6H), 0.72 (s, 3H); **MS m/z [M+H]⁺ (ESI):** 557.55. | Example 5 |
| 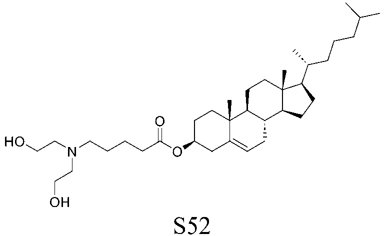 | **¹H NMR** (400 MHz, CDCl₃): δ 5.40-5.35 (m, 1H), 4.78-4.55 (m, 3H), 3.70-3.60 (m, 4H), 2.71-2.63 (m, 4H), 2.63-2.51 (m, 2H), 2.37-2.27 (m, 4H), 2.01-1.88 (m, 8H), 1.70-1.70 (m, 3H), 1.57-1.42 (m, 6H), 1.42-1.30 (m, 4H), 1.19-0.82 (m, 21H), 0.75-0.63 (m, 3H); **MS m/z [M+H]⁺ (ESI):** 574.55. | Example 13 |
| 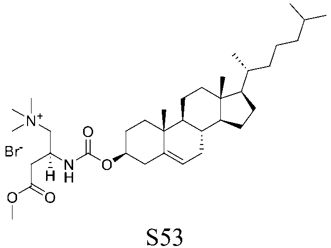 | **¹H NMR** (400 MHz, DMSO-d₆): δ 7.65 (d, *J* = 8.8 Hz, 1H), 5.34 (d, J = 4.9 Hz, 1H), 4.42-4.21 (m, 2H), 3.73-3.43 (m, 5H), 3.12 (s, 9H), 2.63 (d, J = 6.8 Hz, 2H), 2.37-2.13 (m, 2H), 2.03-1.73 (m, 5H), 1.71-0.93 (m, 24H), 0.92-0.87 (s, 3H), 0.87-0.72 (m, 6H), 0.65 (s, 3H); **MS m/z [M+H]⁺ (ESI):** 587.45. | Example 17 |
| 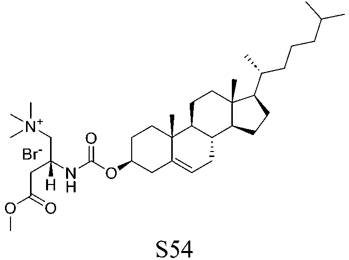 | **¹H NMR** (400 MHz, DMSO-d₆): δ 7.65 (d, *J* = 8.8 Hz, 1H), 5.34 (d, J = 4.9 Hz, 1H), 4.42-4.21 (m, 2H), 3.73-3.43 (m, 5H), 3.12 (s, 9H), 2.63 (d, J = 6.8 Hz, 2H), 2.37-2.13 (m, 2H), 2.03-1.73 (m, 5H), 1.71-0.93 (m, 24H), 0.92-0.87 (s, 3H), 0.87-0.72 (m, 6H), 0.65 (s, 3H); **MS m/z [M+H]⁺ (ESI):** 587.50. | Example 17 |
| 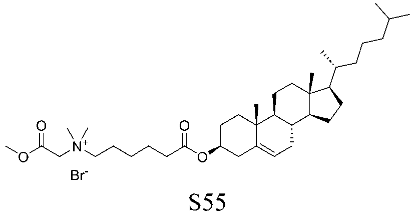 | **¹H NMR** (400 MHz, DMSO-d₆): δ5.38-5.32 (m, 1H), 4.45-4.41 (m, 3H), 3.77 (s, 3H), 3.51-3.43 (m, 2H), 2.3-2.23 (m, 6H), 2.3-2.23 (m, 4H), 2.00-1.88 (m, 2H), 1.88-1.64 (m, 5H), 1.62-1.49 (m, 7H), 1.52-1.21 (m, 9H), 1.17-1.05 (m, 6H), 1.05-0.97 (m, 6H), 0.93-0.82 (m, 3H), 0.82-0.78 (m, 6H), 0.68-0.63 (m, 3H); **MS m/z [M+H]⁺ (ESI):** 600.60. | Example 10 |
| 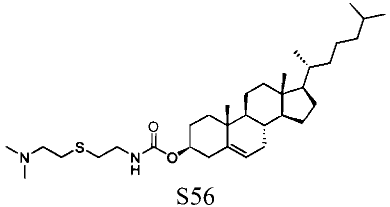 | **¹H NMR** (400 MHz, CDCl₃): δ 5.38 (s, 1H), 4.90-4.30 (m, 2H), 3.47-3.27 (m, 2H), 2.75-2.65 (m, 4H), 2.64-2.54 (m, 2H), 2.45-2.20 (m, 8H), 2.08-1.75 (m, 4H), 1.70-1.42 (m, 7H), 1.42-1.25 (m, 5H), 1.25-1.05 (m, 7H), 1.05-0.95 (m, 5H), 0.95-0.86 (m, 10H), 0.68 (s, 3H); **MS m/z [M+H]⁺ (ESI):** 561.50. | Example 15 |
| 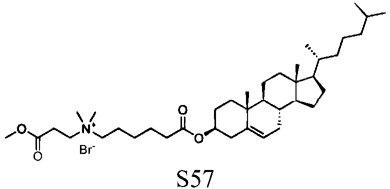 | **¹H NMR** (400 MHz, DMSO-d₆): δ 5.39-5.32 (m, 1H), 4.50-4.41 (m, 1H), 3.68-3.62 (m, 3H), 3.58-3.48 (m, 3H), 3.30-3.21 (m, 2H), 3.04-2.99 (m, 5H), 2.97-2.88 (m, 2H), 2.35-2.23 (m, 4H), 2.02-1.88 (m, 2H), 1.81-1.73 (m,3H), 1.70-1.62 (m, 2H), 1.61-1.44 (m, 7H), 1.44-1.23 (m, 9H), 1.21-1.09 (m, 6H), 1.06-0.97 (m, 5H), 1.00-0.90 (m, 4H), 0.87-0.73 (m, 6H), 0.68-0.62 (m, 3H); **MS m/z [M+H]⁺ (ESI):** 614.55. | Example 10 |
| 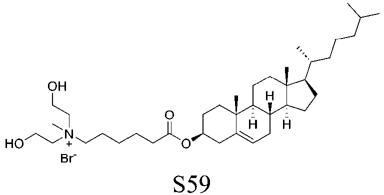 | **¹H NMR** (400 MHz, CDCl₃): δ 5.38-5.37 (m, 1H), 4.61-4.51 (m, 1H), 4.14-4.11 (m, 5H), 3.78-3.74 (m, 5H), 3.58 (m, 2H), 3.33 (s, 4H), 2.33-2.29 (m, 5H), 2.00-0.98 (m, 32H), 0.95-0.85 (m, 10H), 0.68 (s, 3H); **MS m/z [M+H]⁺ (ESI):** 602.45. | Example 10 |
| 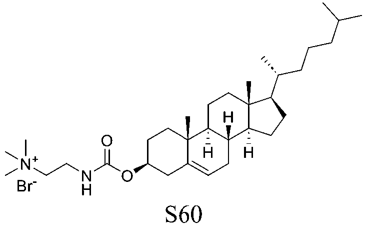 | **¹H NMR** (400 MHz, MeOD): δ 5.38-5.36 (m, 1H), 4.52-4.38 (m, 1H), 3.59-3.56 (m, 2H), 3.48-3.43 (m, 2H), 3.32-3.30 (m, 9H), 2.33-2.31 (m, 2H), 2.07-1.84 (m, 5H), 1.61-0.87 (m, 33H), 0.72 (s, 3H); **MS m/z [M+H]⁺ (ESI):** 515.40. | Example 5 |
| 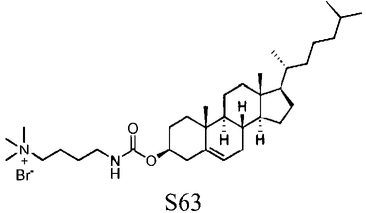 | **¹H NMR** (400 MHz, CDCl₃): δ 5.36-5.35 (m, 1H), 4.46-4.42 (m, 1H), 3.78-3.73 (m, 2H), 3.43 (s, 9H), 3.35-3.19 (m, 2H), 2.31-2.30 (m, 2H), 2.03-1.83 (m, 7H), 1.77-1.28 (m, 35H), 0.72 (s, 3H); **MS m/z [M+H]⁺ (ESI):** 543.40. | Example 17 |
| 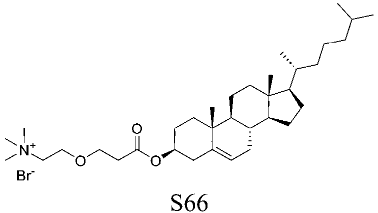 | **¹H NMR** (400 MHz, DMSO-d₆): δ 5.39-5.33 (m, 1H), 4.57-4.50 (m, 1H), 4.13-4.02 (m, 2H), 3.58-3.50 (m, 2H), 3.43-3.35 (m, 2H), 3.07 (s, 9H), 2.33-2.26 (m, 2H), 2.03-1.71 (m, 7H), 1.61-1.20 (m, 11H), 1.18-0.81 (m, 22H), 0.65 (s, 3H); **MS m/z [M+H]⁺ (ESI):** 544.45. | Example 10 |
| 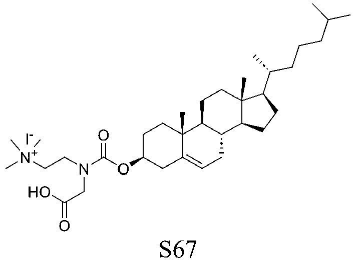 | **¹H NMR** (400 MHz, DMSO-d₆): δ 5.41-526 (m, 1H), 4.40-4.28 (m, 1H), 4.04-3.93 (m, 2H), 3.71-3.63 (m, 2H), 3.51-3.44 (m, 2H), 3.10 (s, 9H), 2.38-2.30 (m, 1H), 2.29-2.16 (m, 1H), 2.00-1.88 (m, 2H), 1.87-1.71 (m, 3H), 1.67-1.45 (m, 5H), 1.44-1.20 (m, 7H), 1.17-0.95 (m, 13H), 0.93-0.88 (m, 3H), 0.87-0.81 (m, 6H), 0.65 (s, 3H); **MS m/z [M+H]⁺ (ESI):** 573.40. | Example 19 |
| 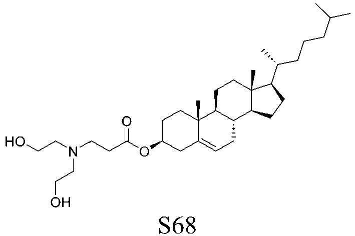 | **¹H NMR** (400 MHz, CDCl₃): δ5.41-5.35 (m, 1H), 4.65 (m, 1H), 3.80-3.40 (m, 4H), 2.90-2.84 (m, 2H), 2.70-2.60 (m, 4H), 2.51-2.47 (m, 2H), 2.36-2.29 (m, 2H), 2.10-1.90 (m, 2H), 1.90-1.76 (m, 3H), 1.67-1.40 (m, 7H), 1.38-1.20 (m, 5H), 1.20-1.15 (m, 6H), 1.08-0.92 (m, 6H), 0.92-0.85 (m, 3H), 0.86-0.70 (m, 6H), 0.70-0.67 (s, 3H)); **MS m/z [M+H]⁺ (ESI):** 546.45. | Example 13 |
| 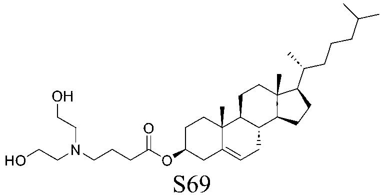 | **¹H NMR** (400 MHz, CDCl₃): δ 5.42-5.30 (m, 1H), 4.72-4.55 (m, 1H), 3.80-3.60 (m, 3H), 3.52-3.40 (m, 1H), 2.82-2.70 (m, 4H), 2.70-2.60 (m, 1H), 2.39-2.28 (m, 4H), 2.06-1.91 (m, 3H), 1.90-1.78 (m, 5H), 1.66-1.41 (m, 7H), 1.41-1.30 (m, 5H), 1.30-0.87 (m, 16H), 0.87-0.84 (m, 6H), 0.68 (s, 3H); **MS m/z [M+H]⁺ (ESI):** 560.60. | Example 13 |
| 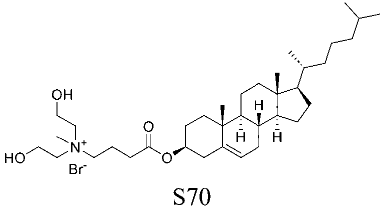 | **¹H NMR** (400 MHz, CDCl₃): δ 5.38-5.37 (m, 1H), 4.78-4.58 (m, 3H), 4.14 (s, 4H), 3.83-3.66 (m, 6H), 3.36 (s, 3H), 2.48-2.46 (m, 2H), 2.32-2.30 (m, 2H), 2.09-1.85 (m, 16H), 1.64-0.86 (m, 26H), 0.68 (s, 3H); **MS m/z [M+H]⁺ (ESI):** 574.40. | Example 10 |
| 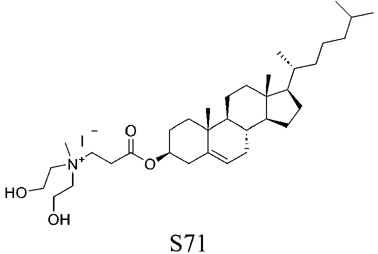 | **¹H NMR** (400 MHz, CDCl₃): δ 5.41-5.34 (m, 1H), 4.68-4.51 (m, 1H), 4.38-4.04 (m, 4H), 3.97-3.77 (m, 6H), 3.38 (s, 3H), 3.09-2.88 (m, 2H), 2.46-2.29 (m, 2H), 2.06-1.83 (m, 5H), 1.54-0.87 (m, 33H), 0.68 (s, 3H); **MS m/z [M+H]⁺ (ESI):** 561.40. | Example 10 |

### Formulation and Biological Studies

### Example 1: Preparation of LNPs

Materials used for lipid nanoparticle assembly include: (1) ionizable lipid compound: ionizable lipids designed and synthesized according to the present invention (e.g. Compound 132) or Lipid5 (purchased from AVT); (2) structural lipid: e.g. Cholesterol (purchased from Sigma-Aldrich); (3) phospholipid: such as DSPC (1,2-distearoyl-sn-glycero-3-phosphocholine, purchased from AVT) or DOPE (1,2-dioleoyl-sn-glycero-3-phosphoethanolamine, commercially available);; (5) polyethylene glycolated lipid: e.g. DMG-PEG2000 (1,2-dimyristoyl-rac-glycero-3-methoxypolyethylene glycol-2000, purchased from AVT); (5) steroid compounds designed and synthesized according to the present invention; and (6) nucleic acid payloads (e.g. Luciferase mRNA, siRNA, or CRISPR Cas 9 mRNA, etc.). Exemplary materials used in LNPs are listed below.

**Table 1**

| No. | Structure name | Structure |
|---|---|---|
| 1 | Luciferase mRNA | Commercially available |
| 2 | Cholesterol | 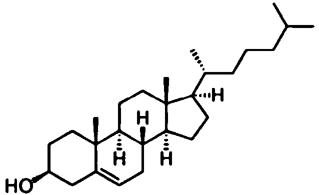 |
| 3 | DSPC | 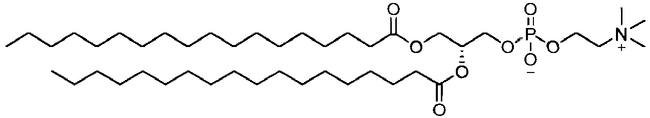 |
| 4 | DOPE | 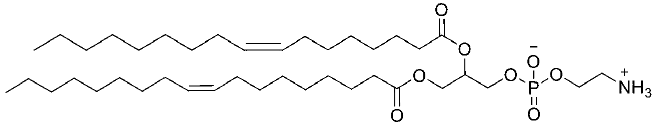 |
| 5 | DMG-PEG2000 | 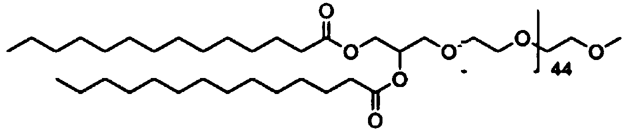 |
| 6 | Compound 132 | 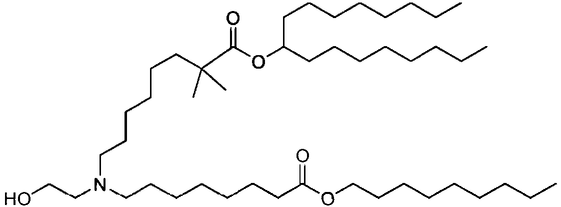 |
| 7 | Lipid5 | 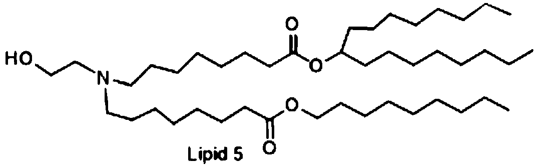 |

The steroid compounds designed and synthesized according to the present invention are listed in Table 2.

**Table 2**

| No. | structure name | structure |
|---|---|---|
| 1 | Compound S16 | 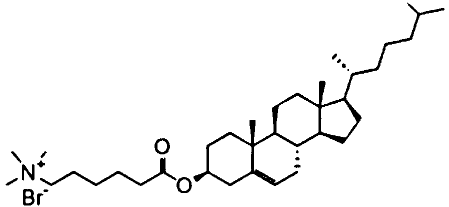 |
| 2 | BHEM-Chol | 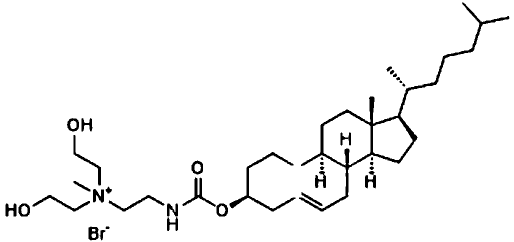 |
| 3 | Compound S20 | 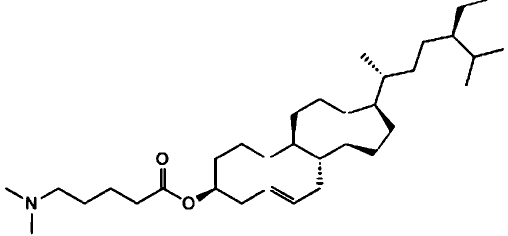 |

Lipid nanoparticles were prepared by (1) dissolving and mixing ionizable lipid compounds, cholesterol, cationic steroid compound, phospholipid, and polyethylene glycolated lipids in ethanol; (2) dissolving the mRNA payload in 25 mM sodium acetate solution (pH = 4.5); (3) using an automated high-throughput microfluidic system to mix the organic phase containing the lipid mixture and the aqueous phase containing the mRNA component in the flow ratio range of 1:1 to 1:4 at a mixing speed of from about 10 mL/min to about 18 mL/min; (4) the prepared lipid nanoparticles (N/P=6) were diluted with phosphate buffer solution and the nanoparticle solutions were ultrafiltered to the original preparation volume using ultrafiltration tubes (purchased from Millipore) with a cut-off molecular weight of 30 kDa; and (5) the obtained nanoparticles were filtered through a sterile 0.2 µm filter membrane and then stored in a sealed glass vial at low temperature.

The preparation method of lipid nanoparticles includes microfluidic mixing systems, but is not limited to this method. Other methods include T-type mixers, and ethanol injection method, and the like.

**Table 3**

| Formulation ID | ionizable lipid | mol % | steroid compound | mol % | Cholesterol (mol %) | Phospho lipid | mol % | DMG-PEG2000 (mol %) |
|---|---|---|---|---|---|---|---|---|
| LNP1 | Lipid5 | 50% | N/A | 0 | 38.5% | DSPC | 10% | 1.5% |
| LNP2 | Compound 132 | 50% | N/A | 0 | 38.5% | DSPC | 10% | 1.5% |
| LNP3 | Lipid5 | 50% | compound S16 | 5 | 33.5% | DSPC | 10% | 1.5% |
| LNP4 | Lipid5 | 50% | BHEM-Chol | 5 | 33.5% | DSPC | 10% | 1.5% |
| LNP5 | Compound 132 | 50% | compound S16 | 5 | 33.5% | DSPC | 10% | 1.5% |
| LNP6 | Compound 132 | 50% | BHEM-Chol | 5 | 33.5% | DSPC | 10% | 1.5% |

**Table4**

| | | | | | | | | |
|---|---|---|---|---|---|---|---|---|
| LNP7 | Compound 132 | 50% | compound S16 | 5 | 33.5% | DOPE | 10% | 1.5% |
| LNP8 | Compound 132 | 50% | compound S16 | 10 | 28.5% | DOPE | 10% | 1.5% |

**Table5**

| | | | | | | | | |
|---|---|---|---|---|---|---|---|---|
| LNP9 | Compound 132 | 50% | compound S16 | 5 | 33.5% | DSPC | 10% | 1.5% |

### Example 2: Characterization of physical properties of LNPs

The particle size and particle size dispersity index (PDI) of the prepared lipid nanoparticles were measured using Dynamic Light Scattering (DLS) with a Zetasizer Pro (from Malvern Instruments Ltd) and DynaPro NanoStar (from Wyatt). The degree of RNA encapsulation by lipid nanoparticles was characterized by the Encapsulation Efficiency % (EE%), which reflects the degree of binding of lipid nanoparticles to RNA fragments. This parameter was measured by the method of Quant-it^{™} RiboGreen RNA Assay (purchased from Invitrogen). Lipid nanoparticle samples were diluted in TE buffer (10 mM Tris-HCl, 1 mM EDTA, pH = 7.5). A portion of the sample solution was removed, to which 0.5% Triton (Triton X-100) was added, and then allowed to stand at 37° C for 120 minutes. Immediately after the addition of RIBOGREEN^{®} reaction solution, the fluorescence values were read on a Varioskan LUX multifunctional microplate reader (from Thermo Fisher) at 485 nm for absorption and 528 nm for emission to give the encapsulation efficiency values.

### Example 3: Animal Experiments

The delivery effect and safety of nanoparticles comprising cationic steroid compound encapsulated with luciferase mRNA (Trilink, L-7202) in mice were evaluated. The test mice were SPF-grade C57BL/6 mice, female, 6-8 weeks old, weighing 18-22 g, and were purchased from SPF (Beijing) Biotechnology Co., Ltd. All animals were acclimatized for more than 7 days prior to the experiment, and had free access to food and water during the experiment. The conditions include alternating light and dark for 12/12 h, the indoor temperature of 20-26 °C and the humidity of 40-70%. The mice were randomly grouped. The lipid nanoparticles formulated above were injected into mice by intravenous administration at a single dose of 0.5 mg/kg mRNA. In vivo bioluminescence imaging was performed at 6 hours post-administration using a small animal in vivo imaging system (IVIS LUMINA III, PerkinElmer). Following imaging, mice were dissected to harvest liver, lungs, and spleen, after which ex vivo bioluminescence imaging of the isolated organs was conducted with the same IVIS system. The protocols are as follows: a D-luciferin with a concentration of 15 mg/mL was prepared with physiological saline, and each mouse was given the D-luciferin solution by intraperitoneal injection. After giving the substrate for 5 minutes, the mice were placed in an anesthesia chamber to perform anesthesia with 2.5% isoflurane concentration. The fully anesthetized mice were put into IVIS, and fluorescence imaging was performed to confirm fluorescence distribution in the living body. After imaging, the mice were taken out, sacrificed by draging the neck and the dissected organs were placed in the IVIS for bioluminescence imaging, data acquisition and analysis were performed on the concentrated distribution area of luminescence.

The organ distribution and efficiency of lipid nanoparticle (LNP) as carriers for in vivo delivery was represented by the average values of fluorescence intensity and total photon counts across organs of different animals within the same subject group, as detailed in Tables 5-1 and 5-2. Higher values of fluorescence intensity and total photon counts indicate enhanced in vivo delivery efficiency of LNPs for the mRNA fragment in the specified organs (liver, lung, or spleen).

**Table 5-1**

| **Formulation ID** | **Size (nm)** | **PDI** | **EE%** | **Liver (Total Flux)** | **Lung (Total Flux)** | **Spleen (Total Flux)** |
|---|---|---|---|---|---|---|
| LNP1 | 97.32 | 0.0868 | 95.51 | 6.81E+09 | 7.02E+06 | 5.09E+07 |
| LNP2 | 119 | 0.148 | 96.20 | 2.42E+09 | 1.44E+07 | 1.09E+08 |
| LNP3 | 168.5 | 0.1314 | 97.80 | 3.34E+07 | 8.62E+06 | 2.50E+08 |
| LNP4 | 152.9 | 0.0524 | 95.72 | 3.21E+07 | 8.18E+06 | 2.63E+08 |
| LNP5 | 131.6 | 0.131 | 91.73 | 3.67E+07 | 1.73E+07 | 4.27E+08 |
| LNP6 | 139.4 | 0.0603 | 96.43 | 6.12E+07 | 7.29E+06 | 2.09E+08 |

**Table 5-2**

| **Formulation ID** | **Size (nm)** | **PDI** | **EE%** | **Liver (Total Flux)** | **Lung (Total Flux)** | **Spleen (Total Flux)** |
|---|---|---|---|---|---|---|
| LNP7 | 101.8 | 0.1179 | 95.34 | 2.70E+07 | 2.13E+07 | 4.14E+08 |
| LNP8 | 91.65 | 0.1865 | 96.71 | 5.05E+07 | 5.23E+07 | 2.67E+08 |

**Table 5-3**

| **Formulation ID** | **Size (nm)** | **PDI** | **EE%** | **Liver (Total Flux)** | **Lung (Total Flux)** | **Spleen (Total Flux)** |
|---|---|---|---|---|---|---|
| LNP9 | 135.7 | 0.197 | 98.59 | 3.07E+07 | 2.26E+07 | 2.37E+08 |

### Example 4

Lipid nanoparticles were prepared according to the same method as described in the preceding examples, using the conditions specified in Table 6. The structures of the corresponding steroid compounds and the results of animal experiments are presented in Table 7.

**Table 6**

| **Formulation ID** | Lipid compound ID | Lipid compound Ratio | Steroid compound ID | Steroid compound Ratio | Cholester ol Ratio | Phospho lipid | Phospholi pid Ratio | polyethylene glycolated lipid Ratio |
|---|---|---|---|---|---|---|---|---|
| Compound 132 + Steroid Compound ID | Compound 132 | 50% | See Table 7 | 5% | 33.5% | DOPE | 10% | 1.5% |

**Table 7**

| Steroid Compound ID | Structure | Liver | Lung | Spleen |
|---|---|---|---|---|
| BHEM-6-Chol | 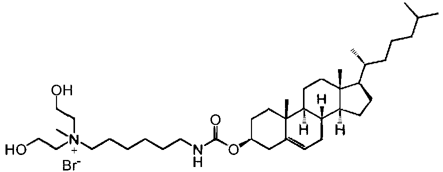 | 2.59E+06 | 1.79E+07 | 5.22E+07 |
| Compound S6 | 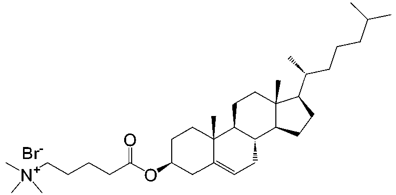 | 2.94E+06 | 6.62E+06 | 6.57E+07 |
| Compound S35 | 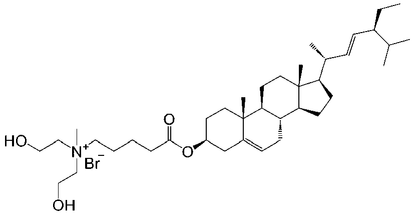 | 3.47E+06 | 3.30E+06 | 7.55E+07 |
| Compound S43 | 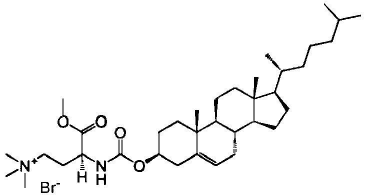 | 2.86E+06 | 1.61E+07 | 3.69E+07 |
| Compound S11 | 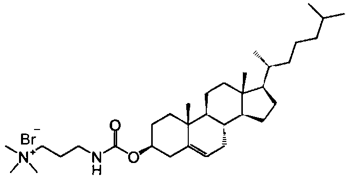 | 3.17E+06 | 7.38E+06 | 5.97E+07 |
| Compound S19 | 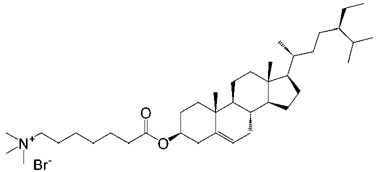 | 2.52E+06 | 1.12E+07 | 7.24E+07 |
| Compound S14 | 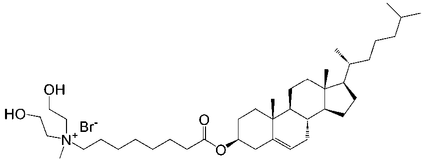 | 2.57E+06 | 6.41E+06 | 4.54E+07 |
| Compound S53 | 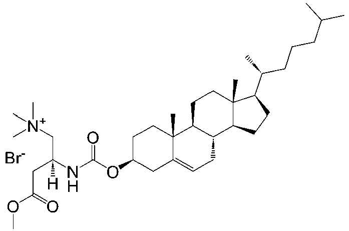 | 4.75E+06 | 3.51E+06 | 1.22E+08 |
| Compound S46 | 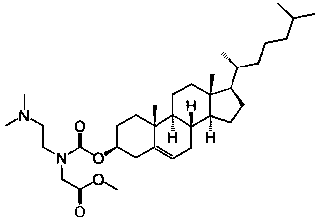 | 4.46E+07 | 4.81E+06 | 8.23E+07 |
| Compound S27 | 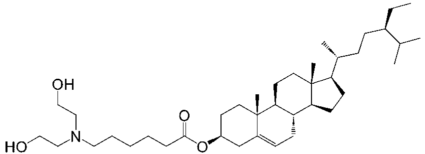 | 4.50E+07 | 3.16E+06 | 1.19E+08 |
| Compound S21 | 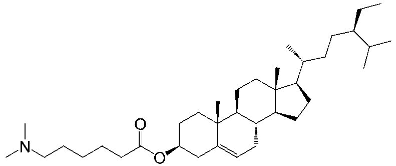 | 4.17E+07 | 3.88E+06 | 1.05E+08 |

The data described above are intended to be merely exemplary, and those skilled in the art will recognize, or will be able to ascertain using no more than routine experimentation, other equivalents of specific compounds, materials, and procedures. All such equivalents are considered to be within the scope of the invention and are encompassed by the appended claims.

### Other embodiments:

1. A lipid nanoparticle, characterized in that it comprises a steroid compound.
2. The lipid nanoparticle according to embodiment 1, wherein the delivery or expression level of the payload in the spleen after administering the lipid nanoparticle to the subject is at least 1 time, more preferably at least 2 times, more preferably at least 5 times, more preferably at least 10 times, or more preferably at least 100 times higher than the delivery or expression level of the payload in the liver.
3. The lipid nanoparticle according to embodiment 1 or 2, wherein the lipid nanoparticle further comprises an ionizable lipid, a structural lipid, and a polymer conjugated lipid.
4. The lipid nanoparticle according to embodiment 2 or 3, wherein the lipid nanoparticle is essentially free of neural phospholipid.
5. The lipid nanoparticle according to any one of embodiments 1-4, wherein the steroid compound is a compound of Formula (I):

M⁻ V₁-[-(R₂₀)ₐ₁-Y₁]ₙ₁-Z₁ (I)

wherein,
Z₁ is an optionally substituted steroid;
each instance of Y₁ is independently absent or is -C(O)O-, -O-, -NH-, -SC(O)O-, -OC(O)NR₂ₐ-, - NR₂ₐC(O)NR₂,-, -OC(O)S-, -OC(O)O-, -NR₂ₐC(O)O-, -OC(O)-, -SC(O)-, -C(O)S-, -NR₂ₐ-, -C(O)NR₂ₐ-, - NR₂ₐC(O)-, -NR₂ₐC(O)S-, -SC(O)NR₂ₐ-, -C(O)-, -OC(S)-, -C(S)O-, -OC(S)NR₂ₐ-, -NR₂ₐC(S)O-, -S-S-, or - S(O)₀₋₂-;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with H, C₁₋₁₀ alkyl, -L_{2b}-OR_{2b}, -L_{b}-SR_{2b}, -L_{2b}-NR_{2b}R'_{2b}, or -L_{2b}N'R_{2b}R'_{2b}R"_{2b}
each instance of R₂₀ is independently -(CR₂₀₁R₂₀₂)-, -NR₂₁- or -N⁺R₂R₂₂-;
each instance of R₂₀₁ and R₂₀₂ are independently H, OH, alkyl, alkoxy, -L_{2b}-NR₂₁R₂₂, -L_{2b}-N⁺R₂₁R₂₂R₂₃;
a1 and n1 are each independently 0, 1, 2, 3, 4, 5, 6, 7, 8, 9, or 10;
provided that, when n1 is greater than 1, each instance of R₂₀, Y₁ and a1 in (R₂₀) ₐ₁-Y₁ moiety can be independently defined as above;
V₁ is a head group and V₁ is -NR₂₁R₂₂, -N⁺R₂₁R₂₂R₂₃, or optionally substituted nitrogen-containing heterocyclyl selected from the group consisting of imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone and (iso)quinoline, or quaternary ammonium salts formed by N-atom substitution with R₂₁; or nitrogen-containing non-heterocyclic group selected from amidine, guanidine and urea;
when the lipid moiety carries no positive charge, M⁻ is absent;
when the lipid moiety carries a positive charge, M⁻ represents the anionic counterion moiety comprising chloride, bromide, iodide, sulfate, nitrate, perchlorate, formate, phosphate, pyrophosphates, citrate, sulfonate, methanesulfonate, triflate and trifluoroacetate;
each instance of R₂₁, R₂₂, R₂₃ are independently H, C₁₋₈ alkyl, -L_{2b}-OR_{2b}, -L_{2b}-SR_{2b}, or -L_{2c}-NR_{2b}R'_{2b}R"_{2b};
L_{2b} is absent or C₁₋₁₀ alkylene; and
each of R_{2b}, R'_{2b} and R"_{2b} are independently H or C₁₋₁₀ alkyl.

6. The lipid nanoparticle according to embodiment 5, wherein in the steroid compound of formula (I),
the steroid is cholesteryl or sitosteyl;
Y₁ is absent, or -SC(O)O-, -OC(O)NR₂ₐ-, -NR₂ₐC(O)NR₂ₐ-, -OC(O)S-, -OC(O)O-, -NR₂ₐC(O)O-, - NR₂ₐC(O)S-, -SC(O)NR₂ₐ-, -OC(S)NR₂ₐ-, or -NR₂ₐC(S)O-;
W₁ is absent, or -C(O)O-, -OC(O)-, -SC(O)-, or -C(O)S-;
R₂ₐ is independently H or C₁₋₁₀ alkyl;
R₂₀₁ and R₂₀₂ are independently H, alkyl, -NR₂₁R₂₂, or -N⁺R₂₁R₂₂R₂₃;
n₁ is 0 or 1;
V₁ is -NR₂₁R₂₂, -N⁺R₂₁R₂₂R₂₃, or optionally substituted nitrogen-containing non-heterocyclic group selected from amidine, guanidine and urea; and
each instance of R₂₁, R₂₂, R₂₃ are independently H, C₁₋₈ alkyl, or -L_{2b}-OH;
each instance of L_{2b} is independently a chemical bond or C₁₋₈ alkylene.

7. The lipid nanoparticle according to embodiment 5 or 6, wherein in the steroid compound of formula (I),
V₁ is -N⁺R₂₁R₂₂R₂₃;
R₂₁, R₂₂ and R₂₃ are independently H, C₁₋₈ alkyl, or -L_{2b}-OH;
each instance of L_{2b} is independently a chemical bond or C₁₋₈ alkylene.

8. The lipid nanoparticle according to embodiment 7, wherein in the steroid compound of formula (I),
R₂₁, R₂₂ and R₂₃ are independently H, C₁₋₄ alkyl, or -L_{2b}-OH;
each instance of L_{2b} is independently a chemical bond or C₁₋₄ alkylene.

9. The lipid nanoparticle according to embodiment 8, wherein in the steroid compound of formula (I),
R₂₁, R₂₂ and R₂₃ are independently H, C₁₋₂ alkyl, or -L_{2b}-OH;
each instance of L_{2b} is independently a chemical bond or C₁₋₂ alkylene.

10. The lipid nanoparticle according to embodiment 7, wherein in the steroid compound of formula (I),
V₁ is 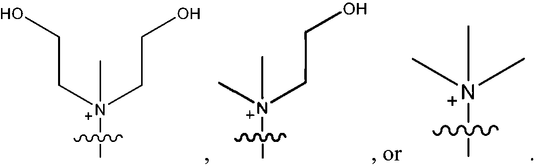
11. The lipid nanoparticle according to embodiment 5, wherein the steroid compound is:

| | |
|---|---|
| DC-Cholesterol (DC-Chol) | 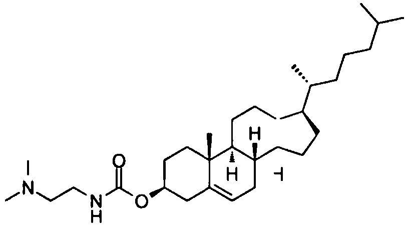 |
| BHEM-Cholesterol (BHEM-Chol) | 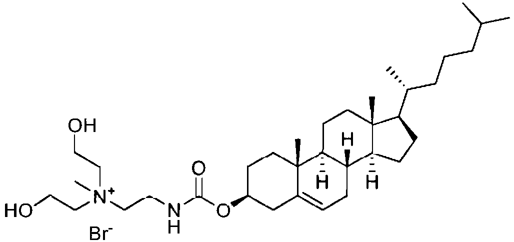 |
| MHEM-Cholesterol (MHEM-Chol) | 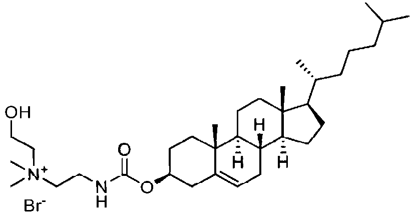 |
| DC-β-Sitosterol (DC-β-Sito) | 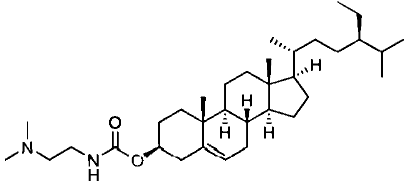 |
| BHEM-β-Sitosterol (BHEM-Sito) | 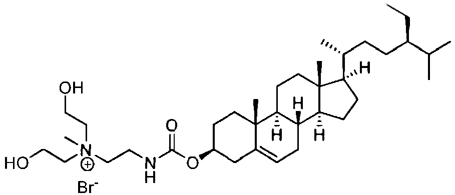 |
| Lys-es-Cholesterol (Lys-es-Chol) | 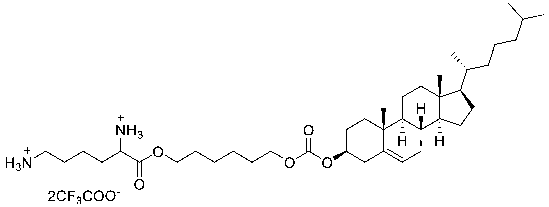 |
| Arg-es-Cholesterol (Arg-es-Chol) | 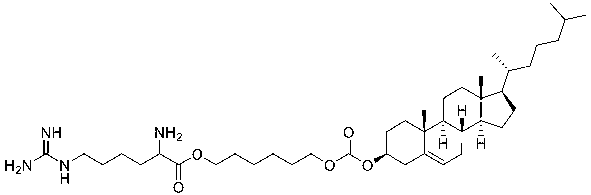 |
| BHEM-6-Chol | 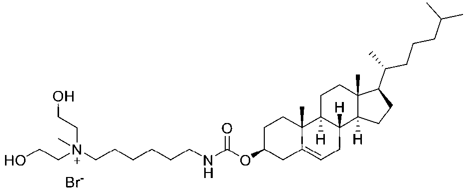 |
| BHEM-4-Chol | 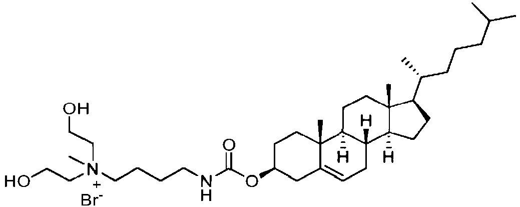 |
| BHEM-6-Sito | 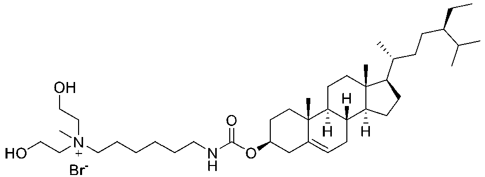 |
| BHEM-4-Sito | 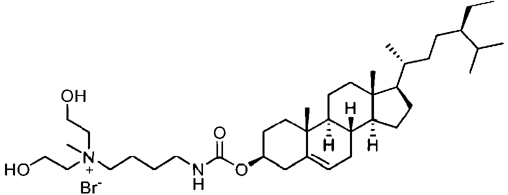 |
| SCL-1 | 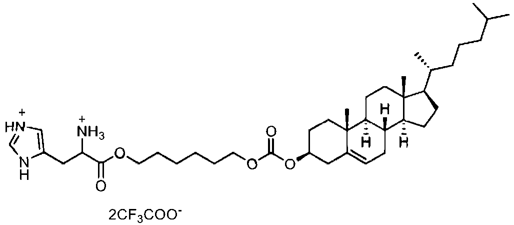 |
| SCL-2 | 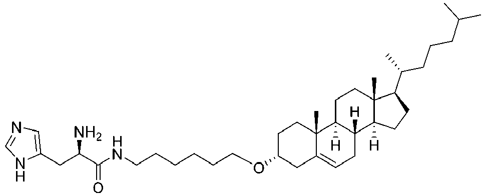 |
| SCL-3 | 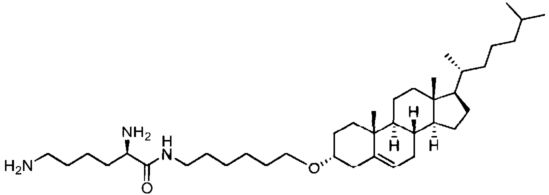 |
| SCL-4 | 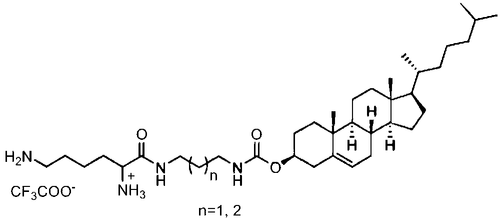 |
| SCL-5 | 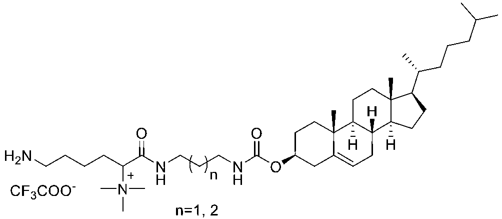 |
| SCL-6 | 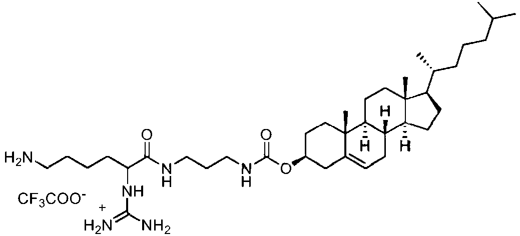 |

12. The lipid nanoparticle according to any one of embodiments 5-11, wherein the steroidal cationic lipid is a cationic lipid containing a quaternary ammonium structure or a cationic lipid with pKa greater than 8, preferably a cationic lipid with pKa greater than 10, more preferably a cationic lipid with pKa greater than 12.
13. The lipid nanoparticle according to any one of embodiments 3-4, wherein the ionizable lipid comprising a primary group and two biodegradable hydrophobic tails, wherein
(a) the primary group includes a head group and a central moiety to which both the biodegradable hydrophobic tails and the head group are directly bonded, wherein the primary group has a protonatable group having a pK a of from about 4 to about 11,
(b) the ionizable lipid has a logP value of at least 10.1, and
(c) each biodegradable hydrophobic tail has the formula -(hydrophobic chain I)-(biodegradable group)-(hydrophobic chain II),
wherein in at least one biodegradable hydrophobic tail,
(i) the biodegradable group is separated from a terminus of the hydrophobic tail by from 6 to 12 carbon atoms;
(ii) the at least one biodegradable hydrophobic tail has the formula -R₁₆ -M₀ -R₁₇, wherein R₁₆ is a C₄₋₁₄ alkylene, C₄₋₁₄ alkenylene or C₄₋₁₄ alknylene, M₀ is the biodegradable group, and R₁₇ is a linear or branched C₁₀₋₂₀ alkyl; and
(iii) the total carbon atom content of the tail -R₁₆-M₀-R₁₇ is 15 to 26.

14. The lipid nanoparticle embodiment 13, wherein the ionizable lipid is a compound of formula (III), or a stereoisomer, tautomer, isotopologue, or a pharmaceutically acceptable salt thereof, 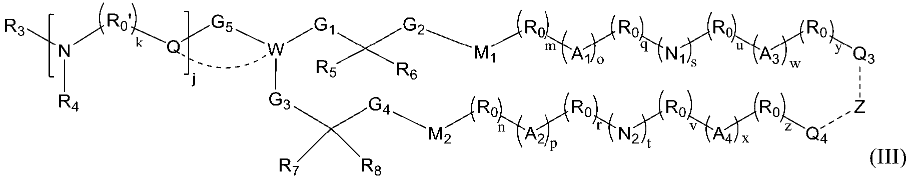 wherein
each instance of R₃ and R₄ are independently C₁₋₁₀ alkyl, C₂₋₁₀ alkenyl, C₂₋₁₀ alkynyl, C₃-C₁₄ cycloalkyl, 3 to 14-membered heterocyclyl, each optionally substituted with one or more R*;
or R₃ and R₄ together with the nitrogen atom they are attached to form a 3 to 14-membered heterocyclyl, which is optionally substituted with one or more R*;
or R₄ and one of the R₀' together with the atoms connecting them form a 4 to 10-membered heterocyclyl or 5 to 10-membered heteroaryl, each optionally substituted with one or more R*;
R* is independently H, halogen, cyano, C₁₋₁₀ alkyl, C₁₋₁₀ haloalkyl, -L_{b}-OR_{b}, -L_{b}-SR_{b}, or -L_{b}-NR_{b}R'_{b};
R₀' is independently a methylene which is optionally substituted by one or two R**, or two substituents of R₀' together with the carb they are attached to form a 3- to 8-membered cycloalkylene;
R** is independently selected from H, C₁₋₈ alkyl, -L_{c}-OR_{c}, -L_{c}SR_{c}, or -L_{c}-NR_{c}R'_{c};
k is 0, 1, 2, 3, 4, 5, or 6;
j is 0 or 1;
the dashed line connecting Q and W is absent or a bond;
W is C, CH, or N, provided that when W is N, j is 0 and the dashed bond connecting Q and W is absent;
G₅ is a bond, C₁₋₂₄ alkylene, C₂₋₂₄ alkenylene, 3- to 8-membered cycloalkylene, C₃-C₈ cycloalkenylene, preferred a bond or C₁₋₈ alkylene, wherein the alkylene, alkenylene, cycloalkylene, cycloalkenylene are optionally substituted with one or more R**;
G₁, G₂, G₃ and G₄ are each independently a bond, C₁₋₁₃ alkylene, C₂₋₁₃ alkenylene, or C₂₋₁₃ alkynylene, wherein the alkylene, alkenylene, and alkynylene are optionally substituted by one or more R^{s};
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R^{s} is independently H, C₁₋₁₄ alkyl, -L_{d}-OR_{d}, -L_{d}-SR_{d}, or -L_{d}-NR_{d}R'_{d};
R₅, R₆, R₇ and R₈ are each independently hydrogen or C₁₋₈ alky, which is optionally substituted by one or more R*;
when the dashed line connecting Q and W is absent, Q is absent or is selected from -C(O)O-, -O-, - NH-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, - C(O)S-, -NR_{b}-, -C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, - NR_{b}C(S)O-, -S-S- or -S(O)₀₋₂-;
when the dashed line connecting Q and W is a chemical bond, G5 is absent, Q and W form a 5- to 10-membered monocyclic or bicyclic ring, which are optionally being substituted with one or more R** groups.
M₁ and M₂ are each independently absent, or selected from -C(O)O-, -O-, -SC(O)O-, -OC(O)NRₐ-, - NRₐC(O)NRₐ-, -OC(O)S-, -OC(O)O-, -NRₐC(O)O-, -OC(O)-, -SC(O)-, -C(O)S-, -NRₐ-, -C(O)NRₐ-, - NRₐC(O)-, -NRₐC(O)S-, -SC(O)NRₐ-, -C(O)-, -OC(S)-, -C(S)O-, -OC(S)NRₐ-, -NRₐC(S)O-, -S-S- or -S(O)₀₋₂₋;
R₀ is independently -(CRR')-;
each instance of R and R' are independently H, C₁₋₂₀ alkyl, -Lₐ-ORₐ, -Lₐ-SRₐ, or -Lₐ-NRₐR'ₐ, preferably R' is H; or R and R' together with the carbon they are attached to form a C₃₋₈ cycloalkylene;
no more than three R₀ or R₀ 'in each chain connected to W are cycloalkylene;
Q₃ and Q₄ are each independently H, -(CRR')-, C₆₋₁₀ aryl, or a steroid moiety, preferably H, or - (CRR')-;
each instance of A₁, A₂, A₃, A₄ are independently -(CR₁₈R₁₈-CR₁₈=CR₁₈)- or -(CR₁₈R₁₈-C≡C)-;
each instance of R₁₈ is independently H or C₁₋₂₀ alkyl;
each instance of N₁ and N₂ are independently a biodegradable group;
Z is absent, C₁₋₁₀ alkylene, or -O-P(O)(OH)-O-;
the dash line connected to Z is absent, or a chemical bond, when Z is absent, Q₃ and Q₄ are not directly connected;
m, n, q, r, u, v, y, and z are each independently 0, 1, 2, 3, 4, 5, 6, 7, 8, 9 or 10;
o, p, w, and x are each independently 0, 1, or 2;
s and t are each independently 0 or 1;
the number of atoms between G₁ and Q₃ or between G₃ and Q₄ is from 8 to 30, preferably from 10 to 25;
Lₐ and Lₑ are each independently a bond or C₁₋₂₀ alkylene;
L_{b} and L_{f} are each independently a bond or C₁₋₁₀ alkylene;
L_{c} is a bond or C₁₋₈ alkylene;
L_{d} is a bond or C₁₋₁₄ alkylene;
Rₐ and R'ₐ are each independently H, C₁₋₂₀ alkyl, C₃₋₁₄ cycloalkyl, or 3- to 14-membered heterocyclyl, wherein the alkyl, cycloalkyl and heterocyclyl are optionally substituted with one or more groups selected from H, C₁₋₂₀ alkyl, -Lₑ-ORₑ, -Lₑ-SRₑ, or -Lₑ-NRₑR'ₑ;
R_{b} and R'_{b} are each independently H, C₁₋₁₀ alkyl, C₃₋₁₄ cycloalkyl, or 3 to 14-membered heterocyclyl, wherein the alkyl, cycloalkyl and heterocyclyl are optionally substituted with one or more groups selected from H, C₁₋₁₀ alkyl, -L_{f}-OR_{f}, -L_{f}-SR_{f}, or -Lᵣ-NR_{f}R'_{f};
R_{c} and R'_{c} are each independently hydrogen or C₁₋₈ alkyl;
R_{d} and R'_{d} are each independently hydrogen or C₁₋₁₄ alkyl;
Rₑ and R'ₑ are each independently hydrogen or C₁₋₂₀ alkyl; and
R_{f} and R'_{f} are each independently hydrogen or C₁₋₁₀ alkyl.

15. The lipid nanoparticle embodiment 14, wherein the ionizable lipid is a compound of formula (VI') or (VII'), or a pharmaceutically acceptable salt, isotopic variants, tautomers, or stereoisomers thereof, 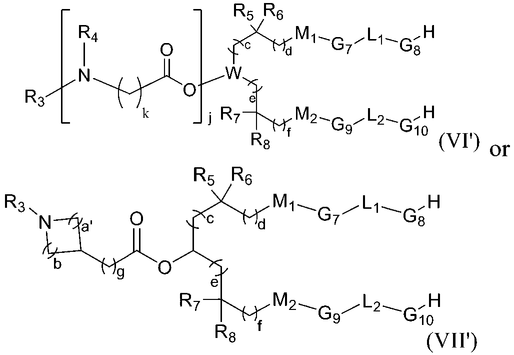 wherein
k is 0, 1, 2, 3, 4 or 5, preferably 1, 2, 3, 4 or 5, preferably 3, 4 or 5, preferably 3 or 4;
a' and b are independently 0, 1, 2, 3, 4 or 5, preferably 0, 1, 2, 3 or 4, preferably 2, provided that, a' and b are 0 at the same time;
g is 0, 1, 2, 3, 4 or 5, preferably 0, 1 or 2, preferably 0 or 1;
a'+g is 0, 1, 2, 3, 4 or 5, preferably a'+g is 2, 3 or 4, preferably a'+g is 2or 3;
the methylene in the 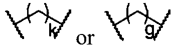 is optionally substituted by 1, 2, 3, 4 or 5 C₁₋₆ alkyl;
W is CH or N;
   (i) when W is N, in the ionizable lipid of formula (VI'):
      j is 0;
      c, d, e, f are independently 0, 1, 2, 3, 4, 5, 6 or 7;
      c+d=4, 5, 6 or 7, preferably c+d=5 or 6; e+f=4, 5, 6 or 7, preferably e+f=5 or 6;
      R₃ is C₁₋₈ alkyl or -C₁₋₈ alkylene-OH, preferably C₁₋₆ alkyl or -C₁₋₆ alkylene-OH, preferably -C₁₋₆ alkylene -OH;
      R₅, R₆, R₇ and R₈ are independently H or C₁₋₆ alkyl;
      M₁ and M₂ are independently -C(O)O-, -OC(O)-, -SC(O)- or -C(O)S-, preferably -C(O)O- or -OC(O)-;
      L₁ and L₂ are independently -(CHR)₂-, -CH=CH- or -C≡C-, preferably -(CHR)₂-;
      G₇ and G₉ are independently C₁₋₄ alkylene;
      G₈ and G₁₀ are independently C₂₋₈ alkylene;
      G₇ and G₈ have a total length of 6, 7, 8 or 9 carbon atoms, preferably 7, 8 or 9 carbon atoms;
      G₉ and G₁₀ have a total length of 6, 7, 8 or 9 carbon atoms, preferably 7, 8 or 9 carbon atoms;
      1, 2, 3 or 4 methylene in G₇, G₈, G₉ or G₁₀ are optionally and independently substituted by one R; R is independently H or C₁₋₁₀ alkyl, preferably H or C₁₋₈ alkyl;
   (ii) in the ionizable lipid of formula (VII'), and when W is CH, in the ionizable lipid of formula (VI'):
      j is 1;
      c, d, e, f are independently 0, 1, 2, 3, 4, 5 or 6;
      c+d=4, 5 or 6, e+f=4, 5 or 6;
      R₃ and R₄ are independently C₁₋₆ alkyl, wherein the alkyl is optionally substituted by 1, 2 or 3 R*;
      R* is independently H, C₁₋₆ alkyl or C₁₋₆ haloalkyl;
      R₅, R₆, R₇ and R₈ are independently H or C₁₋₆ alkyl, preferably C₁₋₆ alkyl;
      M₁ and M₂ are independently -C(O)O-, -OC(O)-, -SC(O)- or -C(O)S-, preferably -C(O)O- or -C(O)S-; L₁ and L₂ are independently -(CHR)₂-, -CH=CH- or -C≡C-;
      G₇ and G₉ are independently C₁₋₄ alkylene;
      G₈ and G₁₀ are independently C₂₋₇ alkylene;
      G₇ and G₈ have a total length of 6, 7 or 8 carbon atoms;
      G₉ and G₁₀ have a total length of 6, 7 or 8 carbon atoms;
      1, 2 or 3 methylene in G₇, G₈, G₉ or G₁₀ are optionally and independently substituted by one R;
      R is independently H or C₁₋₁₀ alkyl; preferably H or C₁₋₇ alkyl;
      provided that, when L₁ is -C≡C-, G₇ is C₁₋₂ alkylene, when L₂ is -C≡C-, G₉ is C₁₋₂ alkylene;
      preferably, in the formula (VII'), L₁ and L₂ are -(CHR)₂-.

16. The lipid nanoparticle embodiment 14, wherein the ionizable lipid of formula (III) is: 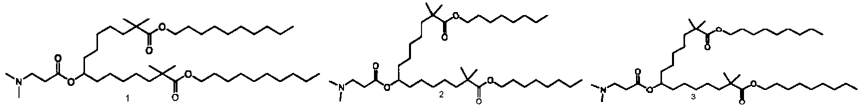 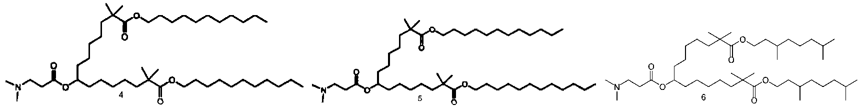 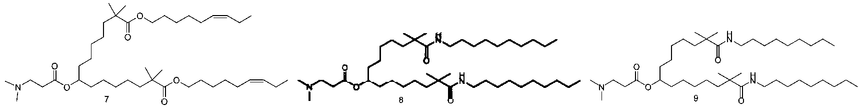 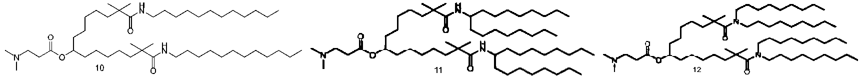 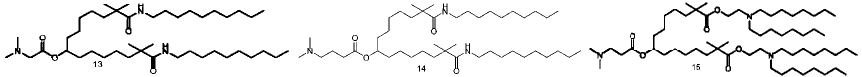 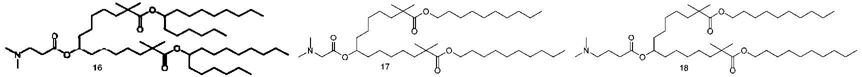 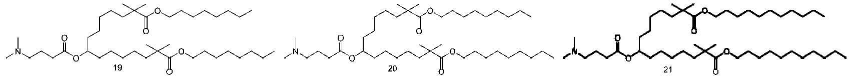 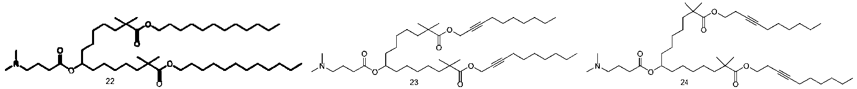 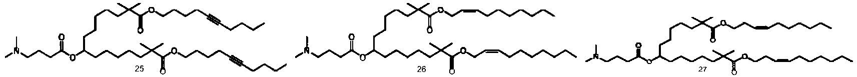 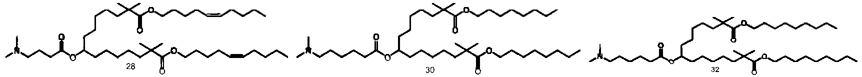 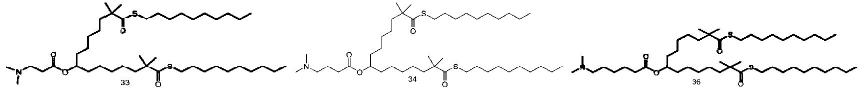 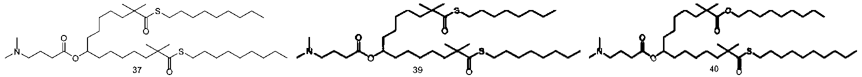 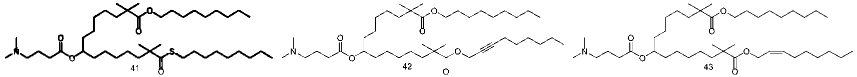 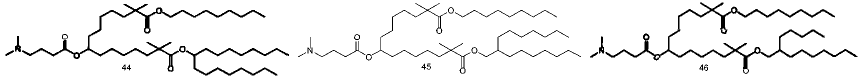 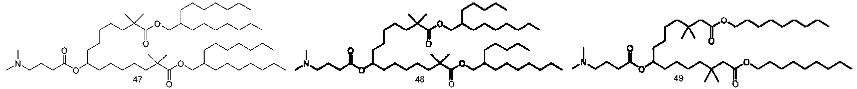 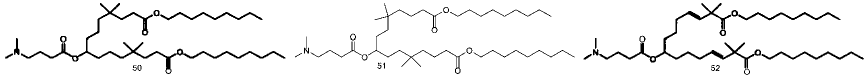 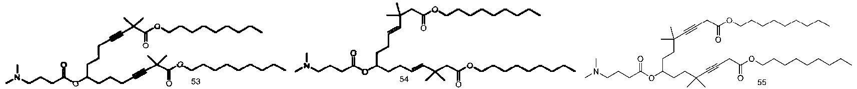 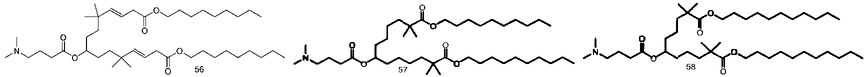 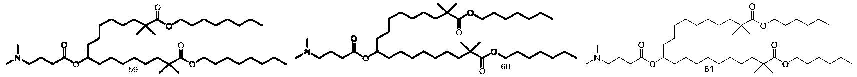 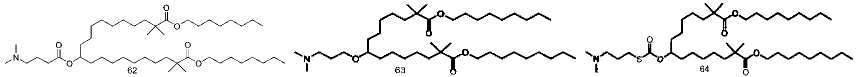 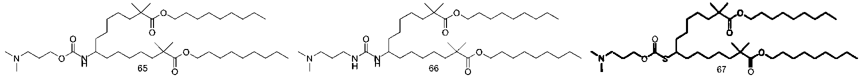 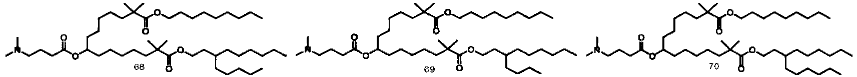 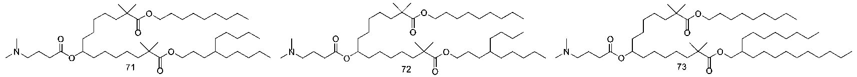 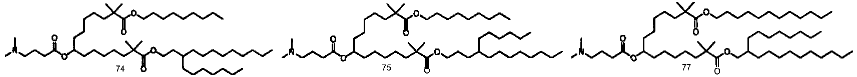 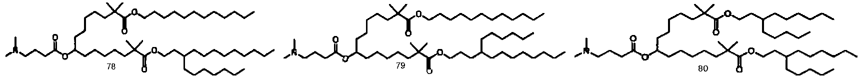 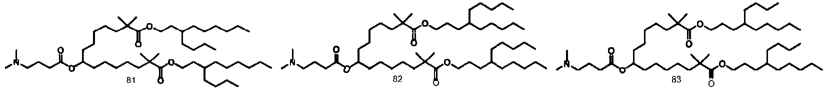 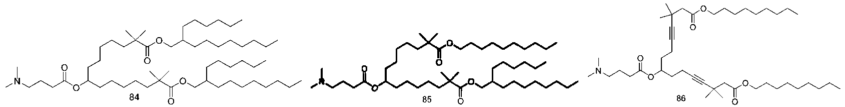 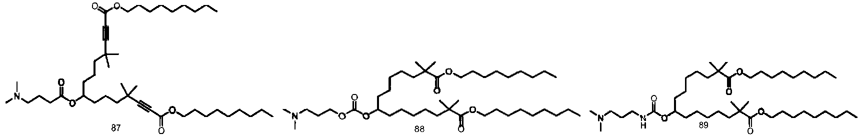 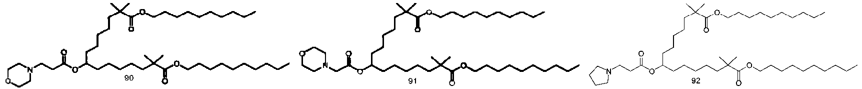 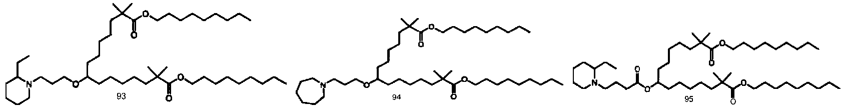 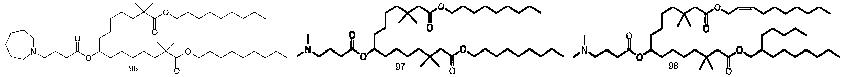 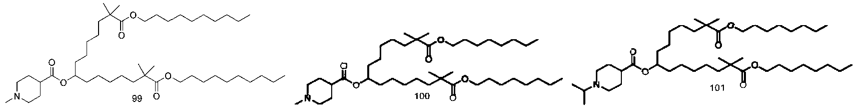 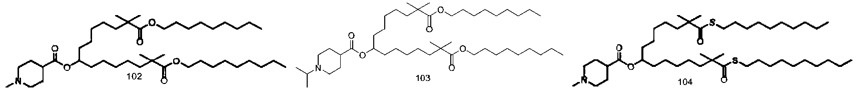 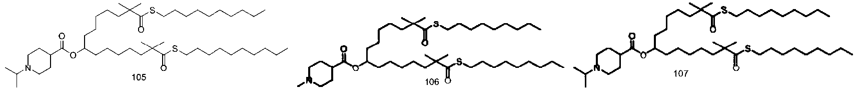 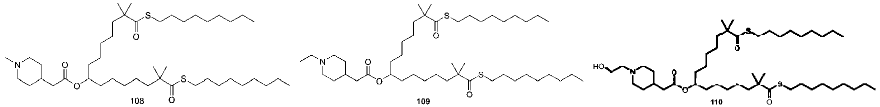 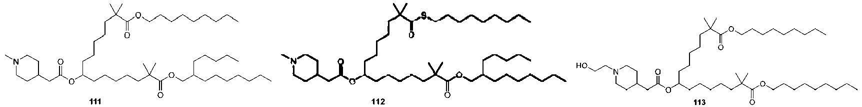 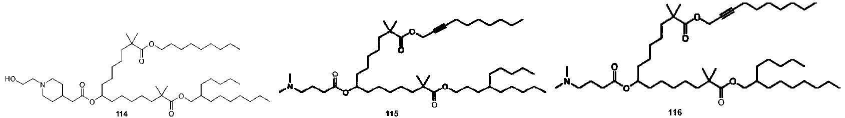 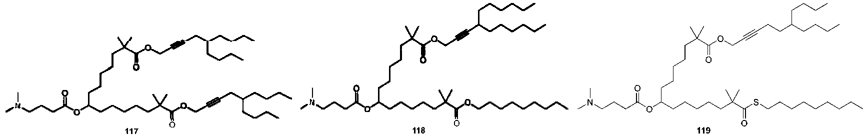 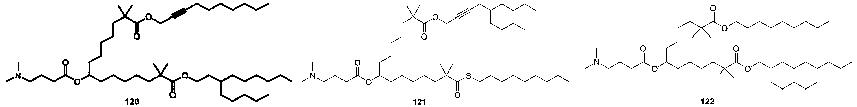 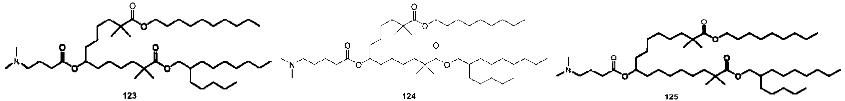 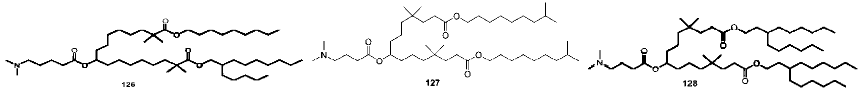 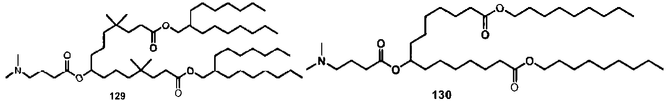 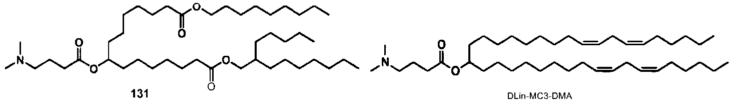 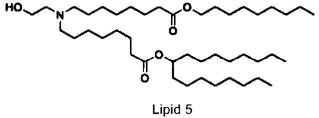 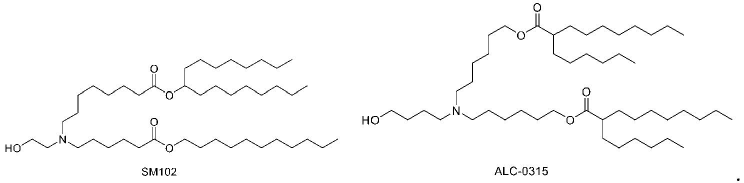
17. The lipid nanoparticle according to any one of embodiments 3-4, wherein the ionizable lipid is selected from one or more of 1,2-dioleyloxy-3-dimethylaminopropane (DODAP), 1,2-dioleyloxy-3-dimethylaminopropane (DODMA), 1,2-dilinoleyloxy-3-dimethylaminopropane (DLinDMA), 2,2-dilinoleyl-4-(2-dimethylaminoethyl)-[1,3]-dioxolane (DLin-KC2-DMA), 4-(N,N-dimethylamino)butanoic acid (dilinoleyl)methyl ester (DLin-MC3-DMA), 8-[(2-hydroxyethyl)(6-oxo-6-decyloxyhexyl)amino]octanoic acid (heptadecyl-9-yl) ester (SM-102) and [(4-hydroxybutyl)azanediyl]bis(hexane-6,1-diyl) bis(2-hexyldecanoate) (ALC-0315); preferably selected from 4-(N,N-dimethylamino)butanoic acid (dilinoleyl)methyl ester (DLin-MC3-DMA) and/or [(4-hydroxybutyl)azanediyl]bis(hexane-6,1-diyl) bis(2-hexyldecanoate) (ALC-0315).
18. The lipid nanoparticle according to any one of embodiments 3-4, wherein the structural lipid is selected from the group consisting of cholesterol, campesterol, stigmasterol, sitosterol, brassicasterol, ergosterol, solanine, ursolic acid, alpha-tocopherol, beta-sitosterol, avenasterol, ergocalciferol, and canola sterol, preferably cholesterol and/or beta-sitosterol, preferably cholesterol.
19. The lipid nanoparticle according to any one of embodiments 3-4, wherein the polymer conjugated lipid is a pegylated lipid.
20. The lipid nanoparticle according to embodiment 19, wherein the pegylated lipid are selected from one or more of the following: PEG modified phosphatidylethanolamine, PEG modified phosphatidic acid, PEG modified ceramide, PEG modified dialkylamine, PEG modified diacylglycerol, and PEG modified dialkylglycerol;
preferably, the pegylated lipid comprises a pegylated moiety having a molecule weight of from about 1000 Da to about 20kDa, or from about 1000 Da to about 5000 Da;
preferably, the polymer conjugated lipid is selected from one or more of the following: DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, DSPE-PEG5000, DSPE-PEG2000 amine and ALC-0159, preferably DMG-PEG2000 and/or ALC-0159.

21. A lipid nanoparticle composition, comprising the lipid nanoparticle of any one of embodiments 1-20 and a payload.
22. The lipid nanoparticle composition according to embodiment 21, wherein the payload is a therapeutic agent, a prophylactic agent, or a diagnostic agent;
preferably, the therapeutic agent, prophylactic agent, or diagnostic agent is nucleic acid;
preferably, the nucleic acid is antisense oligonucleotide (ASO), DNA, or RNA;
preferably wherein the RNA is selected from one or more of RNA interference (RNAi), small interfering RNA (siRNA), short hairpin RNA (shRNA), antisense RNA (aRNA), messenger RNA (mRNA), modified messenger RNA (mmRNA), long noncoding RNA (lncRNA), microRNA (miRNA), small activating RNA (saRNA), multicoding nucleic acid (MCNA), polymer-coded nucleic acid (PCNA), guide RNA (gRNA), CRISPR RNA (crRNA), and ribozyme; preferably mRNA; more preferably modified messenger RNA (mmRNA).

23. A process of preparing lipid nanoparticles comprising the steroid compound of any one of embodiments 1-20, comprising the steps of: mix the lipid components and then mix with the payload;
preferably, including mixing solutions containing lipid components with solutions containing payload;
preferably, the mixing is performed using a microfluidizer system or impacting jet to mix solutions containing various lipid components with solutions containing loads.

24. A pharmaceutical composition comprising the lipid nanoparticle composition according to any one of embodiments 21-22 and pharmaceutically acceptable excipients.
25. Use of the lipid nanoparticle composition according to any one of embodiments 21-22 or the pharmaceutical composition according to embodiment 24 in manufacture of a medicament for the diagnosis, the treatment or the prevention of a disease.
26. A method for the diagnosis, the treatment or the prevention of a disease in a subject, comprising the administration of the lipid nanoparticle composition according to any one of embodiments 21-22 or the pharmaceutical composition according to embodiment 24 to the subject.
27. The method for the diagnosis, the treatment or the prevention of a disease according to embodiment 26, wherein the lipid nanoparticle composition or the pharmaceutical composition is administered via systemic administration route, more preferably, by intravenous injection.
28. A method for delivering a payload to a subject, comprising administering to the subject the lipid nanoparticle composition according to any one of embodiments 21-22 or the pharmaceutical composition according to embodiment 24.
29. The method according to embodiment 28, wherein the payload is a therapeutic agent, a prophylactic agent, or a diagnostic agent.
30. The method according to embodiment 28, wherein the lipid nanoparticle composition or the pharmaceutical composition is administered via systemic administration route, more preferably by intravenous injection.

## Claims

1. A lipid nanoparticle comprising an ionizable lipid and a steroid compound, wherein the steroid compound is a compound of formula (I): 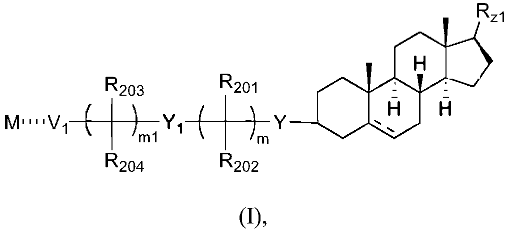 or a stereoisomer, a mixture of stereoisomers, or a pharmaceutically acceptable salt thereof, wherein:
- - - is a single bond or double bond;
Y is -C(O)O-, -O-, -NH-, -SC(O)O-, -OC(O)NR₂:-, -NR₂ₐC(O)NR₂ₐ-, -OC(O)S-, -OC(O)O-, -NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, -C(O)-, -OC(S)-, - C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, -L_{2b}-N⁺R₂₁R₂₂R₂₃, L_{2b}-OC(O)R₂₁, or L_{2b}-C(O)OR₂₁;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl;
R_{z1} is C₁₋₁₄ alkyl or C₁₋₁₄ alkenyl, wherein the alkyl and alkenyl are optionally substituted; and
provided that when Y is -NHC(O)-, at least one of the following conditions are met:
(i) m is an integer from 5 to 12; or
(ii) V₁ is not N(CH₃)₂.

2. A lipid nanoparticle comprising an ionizable lipid and a steroid compound, wherein the steroid compound is a compound of formula (I-P): 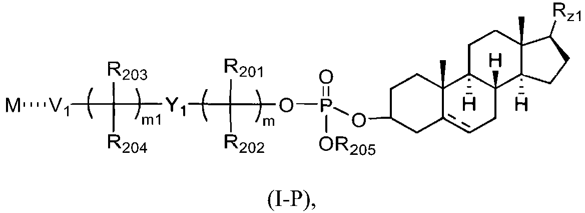 or a stereoisomer, a mixture of stereoisomers, or a pharmaceutically acceptable salt thereof, wherein:
- - - is a single bond or double bond;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, -S(O)₀₋₂-, or -OPO₃-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L₂₆-NR₂₁R₂₂, -L_{2b}-N*R₂₁R₂₂R₂₃, L_{2b}-OC(O)R₂₁, or L_{2b}-C(O)OR₂₁;
R₂₀₅ is absent, a cation, H, C₁₋₆ alkyl, C₁₋₆ haloalkyl, C₂₋₆ alkenyl, or C₂₋₆ alkynyl, and wherein the alkyl, the haloalkyl, the alkenyl, and the alkynyl are optionally substituted;
m is an integer from 1 to 12;
m1 is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole,
oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

3. The lipid nanoparticle of claim 1, wherein Y is -C(O)O-, -OC(O)-, -O-, -NHC(O)O-, -OC(O)NH-, or -OC(O)O-, and wherein the attachment to the left is to the direction of V₁.

4. The lipid nanoparticle of any one of claims 1 to 3, wherein Y₁ is absent, -C(O)O-, -OC(O)-, -O-, -S-, SO, S(O)₂, -O(CO)O-, -NHC(O)-, or -C(O)NH-, and wherein the attachment to the left is to the direction of V₁.

5. The lipid nanoparticle of claim 1, wherein the steroid compound is a compound of Formula (I-A1), (I-A2), (I-A3), (I-A4), or (I-A5): 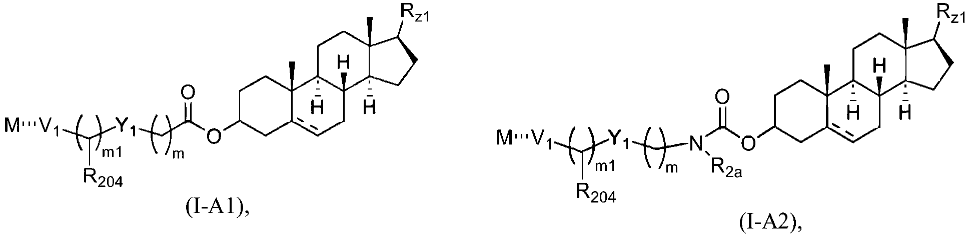 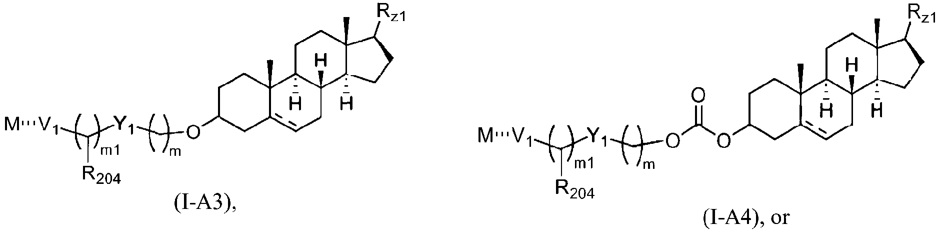 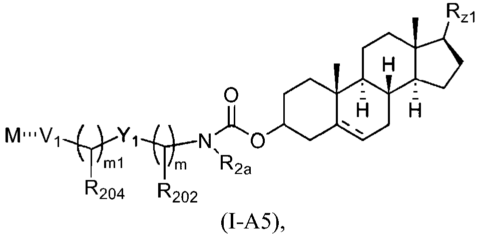 or a stereoisomer, a mixture of stereoisomers, or a pharmaceutically acceptable salt thereof.

6. The lipid nanoparticle of claim 5, wherein the steroid compound is a compound of Formula (I-B1), (I-B2), (I-B3), (I-B4), (I-B5), (I-B6), (I-B7), or (I-B8): 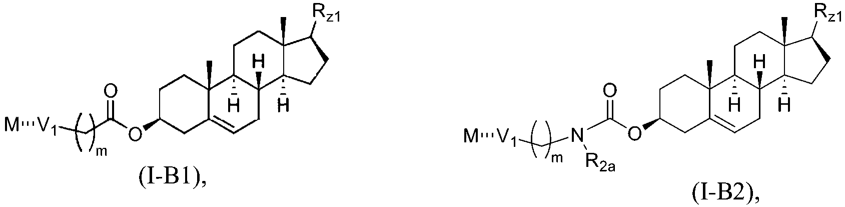 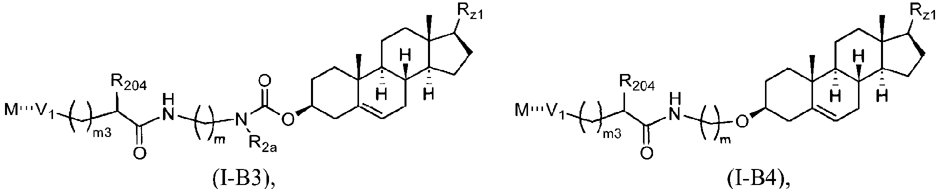 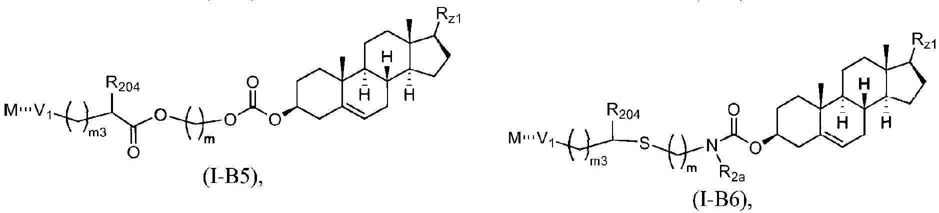 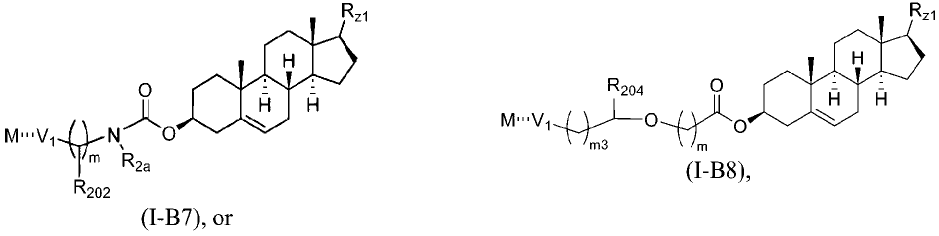 or a stereoisomer, a mixture of stereoisomers, or a pharmaceutically acceptable salt thereof, wherein m3 is an integer from 1 to 11.

7. The lipid nanoparticle of any one of claims 1 to 6, wherein each instance of R₂₀₁, R₂₀₂, R₂₀₃, and R₂₀₄ are independently H, C₁₋₆ alkyl, -NR₂₁R₂₂, -N⁺R₂₁R₂₂R₂₃, -(CH₂)₀₋₆-OC(O)R₂₁, or -(CH₂)₀₋₆-C(O)OR₂₁.

8. The lipid nanoparticle of claim 7, wherein each instance of R₂₀₁, R₂₀₂, R₂₀₃, and R₂₀₄ are independently H, NH₂, NH₃⁺, -CH₂OC(O)CH₃, -CH₂C(O)OCH₃, -OC(O)CH₃, or -C(O)OCH₃.

9. The lipid nanoparticle of any one of claims 1 to 8, wherein m is an integer from 5 to 12.

10. The lipid nanoparticle of claim 2, wherein the steroid compound is a compound of formula (I-P1): 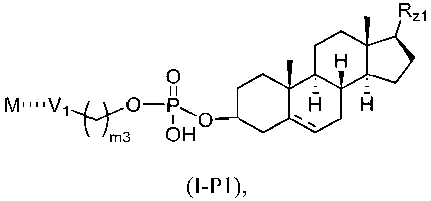 or a stereoisomer, a mixture of stereoisomers, or a pharmaceutically acceptable salt thereof, and wherein m3 is an integer from 1 to 11.

11. The lipid nanoparticle of any one of claims 1 to 10, wherein V₁ is NR₂₁R₂₂, N⁺R₂₁R₂₂R₂₃, or an optionally substituted radical of amidine, guanidine, or urea.

12. The lipid nanoparticle of any one of claims 1 to 11, wherein R₂₁, R₂₂, R₂₃ are each independently H, hydroxyl, C₁₋₃ alkyl, or C₁₋₃ hydroxyalkyl.

13. The lipid nanoparticle of any one of claims 1 to 12, wherein V₁ is NH₂, NH₃⁺, N(CH₃)₂, N⁺(CH₃)₃, N⁺CH₃(CH₂CH₂OH)₂, N⁺(CH₃)₂(CH₂CH₂OH), NHC(=N⁺H₂)NH₂, NHC(O)NH₂, CH₂C(=NH)NH₂, N(CH₃)(CH₂CH₂CO₂CH₃), N⁺(CH₃)₂(CH₂CH₂CO₂CH₃), N(CH₃)(CH₂CO₂CH₃), or N⁺(CH₃)₂(CH₂CO₂CH₃).

14. The lipid nanoparticle of claim 13, wherein V₁ is N⁺CH₃(CH₂CH₂OH)₂, N⁺(CH₃)₂(CH₂CH₂OH), or N⁺(CH₃)₃.

15. The lipid nanoparticle of any one of claims 1 to 14, wherein M is absent, or one or more of bromide, chloride, iodide, or trifluoroacetate.

16. The lipid nanoparticle of any one of claim 1 to 5, wherein R_{z1} is C₁₋₁₄ alkyl or C₁₋₁₄ alkenyl, wherein the alkyl and alkenyl are optionally substituted with one or more hydroxyl, oxo, nitro, cyano, halogen, C₁₋₆ alkoxy, C₂-C₆ alkenyl, C₂-C₆ alkynyl, C₃-C₈ cycloalkyl, C₆-C₁₀ aryl, 5 to 10-membered heteroaryl, or 3 to 8-membered heterocyclyl.

17. The lipid nanoparticle of claim 14, wherein R_{z1} is C₆₋₁₂ alkyl or C₆₋₁₂ alkenyl, and wherein the alkyl and alkenyl are optionally substituted with one or more hydroxyl, oxo, or halogen.

18. The lipid nanoparticle of claim 17, wherein R_{z1} is

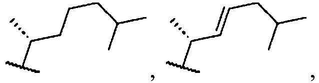

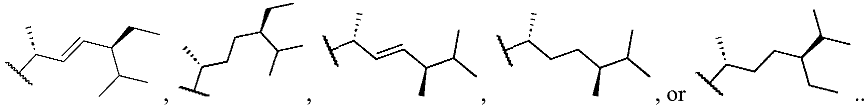

19. A lipid nanoparticle comprising an ionizable lipid and a steroid compound, wherein the steroid compound is: 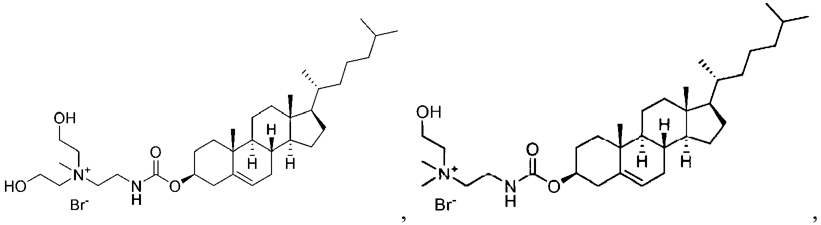 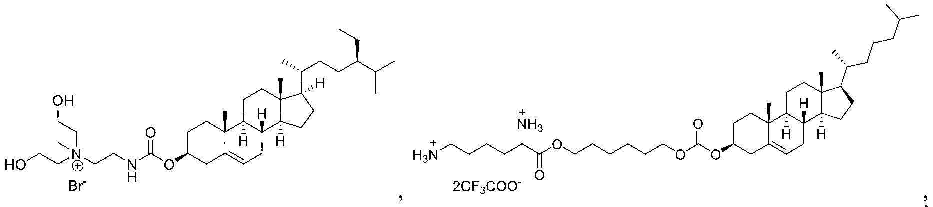 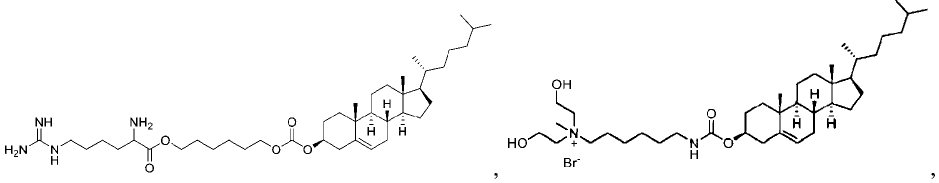 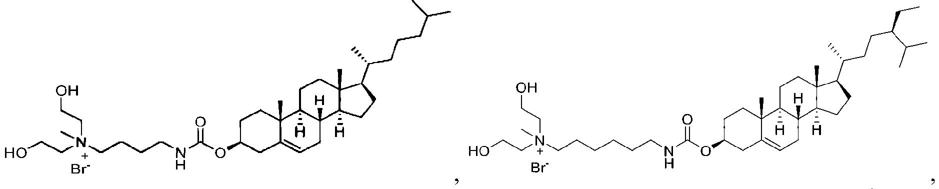 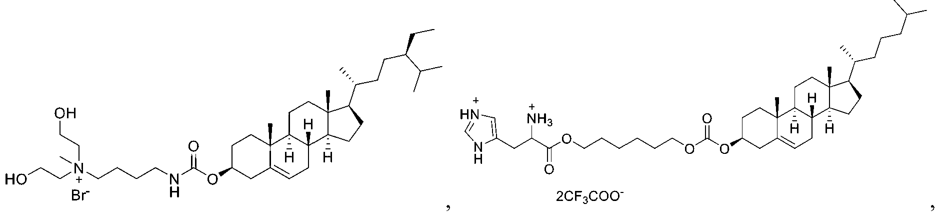 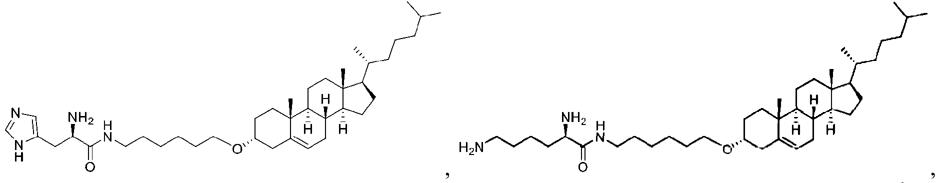 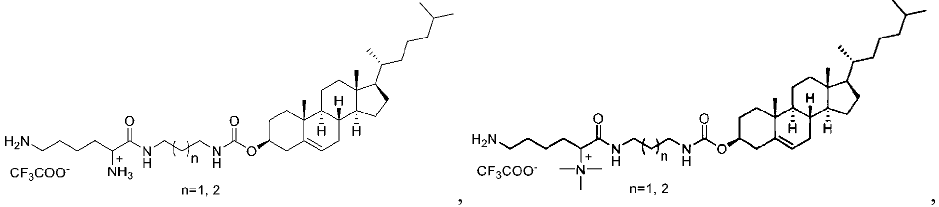 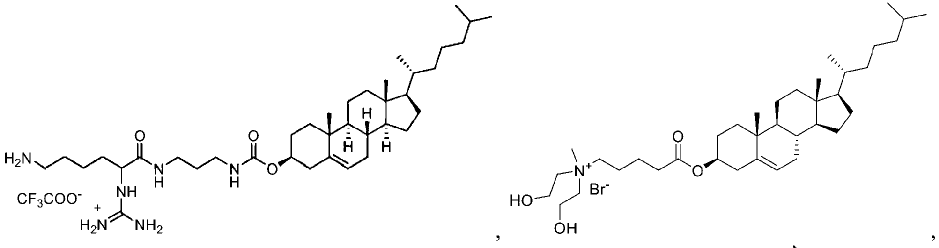 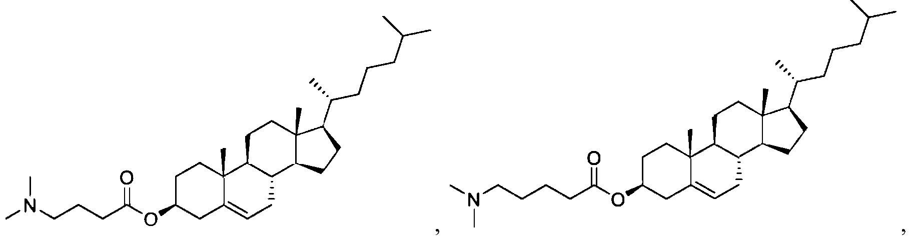 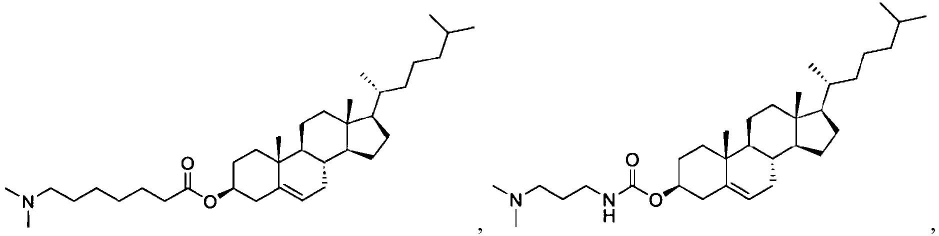 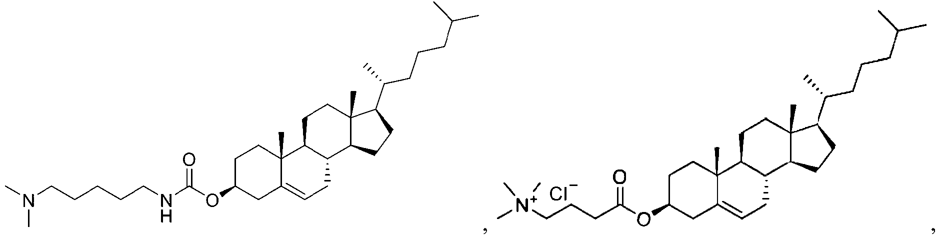 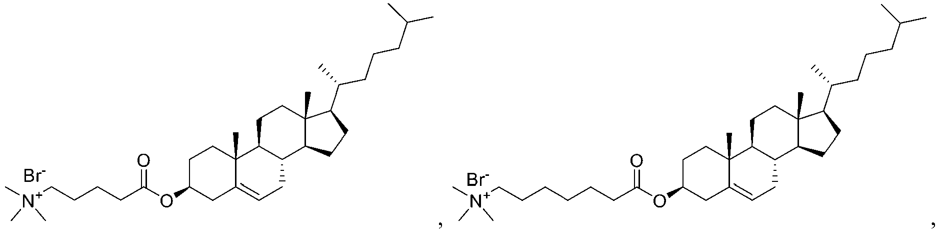 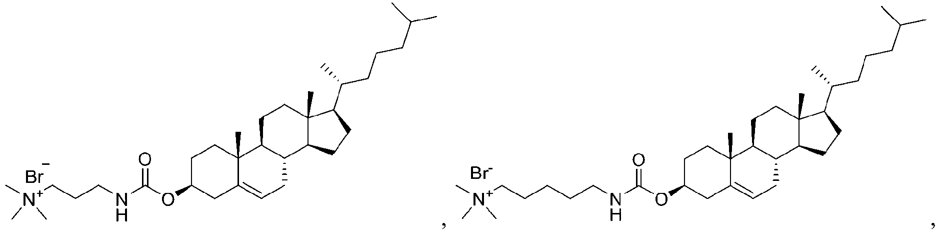 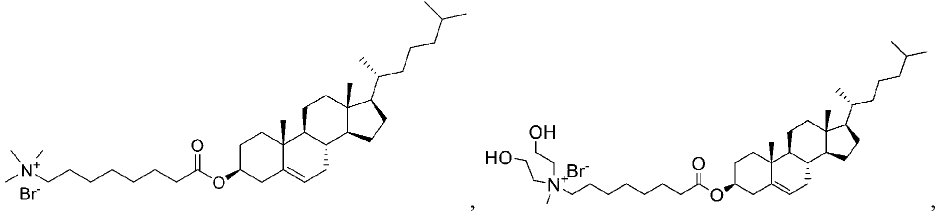 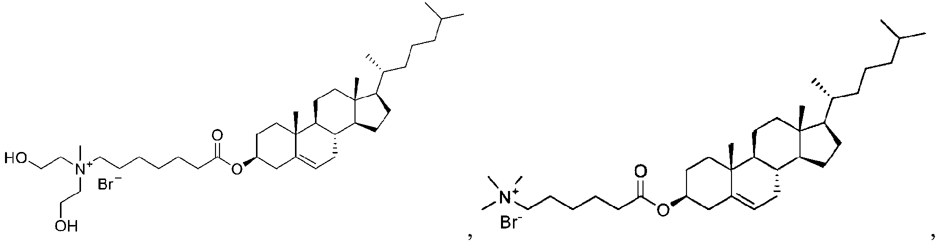 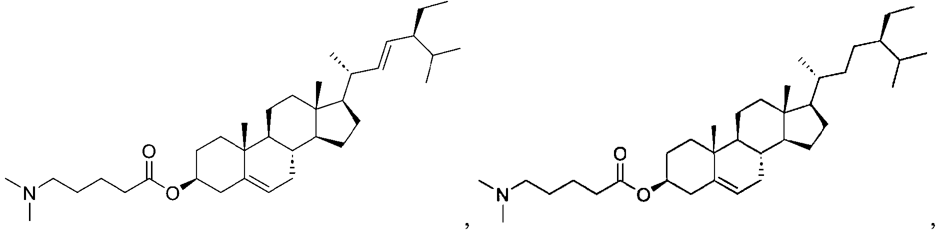 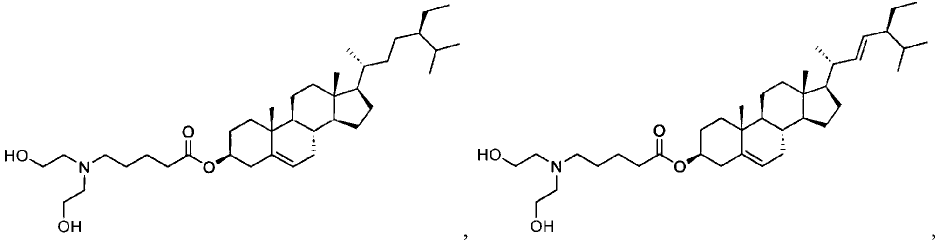 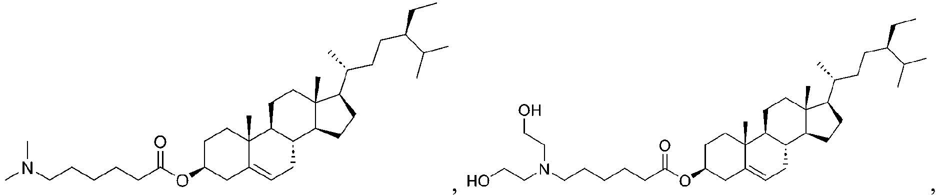 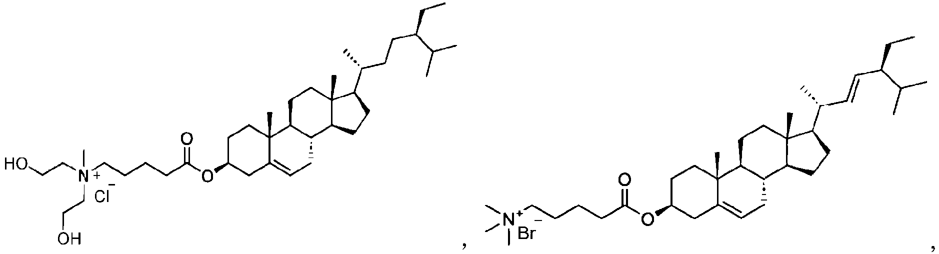 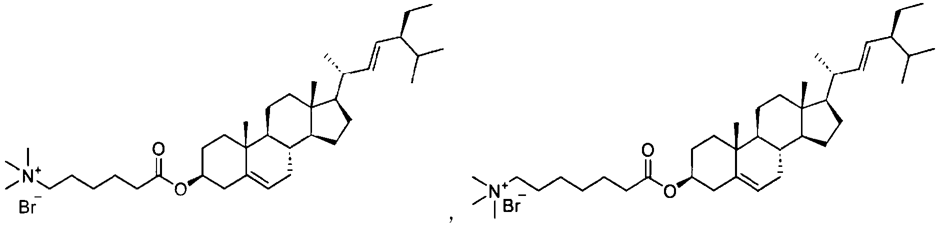 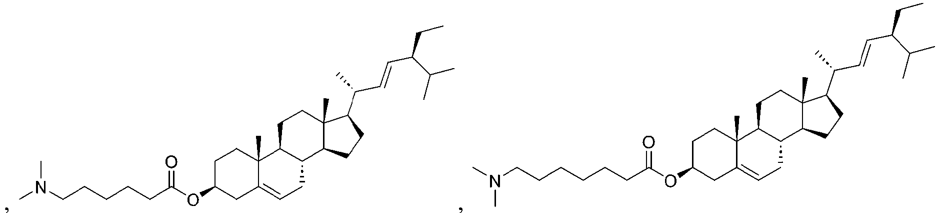 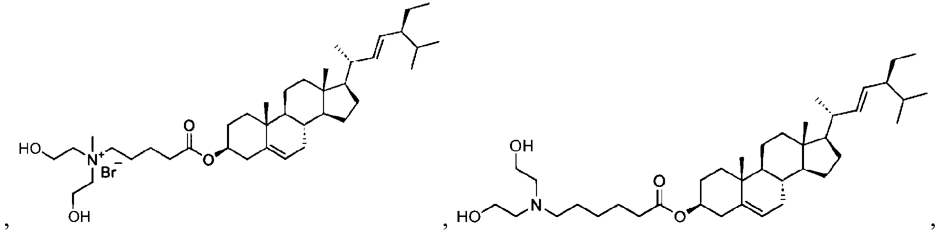 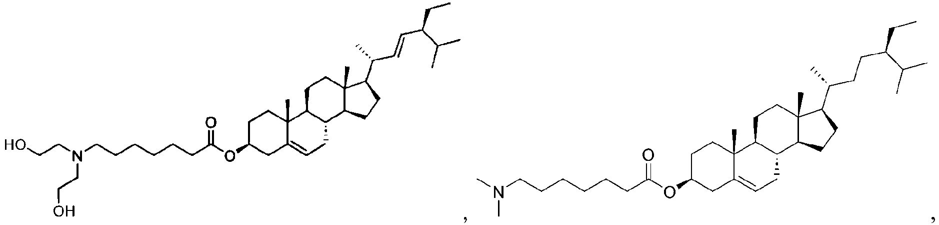 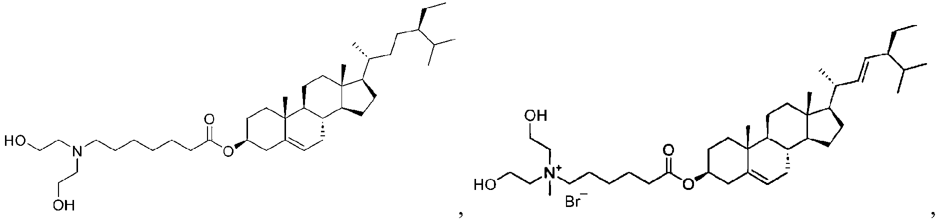 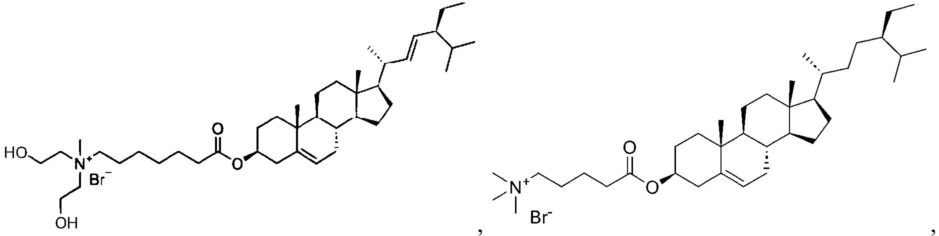 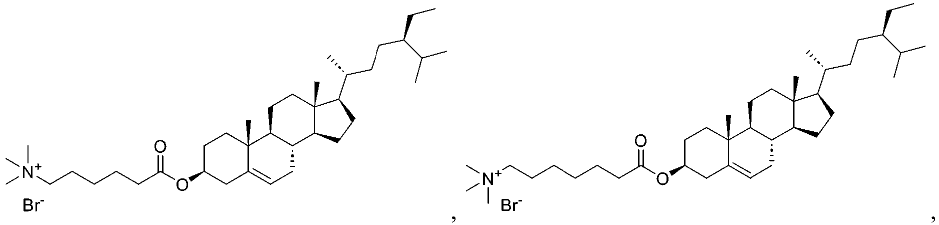 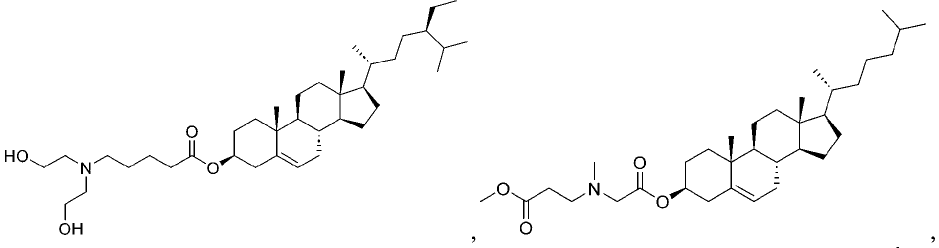 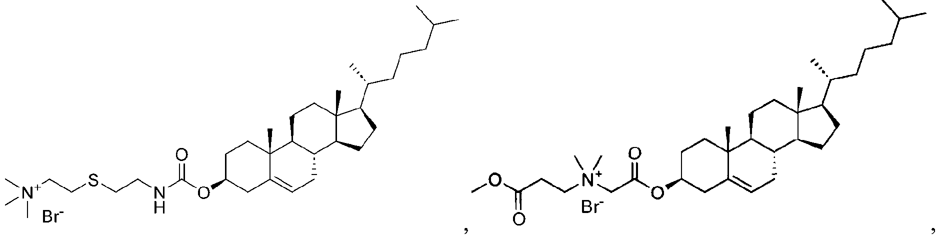 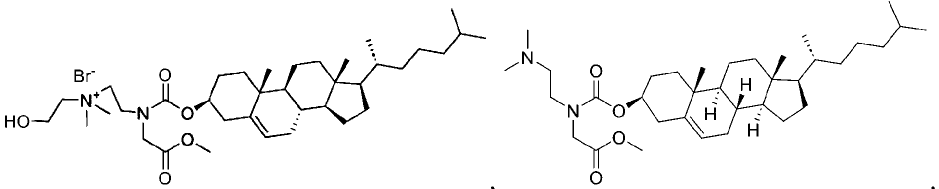 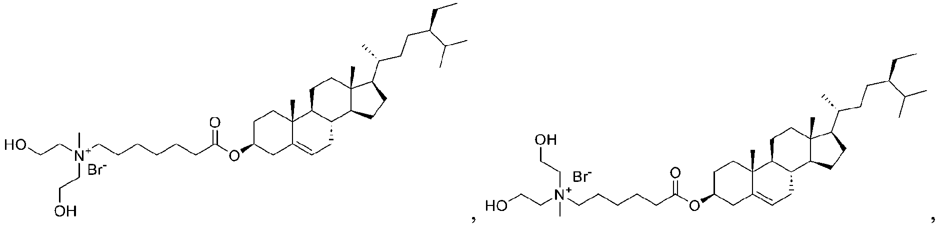 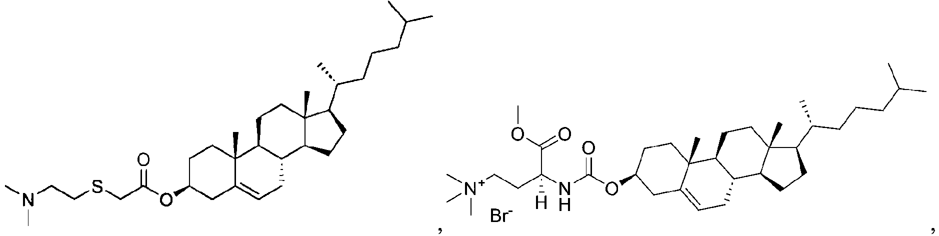 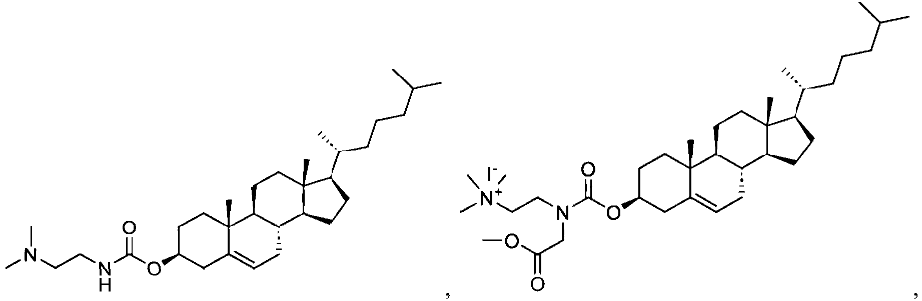 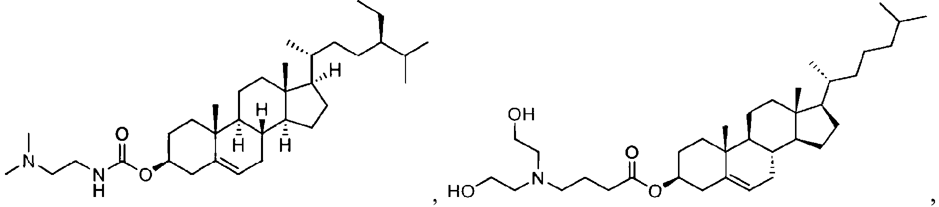 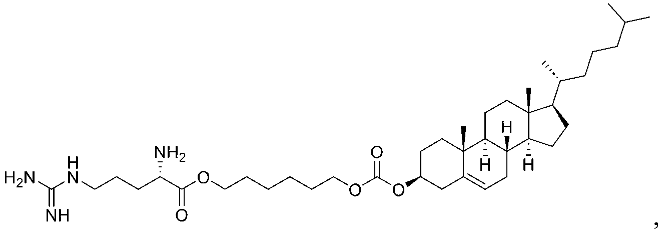 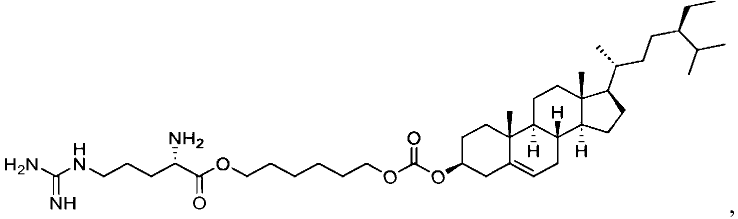 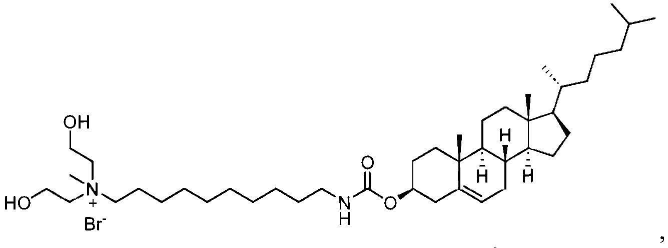 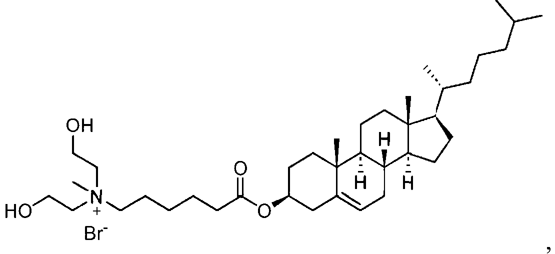 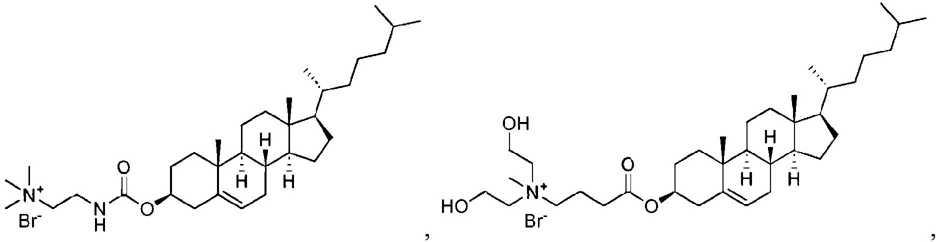 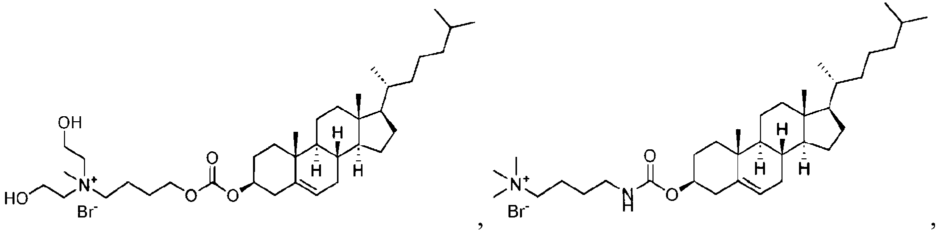 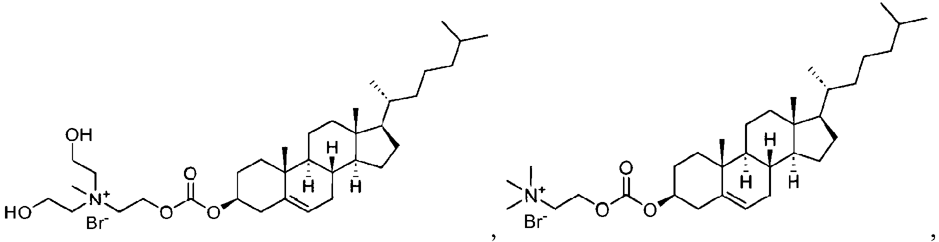 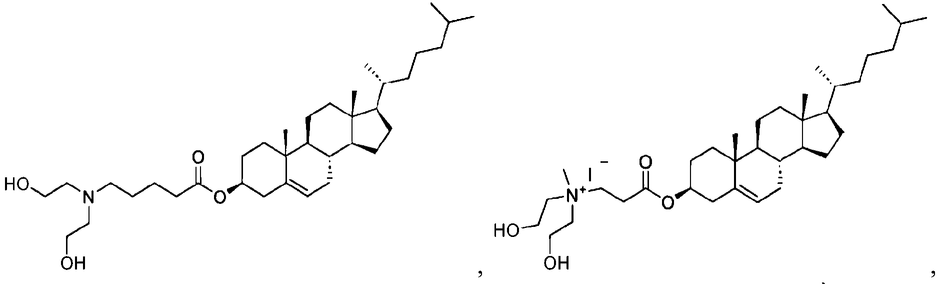 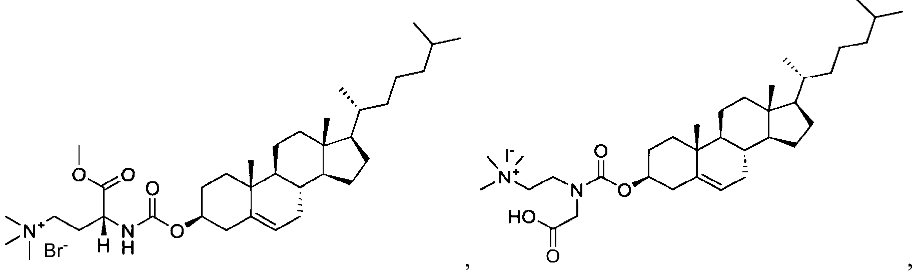 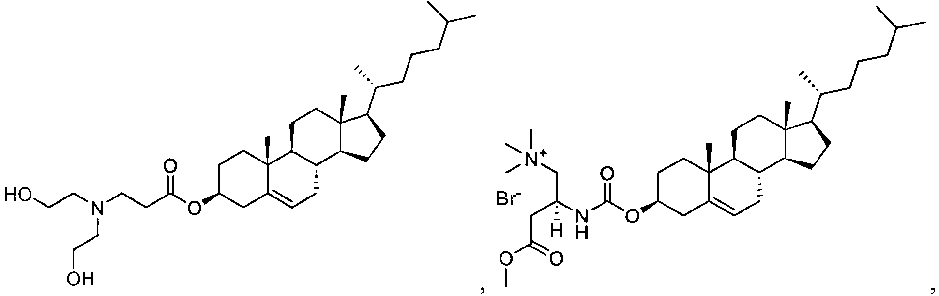 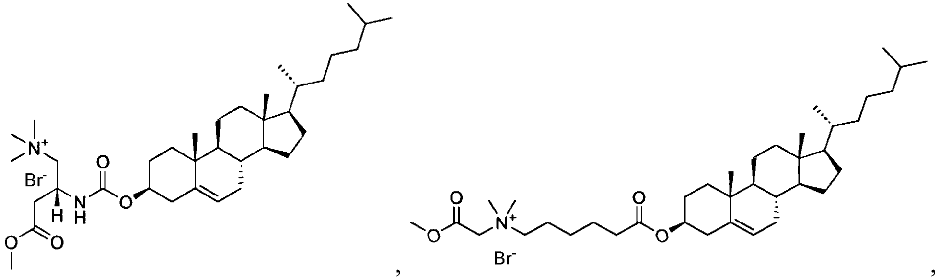 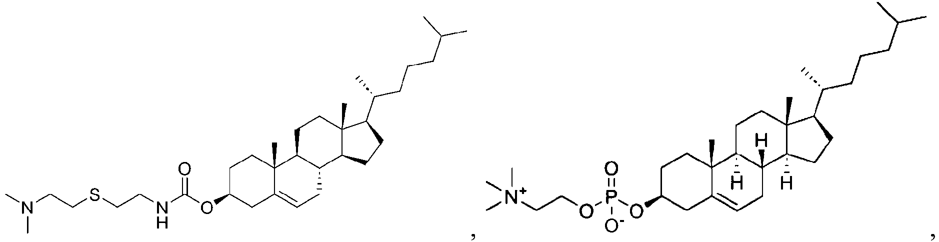 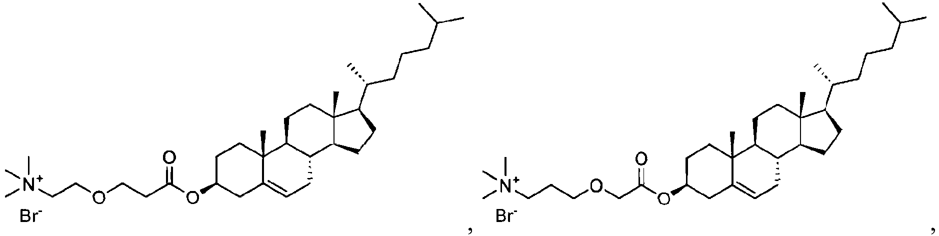 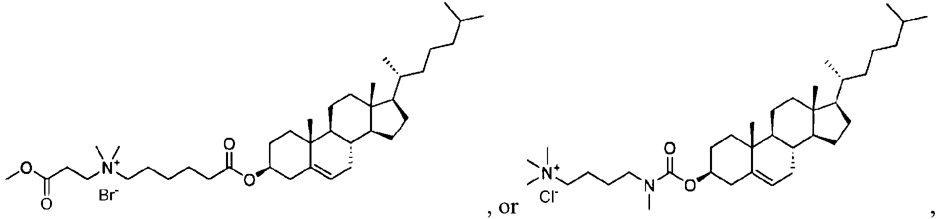 or a stereoisomer, a mixture of stereoisomers, or a pharmaceutically acceptable salt thereof.

20. The lipid nanoparticle of any one of claims 1 to 19, wherein the molar percentage of the steroid compound relative to the total lipid is from 3 mol % to 15 mol % , preferably from 5 mol % to 10 mol %, such as 3 mol %, 4 mol %, 5 mol %, 6 mol %, 7 mol %, 8 mol %, 9 mol %, 10 mol %, 11 mol %, 12 mol %, 13 mol %, 14 mol %, or 15 mol %.

21. The lipid nanoparticle of any one of claims 1 to 20, wherein the ionizable lipid comprises at least one ionizable headgroup and at least one biodegradable hydrophobic chain, wherein:
the ionizable lipid has a logP of at least about 10.1 and a pKa from about 4 to about 11; and
the biodegradable hydrophobic chain has a formula of -R₁₆-M₀-R₁₇; wherein
R₁₆ is a C₄₋₁₄ alkylene, C₄₋₁₄ alkenylene, or C₄₋₁₄ alkynylene; R₁₇ is a C₆-₂₀ alkyl; and M₀ is a biodegradable group, and wherein the alkylene, alkenylene, alkynylene and alkyl are optionally substituted;
preferably M₀ is an ester group, an amide group, a thioester group, a carbonate group, a carbamate group, a carbamothioester group, a urea group, or a disulfide group.

22. The lipid nanoparticle of any one of claims 1 to 21, wherein the ionizable lipid is a compound of formula (III): 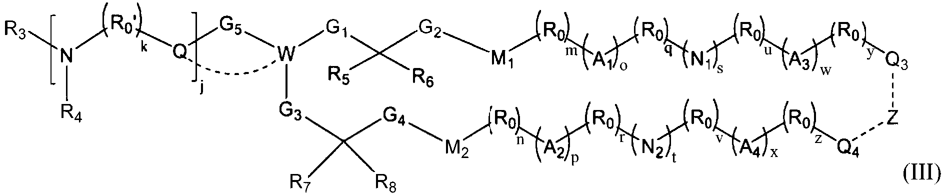 or a stereoisomer, a mixture of stereoisomers, tautomer, isotopologue, or a pharmaceutically acceptable salt thereof, wherein:
each instance of R₃ and R₄ are independently C₁₋₁₀ alkyl, C₂₋₁₀ alkenyl, C₂₋₁₀ alkynyl, C₃-C₁₄ cycloalkyl, 3 to 14-membered heterocyclyl, (C₁₋₁₀ alkylene)-(C₃-C₁₄ cycloalkyl), or (C₁₋₁₀ alkylene)-(3 to 14-membered heterocyclyl), or R₃ and R₄ together with the nitrogen atom they are attached to form a 3 to 14-membered heterocyclyl, or R₄ and one of the R₀' together with the atoms connecting them form a 4 to 10-membered heterocyclyl or 5 to 10-membered heteroaryl, and wherein the alkyl, alkenyl, alkynyl, cycloalkyl, heterocyclyl, heteroaryl, and alkylene are independently optionally substituted with one or more R*;
each instance of R₀' is an independently optionally substituted methylene, or wherein two R₀' together with the carbons they are attached to form a 3 to 8-membered cycloalkylene, and wherein the cycloalkylene is optionally substituted with one or more R**;
k is 0, 1, 2, 3, 4, 5, or 6;
j is 0 or 1;
the dashed line connecting Q and W is absent or a bond;
W is C, CH, or N, provided that when W is N, j is 0 and the dashed bond connecting Q and W is absent;
G₅ is absent, C₁₋₂₄ alkylene, C₂₋₂₄ alkenylene, C₃-C₈ cycloalkylene, C₃-C₈ cycloalkenylene, wherein the alkylene, alkenylene, cycloalkylene, cycloalkenylene are optionally substituted with one or more R**;
G₁, G₂, G₃ and G₄ are each independently absent, C₁₋₁₃ alkylene, C₂₋₁₃ alkenylene, or C₂₋₁₃ alkynylene, wherein the alkylene, alkenylene, and alkynylene are optionally substituted by one or more R^{s};
G₁ and G₂ have a total length of 3, 4, 5, 6, 7, 8, 9, 10, 11, 12 or 13 carbon atoms;
G₃ and G₄ have a total length of 3, 4, 5, 6, 7, 8, 9, 10, 11, 12 or 13 carbon atoms;
R₅, R₆, R₇ and R₈ are each independently hydrogen or optionally substituted C₁₋₈ alkyl;
when the dashed line connecting Q and W is absent, Q is absent or is -C(O)O-, -O-, -NH-, -SC(O)O-, - OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, - C(O)NR_{b}-, -NR_{b}C(O)-, -NR_{b}C(O)S-, -SC(O)NR_{b}-, -C(O)-, -OC(S)-, -C(S)O-, -OC(S)NR_{b}-, -NR_{b}C(S)O-, -S-S-or -S(O)₀₋₂-, wherein the attachment to the right is to the direction of W;
when the dashed line connecting Q and W is a bond, G₅ is absent, and Q and W form a 5 to 10-membered single ring or fused ring, optionally wherein the ring is substituted with one or more R**;
M₁ and M₂ are each independently absent, or are -C(O)O-, -O-, -SC(O)O-, -OC(O)NRₐ-, -NRₐC(O)NRₐ-, -OC(O)S-, -OC(O)O-, -NRₐC(O)O-, -OC(O)-, -SC(O)-, -C(O)S-, -NRₐ-, -C(O)NRₐ-, -NRₐC(O)-, -NRₐC(O)S-, -SC(O)NRₐ-, -C(O)-, -OC(S)-, -C(S)O-, -OC(S)NRₐ-, -NRₐC(S)O-, -S-S-or -S(O)₀₋₂-;
each instance of R₀ is independently -(CRR')-;
each instance of R and R' are independently H, C₁₋₂₀ alkyl, Lₐ-ORₐ, -Lₐ-SRₐ, or -Lₐ-NRₐR'ₐ, or R and R' together with the carbon they are attached to form a C₃₋₈ cycloalkylene;
Q₃ and Q₄ are each independently H, -(CRR')-, C₆₋₁₀ aryl, or a steroid moiety;
each instance of A₁, A₂, A₃, A₄ are independently -(CR₁₈R₁₈-CR₁₈=CR₁₈)- or -(CR₁₈R₁₈-C≡C)-;
each instance of R₁₈ is independently H or C₁₋₂₀ alkyl;
each instance of N₁ and N₂ are independently a biodegradable group;
Z is absent, C₁₋₁₀ alkylene, or -O-P(O)(OH)-O-;
each of the dashed lines between Z-Q₃ or Z-Q₄ is independently absent or a bond, provided that when Z is absent, both dashed lines are absent;
m, n, q, r, u, v, y, and z are each independently 0, 1, 2, 3, 4, 5, 6, 7, 8, 9 or 10;
o, p, w, and x are each independently 0, 1, or 2;
s and t are each independently 0 or 1;
the number of carbon atoms between G₁ and Q₃ or between G₃ and Q₄ is from 8 to 30;
Lₐ and L_{c} are each independently absent or C₁₋₂₀ alkylene;
L₈ and L_{f} are each independently absent or C₁₋₁₀ alkylene;
L_{c} is absent or C₁₋₈ alkylene;
L_{d} is absent or C₁₋₁₄ alkylene;
R* is hydroxyl, halogen, C₁₋₁₀ alkyl, C₁₋₁₀ haloalkyl, C₁₋₁₀ hydroxyalkyl, -L_{b}-OR_{b}, -L_{b}-SR_{b}, or -L_{b}-NR_{b}R'_{b};
R** is hydroxyl, halogen, C₁₋₈ alkyl, C₁₋₈ haloalkyl, C₁₋₈ hydroxyalkyl, -L_{b}-OR_{b}, -L_{b}-SR_{b}, or -L_{b}-NR_{b}R'_{b};
R^{s} is C₁₋₁₄ alkyl, -L_{b}-OR_{b}, -L_{b}-SR_{b}, or -L_{b}-NR_{b}R'_{b};
Rₐ and R'ₐ are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
R_{b} and R'_{b} are each independently H, C₁₋₁₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₁₀ alkyl, -L_{f}-OR_{f}, -L_{f}-SR_{f}, or -L_{f}-NR_{f}R'_{f};
R_{c} and R'_{c} are each independently hydrogen or C₁₋₈ alkyl;
R_{d} and R'_{d} are each independently hydrogen or C₁₋₁₄ alkyl;
Rₑ and R'ₑ are each independently hydrogen or C₁₋₂₀ alkyl; and
R_{f} and R'_{f} are each independently hydrogen or C₁₋₁₀ alkyl.

23. The lipid nanoparticle of claim 22, wherein the ionizable lipid is a compound of formula (IV'): 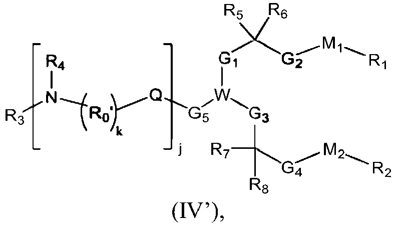 or a stereoisomer, a mixture of stereoisomers, or a pharmaceutically acceptable salt thereof, wherein
j is 0 or 1;
W is CH or N, provided that when W is N, j is 0;
k is 0, 1, 2, 3, 4, 5, 6, 7, or 8;
each instance of R₀' is an independently optionally substituted methylene, or wherein two R₀' together with the carbons they are attached to form a 3 to 8-membered cycloalkylene, and wherein the cycloalkylene is optionally substituted with one or more R**;
M₁ and M₂ are each independently -C(O)O-, -O-, -SC(O)O-, -OC(O)NRₐ-, -NRₐC(O)NRₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, or -S(O)₀₋₂-;
Q is absent, -C(O)O-, -O-, -SC(O)O-, -OC(O)NR_{b}-, -NR_{b}C(O)NR_{b}-, -OC(O)S-, -OC(O)O-, -NR_{b}C(O)O-, -OC(O)-, -SC(O)-, -C(O)S-, -NR_{b}-, -C(O)NR_{b}-, -NR_{b}C(O)-, -NR_{b}C(O)S-, -SC(O)NR_{b}-, -C(O)-, -OC(S)-, - C(S)O-, -OC(S)NR_{b}-, -NR_{b}C(S)O-, -S-S-, or -S(O)₀₋₂-;
Rₐ and R_{b} are each independently H, C₁₋₂₀ alkyl, C₃₋₁₄ cycloalkyl, or 3 to 14-membered heterocyclyl, wherein the alkyl, cycloalkyl and heterocyclyl are optionally substituted with one or more groups selected from hydroxyl, halogen, C₁₋₂₀ alkyl, -Lₑ-ORₑ, -Lₑ-SRₑ, or -Lₑ-NRₑR'ₑ;
G₅ is absent or optionally substituted C₁₋₈ alkylene;
R₁ and R₂ are each independently C₄₋₂₀ alkyl, C₄₋₂₀ alkenyl or C₄₋₂₀ alkynyl, wherein one or more methylene units in R₁ and R₂ are independently optionally replaced by -NH- or -N(C₁₋₂₀ alkyl), wherein the alkyl, alkenyl and alkynyl are optionally substituted;
each instance of R₃ and R₄ are independently H, C₁₋₁₀ alkyl, C₂₋₁₀ alkenyl, C₂₋₁₀ alkynyl, C₃-C₁₄ cycloalkyl, or 3- to 14-membered heterocyclyl, or R₃ and R₄ together with the N atom to which they are attached to form 3 to 14-membered heterocyclyl, or R₄ and one R₀' together with the atoms connecting them form a 4 to 10-membered heterocyclyl; and wherein the alkyl, alkenyl, alkynyl, cycloalkyl, heterocyclyl are optionally substituted with one or more R*; and
R₅, R₆, R₇ and R₈ are each independently hydrogen or optionally substituted C₁₋₈ alkyl.

24. The lipid nanoparticle of claim 23, wherein the ionizable lipid is a compound of formula (V'), (VI') or (VII'): 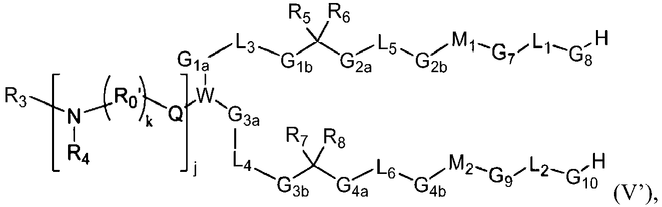 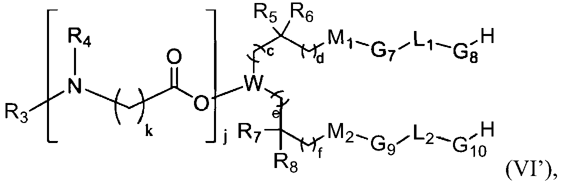 or 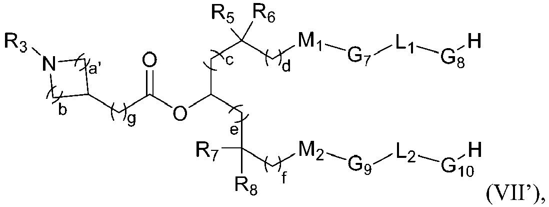 or a stereoisomer, a mixture of stereoisomers, or a pharmaceutically acceptable salt thereof, wherein
k is 0, 1, 2, 3, 4, or 5;
L₁ and L₂ are each independently -(CRR')₂-, -CH=CH-, -C≡C- or -NR"-;
one of L₃ and L₅ is -(CR^{s}R^{s}')₂-, -CH=CH-, or -C≡C-, and the other is absent;
one of L₄ and L₆ is -(CR^{s}R^{s}')₂-, -CH=CH-, or -C≡C-, and the other is absent;
each of G₁ₐ, G_{1b}, G₂ₐ, G_{2b}, G₃ₐ, G_{3b}, G₄ₐ and G_{4b} is independently absent or optionally substituted C₁₋₇ alkylene;
G₁ₐ, G_{1b}, G₂ₐ and G_{2b} have a total length of 1, 2, 3, 4, 5, 6 or 7 carbon atoms;
G₃ₐ, G_{3b}, G₄ₐ and G_{4b} have a total length of 1, 2, 3, 4, 5, 6 or 7 carbon atoms;
R₅, R₆, R₇ and R₈ are each independently hydrogen or optionally substituted C₁₋₆ alkyl;
G₇, G₈, G₉ and G₁₀ are each independently absent or optionally substituted C₁₋₁₂ alkylene, provided that G₇ and G₈ have a total length of 4, 5, 6, 7, 8, 9, 10, 11 or 12 carbon atoms, and G₉ and G₁₀ have a total length of 4, 5, 6, 7, 8, 9, 10, 11 or 12 carbon atoms;
R^{s} and R^{s'} are each independently H, C₁₋₁₀ alkyl, -L_{d}-OR_{d} or -L_{d}-NR_{d}R'_{d};
R' is H, C₁₋₁₄ alkyl, -Lₐ-ORₐ or -Lₐ-NRₐR'ₐ;
R" is H or C₁₋₁₄ alkyl;
L_{d} is absent or C₁₋₁₀ alkylene;
Lₐ is absent or C₁₋₁₄ alkylene;
Rₐ and R'ₐ are each independently H, C₁₋₁₄ alkyl, C₃₋₁₀ cycloalkyl or 3- to 10-membered heterocyclyl, and wherein the alkyl, cycloalkyl and heterocyclyl are optionally substituted;
R_{d} and R'_{d} are each independently H or C₁₋₁₀ alkyl;
a' and b are each independently 0, 1, 3, 4, 5, provided that at least one of a' and b is not 0; g is 0, 1, 2, 3, 4, or 5;
a'+g is 0, 1, 2, 3, 4, or 5; and
c, d, e, and f are each independently 0, 1, 2, 3, 4, 5, 6, or 7, provided that c+d is an integer between 2 and 9, and e+f is an integer between 2 and 9.

25. The lipid nanoparticle of any one of claim 1 to 24, wherein the ionizable lipid is a compound of formula (VIII): 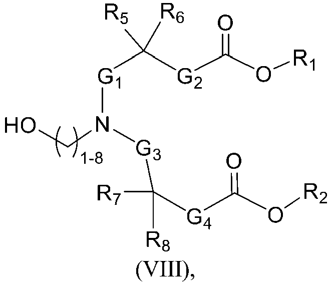 or a stereoisomer, a mixture of stereoisomers, or a pharmaceutically acceptable salt thereof.

26. The lipid nanoparticle of claim 25, wherein R₅, R₆, R₇ and R₈ are each independently hydrogen or methyl.

27. The lipid nanoparticle of claim 25, wherein G₁, G₂, G₃ and G₄ are each independently absent or C₁₋₈ alkylene.

28. The lipid nanoparticle of any one of claims 1 to 27, wherein the ionizable lipid is a compound of formula (IX): 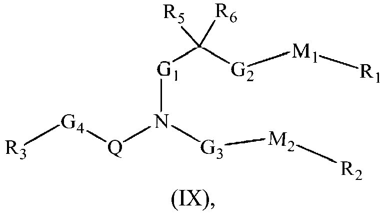 or a stereoisomer, a mixture of stereoisomers, or a pharmaceutically acceptable salt thereof, wherein:
G₁ and G₂ are each independently a bond, C₁₋₁₃ linear alkylene, C₂₋₁₃ linear alkenylene, or C₂₋₁₃ linear alkynylene, wherein the alkylene, alkenylene, and alkynylene are optionally substituted by one or more R_{G1};
G₁ and G₂ have a total length of 3, 4, 5, 6, 7, 8, 9, 10, 11, 12 or 13 carbon atoms;
each instance of R_{G1} is independently H, C₁₋₁₄ alkyl, -Lₐ-ORₐ, -Lₐ-SRₐ, or -Lₐ-NRₐR'ₐ;
G₃ is C₄₋₁₄ linear alkylene, C₄₋₁₄ linear alkenylene, or C₄₋₁₄ linear alkynylene, wherein the alkylene, alkenylene, and alkynylene are optionally substituted by one or more R_{G3};
each instance of R_{G3} is independently H, -Lₐ-ORₐ, -Lₐ-SRₐ or -Lₐ-NRₐR'ₐ;
Lₐ is independently a bond or C₁₋₁₄ alkylene;
Rₐ and R'ₐ are each independently H, C₁₋₁₄ alkyl, C₃₋₁₄ cycloalkyl, or 3- to 14-membered heterocyclyl;
G₄ is a bond, C₁₋₆ alkylene, C₂₋₆ alkenylene, or C₂₋₆ alkynylene, and wherein the alkylene, alkenylene, and alkynylene are optionally substituted by one or more R_{G4};
each of instance of R_{G4} is independently H, C₁₋₆ alkyl, -L_{b}-OR_{b}, -L_{b}-SR_{b} or -L_{b}-NR_{b}R'_{b};
L_{b} is independently a bond or C₁₋₆ alkylene;
R_{b} and R'_{b} are independently H, C₁₋₆ alkyl, C₃₋₁₀ cycloalkyl or 3-to 10-membered heterocyclyl;
or two R_{G4} together with the same carbon atom they are attached to form a C₃₋₁₄ cycloalkylene or 3- to 14-membered heterocyclylene, wherein the cycloalkylene and heterocyclylene are optionally substituted by one or more R_{4g};
each of instance of R_{4g} is independently H, halogen, cyano, C₁₋₈ alkyl, C₁₋₈ haloalkyl, -Lₑ-ORₑ, -Lₑ-SRₑ, or -Lₑ-NRₑR'ₑ;
Lₑ is independently a bond or C₁₋₈ alkylene;
Rₑ and R'ₑ are independently H, C₁₋₈ alkyl, C₃₋₁₄ cycloalkyl, or 3- to 14-membered heterocyclyl;
M₁ and M₂ are each independently -C(O)O-, -OC(O)-, -O-, -SC(O)O-, -OC(O)NR-, -NRC(O)NR-, - OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S- or -S(O)₀₋₂-;
Q is a bond, -C(O)O-, -O-, -SC(O)O-, -OC(O)NR_{f}-, -NR_{f}C(O)NR_{f}-, -OC(O)S-, -OC(O)O-, -NRᵣC(O)O-, -OC(O)-, -SC(O)-, -C(O)S-, -NR_{f}-, -C(O)NR_{f}-, -NR_{f}C(O)-, -NR_{f}C(O)S-, -SC(O)NR_{f}-, -C(O)-, -OC(S)-, - C(S)O-, -OC(S)NR_{f}-, -NR_{f}C(S)O-, -S-S-, -S(O)₀₋₂-, phenylene, or pyridinylene, wherein the phenylene and pyridinylene are optionally substituted with one or more R*;
R* is independently H, halogen, cyano, C₁₋₁₀ alkyl, C₁₋₁₀ haloalkyl, -L_{f}-OR_{f}, -L_{f}-SR_{f}, or -L_{f}-NR_{f}R'_{f},
L_{f} is independently a bond or C₁₋₈ alkylene;
R_{f} and R'_{f} are independently H, C₁₋₁₀ alkyl, C₃₋₁₄ cycloalkyl, or 3- to 14-membered heterocyclyl;
R₁ and R₂ are independently C₄₋₂₀ alkyl, C₄₋₂₀ alkenyl, or C₄₋₂₀ alkynyl, which is optionally substituted with one or more R₁ₛ, and wherein one or more methylene units are optionally and independently replaced with -NR'-;
R₁ₛ is independently H, C₁₋₂₀ alkyl, -L_{c}-OR_{c}, -L_{c}-SR_{c}, or -L_{c}-NR_{c}R'_{c};
R and R' are each independently H or C₁₋₂₀ alkyl;
L_{c} is independently a bond or C₁₋₂₀ alkylene;
R_{c} and R'_{c} are each independently H, C₁₋₂₀ alkyl, C₃₋₁₄ cycloalkyl, or 3- to 14-membered heterocyclyl;
R₃ is -CN, -OR_{g}, -C(O)R_{g}, -OC(O)R_{g}, -NR"C(O)R_{g}, -NR_{g}R'_{g}, -NR"C(O)NR_{g}R'_{g}, -NR"C(O)R_{g}, - NR"S(O)₂R_{g}, -OC(O)NR_{g}R'_{g}, -NR"C(O)OR_{g}, -N(OR_{g})C(O)R_{g}, -N(OR_{g})S(O)₂R_{g}, -N(OR_{g})C(O)OR_{g}, - N(OR_{g})C(O)R_{g}R'_{g}, 3- to 14-membered heterocyclyl, or 5- to 14-membered heteroaryl;
R_{g} and R'_{g} are each independently H, C₁₋₁₀ alkyl, C₃₋₁₀ cycloalkyl, or 3- to 10-membered heterocyclyl;
R" is independently H or C₁₋₆ alkyl;
R₅ and R₆ are independently C₁₋₈ alkyl, wherein the alkyl is optionally substituted by one or more R₄ₛ;
or R₅ and R₆ together with the same carbon atom they are attached to form a C₃₋₁₄ cycloalkylene or 3- to 14-membered heterocyclylene, wherein the cycloalkylene and heterocyclylene are optionally substituted by one or more R₄ₛ;
R₄ₛ is independently H, halogen, cyano, C₁₋₈ alkyl, C₁₋₈ haloalkyl, -Lₐ-ORₐ, -Lₐ-SRₐ, or -L_{d}-NR_{d}R'_{d};
L_{d} is independently a bond or C₁₋₈ alkylene; and
R_{d} and R'_{d} are independently H, C₁₋₈ alkyl, C₃₋₁₄ cycloalkyl or 3- to-14-membered heterocyclyl.

29. The lipid nanoparticle of any one of claims 1 to 28, wherein the ionizable lipid is a compound of formula (X): 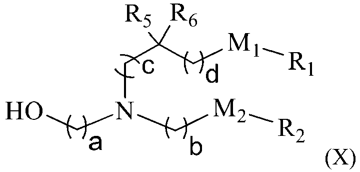 or a stereoisomer, a mixture of stereoisomers, or a pharmaceutically acceptable salt thereof, wherein:
a is 1, 2, 3, 4, 5 or 6;
b is 4, 5, 6, 7, 8, 9 or 10;
c is 1, 2, 3, 4, 5 or 6;
d is 0, 1, 2, 3 or 4; and
c+d is 3, 4, 5, 6, 7, 8 or 9.

30. The lipid nanoparticle of any one of claims 1 to 29, wherein the ionizable lipid is: 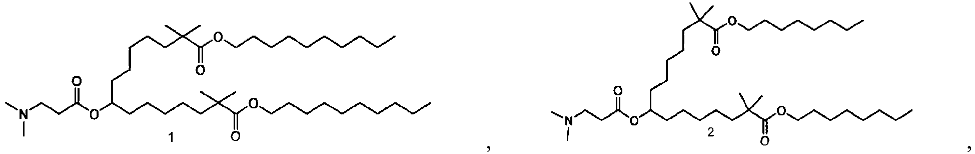 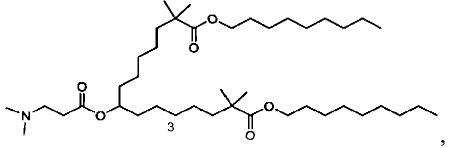 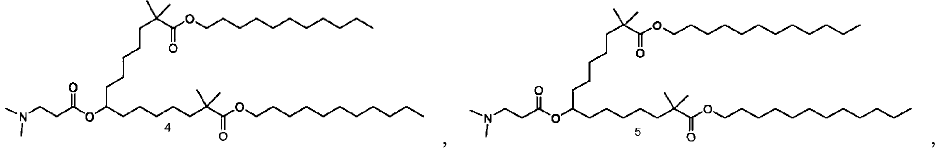 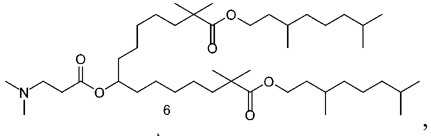 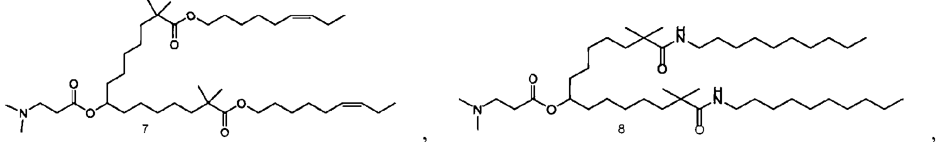 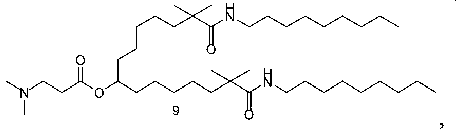 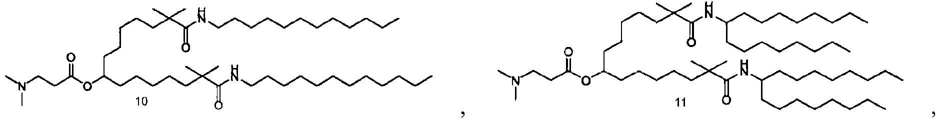 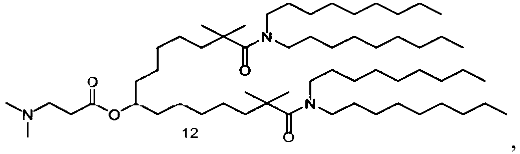 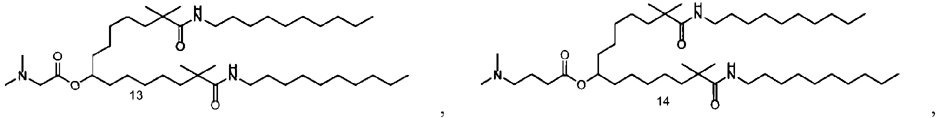 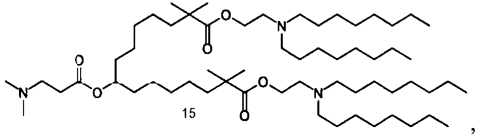 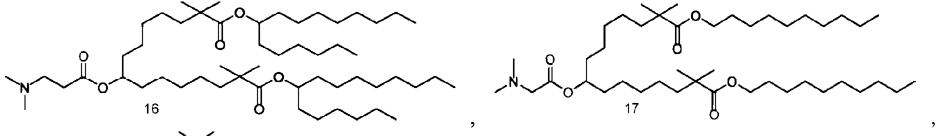 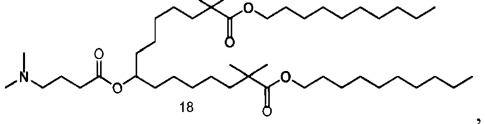 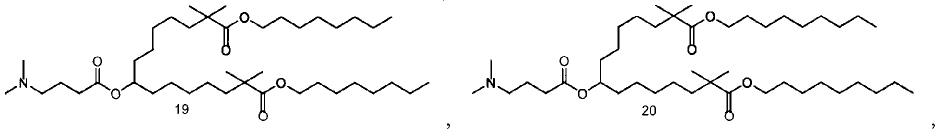 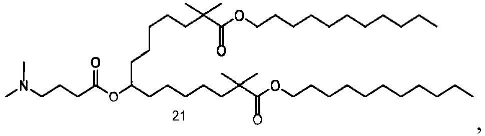 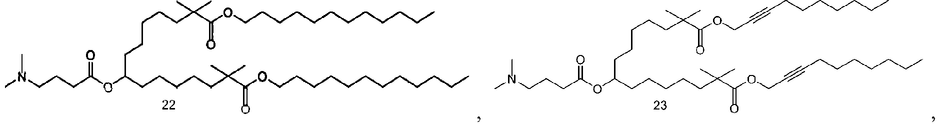 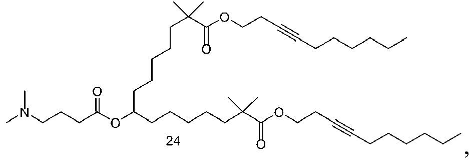 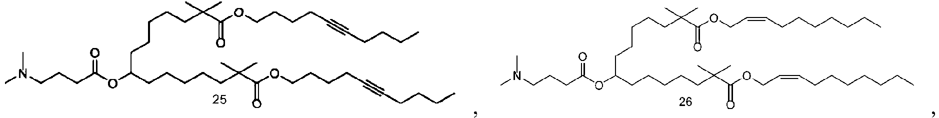 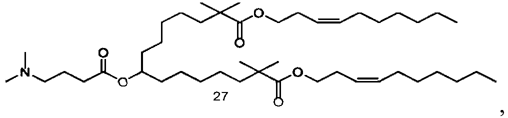 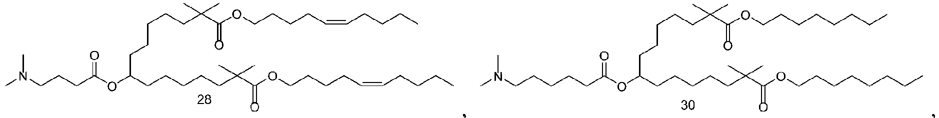 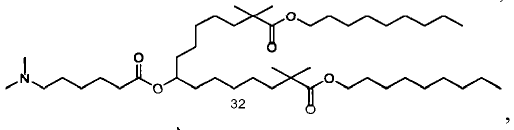 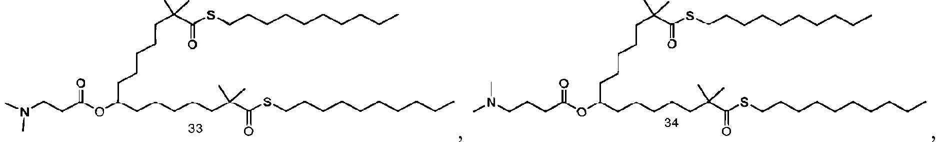 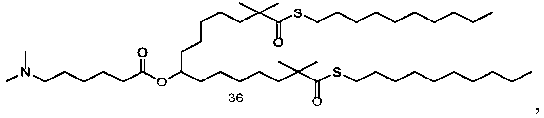 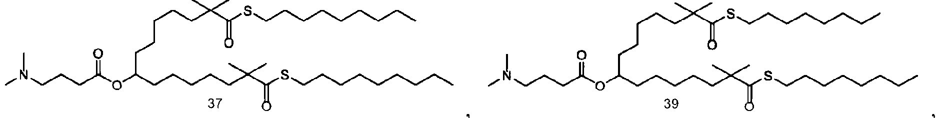 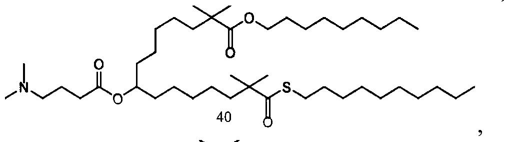 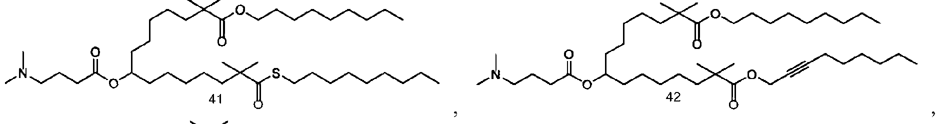 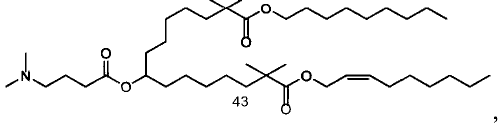 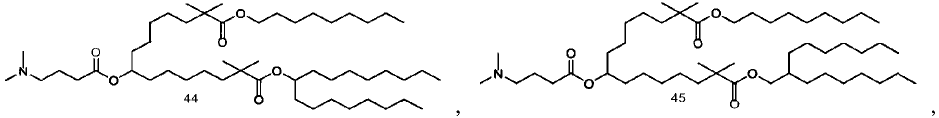 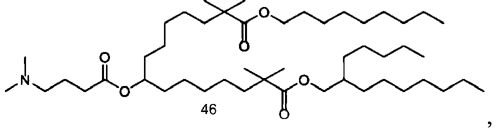 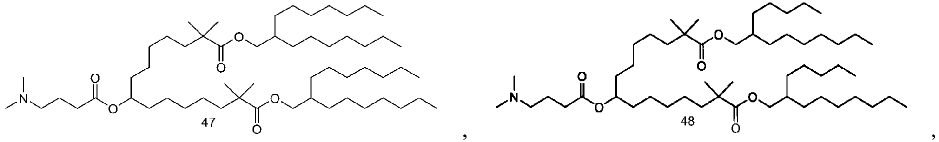 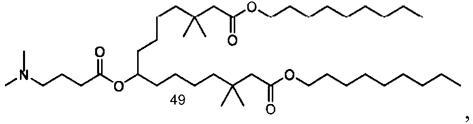 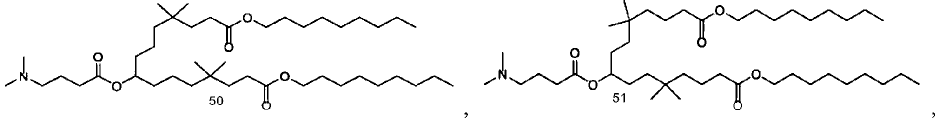 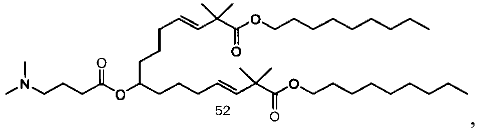 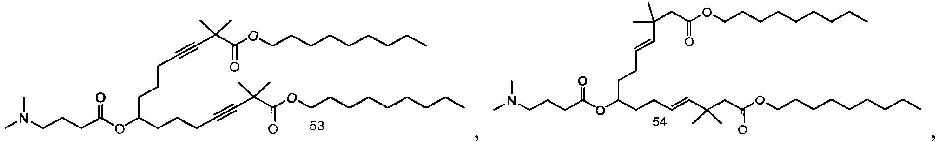 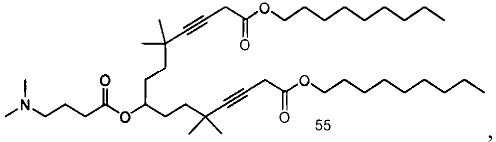 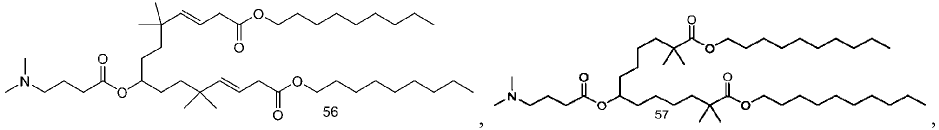 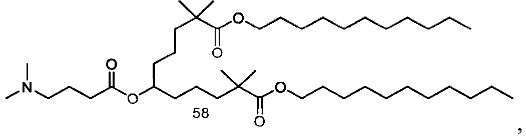 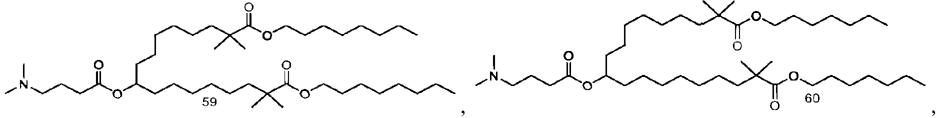 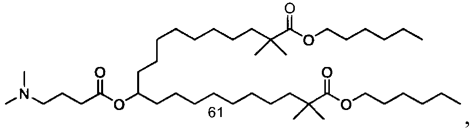 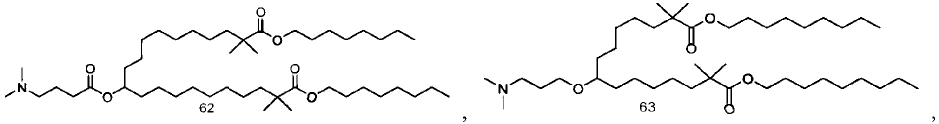 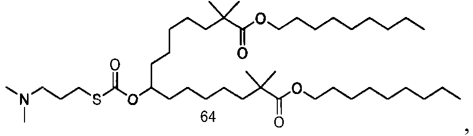 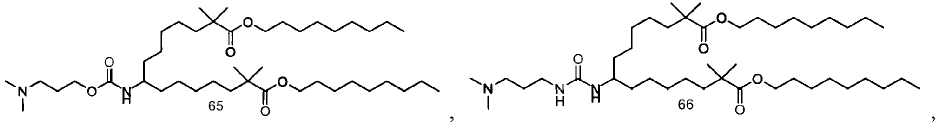 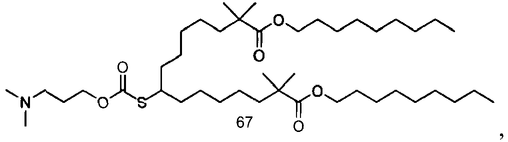 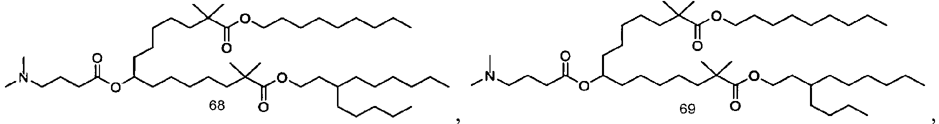 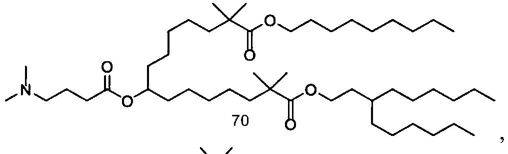 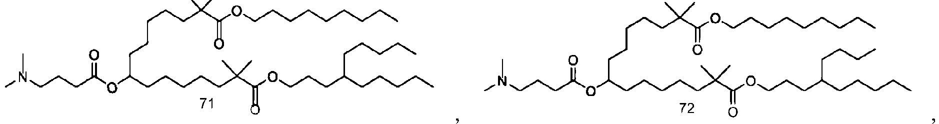 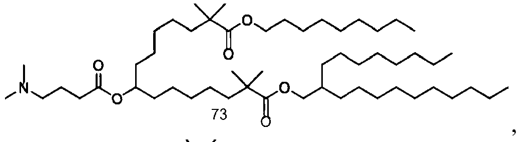 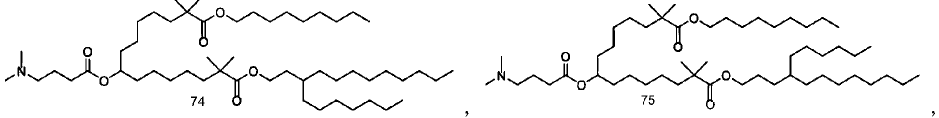 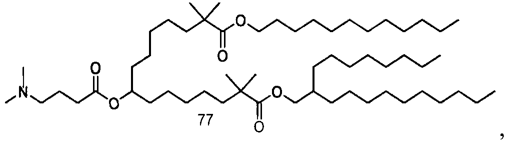 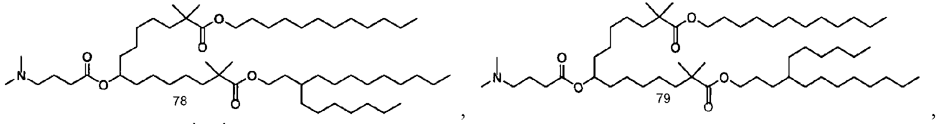 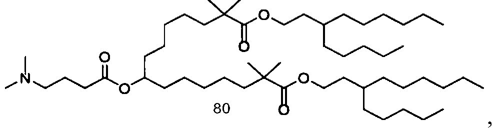 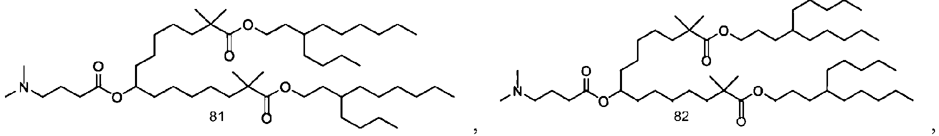 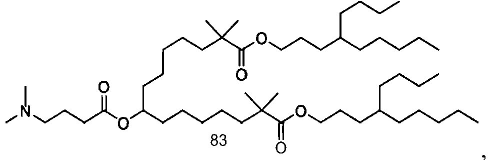 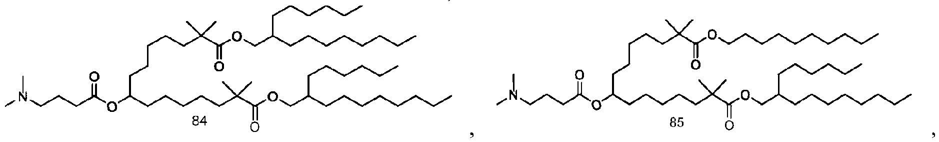 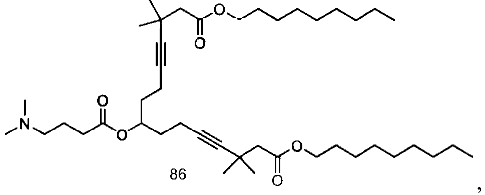 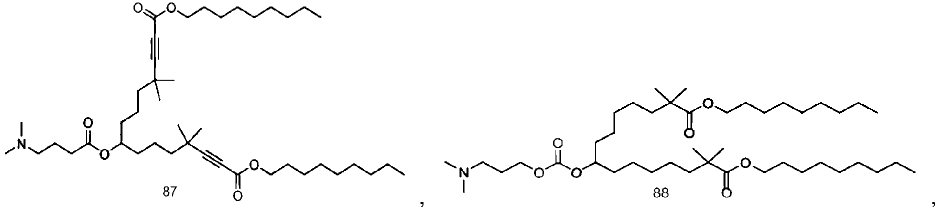 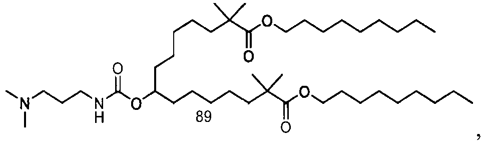 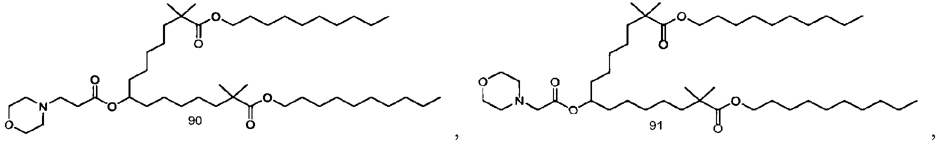 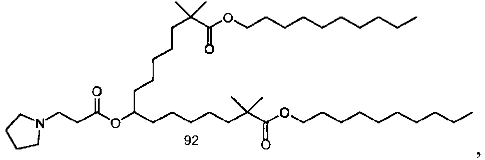 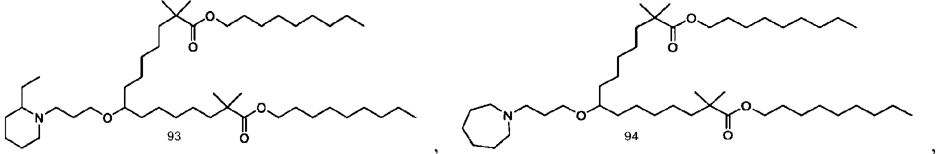 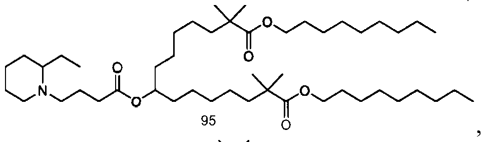 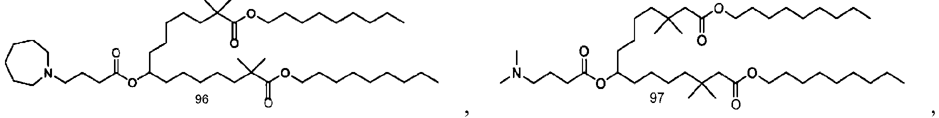 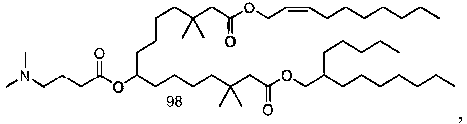 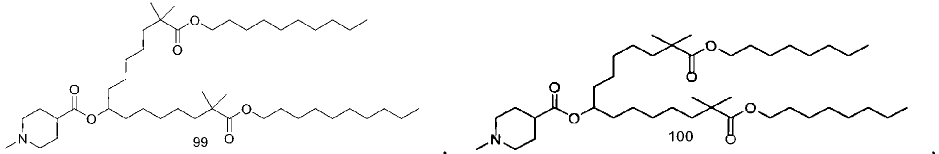 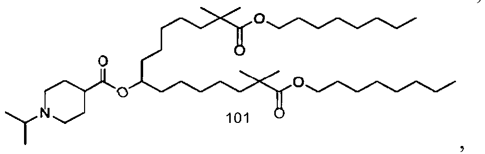 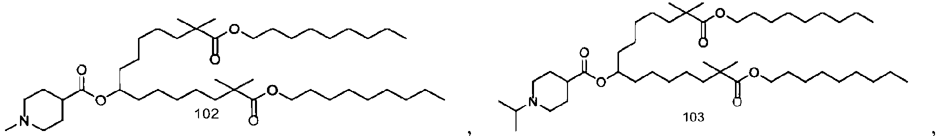 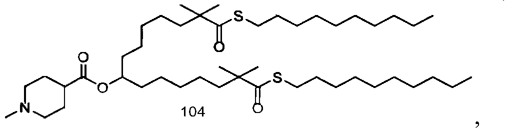 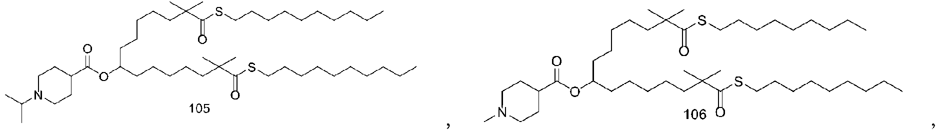 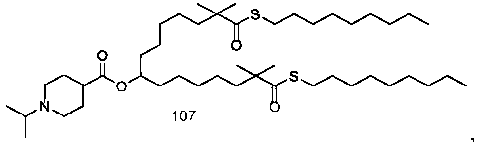 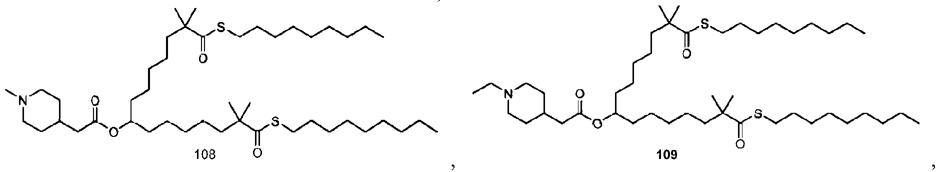 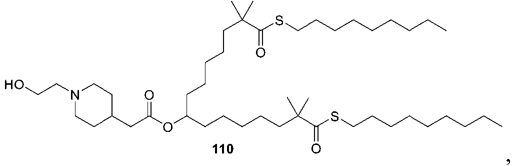 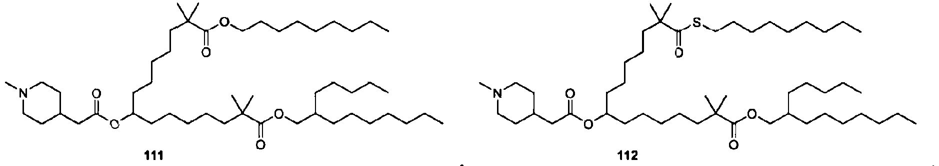 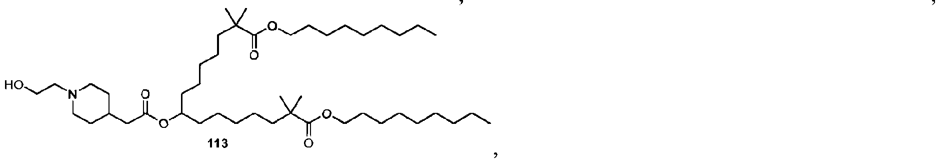 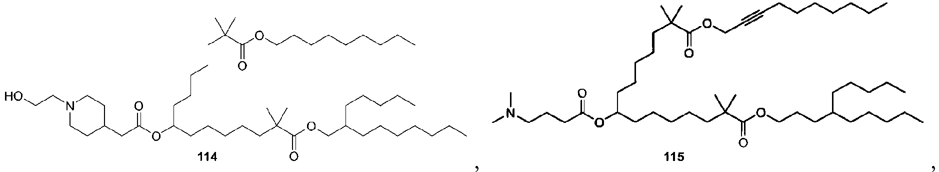 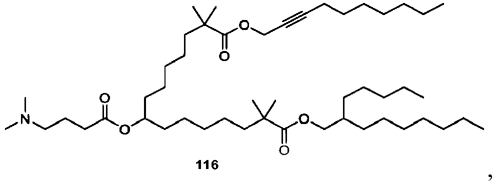 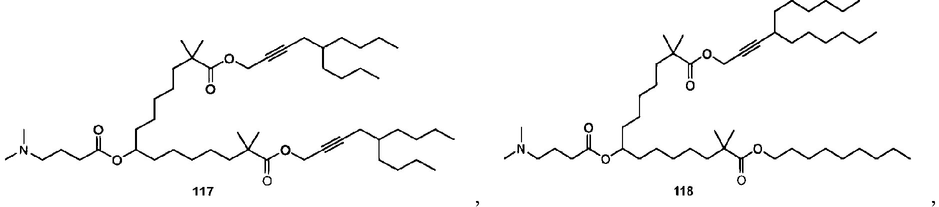 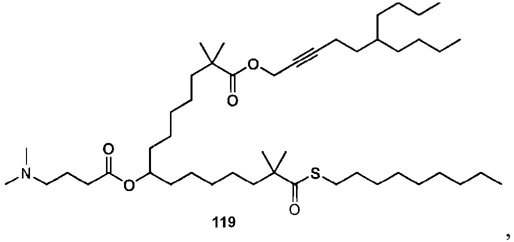 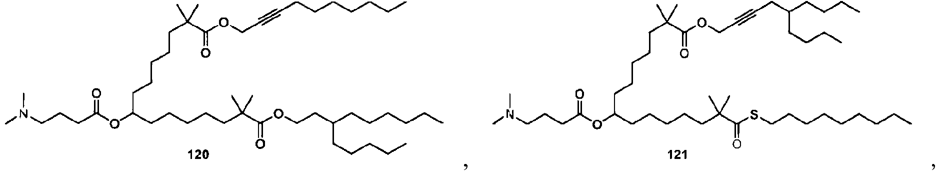 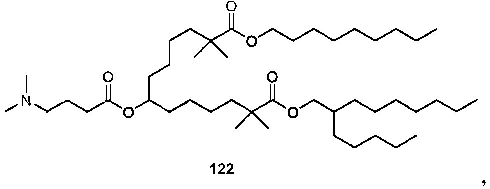 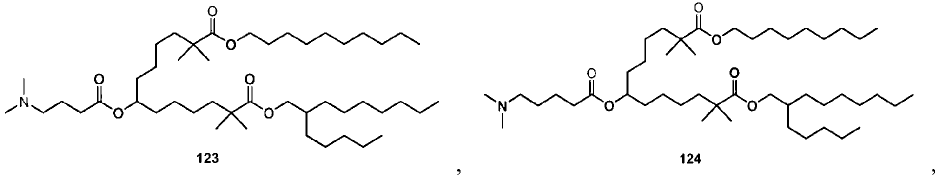 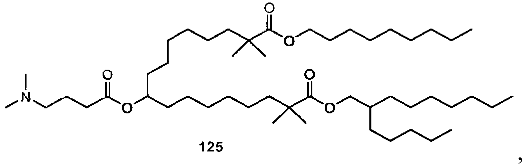 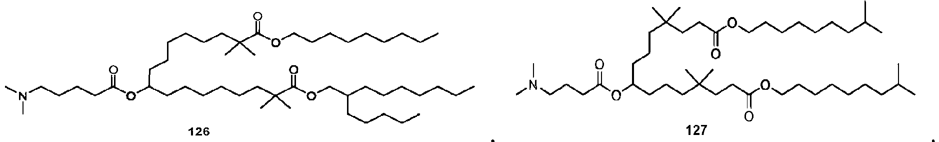 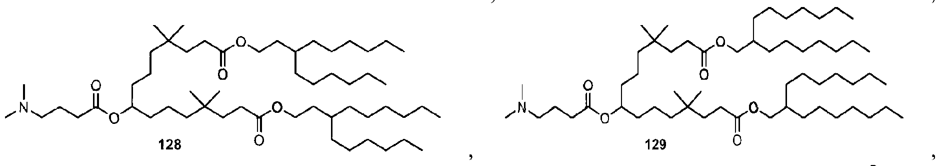 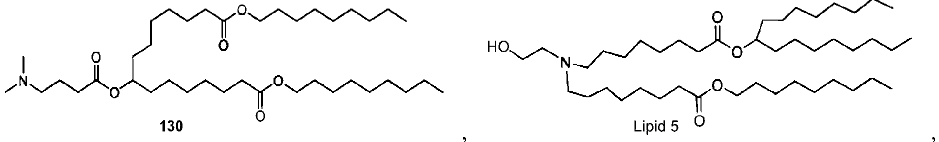 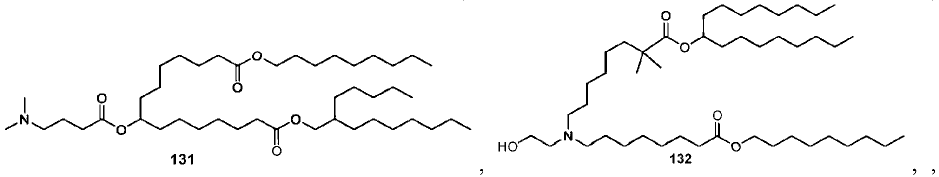 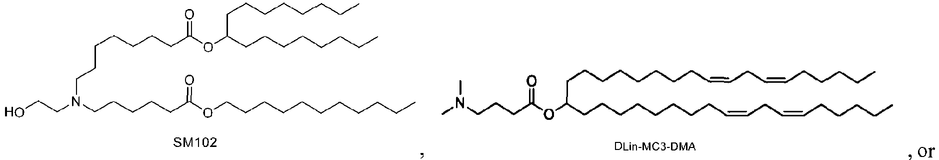 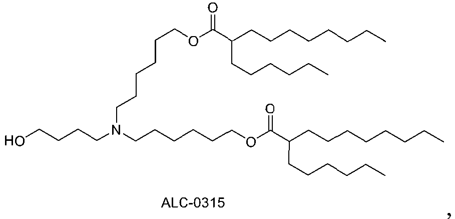 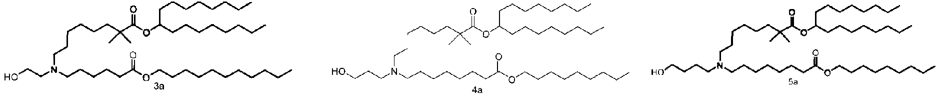 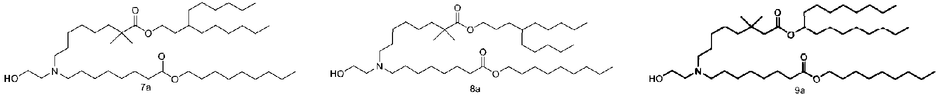 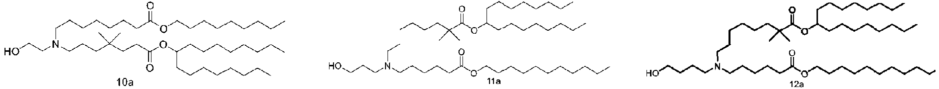 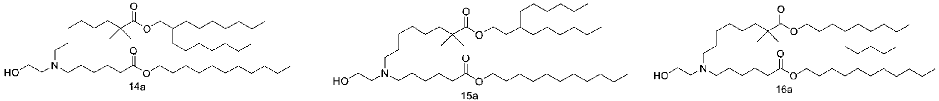 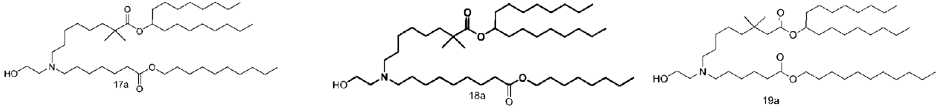 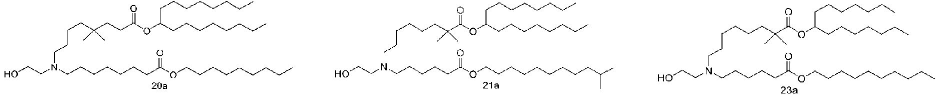 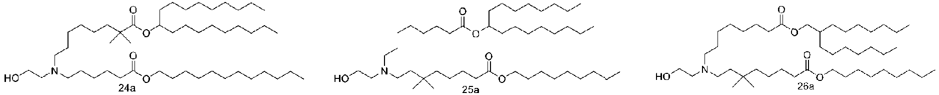 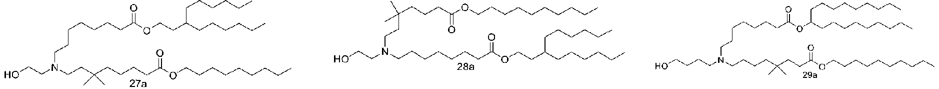 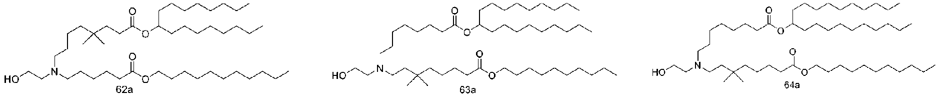 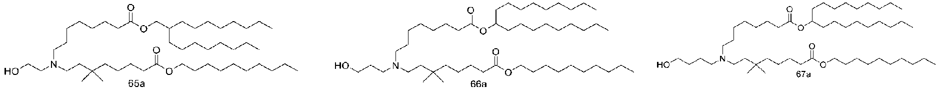 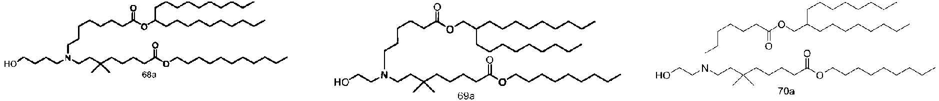 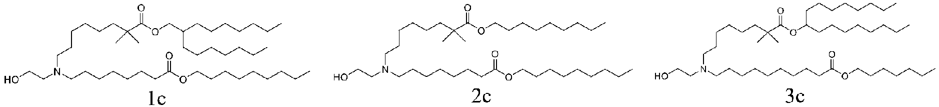 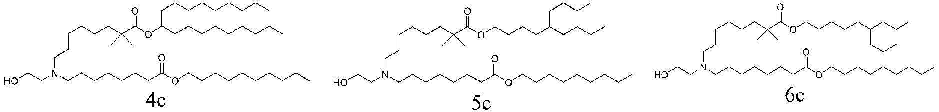 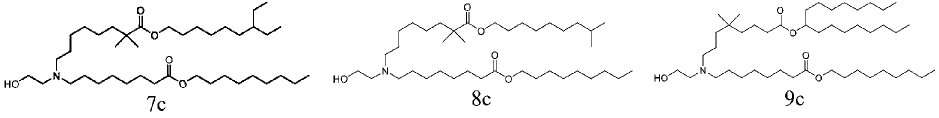 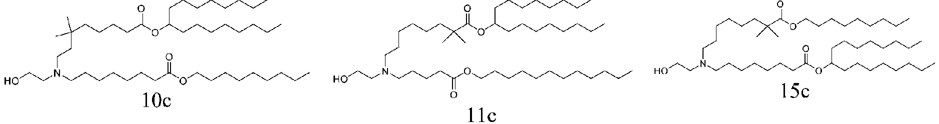 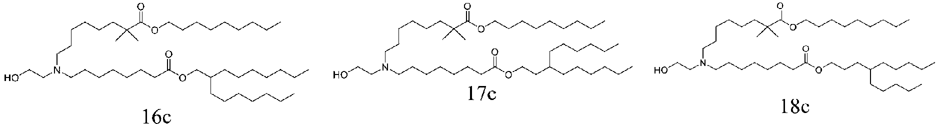 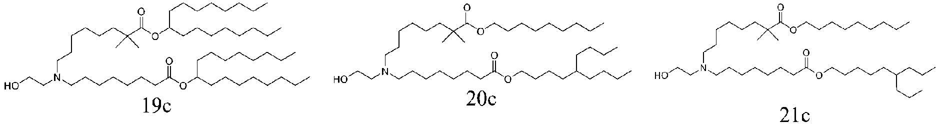 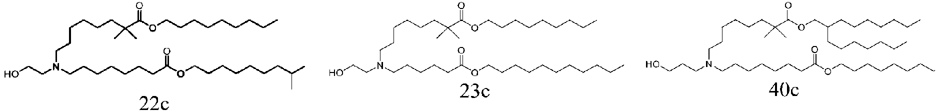 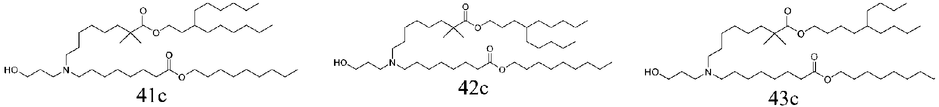 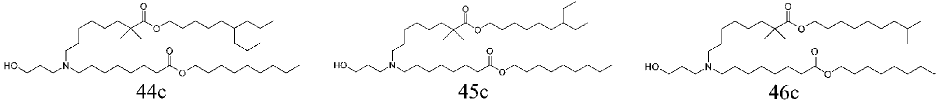 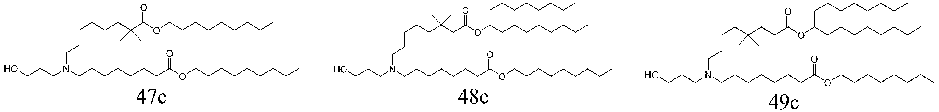 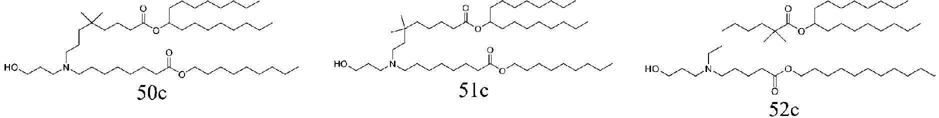 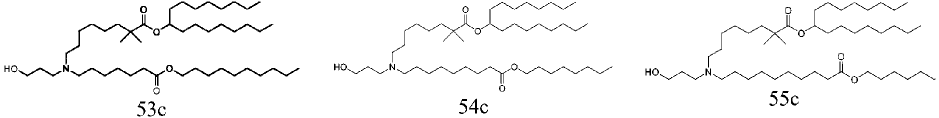 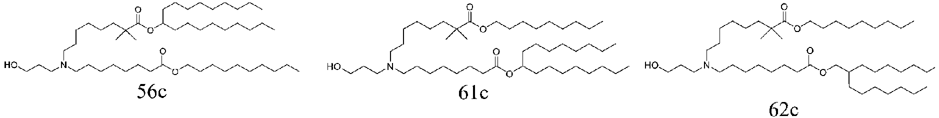 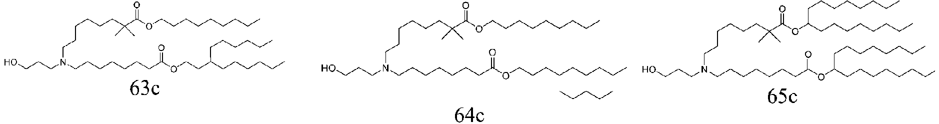 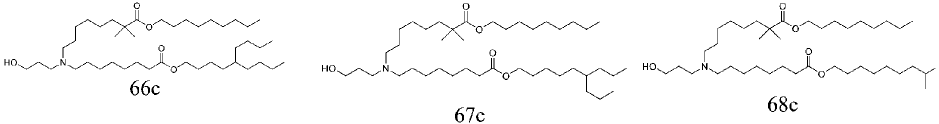 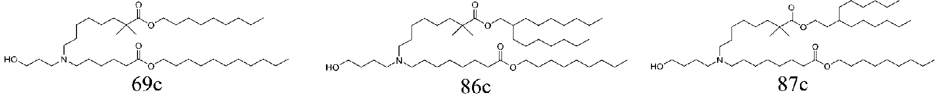 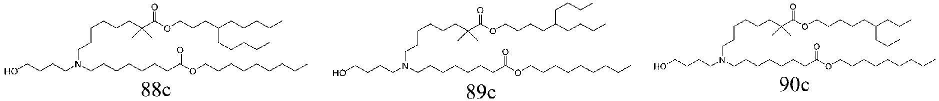 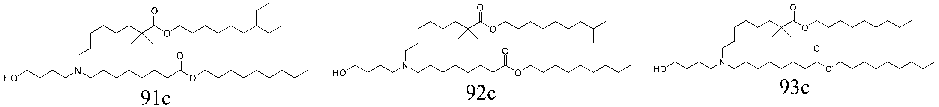 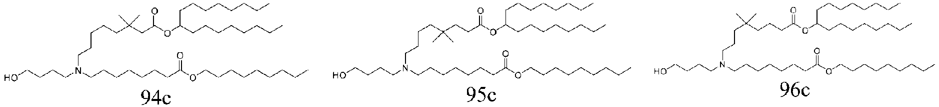 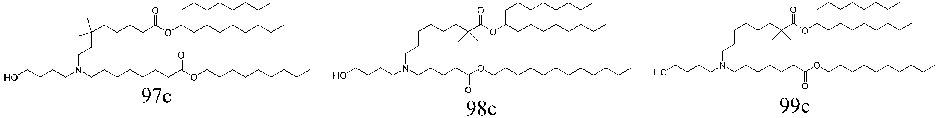 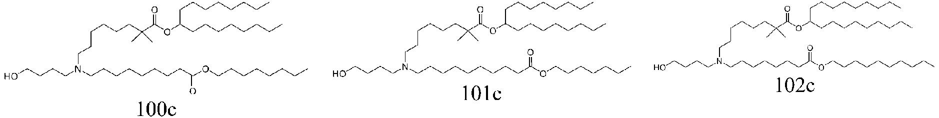 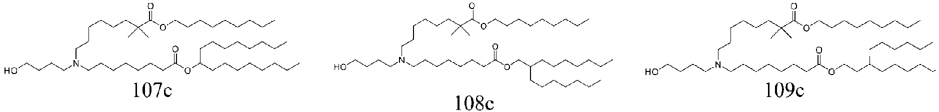 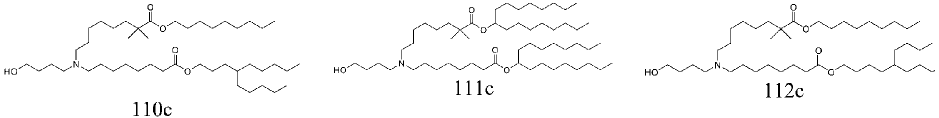 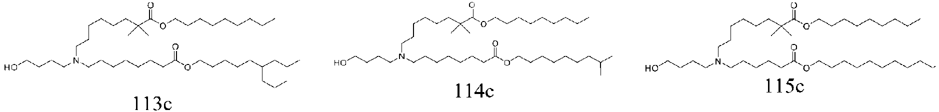 or a stereoisomer, a mixture of stereoisomers, or a pharmaceutically acceptable salt thereof.

31. The lipid nanoparticle of any one of claims 1 to 30, wherein the ionizable lipid is DODAP, DODMA, DLinDMA, DLin-KC2-DMA, DLin-MC3-DMA, SM-102, or ALC-0315, or a combination thereof, or a stereoisomer, a mixture of stereoisomers, or a pharmaceutically acceptable salt thereof.

32. The lipid nanoparticle of any one of claims 1 to 31, wherein the molar percentage of the ionizable lipid relative to the total lipid is from about 35 mol % to about 70 mol %, more preferably 40 mol% to 60 mol%, such as 35 mol%, 40 mol%, 45 mol%, 50% mol%, 55 mol% or 60%.

33. The lipid nanoparticle of any one of claims 1 to 32, further comprising phospholipid.

34. The lipid nanoparticle of claim 33, wherein the phospholipid is DSPC, DMPC, DOPC, DPPC, POPC, DOPE, DMPE, POPOE, or DPPE.

35. The lipid nanoparticle of any one of claims 1 to 34, wherein the molar percentage of the phospholipidis relative to the total lipid is from 5 mol% to 30 mol%, preferably from 5 mol% to 20 mol%, more preferably from 5 mol% to 15 mol%, such as 5 mol%, 6 mol%, 7 mol%, 8 mol%, 9 mol%, 10 mol%, 11 mol%, 12 mol%, 13 mol%, 14 mol%, or 15 mol%.

36. The lipid nanoparticle of any one of claims 1 to 35, further comprising a structural lipid.

37. The lipid nanoparticle of claim 36, wherein the structural lipid is selected from the group consisting of cholesterol, campesterol, stigmasterol, sitosterol, brassicasterol, ergosterol, solanine, ursolic acid, alpha-tocopherol, beta-sitosterol, avenasterol, and canola sterol.

38. The lipid nanoparticle of any one of claims 1 to 37, wherein the molar percentage of the structural lipid relative to the total lipid is from 10 mol % to 80 mol %, more preferably from 15 mol % to 70 mol %, more preferably from 30 mol % to 50 mol % , more preferably from 30 mol % to 40 mol %, such as 30 mol %, 35 mol %, 40 mol %, 45 mol %, 50 mol %, 55 mol % or 60 mol %.

39. The lipid nanoparticle of any one of claims 1 to 38, further comprising a polymer conjugated lipid, such as a pegylated lipid.

40. The lipid nanoparticle of claim 39, wherein the pegylated lipid comprises a pegylated moiety having a molecule weight of from about 1000 Da to about 20,000 Da, from about 1000 Da to about 5000 Da, or from about 1000 Da to about 2000 Da.

41. The lipid nanoparticle of claim 39, wherein the polymer conjugated lipid is ALC-0159, DMG-PEG2000, DMPE-PEG1000, DPPE-PEG1000, DSPE-PEG1000, DOPE-PEG1000, Ceramide-PEG2000, DMPE-PEG2000, DPPE-PEG2000, DSPE-PEG2000, DSPE-PEG2000-Mannose, Ceramide-PEG5000, DSPE-PEG5000, or DSPE-PEG2000 amine.

42. The lipid nanoparticle of any one of claims 1 to 41, wherein the molar percentage of the polymer conjugated lipid relative to the total lipid is from 0.25 mol % to 10 mol %, more preferably from 0.5 mol % to 5 mol %, more preferably from 0.5 mol % to 3 mol %, such as 0.5 mol%, 0.6 mol%, 0.7 mol%, 0.8 mol%, 0.9 mol%, 1.0 mol%, 1.1 mol%, 1.2 mol%, 1.3 mol%, 1.4 mol%, 1.5 mol%, 1.6 mol%, 1.7mol%, 1.8 mol%, 1.9 mol%, 2.0 mol%, 2.1 mol%, 2.2 mol%, 2.3 mol%, 2.4 mol%, 2.5 mol%, 2.6 mol%, 2.7 mol%, 2.8 mol%, 2.9 mol%, 3.0 mol%, 3.1 mol%, 3.2 mol%, 3.3 mol%, 3.4 mol%, 3.5 mol%, 3.6 mol%, 3.7 mol%, 3.8 mol%, 3.9 mol%, or 4.0 mol%.

43. The lipid nanoparticle of any one of claims 1 to 42, wherein the lipid nanoparticle targeting spleen comprises: (1) a steroid compound; (2) an ionizable lipid; (3) phospholipid; (4) structural lipid; and (5) polymer conjugated lipid.

44. The lipid nanoparticle of any one of claims 1 to 43, the lipid nanoparticle comprises the following components in the molar percentages:
1) Steroid compound 3 mol%-15 mol%, preferably 5 mol%-10 mol%;
2) Ionizable lipid 35 mol%-70 mol%, preferably 40 mol%-60 mol%;
3) Phospholipid 5 mol%-30 mol%, preferably 5 mol%-20 mol%;
4) Structural lipid 10 mol%-80 mol%, preferably 15 mol%-70 mol%, more preferably 30 mol%-50 mol%; and
5) Polymer conjugated lipid 0.25 mol%-10 mol%, preferably 0.5 mol%-5 mol%, more preferably 0.5 mol%-3 mol%.

45. The lipid nanoparticle of any one of claims 1 to 44, wherein the lipid nanoparticle further comprises a payload, and wherein the payload is a therapeutic agent, a prophylactic agent, or a diagnostic agent.

46. The lipid nanoparticle of claim 45, wherein the payload is nucleic acid.

47. The lipid nanoparticle of claim 46, wherein the nucleic acid is antisense oligonucleotide (ASO), DNA, or RNA, optionally wherein the RNA is RNA interference (RNAi), small interfering RNA (siRNA), short hairpin RNA (shRNA), antisense RNA (aRNA), messenger RNA (mRNA), modified messenger RNA (mmRNA), long noncoding RNA (lncRNA), microRNA (miRNA), small activating RNA (saRNA), multicoding nucleic acid (MCNA), polymer-coded nucleic acid (PCNA), guide RNA (gRNA), CRISPR RNA (crRNA), or any other RNA in the ribozyme.

48. A lipid nanoparticle composition comprising the lipid nanoparticle of any one of claims 1 to 47.

49. A pharmaceutical composition comprising the lipid nanoparticle of any one of claims 1 to 47 or the lipid nanoparticle composition of claim 48 and a pharmaceutically acceptable carrier.

50. A method of treating or preventing a splenic disease or spleen-related disease in a subject having the splenic disease or spleen-related disease, comprising administering to the subject a therapeutically effective amount of the lipid nanoparticle of any one of claims 1 to 47, the lipid nanoparticle composition of claim 48, or the pharmaceutical composition of claim 49.

51. A use of the lipid nanoparticle of any one of claims 1 to 47, the lipid nanoparticle composition of claim 48, or the pharmaceutical composition of claim 49 in manufacture of a medicament for the treatment or prevention of splenic disease or spleen-related disease.

52. The lipid nanoparticles of any one of claims 1 to 47, the lipid nanoparticle composition of claim 48, or the pharmaceutical composition of claim 49 for use in the treatment or prevention of splenic disease or spleen-related disease;
preferably, the splenic disease or spleen-related disease includes hematologic disorders, infectious diseases, immune diseases, or cancer;
more preferably, the splenic disease or spleen-related disease includes hemolytic anemia; thrombocytopenic purpura; chronic leukemia; lymphoma such as Hodgkin lymphoma or non-Hodgkin lymphoma; myelodysplastic syndrome; myelofibrosis; splenomegaly; lipid metabolism disorders such as Gaucher disease and sphingomyelinosis; splenomegaly and hypersplenism associated with sepsis, typhoid, infectious mononucleosis or subacute bacterial endocarditis; splenomegaly and hypersplenism associated with malaria, tuberculosis, Kala-azar or other related diseases; congestive splenomegaly and hypersplenism due to liver cirrhosis and portal hypertension; splenic cysts such as parasitic splenic cysts or pseudosplenic cysts; splenic abscesses; primary splenic tumors; splenic metastases; splenic artery aneurysms; splenic infarction; splenic purpura; splenic rupture; and the like.

53. The method of claim 50 or the use of claim 51, wherein the splenic disease or spleen-related disease includes hematologic disorders, infectious diseases, immune diseases, or cancer.

54. The method of claim 50 or the use of claim 51, wherein the splenic disease or spleen-related disease includes hemolytic anemia; thrombocytopenic purpura; chronic leukemia; lymphoma such as Hodgkin lymphoma or non-Hodgkin lymphoma; myelodysplastic syndrome; myelofibrosis; splenomegaly; lipid metabolism disorders such as Gaucher disease and sphingomyelinosis; splenomegaly and hypersplenism associated with sepsis, typhoid, infectious mononucleosis or subacute bacterial endocarditis; splenomegaly and hypersplenism associated with malaria, tuberculosis, Kala-azar or other related diseases; congestive splenomegaly and hypersplenism due to cirrhosis of the liver and portal hypertension; splenic cysts such as parasitic splenic cysts or pseudosplenic cysts; splenic abscesses; primary splenic tumors; splenic metastases; splenic artery aneurysms; splenic infarction; splenic purpura; splenic rupture; and the like.

55. A process of preparing the lipid nanoparticle of any one of claims 1 to 47 or the lipid nanoparticle composition of claim 48, comprising the steps of:
(i) dissolving in a first solution a mixture comprising the lipids, wherein the lipid solution is formed in an organic solvent;
(ii) dissolving in a second solution a payload to form a payload solution; and
(iii) mixing the lipid solution and the payload solution to form the lipid nanoparticles.

56. The process of claim 55, wherein the mixing is performed using a microfluidizer system.

57. A steroid compound of formula (II-A): 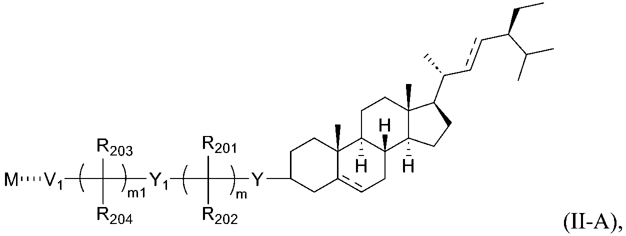 or a stereoisomer, a mixture of stereoisomers, or a pharmaceutically acceptable salt thereof, wherein:
each - - - is independently a single bond or double bond;
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 3 to 12;
ml is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted; and provided that the compound is not:

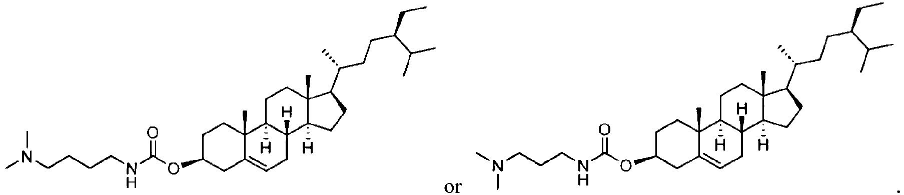

58. A steroid compound of formula (II-B): 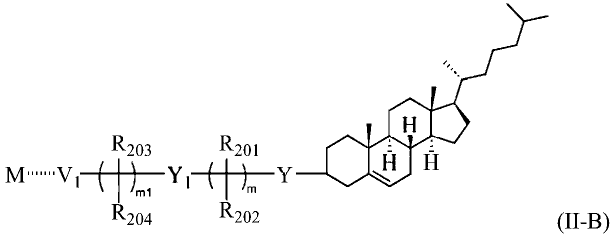 or a stereoisomer, a mixture of stereoisomers, or a pharmaceutically acceptable salt thereof, wherein:
Y is -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, or -S(O)₀₋₂-, and wherein the attachment to the left is to the direction of V₁;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂ₐ-, -NR₂ₐC(O)-, -NR₂ₐC(O)S-, -SC(O)NR₂,-, - C(O)-, -OC(S)-, -C(S)O-, -OC(S)NR₂ₐ-, -NR₂ₐC(S)O-, or -S(0)₀₋₂-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, or -L_{2b}-N⁺R₂₁R₂₂R₂₃, L_{2b}-OC(O)R₂₁, or L_{2b}-C(O)OR₂₁;
m is an integer from 5 to 12;
ml is integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene; and
each of R_{2b}, R'_{2b} and R"_{2b} are independently H or C₁₋₁₀ alkyl;
preferably, Y₁ is -C(O)O-, -OC(O)-, -O-, -S-, -NR₂ₐ-, -OC(O)NR₂ₐ₋, or -NR₂ₐC(O)-.

59. A steroid compound of formula (I-P): 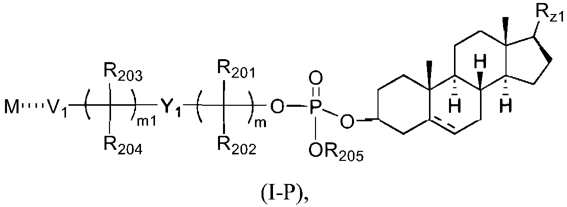 or a stereoisomer, a mixture of stereoisomers, or a pharmaceutically acceptable salt thereof, wherein:
- - - is a single bond or double bond;
Y₁ is absent, -C(O)O-, -O-, -NH-, -SC(O)O-, -OC(O)NR₂ₐ-, -NR₂ₐC(O)NR₂ₐ-, -OC(O)S-, -OC(O)O-, - NR₂ₐC(O)O-, -OC(O)-, -SC(O)-, -C(O)S-, -NR₂ₐ-, -C(O)NR₂,-, -NR₂ₐC(O)-, -NR₂ₐC(O)S-, -SC(O)NR₂ₐ-, - C(O)-, -OC(S)-, -C(S)O-, -OC(S)NR₂ₐ-, -NR₂ₐC(S)O-, -S(O)₀₋₂-, or -OPO₃-, and wherein the attachment to the left is to the direction of V₁;
each instance of R₂ₐ is independently H, C₁₋₁₀ alkyl, C₃₋₁₄ cycloalkyl, or 3 to 14-membered heterocyclyl, and wherein the alkyl, cycloalkyl, and heterocyclyl are independently optionally substituted with C₁₋₁₀ alkyl, L_{2b}-OR_{2b}, L_{b}-SR_{2b}, L_{2b}-NR_{2b}R'_{2b}, L_{2b}N⁺R_{2b}R'_{2b}R"_{2b}, L_{2b}-OC(O)R_{2b}, or L_{2b}-C(O)OR_{2b};
each instance of R₂₀₁, R₂₀₂, R₂₀₃, R₂₀₄ are independently H, OH, alkyl, alkoxy, -L_{2b}-NR₂₁R₂₂, -L_{2b}-N⁺R₂₁R₂₂R₂₃, L_{2b}-OC(O)R₂₁, or L_{2b}-C(O)OR₂₁;
R₂₀₅ is absent, a cation, H, C₁₋₆ alkyl, C₁₋₆ haloalkyl, C₂₋₆ alkenyl, or C₂₋₆ alkynyl, and wherein the alkyl, haloalkyl, alkenyl, and alkynyl are optionally substituted;
m is an integer from 1 to 12;
ml is an integer from 0 to 12;
V₁ is NR₂₁R₂₂, N⁺R₂₁R₂₂R₂₃, imidazole, pyridine, pyrrole, pyrrolidine, pyrazole, (iso)thiazole, (iso)oxazole, oxadiazine, oxazoline, dithiourazole, (iso)triazole, tetrazole, pentazole, indole, dihydroindole, pyrimidine, pyrazine, quinazoline, piperazine, piperidine, morpholine, pyran, quinoxaline, quinoline, quinolinone, (iso)quinoline, amidine, guanidine, or urea;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate;
each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b};
L_{2b} is absent or optionally substituted C₁₋₁₀ alkylene;
each instance of R_{2b}, R'_{2b} and R"_{2b} are independently H or C₁₋₁₀ alkyl; and
R_{z1} is C₁₋₁₄ alkyl or C₁₋₁₄ alkenyl, wherein the alkyl and alkenyl are optionally substituted.

60. A steroid compound of formula (II-C1): 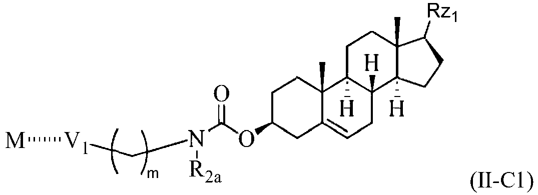 or a stereoisomer, a mixture of stereoisomers, or a pharmaceutically acceptable salt thereof, wherein:
R_{z1} is 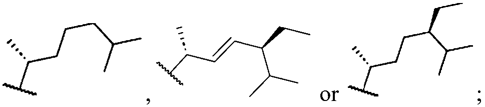
R₂ₐ is H, optionally substituted C₁₋₈ alkyl, -L_{2b}-OR_{2b}, L_{2b}-OC(O)R_{2b}, or L_{2b}-C(O)OR_{2b}, preferably H, -CH₃, -CH₂C(O)OCH₃, or -CH₂C(O)OH;
V₁ is NR₂₁R₂₂ or N⁺R₂₁R₂₂R₂₃, wherein each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}; provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl;
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 10, such as 1, 2, 3, 4, 5, 6, 7, 8, 9, or 10.

61. A steroid compound of formula (II-C2): 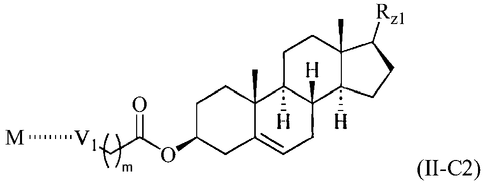 or a stereoisomer, a mixture of stereoisomers, or a pharmaceutically acceptable salt thereof, wherein:
R_{z1} is 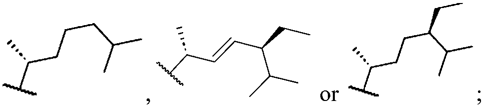
V₁ is NR₂₁R₂₂ or N⁺R₂₁R₂₂R₂₃, wherein each instance of R₂₁, R₂₂, R₂₃ are independently H, optionally substituted C₁₋₈ alkyl, -L_{2b}-OR_{2b}, -L_{2b}-SR_{2b}, -L_{2b}-NR_{2b}R'_{2b}R"_{2b}, L_{2b}-OC(O)R_{2b}, or L_{2b}-C(O)OR_{2b}; provided that R₂₁ and R₂₂ are not both hydrogen;
L_{2b} is absent or optionally substituted C₁₋₁₀ alkylene;
each of R_{2b}, R'_{2b} and R"_{2b} are independently H or C₁₋₁₀ alkyl; and
M is absent or one or more anions independently selected from chloride, bromide, iodide, sulfate, nitrate, perchlorate, formate, citrate, sulfonate, methanesulfonate, triflate, trifluoroacetate, pyrosulfate, bisulfate, sulfite, bisulfite, phosphate, pyrophosphate, monohydrogen phosphate, dihydrogen phosphate, acetate, propionate, decanoate, caprylate, acrylate, isobutyrate, caproate, heptanoates, oxalate, malonate, succinate, fumarates, maleate, benzoates, phthalates, phenylacetate, lactate, glycolate, tartrate, mandelate, mesylate, gluconate, aspartate, or glutamate; and
m is an integer from 1 to 10, such as 1, 2, 3, 4, 5, 6, 7, 8, 9, or 10.

62. A steroid compound, which is selected from the following compounds: 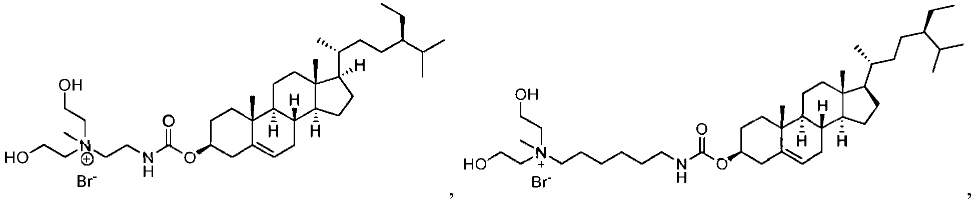 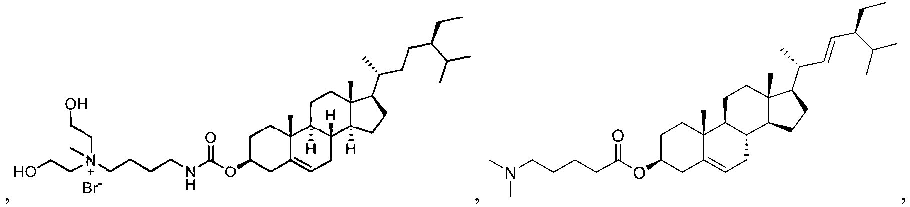 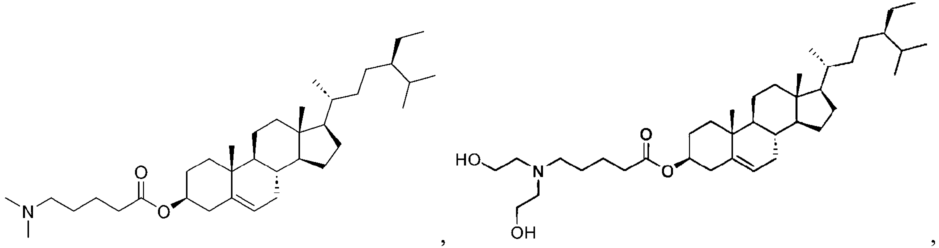 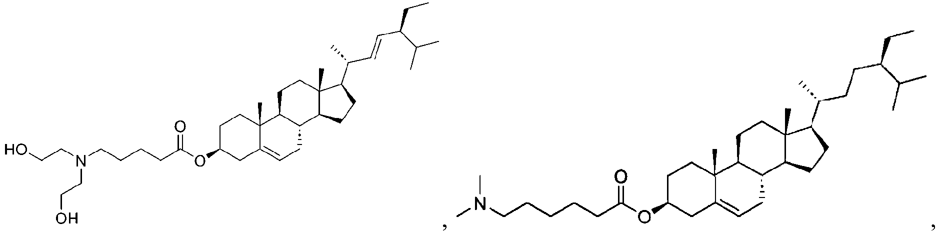 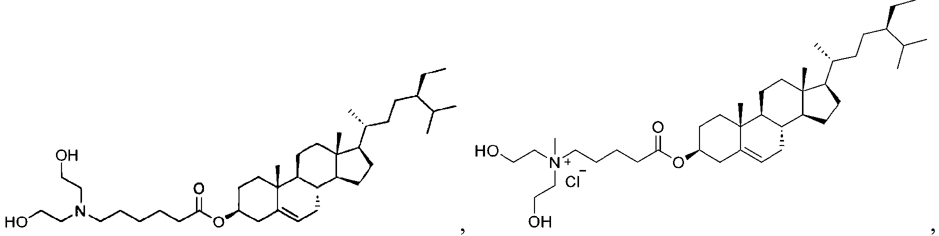 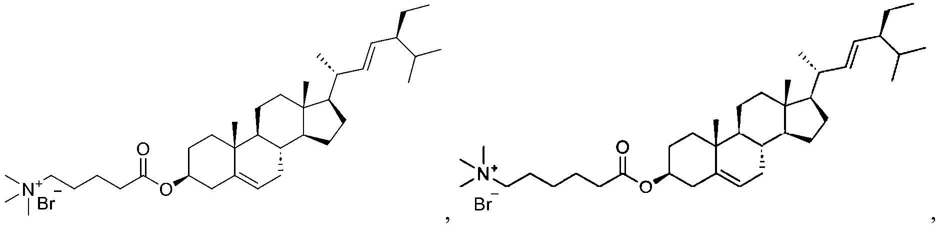 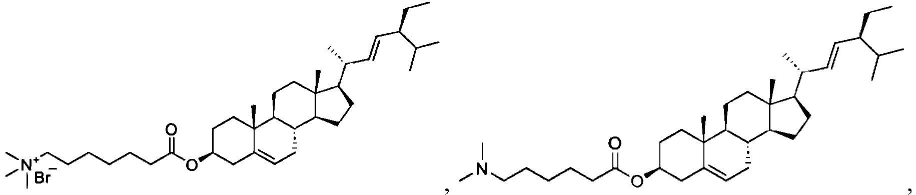 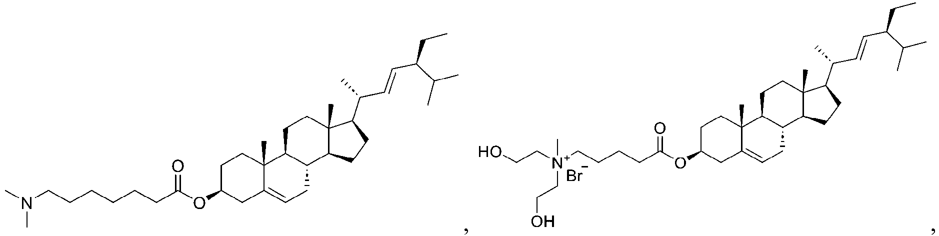 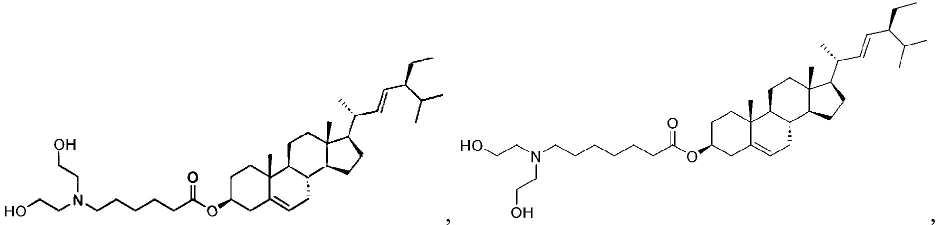 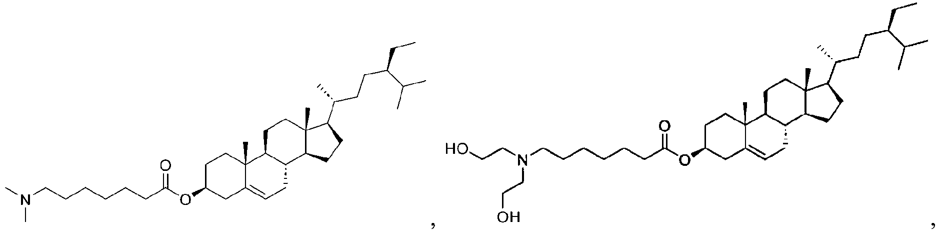 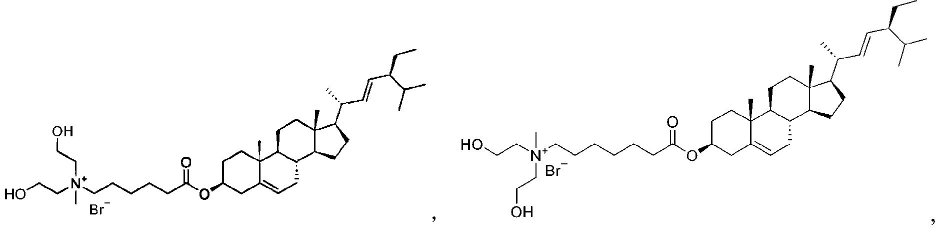 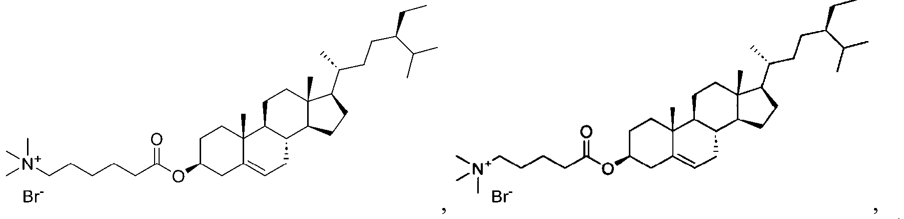 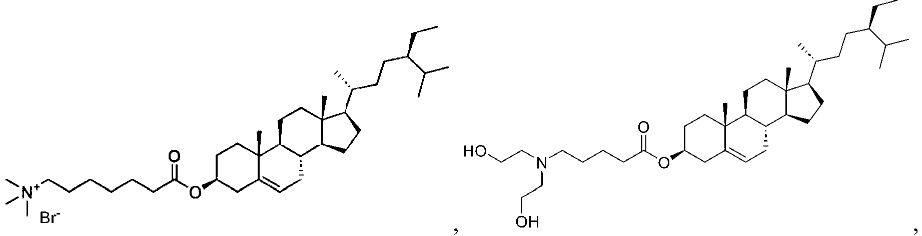 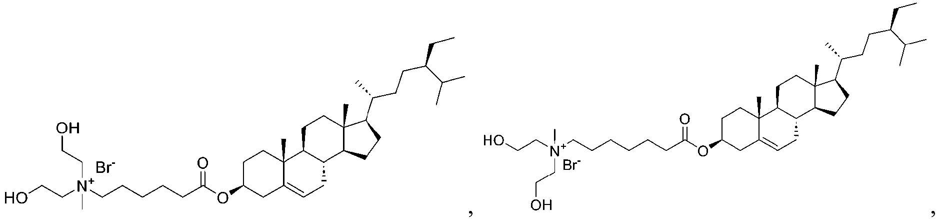 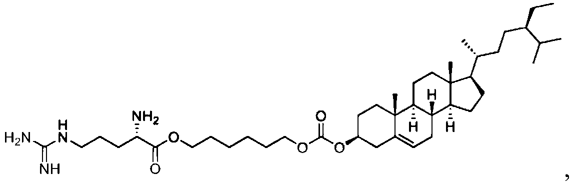 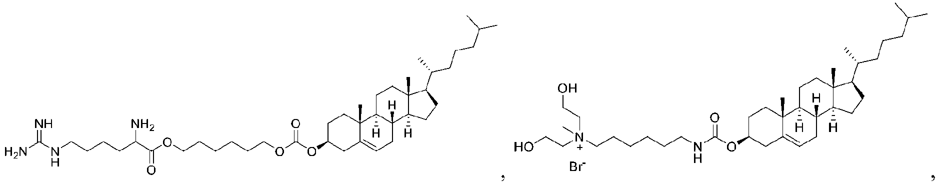 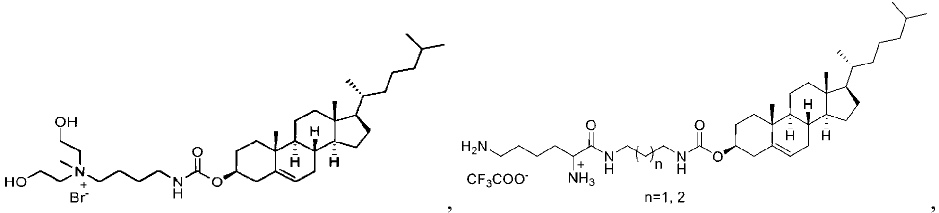 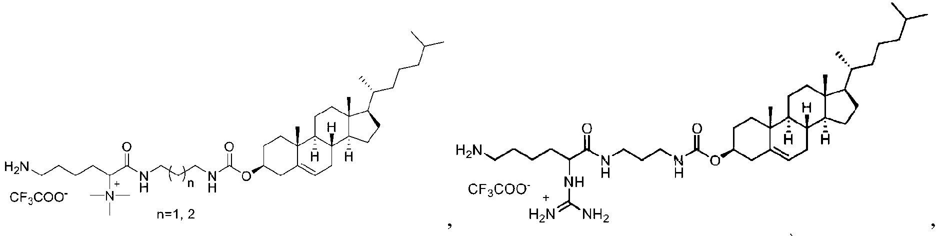 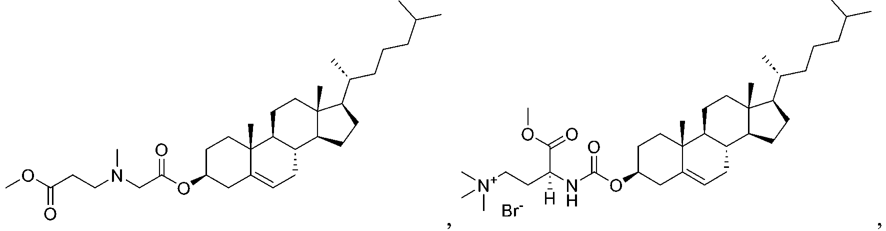 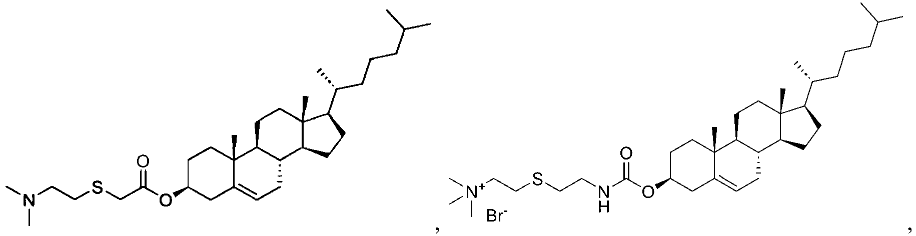 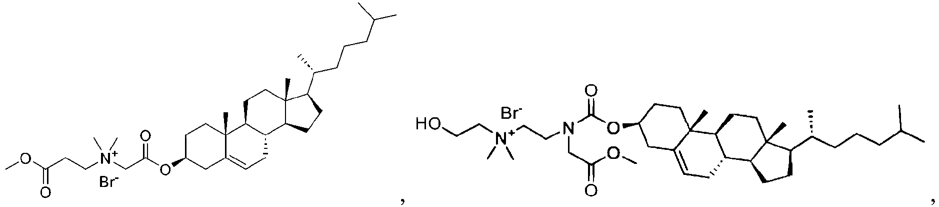 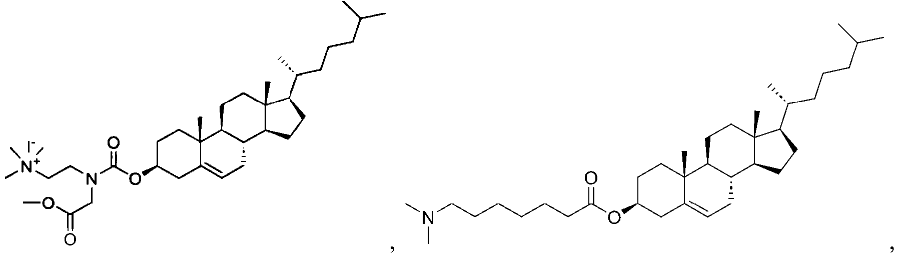 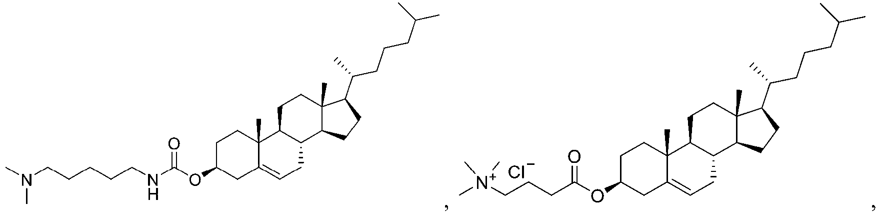 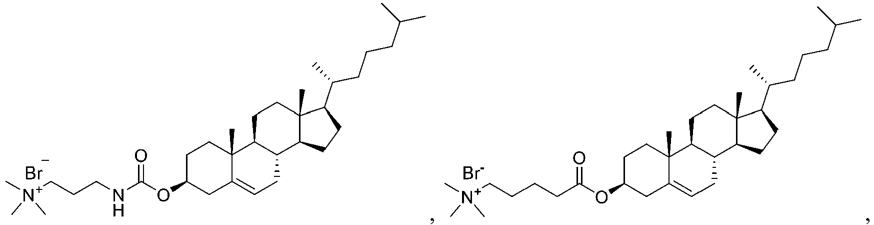 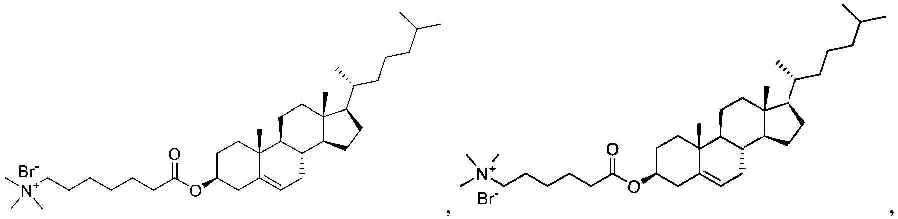 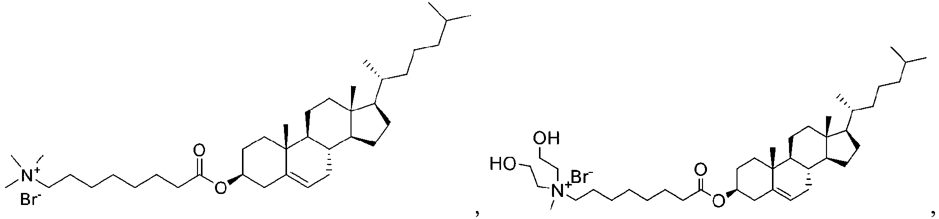 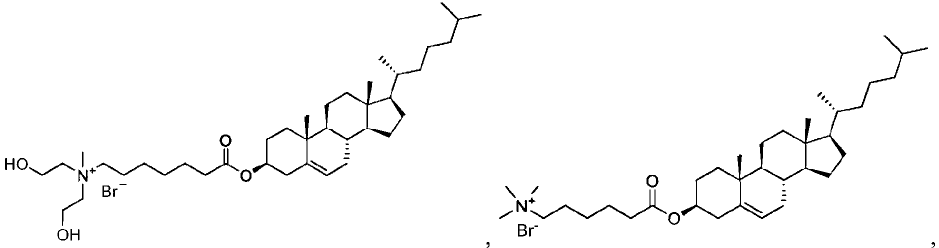 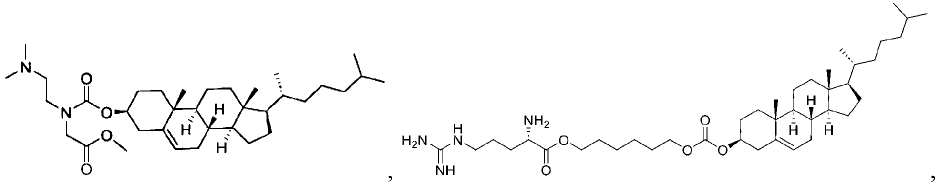 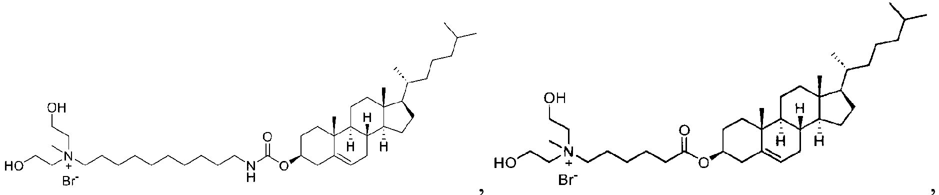 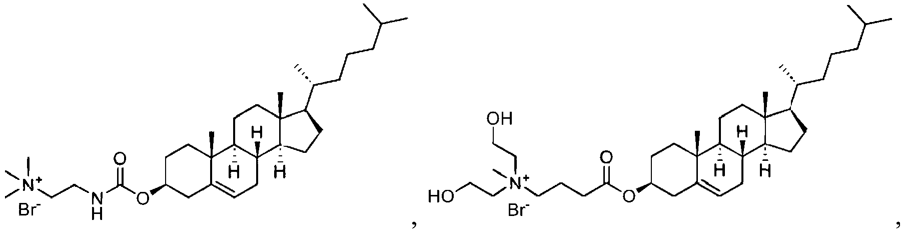 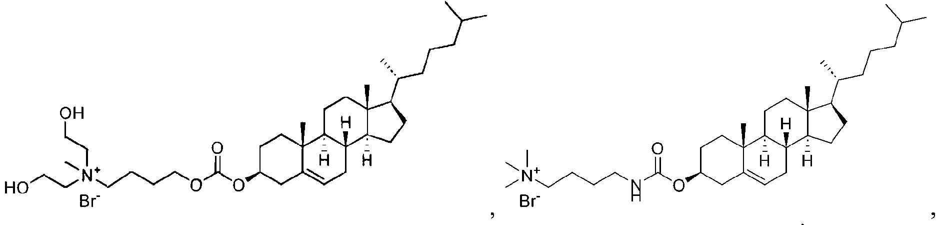 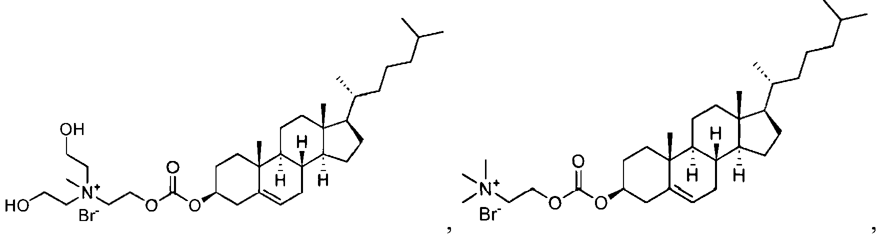 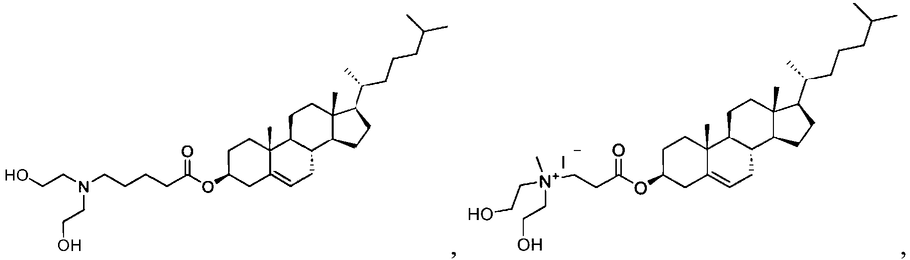 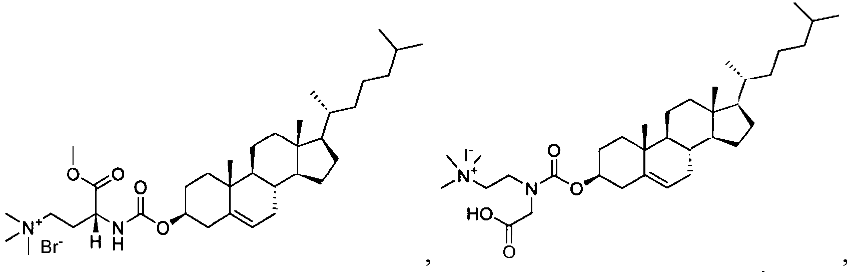 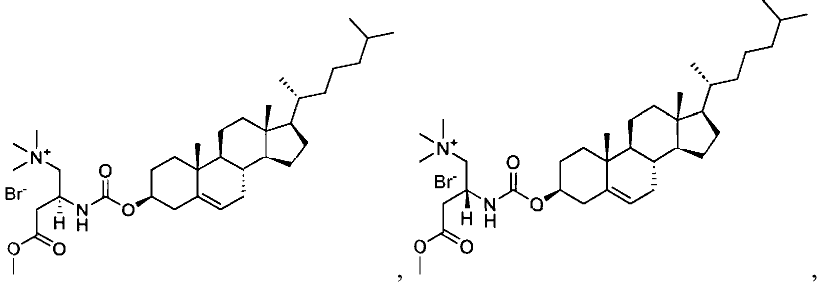 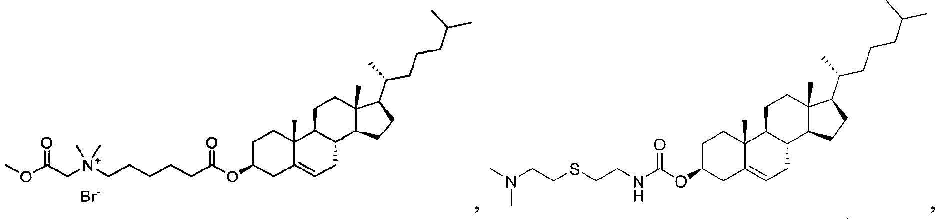 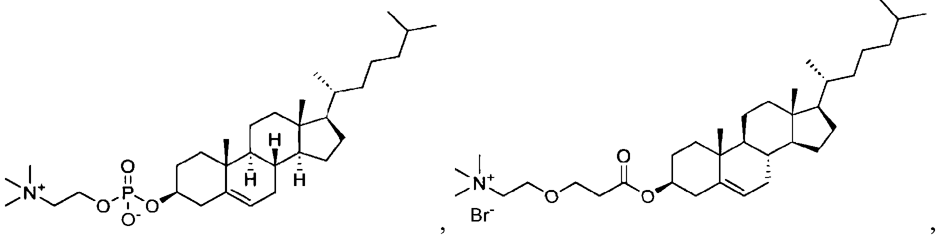 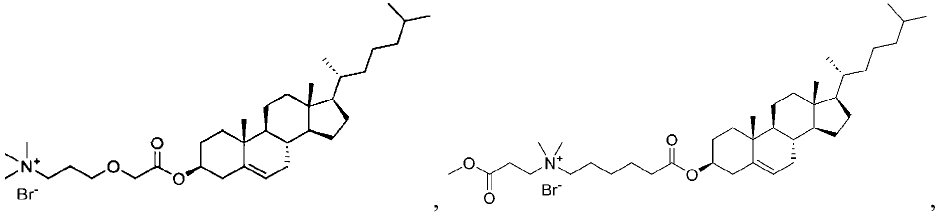 or 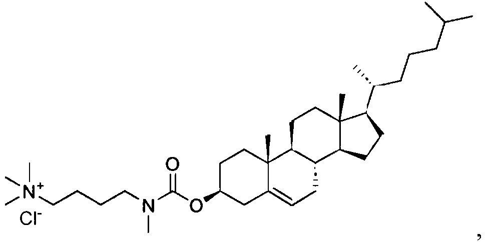 or a stereoisomer, a mixture of stereoisomers, or a pharmaceutically acceptable salt thereof.

63. A lipid nanoparticle comprising the compound of any one of claims 57 to 62.
